# EUROPEAN PATENT APPLICATION

(11) **EP 3 375 877 A1**
(43) Date of publication of application: **19.09.2018**
(21) Application number: 18155396.7
(22) Date of filing: 17.11.2014
(51) Int. Cl.: C12N 15/113, C12N 9/22, C12N 15/90

(54) **CRISPR-CAS SYSTEM MATERIALS AND METHODS**

(30) Priority: 18.11.2013 US 201361905835 P
(62) Divisional of application: 14825102.8
(71) Applicant: Crispr Therapeutics AG, 4051 Basel (CH)
(72) Inventor: CHARPENTIER,, Emmanuelle, 38102 Braunschweig (DE); CHYLINSKI,, Krzysztof, A-1110 Vienna (AT); FONFARA,, Ines, 13189 Berlin (DE)
(74) Representative: Potter Clarkson LLP

(57) **Abstract**

The invention relates to Type II CRISPR-Cas systems of Cas9 enzymes, guide RNAs and associated specific PAMs.

## Description

### Field of the Invention

The invention relates to type II CRISPR-Cas systems of Cas9 enzymes, guide RNAs and associated specific PAMs. This application claims the benefit of the filing date of U.S. Provisional Patent Application No. 61/905,835 filed November 18, 2013, which is incorporated by reference herein in its entirety.

### Incorporation by Reference of the Sequence Listing

This application contains, as a separate part of disclosure, a Sequence Listing in computer-readable form (filename: 48128_SeqListing.txt; 7,869,256 bytes - ASCII text file; created November 14, 2014) which is incorporated by reference herein in its entirety.

### Background

Editing genomes using the RNA-guided DNA targeting principle of CRISPR-Cas (Clustered Regularly Interspaced Short Palindromic Repeats-CRISPR associated proteins) immunity has been exploited widely over the past few months (1-13). The main advantage provided by the bacterial type II CRISPR-Cas system lies in the minimal requirement for programmable DNA interference: an endonuclease, Cas9, guided by a customizable dual-RNA structure (14). As initially demonstrated in the original type II system of Streptococcus pyogenes, trans-activating CRISPR RNA (tracrRNA) (15,16) binds to the invariable repeats of precursor CRISPR RNA (pre-crRNA) forming a dual-RNA (14-17) that is essential for both RNA co-maturation by RNase III in the presence of Cas9 (15-17), and invading DNA cleavage by Cas9 (14,15,17-19). As demonstrated in Streptococcus, Cas9 guided by the duplex formed between mature activating tracrRNA and targeting crRNA (14-16) introduces site-specific double-stranded DNA (dsDNA) breaks in the invading cognate DNA (14,17-19). Cas9 is a multi-domain enzyme (14,20,21) that uses an HNH nuclease domain to cleave the target strand (defined as complementary to the spacer sequence of crRNA) and a RuvC-like domain to cleave the non-target strand (14,22,23), enabling the conversion of the dsDNA cleaving Cas9 into a nickase by selective motif inactivation (2,8,14,24,25). DNA cleavage specificity is determined by two parameters: the variable, spacer-derived sequence of crRNA targeting the protospacer sequence (a protospacer is defined as the sequence on the DNA target that is complementary to the spacer of crRNA) and a short sequence, the Protospacer Adjacent Motif (PAM), located immediately downstream of the protospacer on the non-target DNA strand (14,18,23,26-28).

Recent studies have demonstrated that RNA-guided Cas9 can be employed as an efficient genome editing tool in human cells (1,2,8,11), mice (9,10), zebrafish (6), drosophila (5), worms (4), plants (12,13), yeast (3) and bacteria (7). The system is versatile, enabling multiplex genome engineering by programming Cas9 to edit several sites in a genome simultaneously by simply using multiple guide RNAs (2,7,8,10). The easy conversion of Cas9 into a nickase was shown to facilitate homology-directed repair in mammalian genomes with reduced mutagenic activity (2,8,24,25). In addition, the DNA-binding activity of a Cas9 catalytic inactive mutant has been exploited to engineer RNA-programmable transcriptional silencing and activating devices (29,30).

To date, RNA-guided Cas9 from *S. pyogenes, Streptococcus thermophilus,Neisseria meningitidis* and *Treponema denticola* have been described as tools for genome manipulation (1-13,24,25,31-34 and Esvelt et al. PMID: 24076762).

### Summary

The present invention expands the RNA-programmable Cas9 toolbox to additional orthologous systems. The diversity and interchangeability of dual-RNA:Cas9 in eight representatives of phylogenetically defined type II CRISPR-Cas groups was examined herein. The results of this work not only introduce a wider range of Cas9 enzymes, guide RNA structures and associated specific PAMs but also enlighten the evolutionary aspects of type II CRISPR-Cas systems, including coevolution and horizontal transfer of the system components.

In an aspect, the present disclosure provides guide RNAs, both single-molecule and double-molecule guide RNAs, as well as methods for manipulating DNA in a cell using the guide RNAs and/or DNAs (including vectors) encoding the guide RNAs. Complexes comprising the guide RNAs and Cas9 endonucleases are also provided

In some embodiments, the single-molecule guide RNAs comprise a DNA-targeting segment and a protein-binding segment, wherein the protein-binding segment comprises a tracrRNA set out in Supplementary Table S5 or wherein the protein-binding segment comprises a tracrRNA at least 80% identical over at least 20 nucleotides to a tracrRNA set out in Supplementary Table S5. In some embodiments, the protein-binding segment comprises a CRISPR repeat set out in Supplementary Table S5 that is the CRISPR repeat cognate to the tracrRNA of the protein-binding segment. In some embodiments, the DNA-targeting segment comprises RNA complementary to a protospacer-like sequence in a target DNA 5' to a PAM sequence. In some embodiments, the tracrRNA and CRISPR repeat are respectively the C. jejuni tracrRNA and its cognate CRISPR repeat set out in Supplementary Table S5 and the PAM sequence is NNNNACA. In some embodiments, the tracrRNA and CRISPR repeat are respectively at least 80% identical to the C. jejuni tracrRNA and its cognate CRISPR repeat set out in Supplementary Table S5 and the PAM sequence is NNNNACA.

In some embodiments, the single-molecule guide RNA comprises a sequence that hybridizes to a protospacer-like sequence set out in one of SEQ ID NOs: 801-2701.

In another aspect, the disclosure provides a DNA encoding a single-molecule guide RNA of the invention.

In yet another aspect, the disclosure provides a vector comprising a DNA encoding a single-molecule guide RNA of the invention.

In still another aspect, the disclosure provides a cell comprising a DNA encoding a single-molecule guide RNA of the invention.

In an aspect, the disclosure provides a double-molecule guide RNA comprising: a targeter-RNA and an activator-RNA complementary thereto, wherein the activator-RNA comprises a tracrRNA set out in Supplementary Table S5 or wherein the activator-RNA comprises a tracrRNA at least 80% identical over at least 20 nucleotides to a tracrRNA set out in Supplementary Table S5. In some embodiments, the double-molecule guide RNA comprises a modified backbone, a non-natural internucleoside linkage, a nucleic acid mimetic, a modified sugar moiety, a base modification, a modification or sequence that provides for modified or regulated stability, a modification or sequence that provides for subcellular tracking, a modification or sequence that provides for tracking, or a modification or sequence that provides for a binding site for a protein or protein complex. In some embodiments, the targeter-RNA comprises a CRISPR repeat set out in Supplementary Table S5. In some embodiments, the targeter-RNA comprises a CRISPR repeat set out in Supplementary Table S5 that is the cognate CRISPR repeat of the tracrRNA of the activator-RNA. In some embodiments, the targeter-RNA further comprises RNA complementary to a protospacer-like sequence in a target DNA 5' to a PAM sequence. In some embodiments, the tracrRNA and CRISPR repeat are respectively the C. jejuni tracrRNA and its cognate CRISPR repeat set out in Supplementary Table S5 and the PAM sequence is NNNNACA. In some embodiments, the tracrRNA and CRISPR repeat are at least 80% identical to respectively the C. jejuni tracrRNA and its cognate CRISPR repeat set out in Supplementary Table S5 and the PAM sequence is NNNNACA.

In some embodiments, the double-molecule guide RNA comprises a sequence that hybridizes to a protospacer-like sequence set out in one of SEQ ID NOs: 801-2701.

In another aspect, the disclosure provides a DNA encoding a double-molecule guide RNA of the invention.

In yet another aspect, the disclosure provides a vector comprising a DNA encoding a double-molecule guide RNA of the invention.

In still another aspect, the disclosure provides a cell comprising a DNA encoding a double-molecule guide RNA of the invention.

In an aspect, the disclosure provides methods for manipulating DNA in a cell, comprising contacting the DNA with a Cas9 ortholog-guideRNA complex, wherein the complex comprises: (a) a C. jejuni Cas9 endonuclease heterologous to the cell or an endonuclease with an activity portion at least 90% identical to the activity portion of the C. jejuni Cas9 endonuclease, and a guide RNA targeting the complex to a protospacer-like sequence in the DNA 5' to the PAM sequence NNNNACA; (b) a P. multocida Cas9 endonuclease heterologous to the cell or an endonuclease with an activity portion at least 90% identical to the activity portion of the P. multocida Cas9 endonuclease, and a guide RNA targeting the complex to a protospacer-like sequence in the DNA 5' to the PAM sequence GNNNCNNA or NNNNC; (c) an F. novicida Cas9 endonuclease heterologous to the cell or an endonuclease with an activity portion at least 90% identical to the activity portion of the F. novicida Cas9 endonuclease, and a guide RNA targeting the complex to a protospacer-like sequence in the DNA 5' to the PAM sequence NG; (d) an S. thermophilus** Cas9 endonuclease heterologous to the cell or an endonuclease with an activity portion at least 90% identical to the activity portion of the S. thermophilus** Cas9 endonuclease, and a guide RNA targeting the complex to a protospacer-like sequence in the DNA 5' to the PAM sequence NNAAAAW; (e) an L. innocua Cas9 endonuclease heterologous to the cell or an endonuclease with an activity portion at least 90% identical to the activity portion of the L. innocua Cas9 endonuclease, and a guide RNA targeting the complex to a protospacer-like sequence in the DNA 5' to the PAM sequence NGG; or (f) an S. dysgalactiae Cas9 endonuclease heterologous to the cell or an endonuclease with an activity portion at least 90% identical to the activity portion of the S. dysgalactiae Cas9 endonuclease, and a guide RNA targeting the complex to a protospacer-like sequence in the DNA 5' to the PAM sequence NGG. In some embodiments, the guide is a single-molecule guide RNA. In some embodiments, the guide RNA is a double-molecule guide RNA. The complexes used in the methods are also provided.

In some embodiments of the methods, the protospacer-like sequence targeted is in a CCR5, CXCR4, KRT5, KRT14, PLEC or COL7A1 gene. In some embodiments, the protospacer-like sequence is in a chronic granulomatous disease (CGD)-related gene CYBA, CYBB, NCF1, NCF2 or NCF4. In some embodiments, the protospacer-like sequence targeted is in a gene encoding B-cell lymphoma/leukemia IIA (BCL11A) protein, an erythroid enhancer of BCL11A or a BCL11A binding site. In some embodiments, the protospacer-like sequence targeted is up to 1000 nucleotides upstream of the above mentioned genes. In some embodiments of the methods, the guide RNA comprises a sequence complementary to a protospacer-like sequence set out in one of SEQ ID NOs: 801-2701.

In an aspect, the disclosure provides a recombinant vector encoding: (a) a guide RNA, wherein the guide RNA comprises a DNA-targeting segment complementary to a protospacer-like sequence in the DNA 5' to the PAM sequence NNNNACA; and (b) a C. jejuni Cas9 endonuclease (for example, set out in SEQ ID NO: 50) or an endonuclease with an activity portion at least 90% identical to the activity portion of the C. jejuni Cas9 endonuclease. In some embodiments, the DNA-targeting segment complementary to the protospacer-like sequence is RNA complementary to the target sequences set out in one of SEQ ID NOs: 801-973, 1079-1222, 1313-1348, 1372-1415, 1444-1900, 2163-2482 or 2667-2686. Methods of using the vectors to manipulate DNA in a cell are also provided.

In another aspect, the disclosure provides a recombinant vector encoding: (a) a guide RNA, wherein the guide RNA comprises a DNA-targeting segment complementary to a protospacer-like sequence in the DNA 5' to the PAM sequence GNNNCNNA or NNNNC; and (b) a P. multocida Cas9 endonuclease (for example, set out in SEQ ID NO: 1) or an endonuclease with an activity portion at least 90% identical to the activity portion of the P. multocida Cas9 endonuclease. In some embodiments, the DNA-targeting segment complementary to the protospacer-like sequence is RNA complementary to the target sequences set out in one of SEQ ID NOs:974-1078, 1223-1312, 1349-1371, 1416-1443, 1901-2162, 2483-2666 or 2687-2701. Methods of using the vectors to manipulate DNA in a cell are also provided.

In yet another aspect, the disclosure provides a recombinant vector encoding: (a) a guide RNA, wherein the guide RNA comprises a DNA-targeting segment complementary to a protospacer-like sequence in the DNA 5' to the PAM sequence NG; and (b) a F. novicida Cas9 endonuclease (fore example, set out in SEQ ID NO: 43) or an endonuclease with an activity portion at least 90% identical to the activity portion of the F. novicida Cas9 endonuclease. Methods of using the vectors to manipulate DNA in a cell are also provided.

In still another aspect, the disclosure provides a recombinant vector encoding: (a) a guide RNA, wherein the guide RNA comprises a DNA-targeting segment complementary to a protospacer-like sequence in the DNA 5' to the PAM sequence NNAAAAW; and (b) a S. thermophilus** Cas9 endonuclease or an endonuclease with an activity portion at least 90% identical to the activity portion of the S. thermophilus** Cas9 endonuclease. Methods of using the vectors to manipulate DNA in a cell are also provided.

In yet another aspect, the disclosure provides a recombinant vector encoding:(a) a guide RNA, wherein the guide RNA comprises a DNA-targeting segment complementary to a protospacer-like sequence in the DNA 5' to the PAM sequence NGG; and (b) a L. innocua Cas9 endonuclease (for example, set out in SEQ ID NO: 3) or an endonuclease with an activity portion at least 90% identical to the activity portion of the L. innocua Cas9 endonuclease. Methods of using the vectors to manipulate DNA in a cell are also provided.

In still another aspect, the disclosure provides a recombinant vector encoding: (a) a guide RNA, wherein the guide RNA comprises a DNA-targeting segment complementary to a protospacer-like sequence in the DNA 5' to the PAM sequence NGG; and (b) a S. dysgalactiae Cas9 endonuclease (for example, set out in SEQ ID NO: 105) or an endonuclease with an activity portion at least 90% identical to the activity portion of the S. dysgalactiae Cas9 endonuclease.

In some embodiments of the vectors, the guide RNA comprises a sequence complementary to a protospacer-like sequence set out in one of SEQ ID NOs: 801-2701.

In a related aspect, the disclosure provides a method comprising (a) identifying at least 7-20 bases of mammalian genomic DNA adjacent to any of the preceding protospacer-like sequences, and (b) manipulating the mammalian genomic DNA sequence by contacting a mammalian cell with, or administering to a mammal, (i) a DNA-targeting segment complementary to the DNA sequence identified in step (a) and (ii) a protein-binding segment, or nucleic acid(s) encoding (i) and (ii), and (iii) a cas9 endonuclease or a nucleic acid encoding said cas9 endonuclease; and (c) detecting cleavage of the mammalian genomic DNA.

In an aspect, the disclosure provides a modified Cas9 endonuclease, modified from any of the Cas9 orthologs disclosed herein, comprising one or more mutations corresponding to S. pyogenes Cas9 mutation E762A, HH983AA or D986A. In some embodiments, the modified Cas 9 endonuclease further comprises one or more mutations corresponding to S. pyogenes Cas9 mutation D10A, H840A, G12A, G17A, N854A, N863A, N982A or A984A.

In an aspect, the disclosure provides a method for manipulating DNA in a cell, comprising contacting the DNA with a Cas9 ortholog-guide RNA complex, wherein the complex comprises: (a) a Cas9 endonuclease heterologous to the cell and (b) a cognate guide RNA of the Cas9 endonuclease comprising a tracrRNA set out in Supplementary Table S5 or a guide RNA comprising a tracrRNA at least 80% identical to a cognate tracrRNA set out in Supplementary Table S5 over at least 20 nucleotides. In some embodiments, the guide is a single-molecule guide RNA. In some embodiments, the guide RNA is a double-molecule guide RNA. In some embodiments of the methods, the guide RNA comprises a sequence complementary to a protospacer-like sequence set out in one of SEQ ID NOs: 801-2701. Complexes used in the methods are also provided.

In an aspect, the disclosure provides a method for manipulating DNA in a cell, comprising contacting the DNA with a Cas9 ortholog-guide RNA complex, wherein the complex comprises: (a) a cognate guide RNA for a first Cas9 endonuclease from a cluster in Supplementary Table S2 and (b) a second Cas9 endonuclease from the same cluster that is exchangeable with preserved high cleavage efficiency with the first endonuclease and shares at least 80% identity with the first endonuclease over 80% of their length. In some embodiments, the guide is a single-molecule guide RNA. In some embodiments, the guide RNA is a double-molecule guide RNA. In some embodiments, the first Cas9 endonuclease is from S. pyogenes and the second Cas9 endonuclease is from S. mutans. In some embodiments, the first Cas9 endonuclease is from S. thermophilus* and the second Cas9 endonuclease is from S. mutans. In some embodiments, the first Cas9 endonuclease is from N. meningitidis and the second Cas9 endonuclease is from P. multocida. Complexes used in the methods are also provided.

In an aspect, the disclosure provides a method for manipulating DNA in a cell, comprising contacting the DNA with a Cas9 ortholog-guide RNA complex, wherein the complex comprises: (a) a cognate guide RNA of a first Cas9 endonuclease from a cluster in Supplementary Table S6 and (b) an Cas9 endonuclease from the same cluster in Supplementary Table S6 that is exchangeable with the same or lowered cleavage efficiency with the first endonuclease and shares at least 50% amino acid sequence identity with the first endonuclease over 70% of their length. In some embodiments, the guide is a single-molecule guide RNA. In some embodiments, the guide RNA is a double-molecule guide RNA. In some embodiments, the first Cas9 endonuclease is from C. Jejuni and the second Cas9 endonuclease is from P. multocida. In some embodiments, the first Cas9 endonuclease is from N. meningitidis and the second Cas9 endonuclease is from P. multocida. Complexes used in the methods are also provided.

In an aspect, the disclosure provides a method for manipulating DNA in a cell, comprising contacting the DNA with two or more Cas9-guide RNA complexes, wherein each Cas9-guideRNA complex comprises: (a) a Cas9 endonuclease from a different cluster in Supplementary Table S6 exhibiting less than 50% amino acid sequence identity with the other endonucleases of the method over 70% of their length, and (b) a guide RNA specifically complexed with each Cas9 endonuclease. In some embodiments, the guide is a single-molecule guide RNA. In some embodiments, the guide RNA is a double-molecule guide RNA.In some embodiments, the Cas9 endonucleases are from F. novicida and S. pyogenes. In some embodiments, the Cas9 endonucleases are from N. meningitidis and S. mutans. In some embodiments, the Cas9 endonucleases are the S. thermophilus* Cas9 and the S. thermophilus** Cas9. Complexes used in the methods are also provided.

In some embodiments of the manipulation methods, the DNA targeted in the cell is a CCR5, CXCR4, KRT5, KRT14, PLEC or COL7A1 gene. In some embodiments, the DNA targeted in the cell is a chronic granulomatous disease (CGD)-related gene CYBA, CYBB, NCF1, NCF2 or NCF4. In some embodiments, the protospacer-like sequence targeted is in a gene encoding B-cell lymphoma/leukemia IIA (BCL11A) protein, an erythroid enhancer of BCL11A or a BCL11A binding site. In some embodiments, the protospacer-like sequence targeted is up to 1000 nucleotides upstream of the above mentioned genes. In some embodiments of the methods, the guide RNA comprises a sequence complementary to a protospacer-like sequence set out in one of SEQ ID NOs: 801-2701.

It is contemplated that any of the methods provided herein may ex vivo or in vivo.

### Brief Description of the Drawings

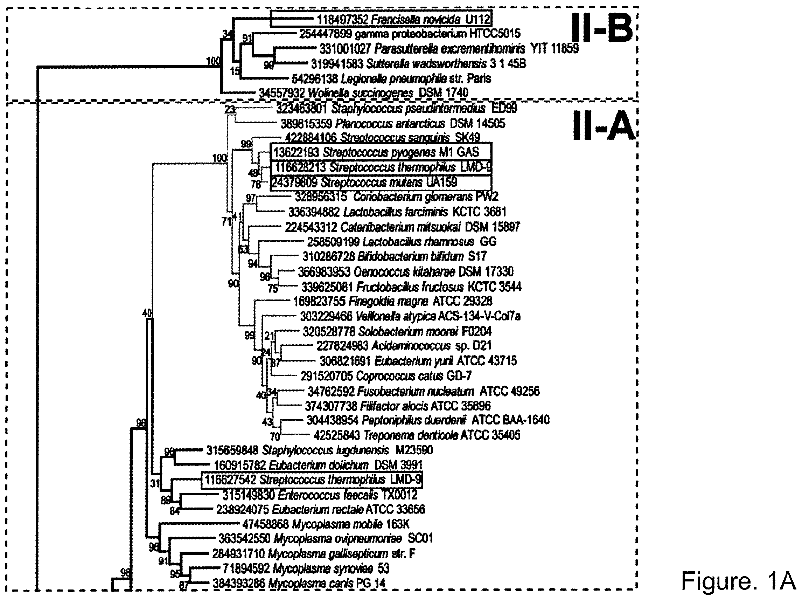

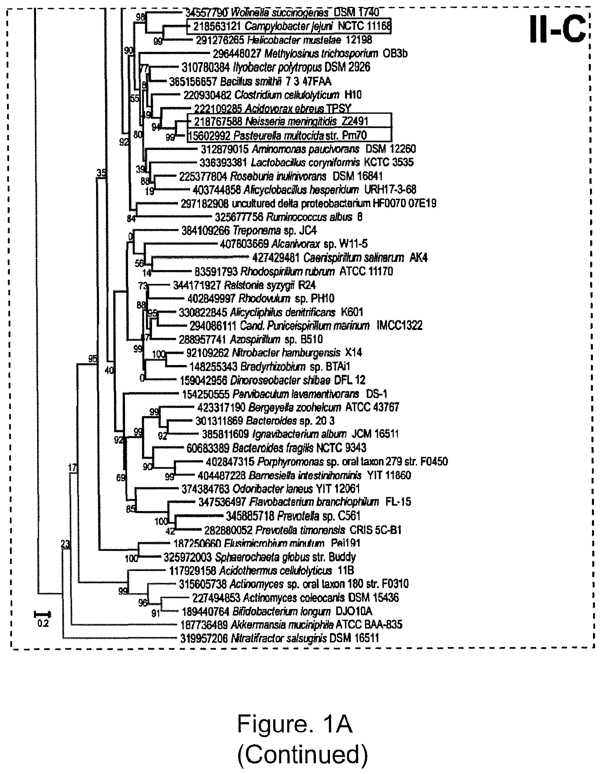

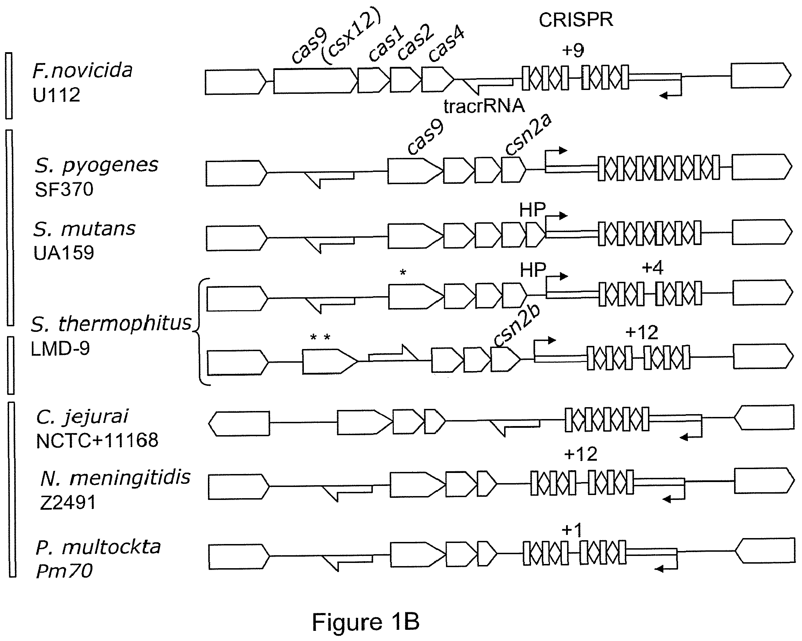

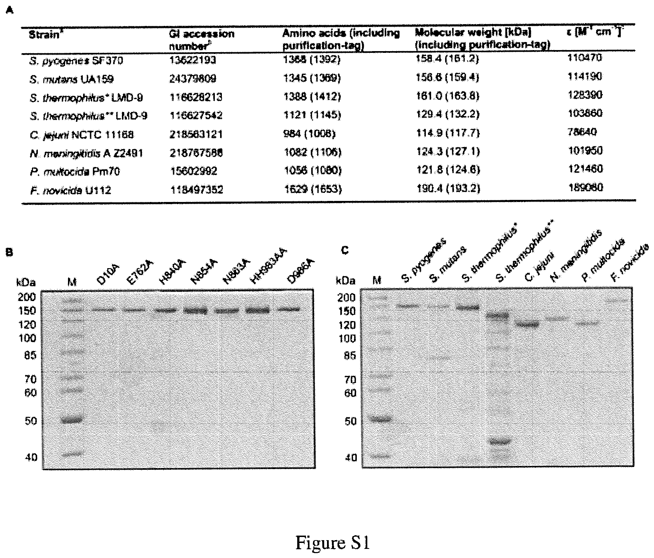

**Figure 1****.** Phylogeny of representative Cas9 orthologs and schematic representation of selected bacterial type II CRISPR-Cas systems. (**A**) Phylogenetic tree of Cas9 reconstructed from selected, informative positions of representative Cas9 orthologs multiple sequence alignment is shown (see Supplementary Figure S2 and Supplementary Table S2). The Cas9 orthologs of the subtypes classified as II-A, II-B and II-C are highlighted with shaded boxes. The colored branches group distinct proteins of closely related loci with similar locus architecture (15). Each protein is represented by the Genlnfo (GI) identifier followed by the bacterial strain name. The bootstrap values are given for each node (see Materials and Methods). Note that the monophyletic clusters of subtypes II-A and II-B are supported by high bootstrap values. The scale bar for the branch length is given as the estimated number of amino acid substitution per site. (**B**) Genetic loci of type II (Nmeni/CASS4) CRISPR-Cas in *Streptococcus pyogenes* SF370, *Streptococcus mutans* UA159, *Streptococcus thermophilus* LMD-9 *(CRISPR3), **(CRISPR1), *Campylobacter jejuni* NCTC 11168, *Neisseria meningitidis* Z2491, *Pasteurella multocida* Pm70 and *Francisella novicida* U112. Red arrow, transcription direction of tracrRNA; blue arrows, cas genes; black rectangles, CRISPR repeats; green diamonds, spacers; thick black line, leader sequence; black arrow, putative pre-crRNA promoter; HP, Hypothetical Protein. The colored bars represented on the left correspond to Cas9 tree branches colors. The transcription direction and putative leader position of *C. jejuni* and *N. meningitidis* pre-crRNAs were derived from previously published RNA sequencing data (15). The CRISPR-Cas locus architecture of *P*. *multocida* was predicted based on its close similarity to that of *N. meningitidis* and further confirmed by bioinformatics prediction of tracrRNA based on a strongly predicted promoter and a transcriptional terminator as described in (15). Type II CRISPR-Cas loci can differ in the cas gene composition, mostly with *cas9, cas1* and cas2 being the minimal set of genes (type II-C, blue), sometimes accompanied with a fourth gene *csn2a*/*b* (type II-A, yellow and orange) or *cas4* (type II-B, green). The CRISPR array can be transcribed in the same (type II-A, yellow and orange) or in the opposite (types II-B and C, blue and green) direction of the cas operon. The location of tracrRNA and the direction of its transcription differ within the groups (compare type II-A of *S. thermophilus*** with type II-A from the other species indicated here (yellow) and compare type II-C of C. *jejuni* with type II-C of *N. meningitidis* and *P*. *multocida* (blue)).

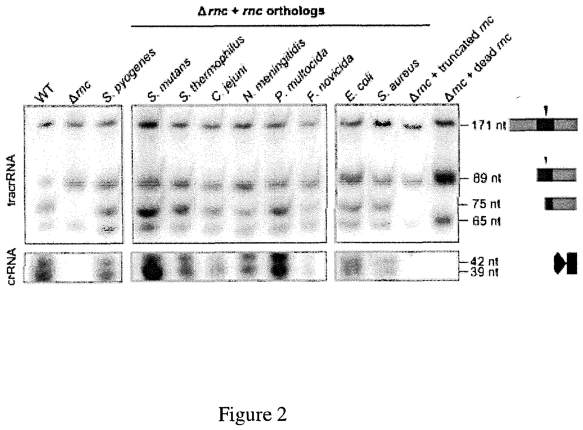

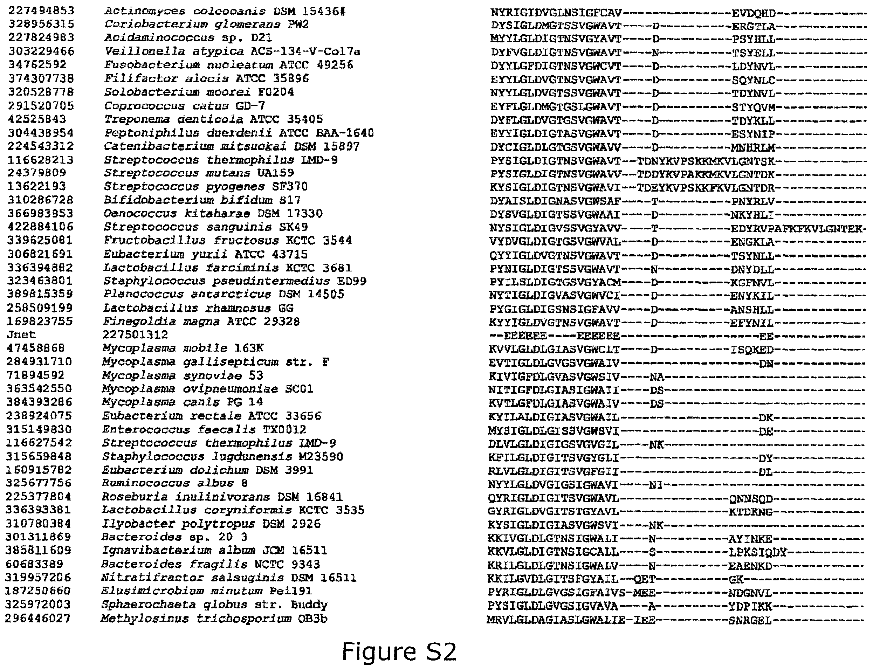

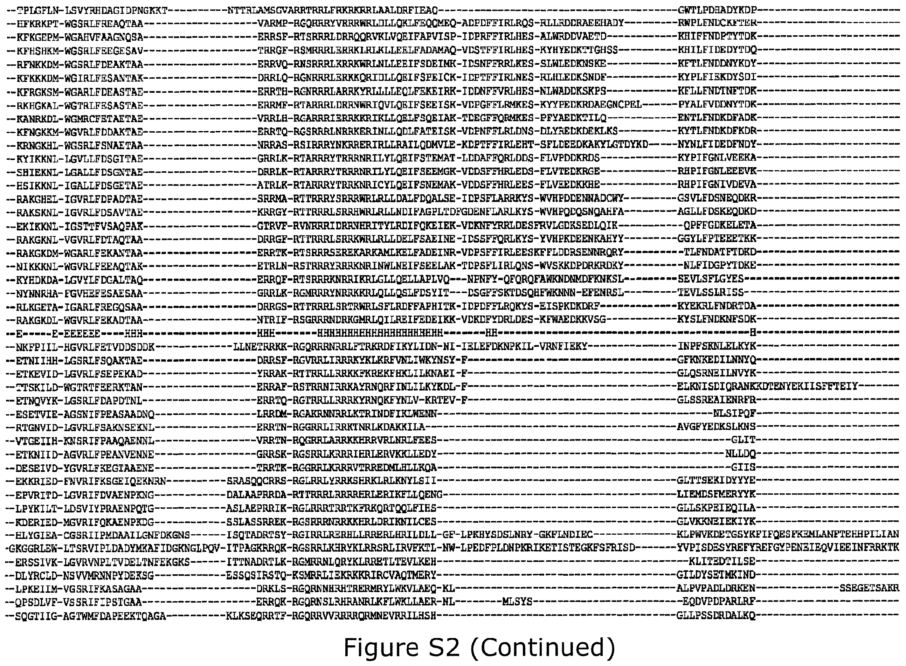

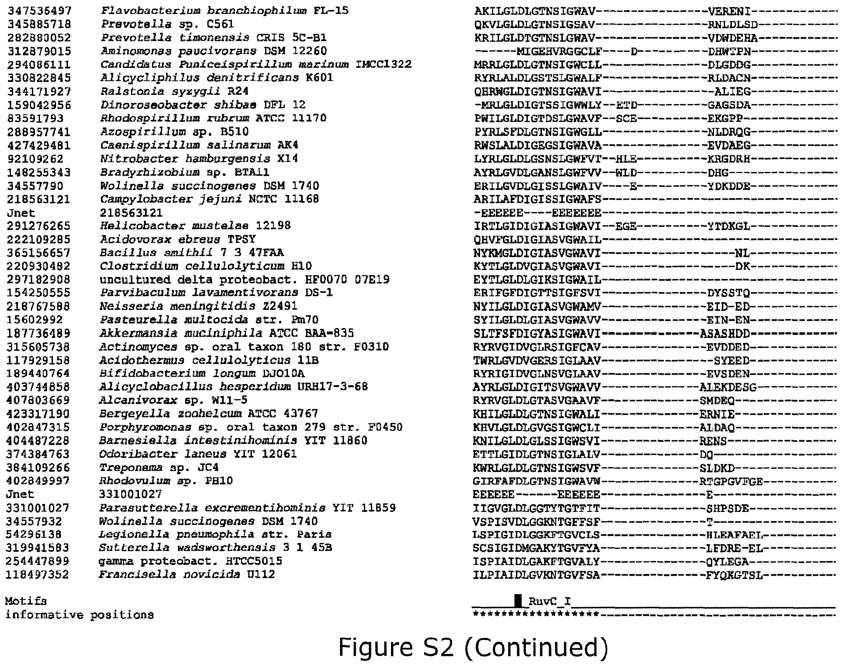

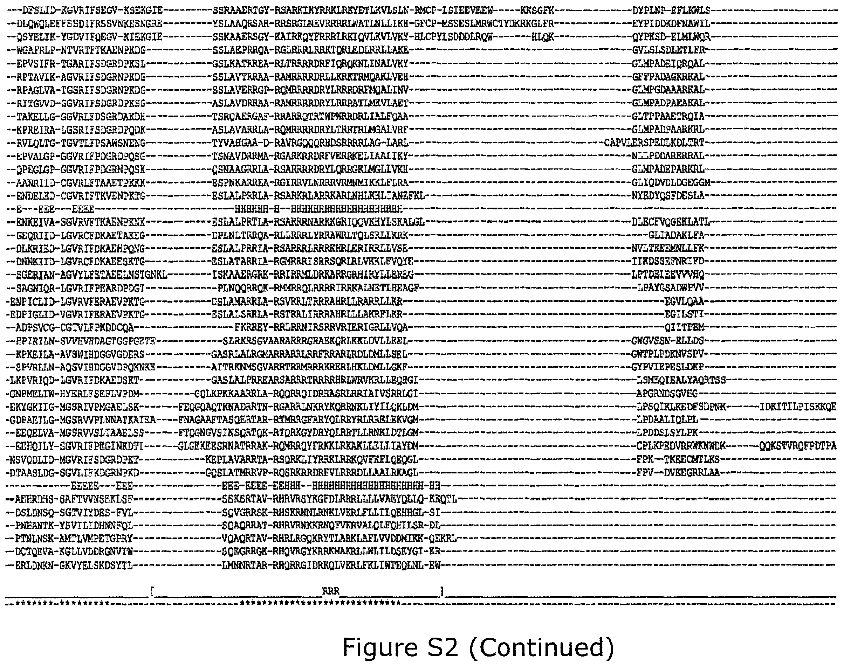

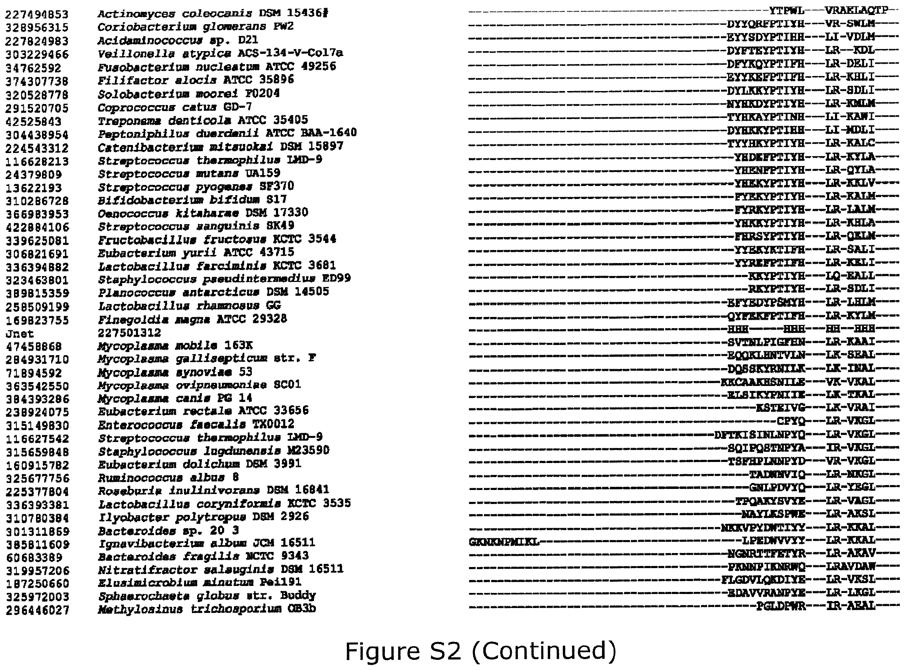

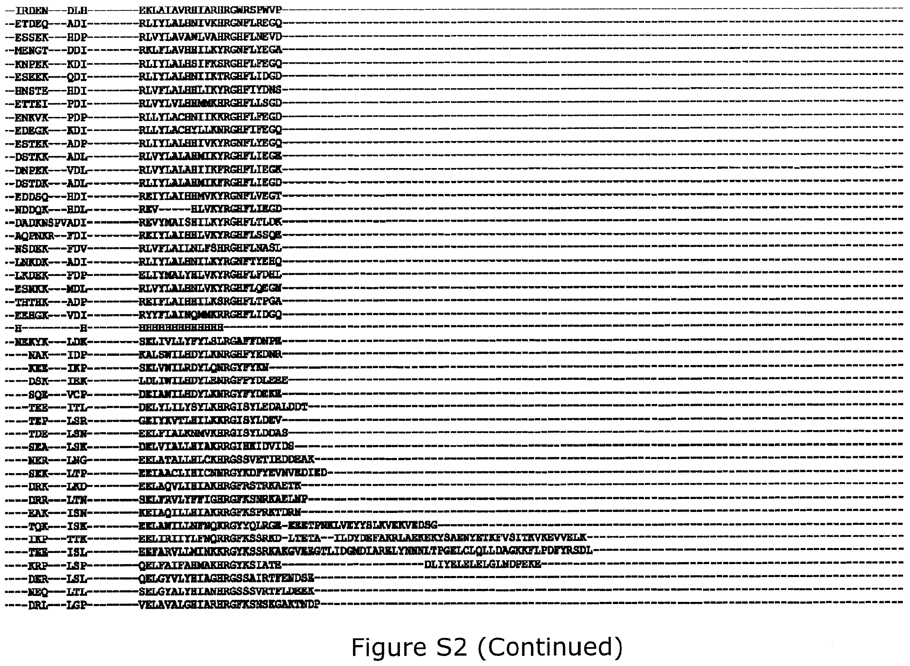

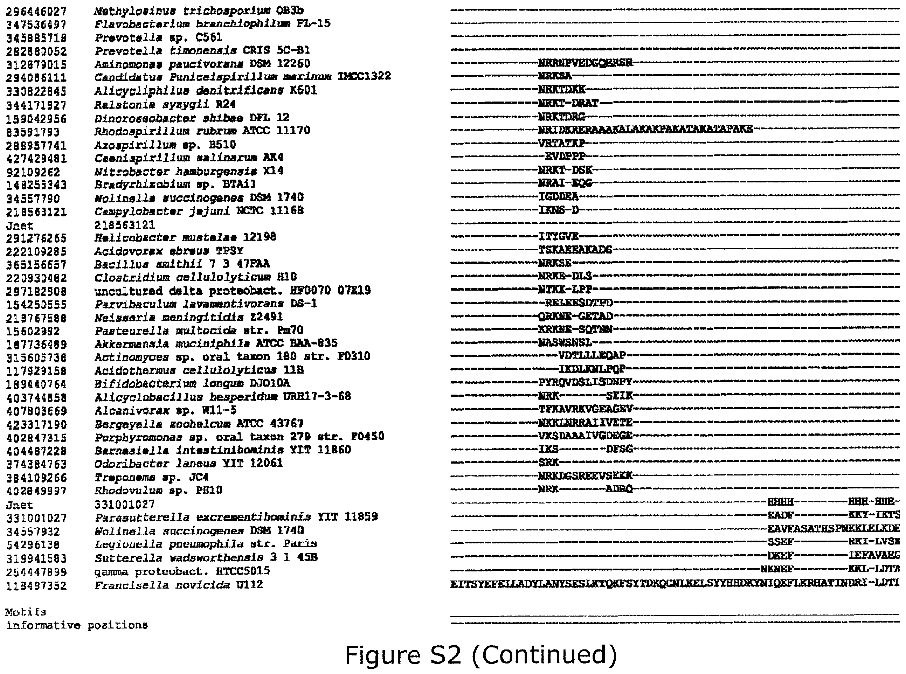

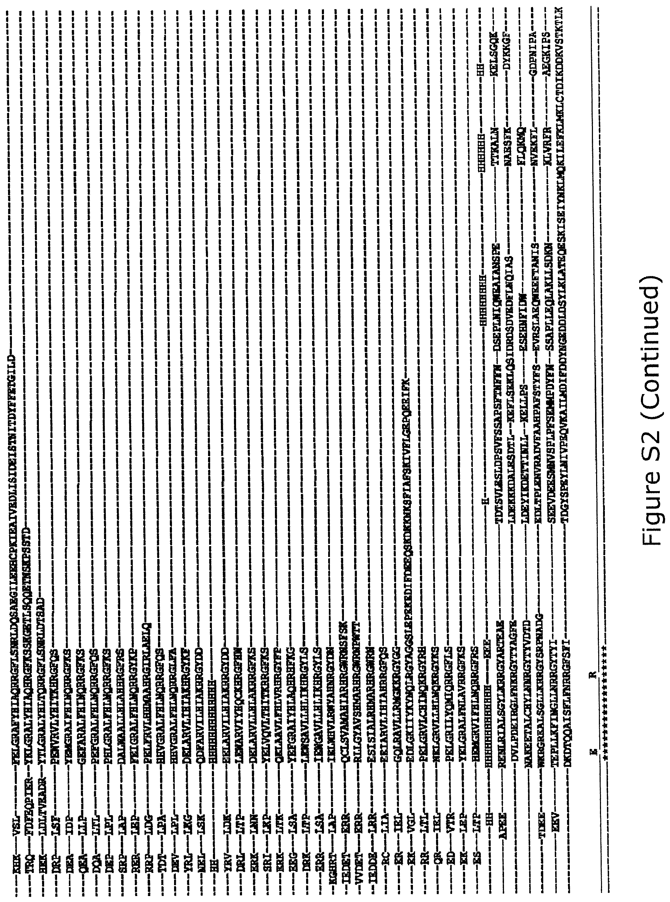

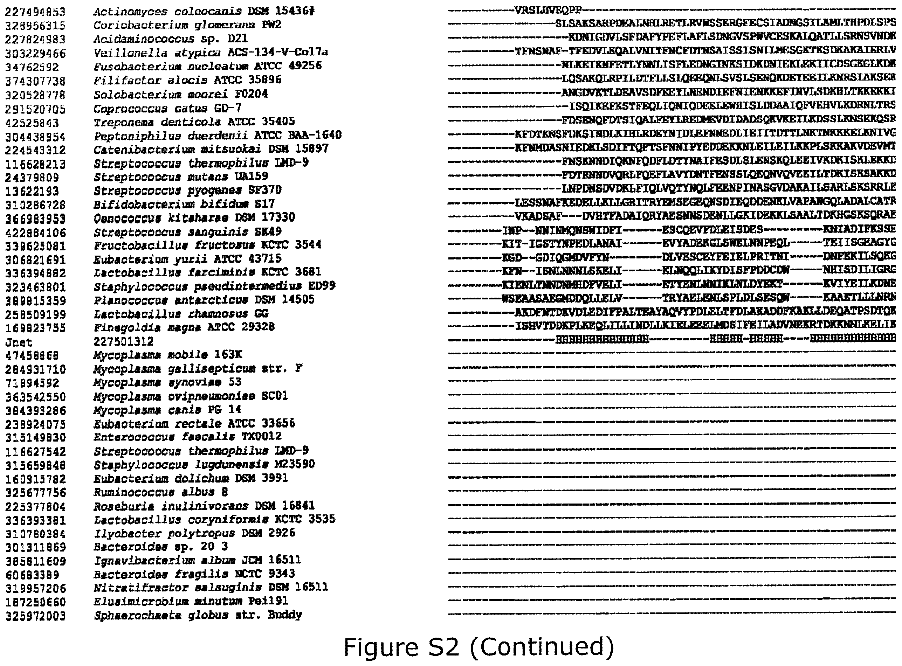

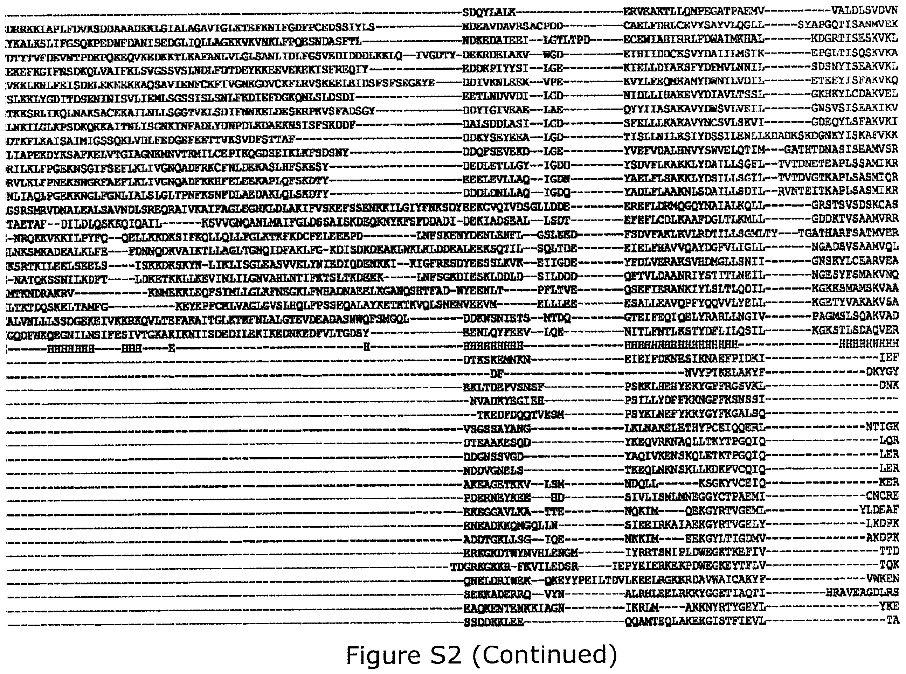

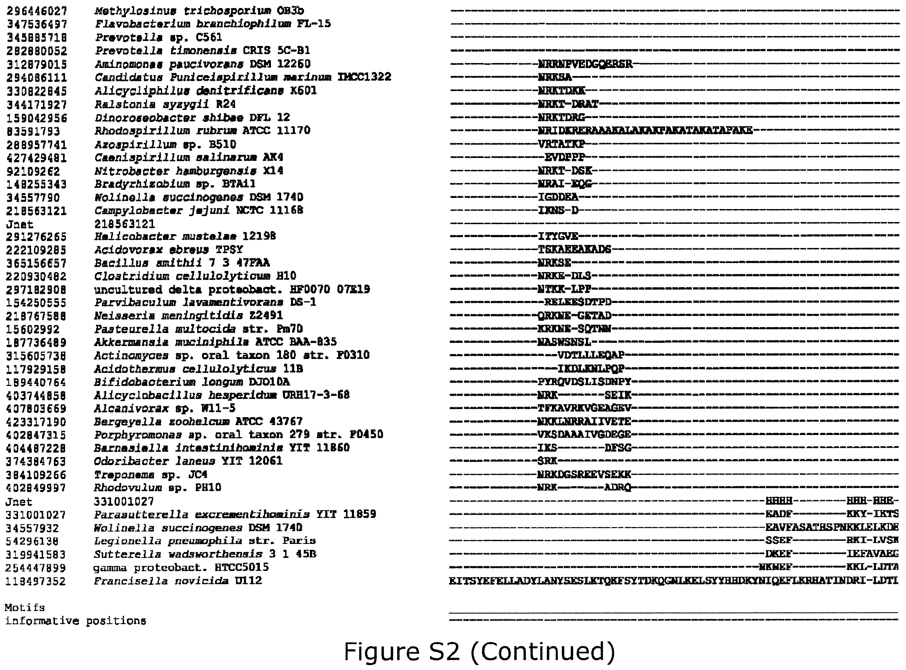

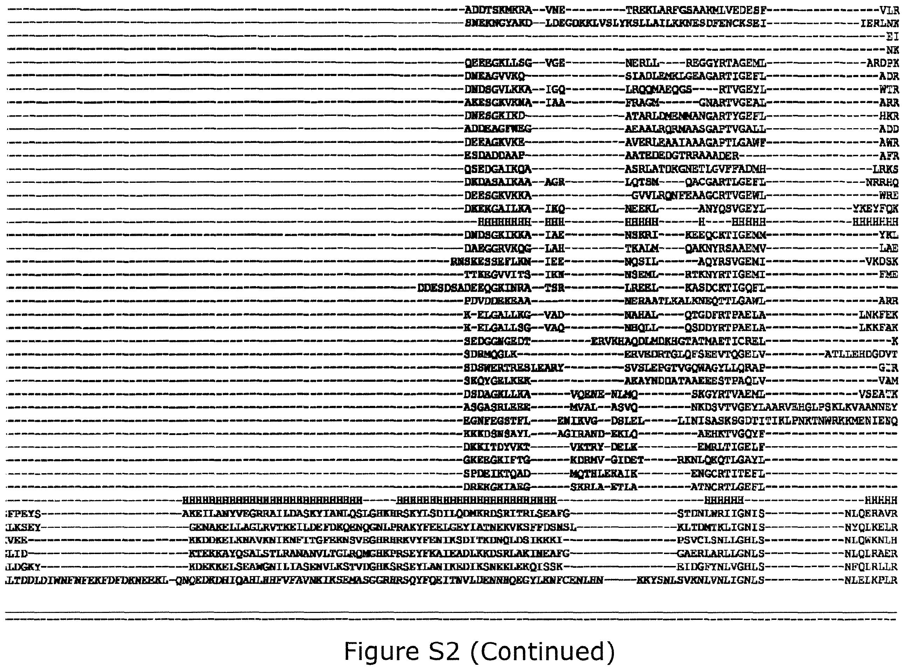

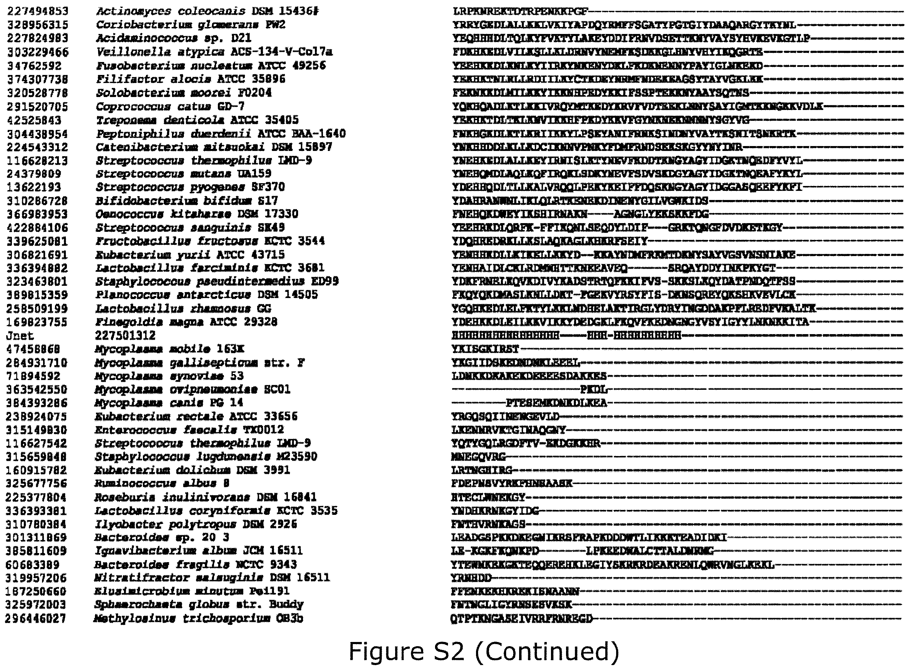

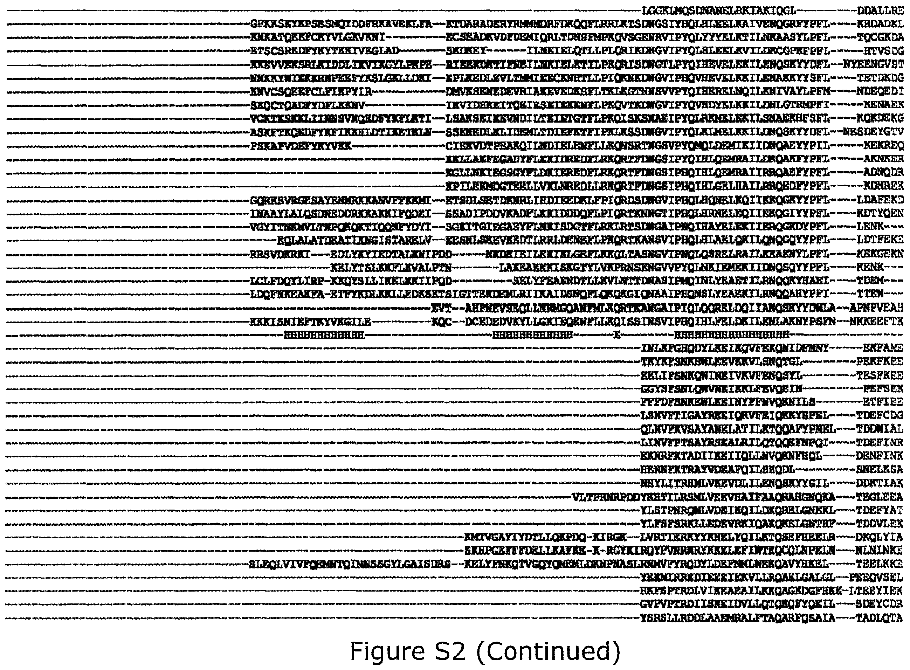

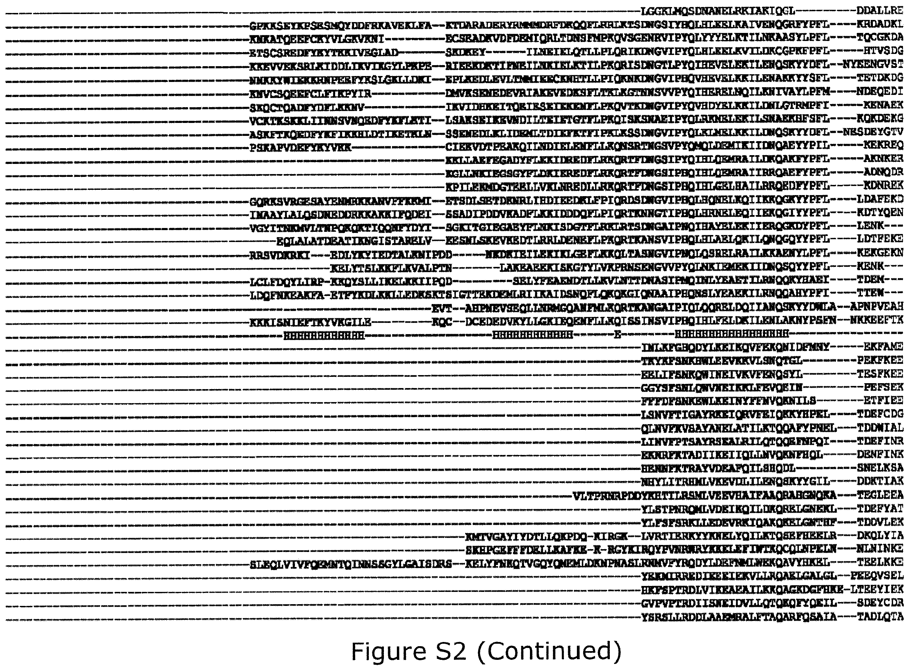

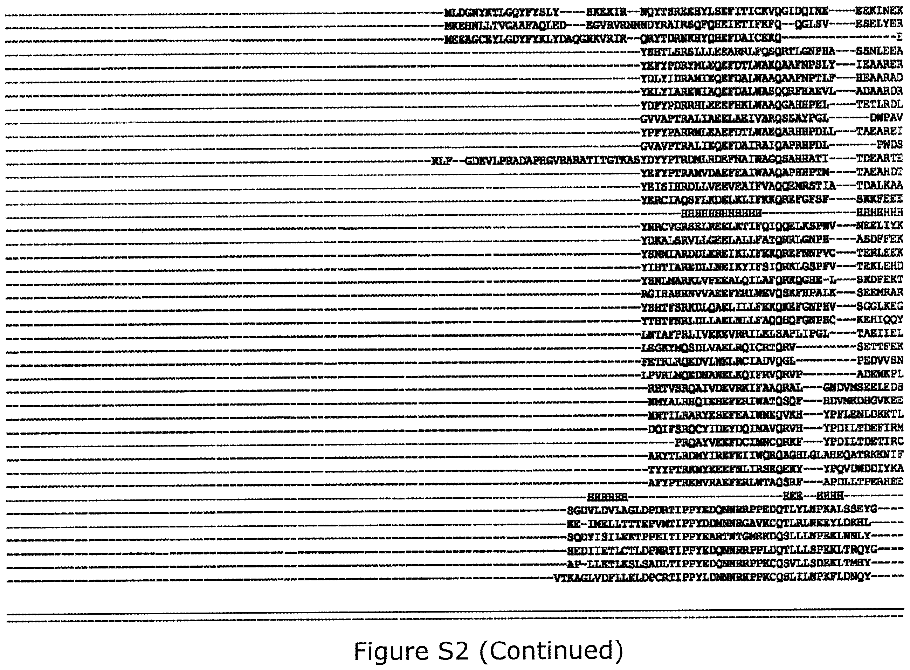

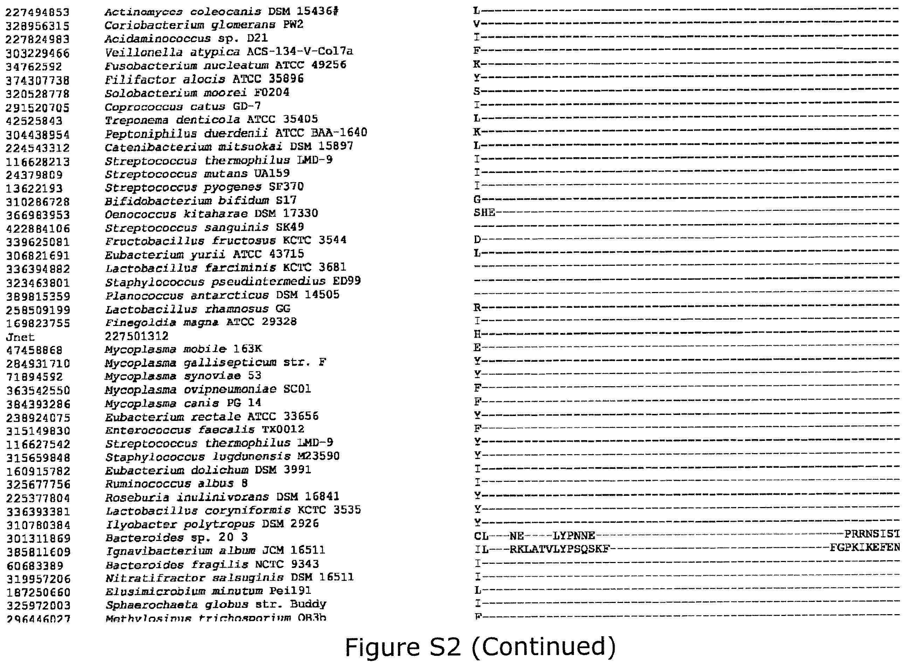

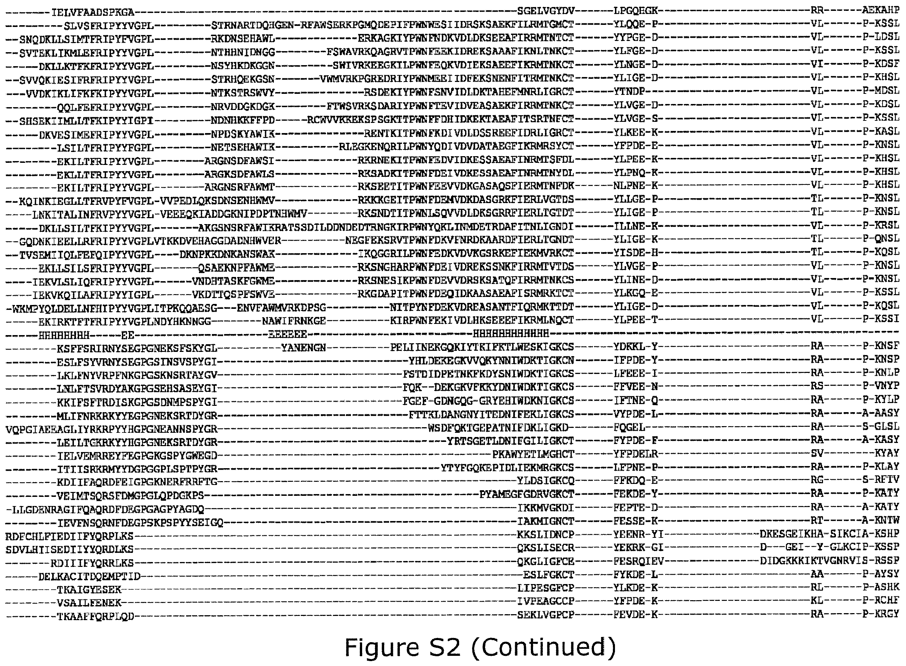

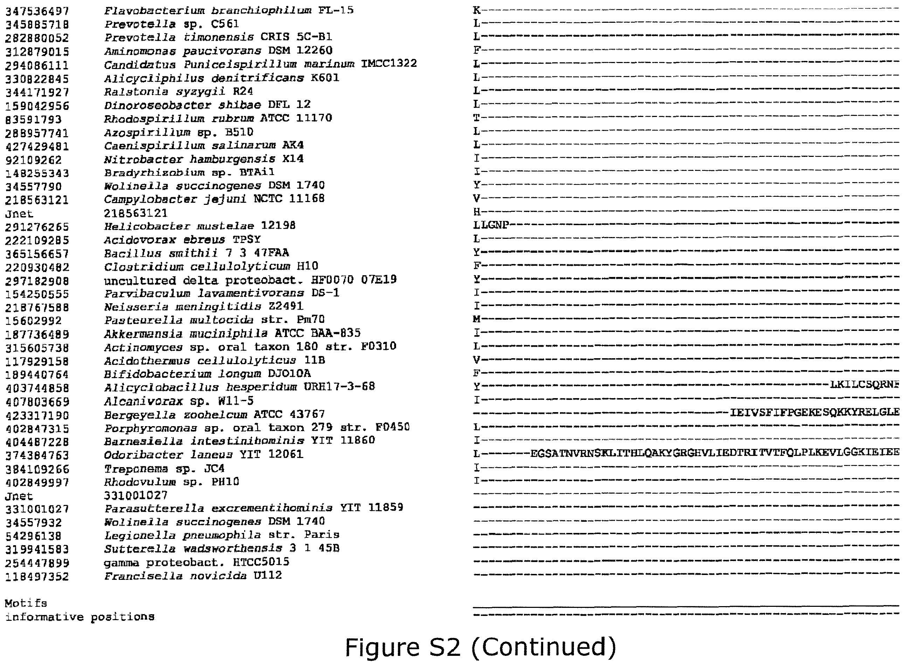

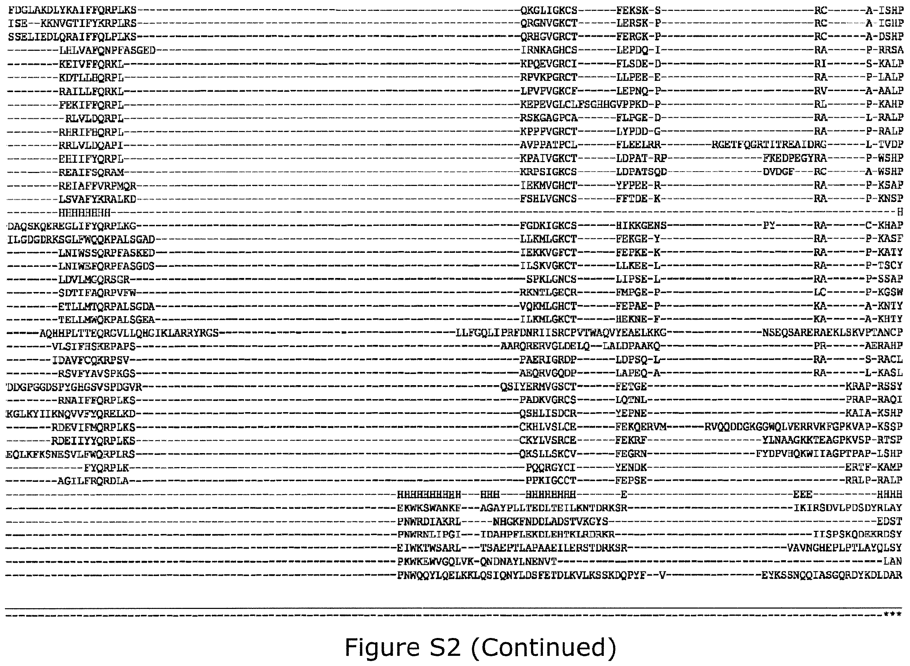

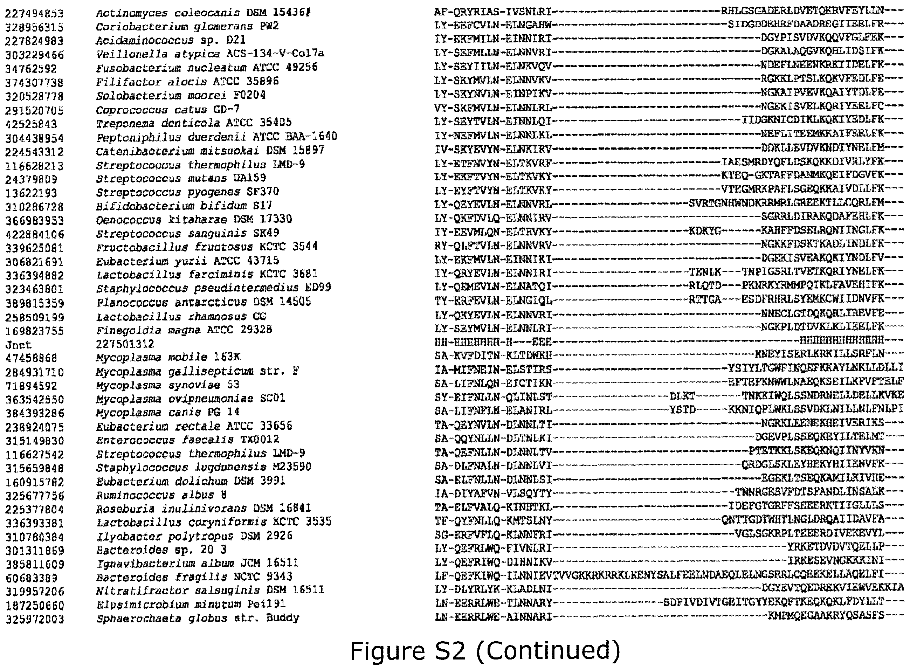

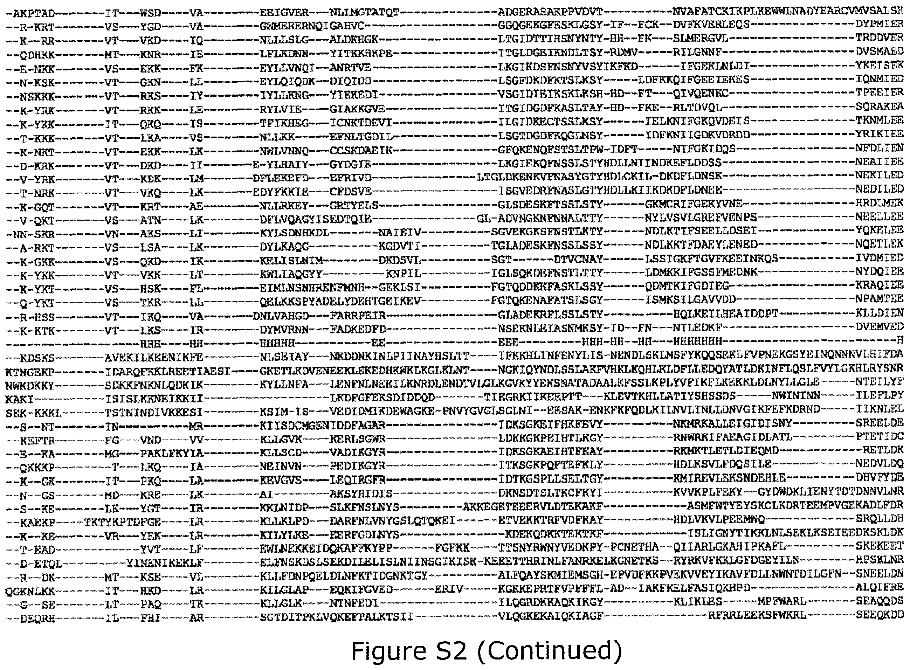

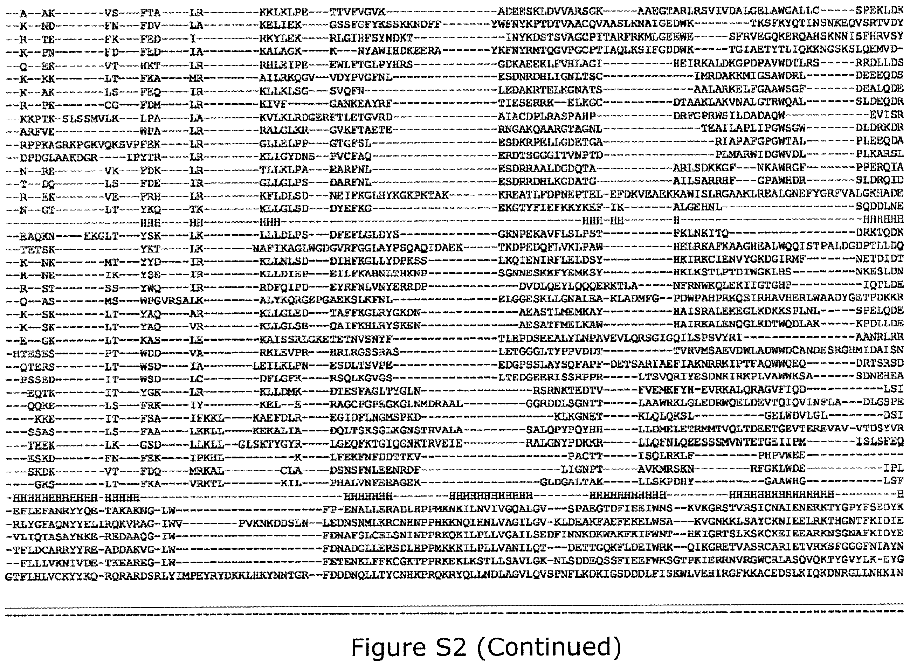

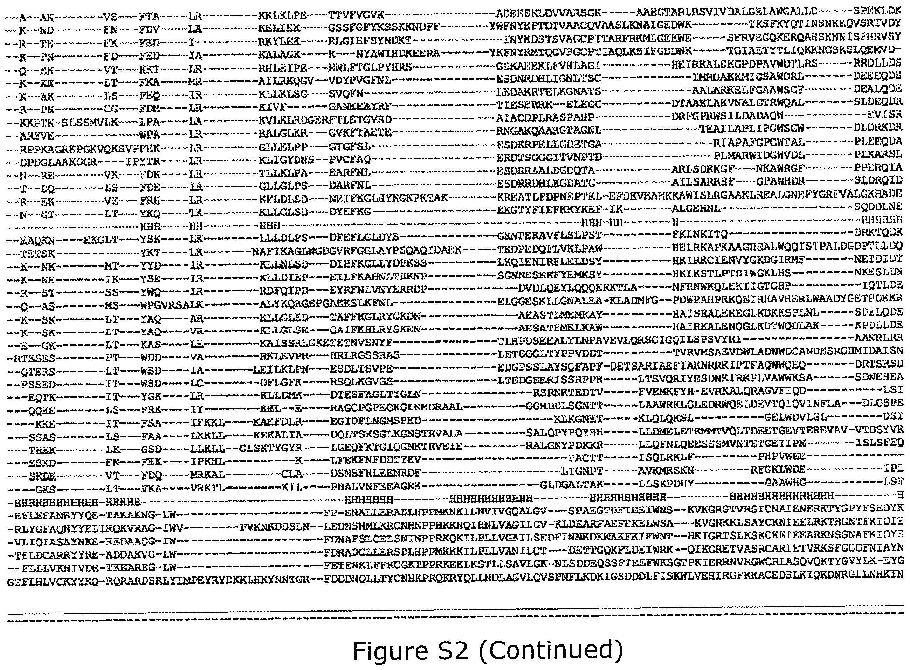

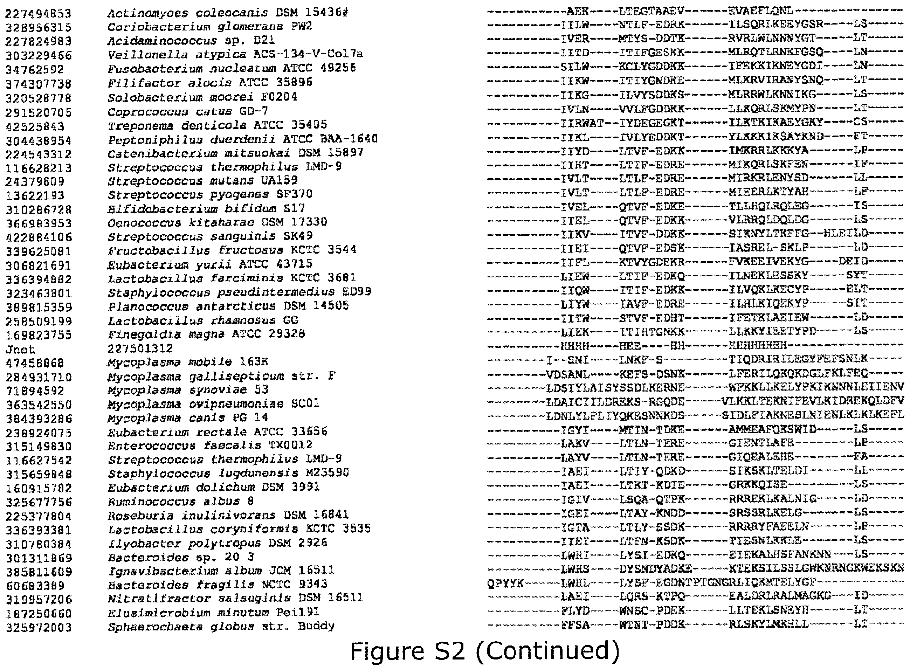

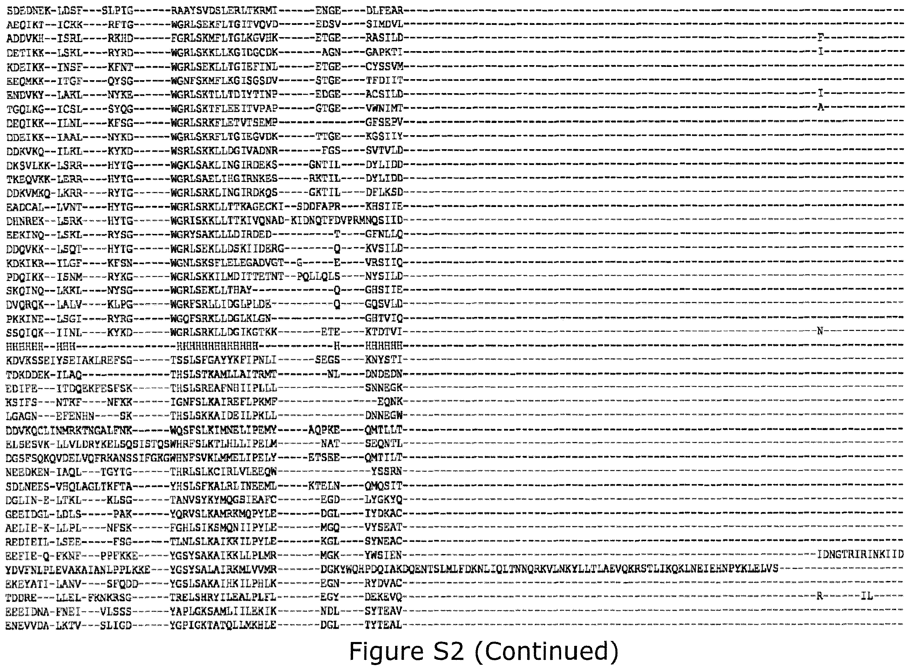

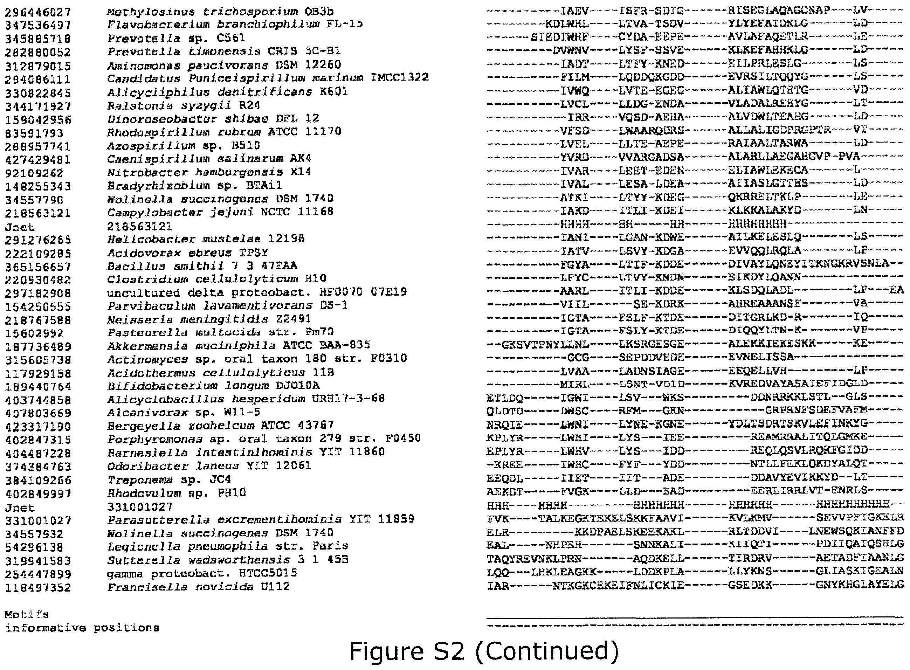

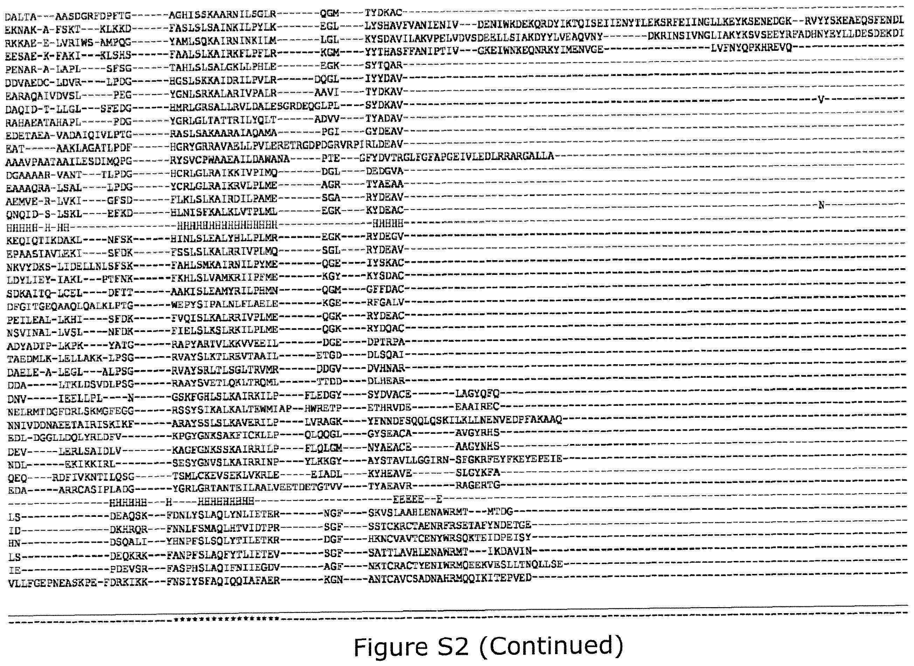

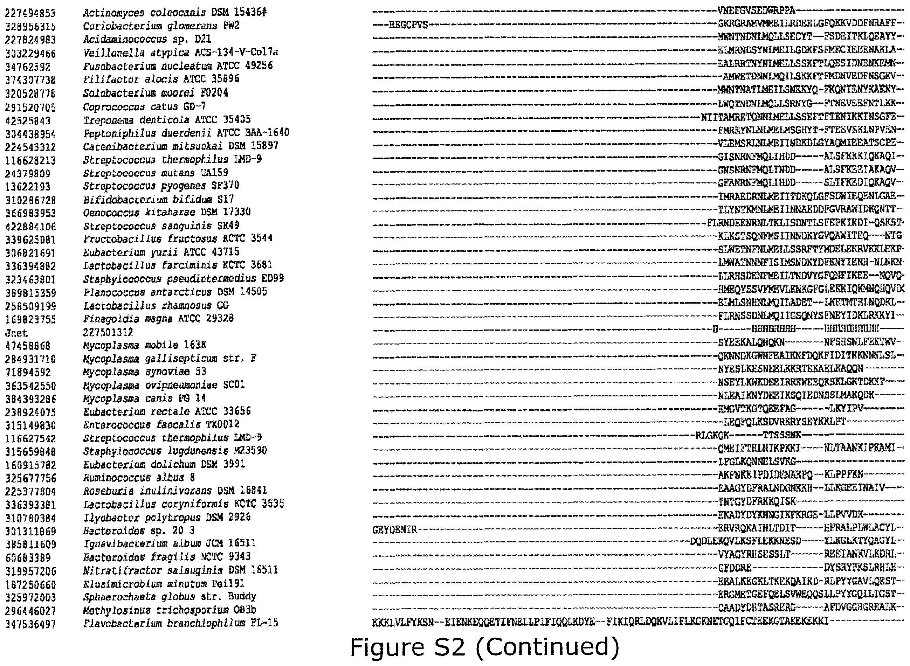

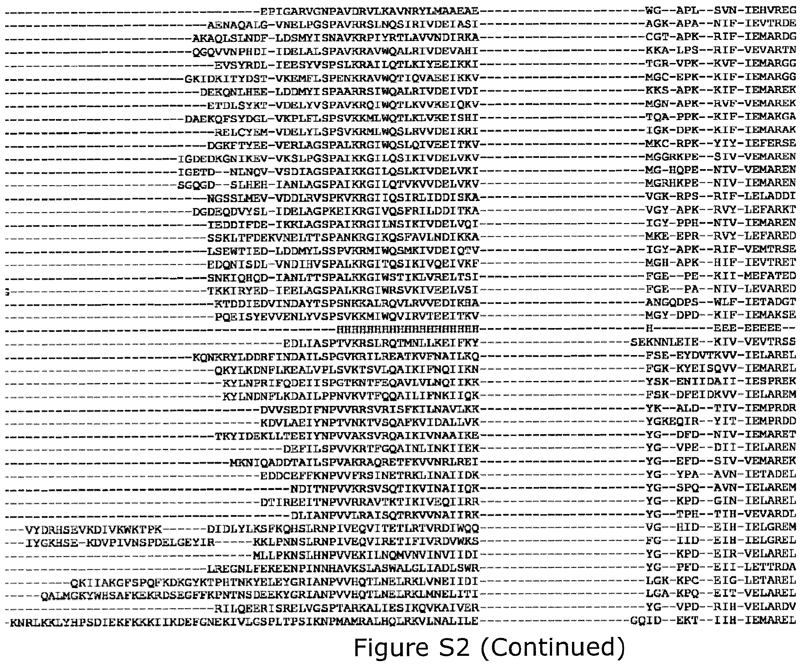

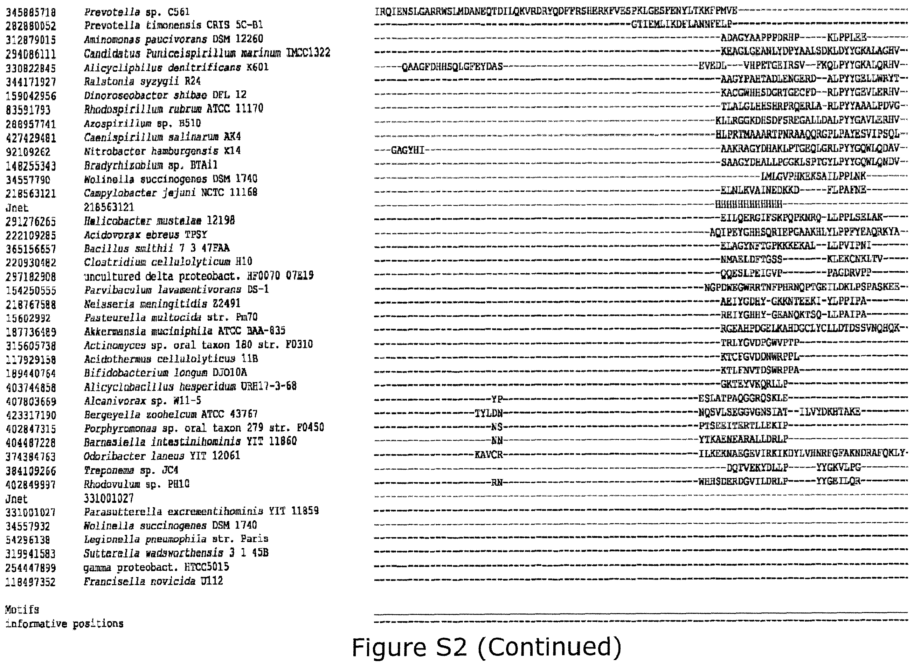

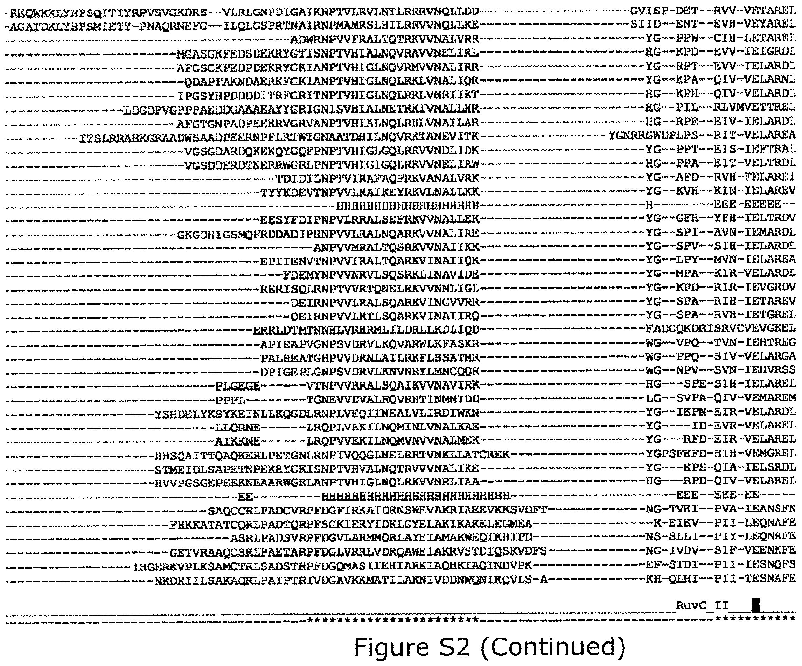

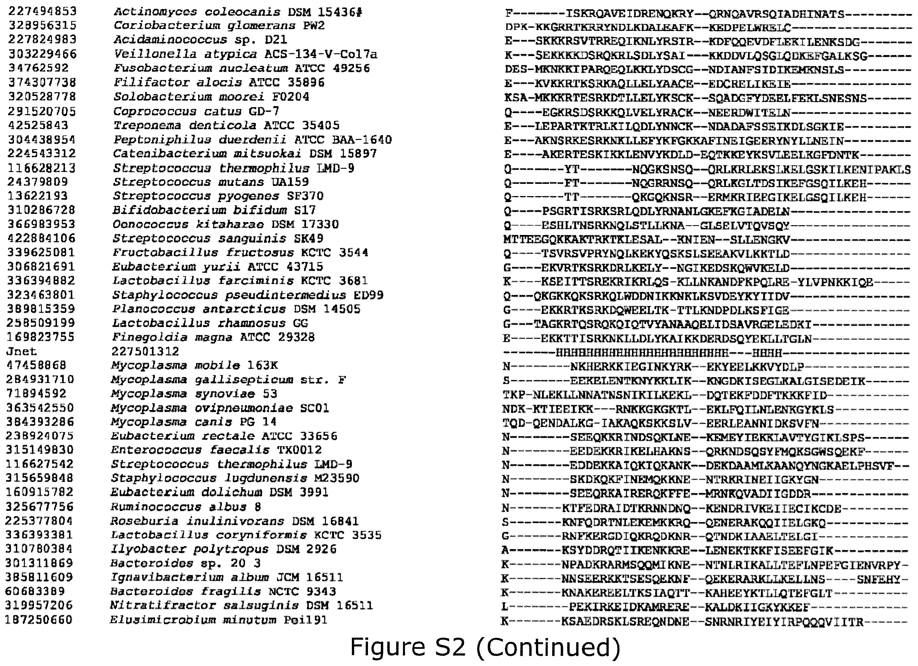

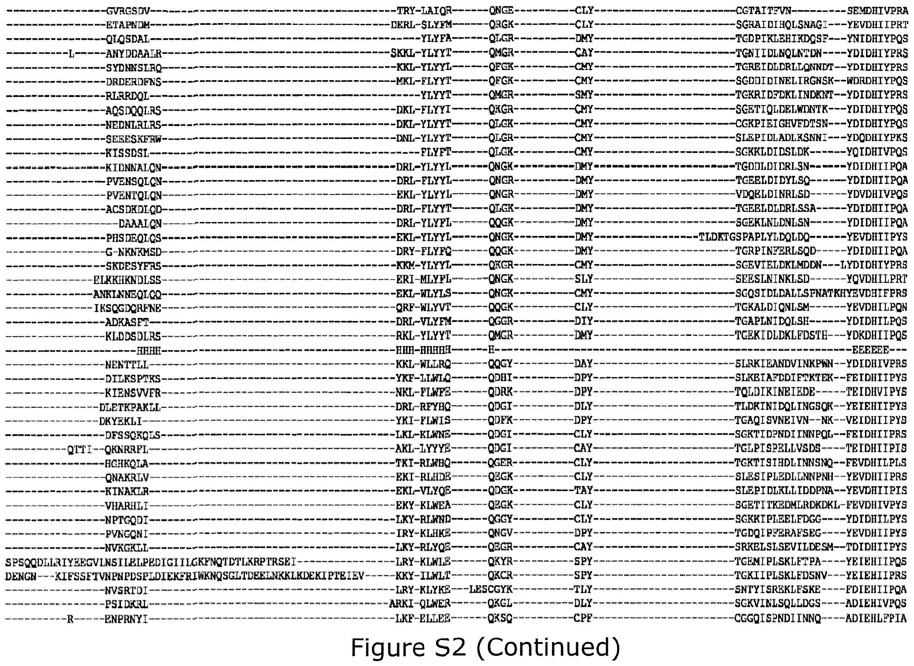

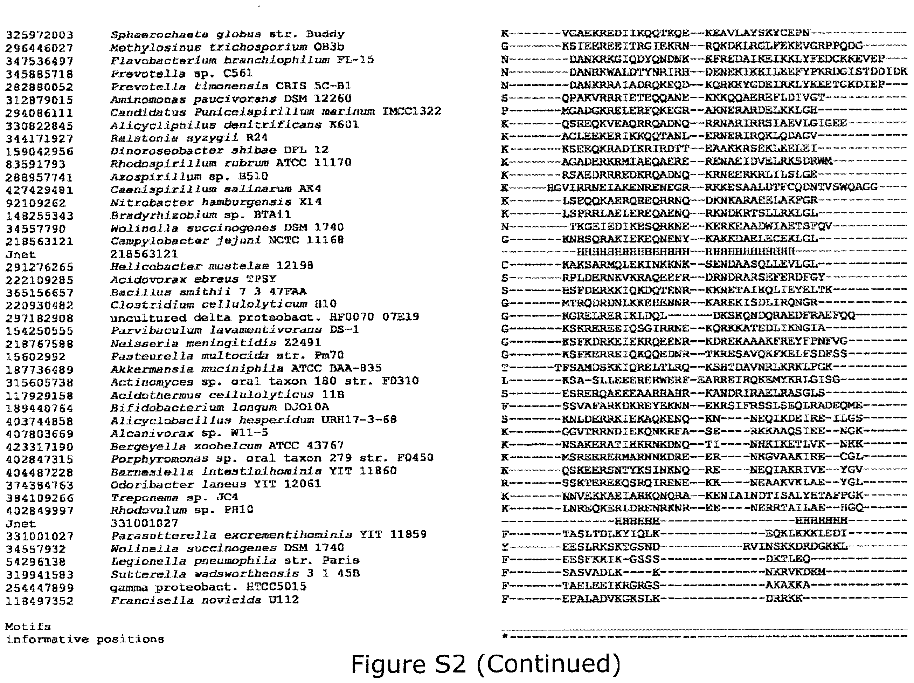

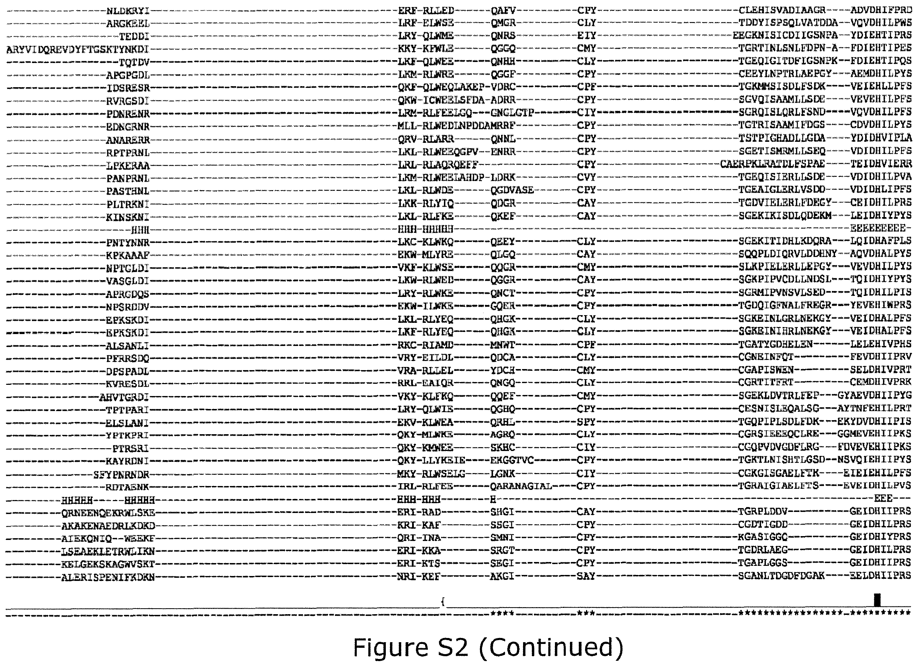

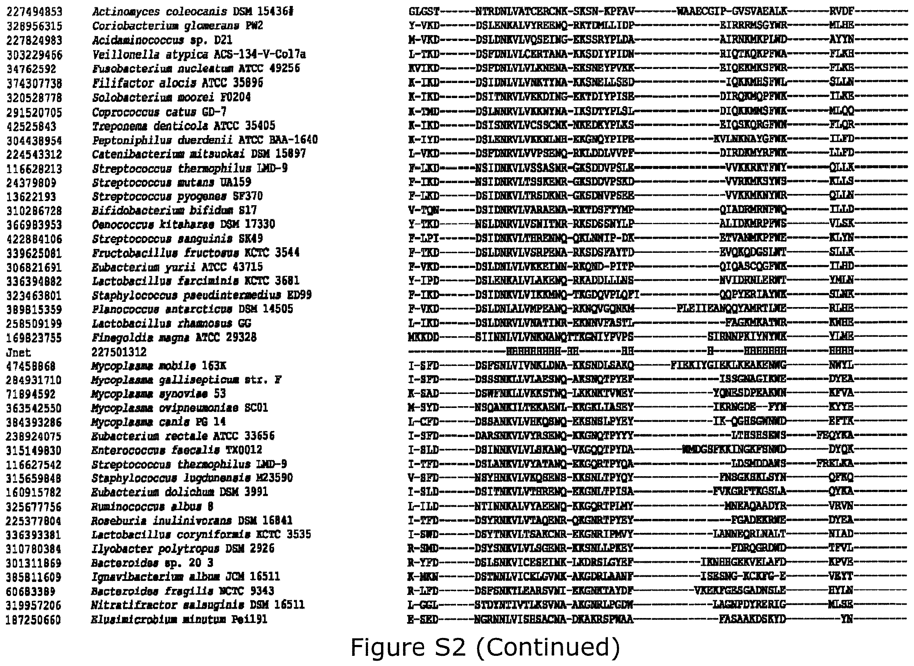

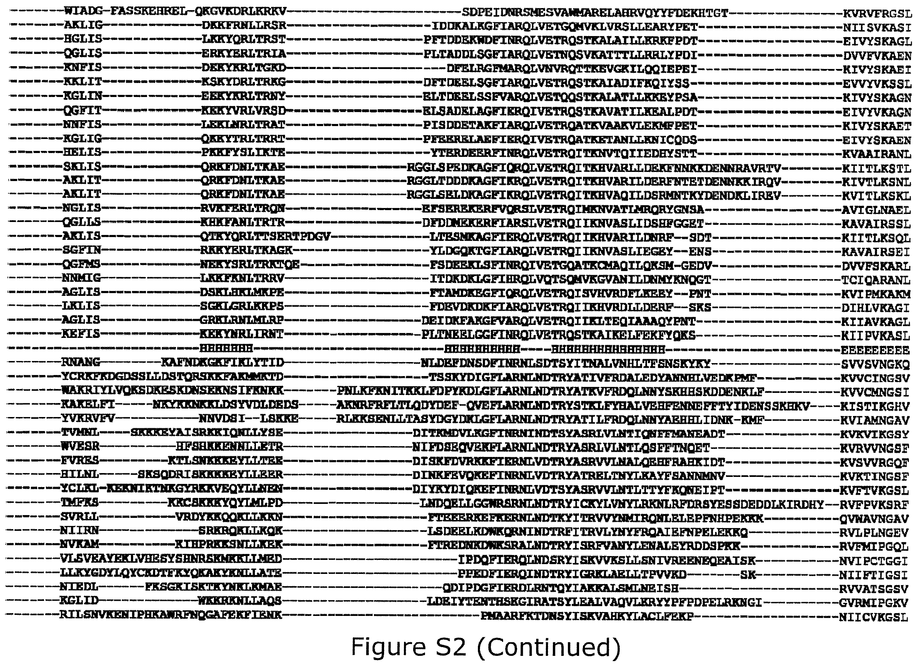

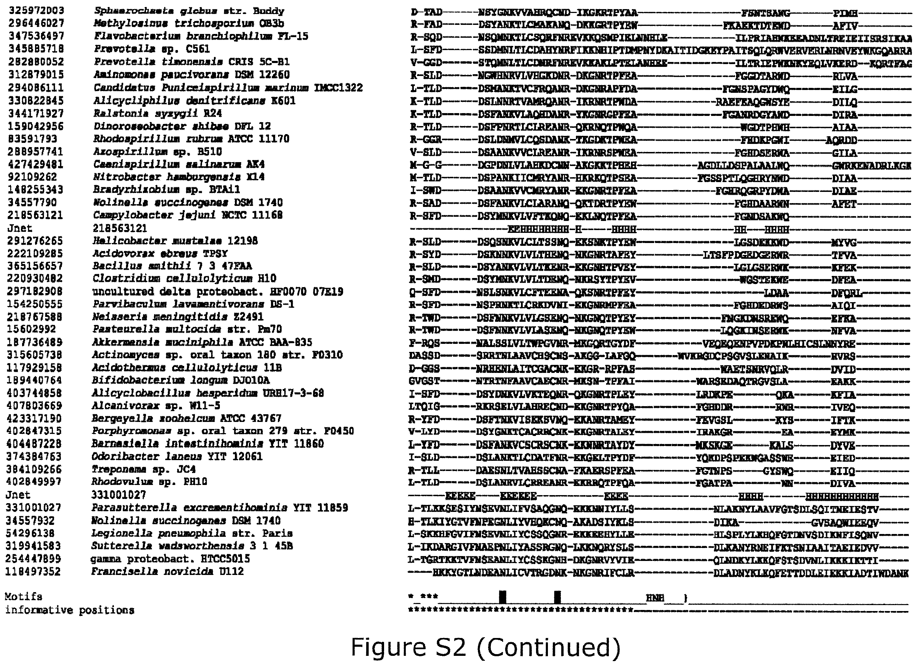

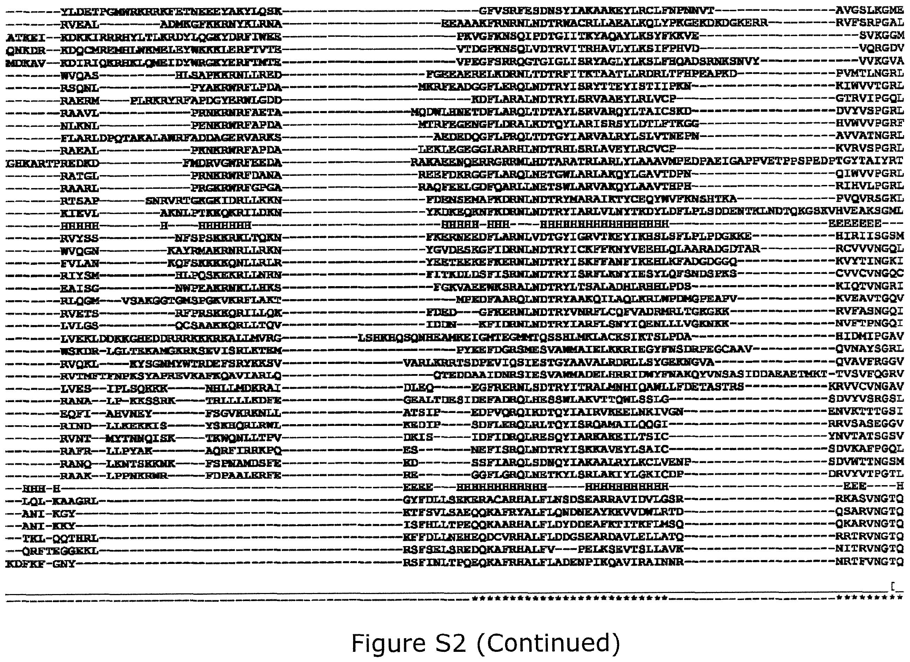

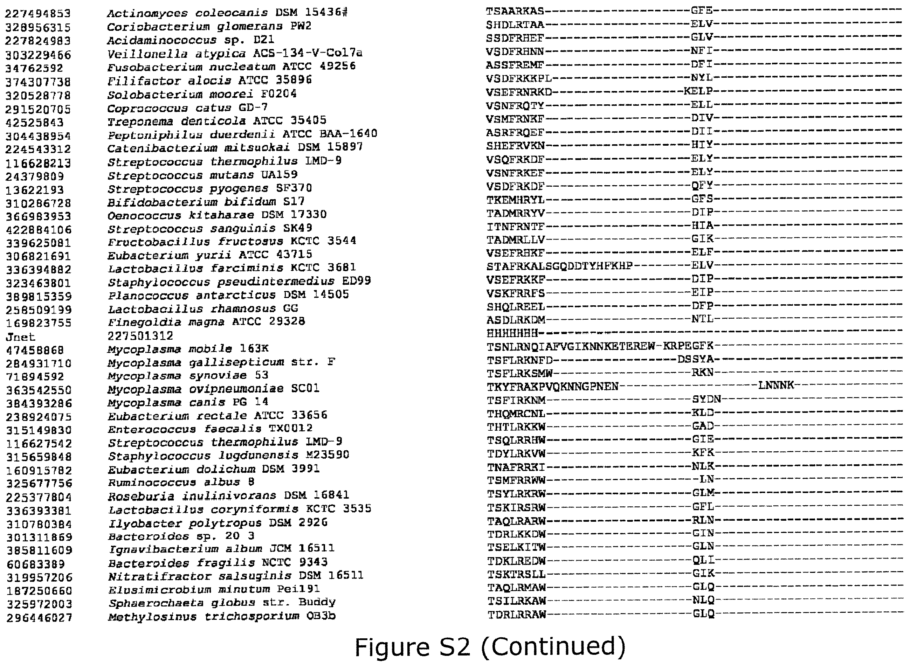

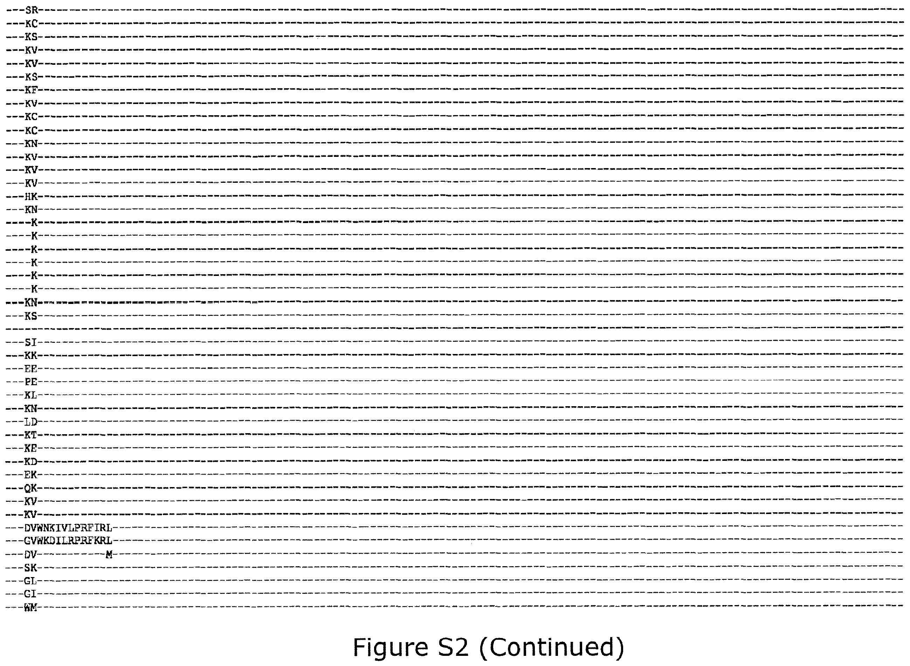

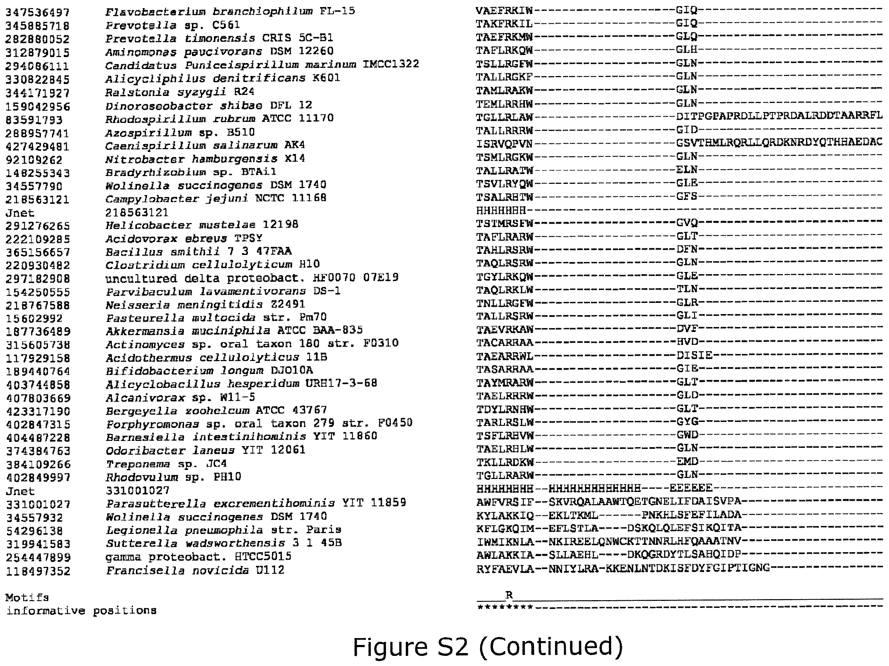

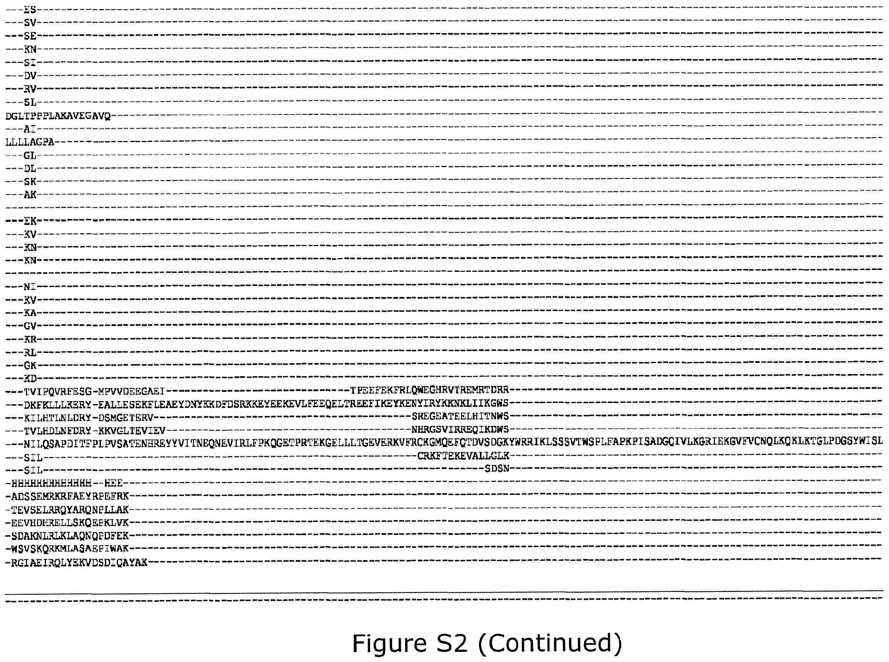

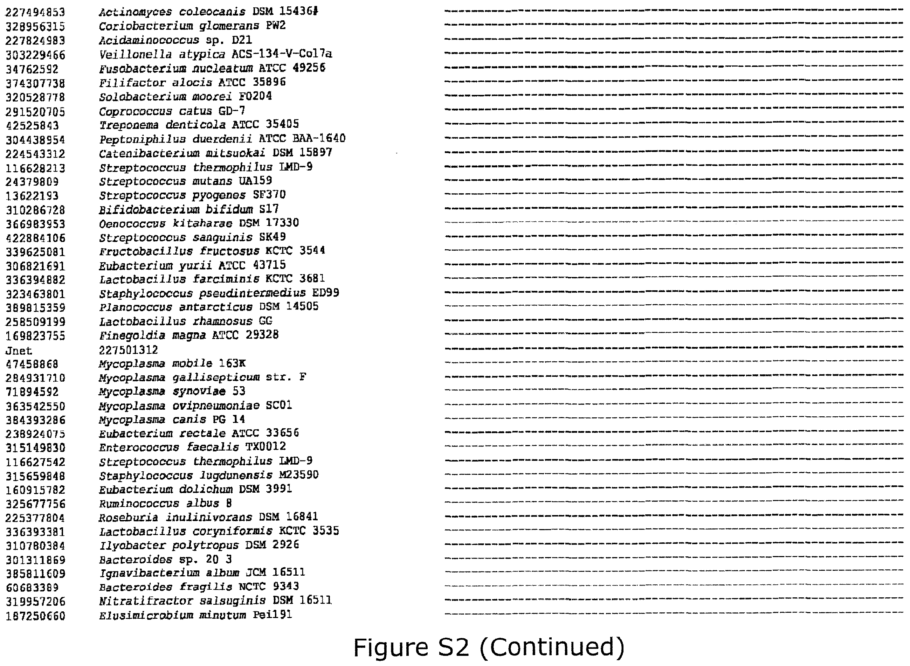

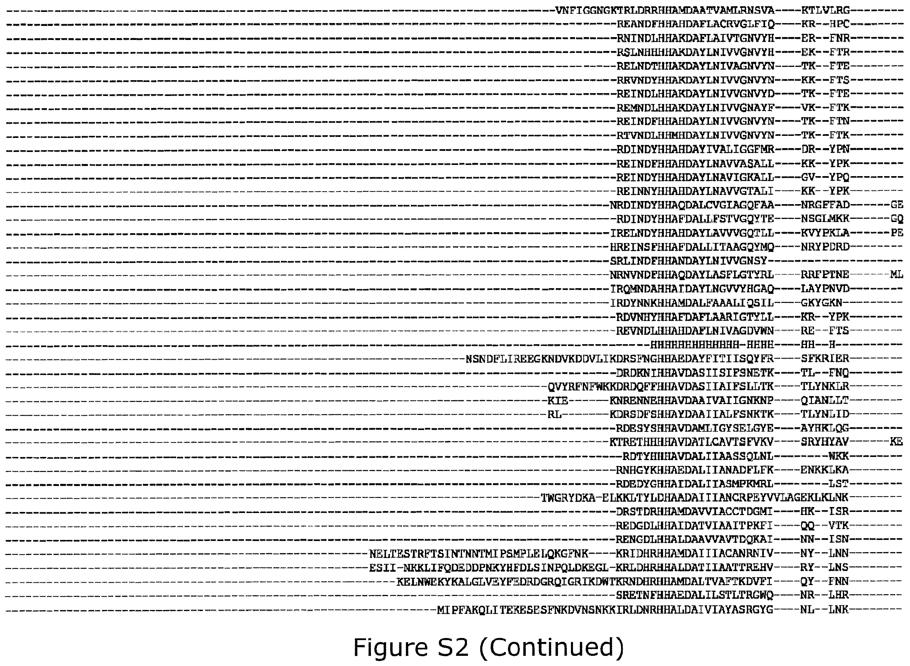

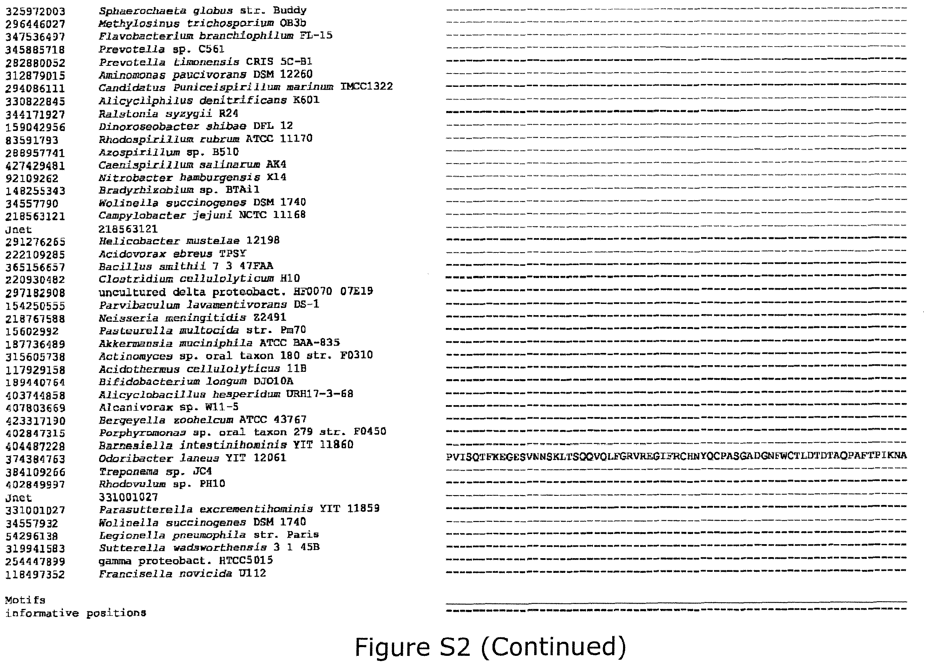

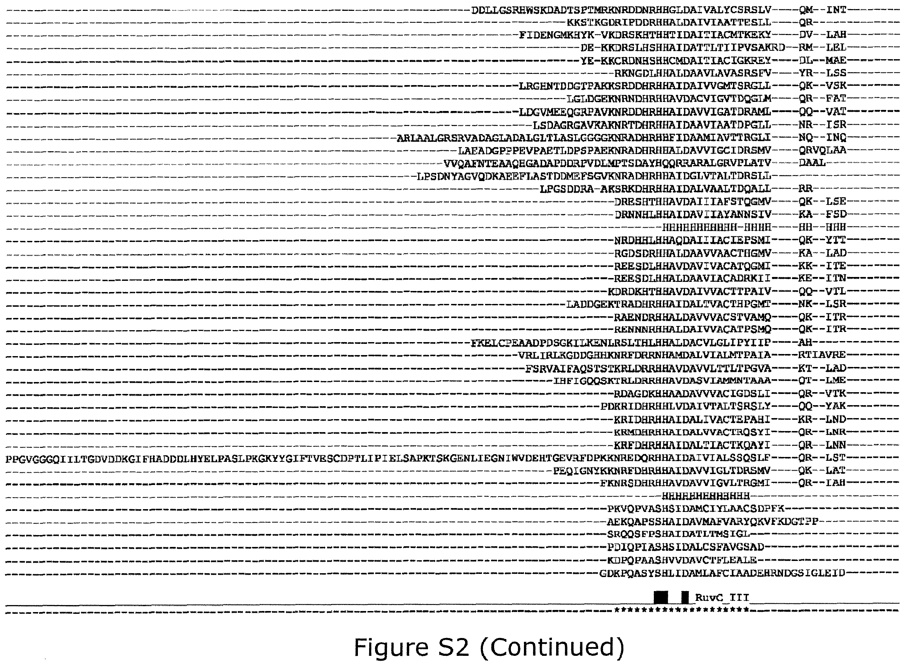

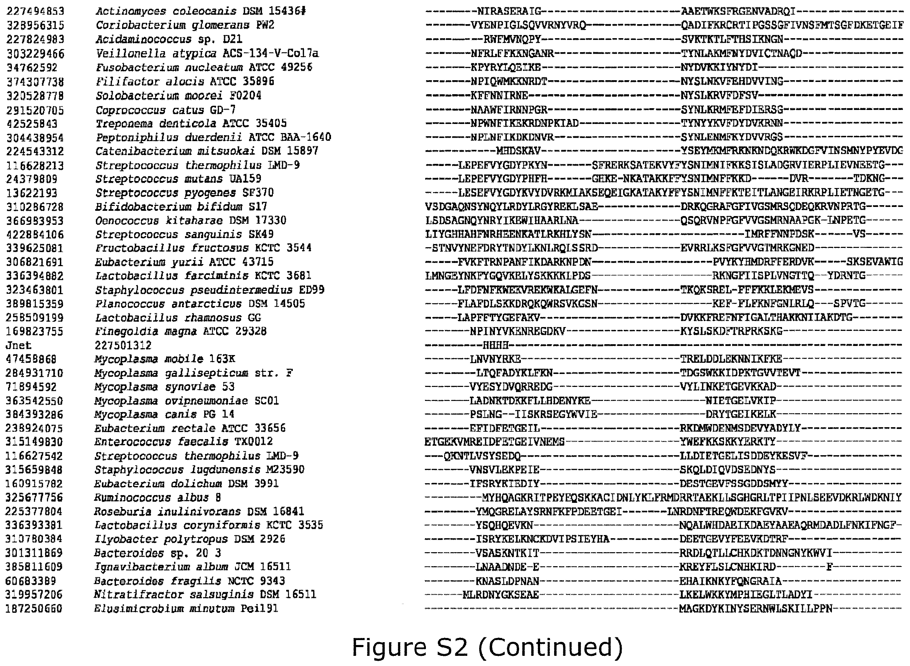

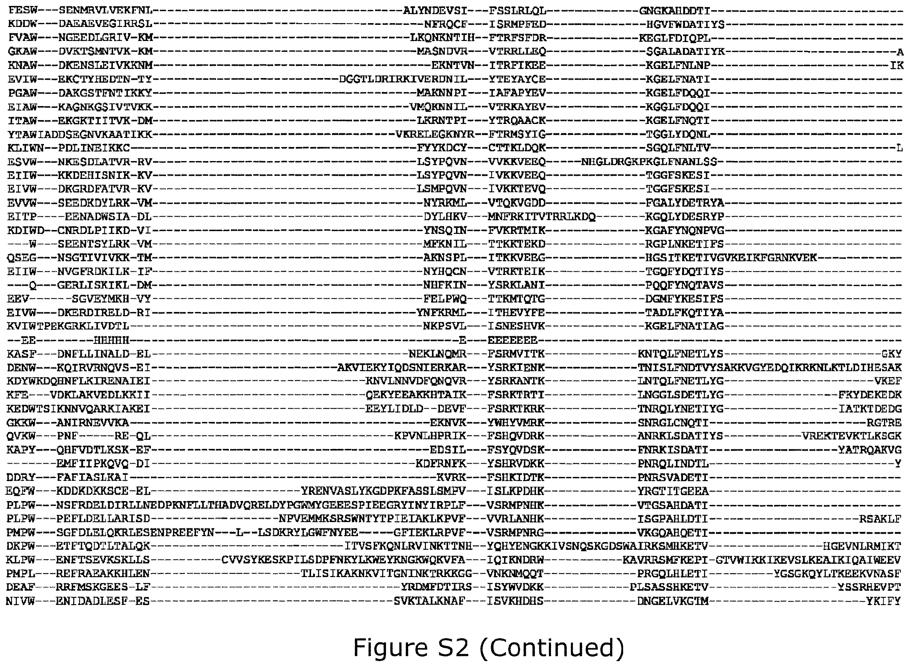

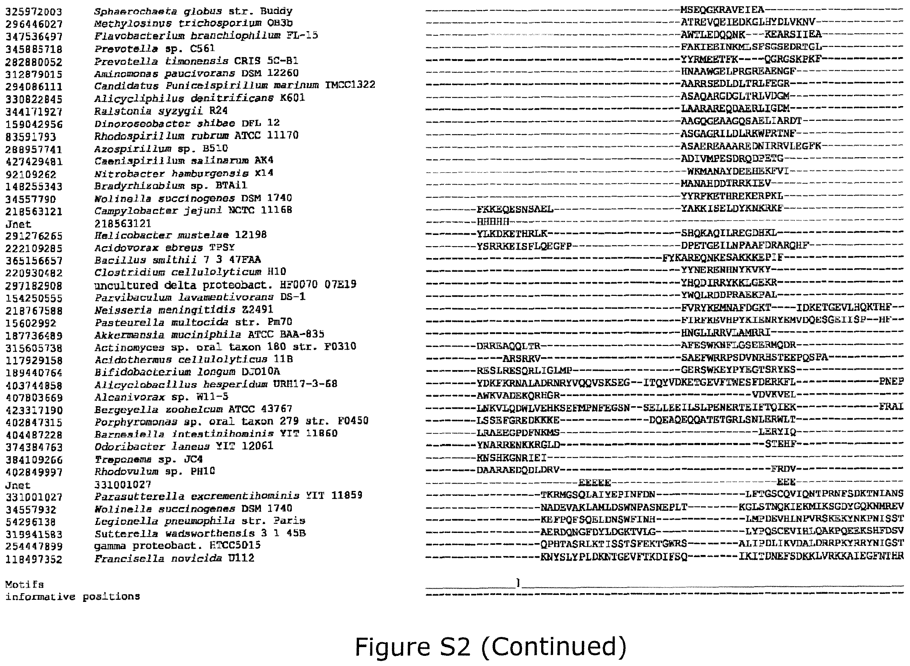

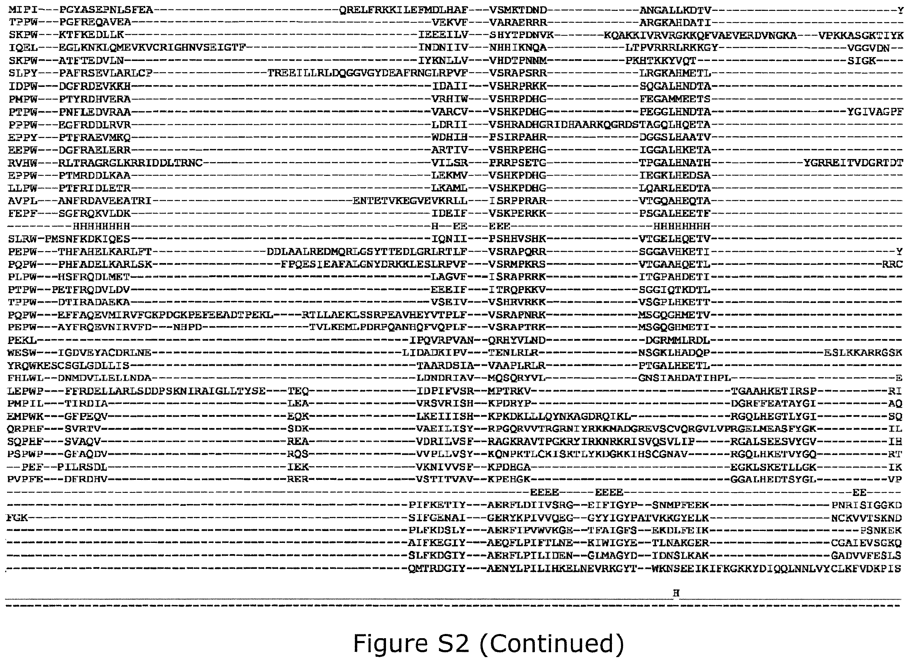

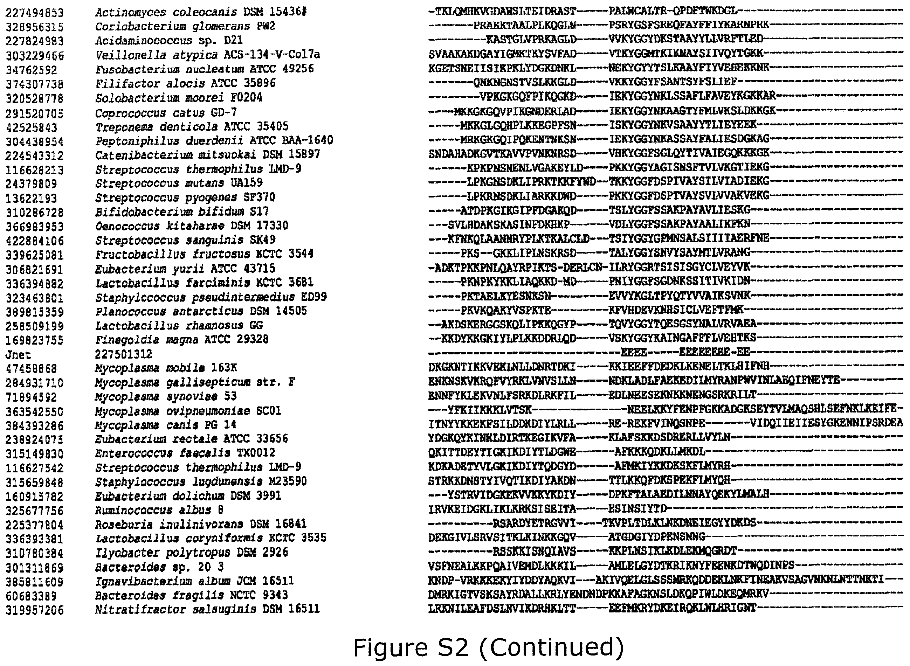

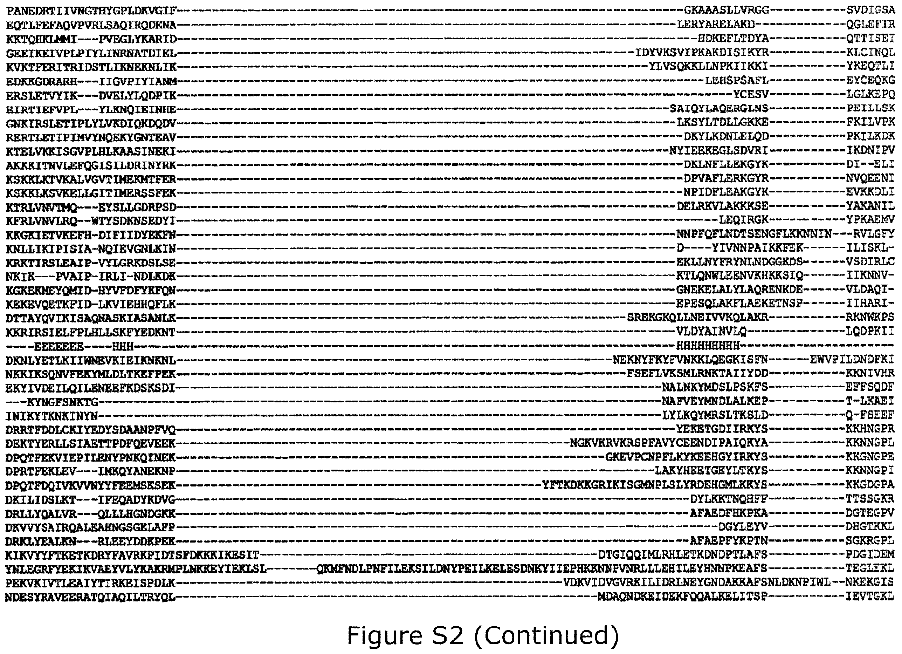

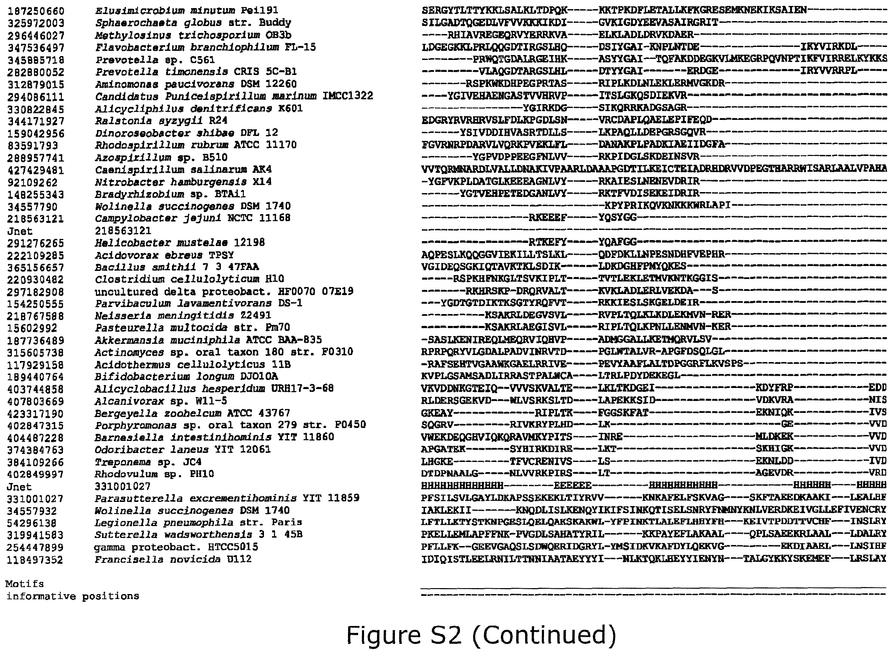

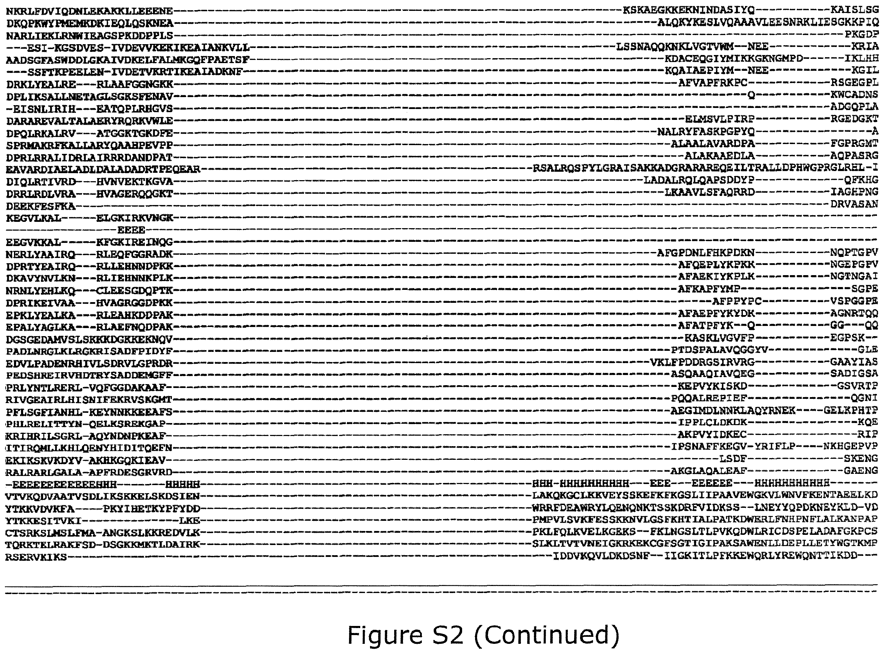

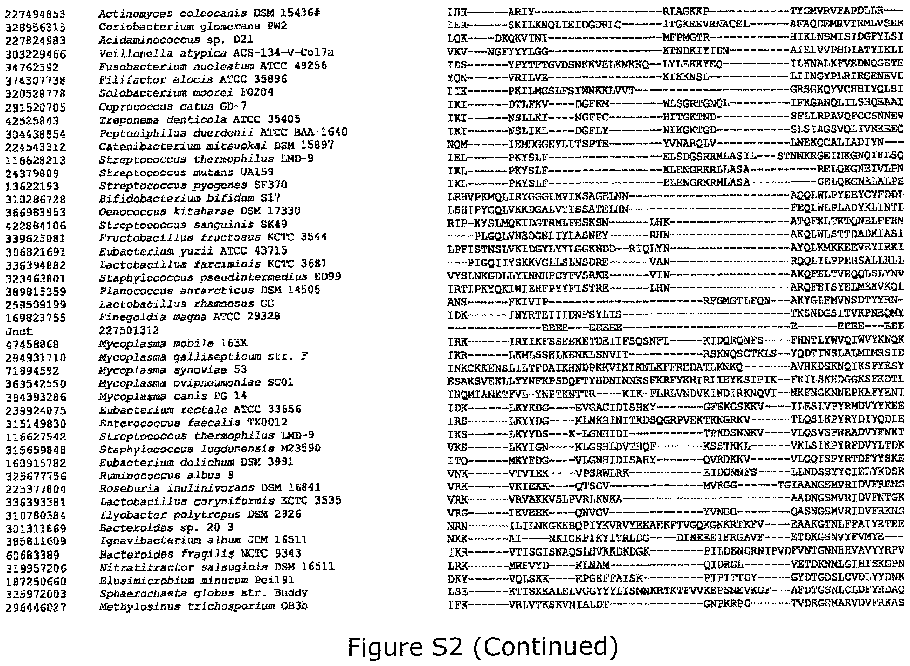

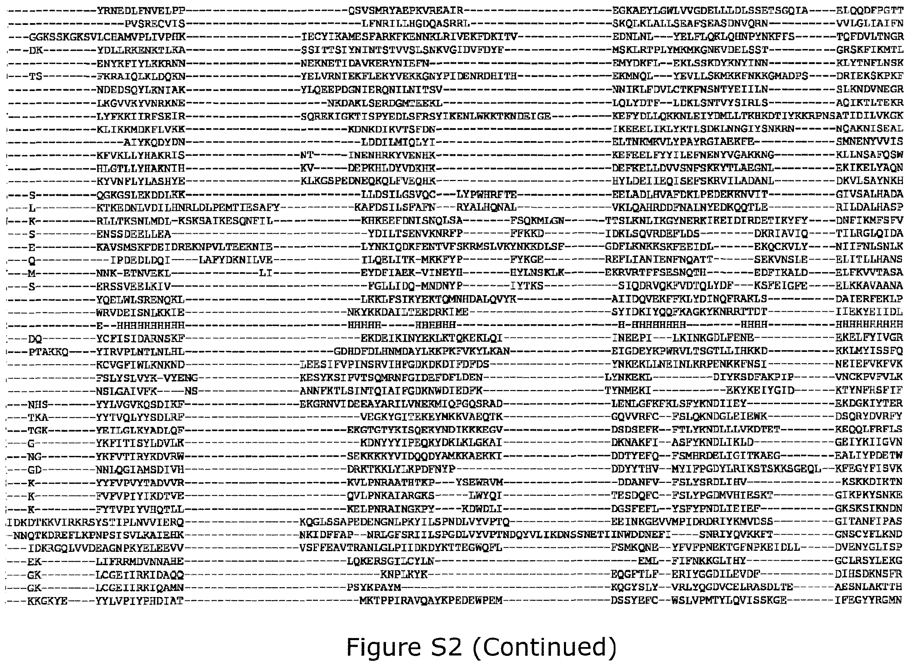

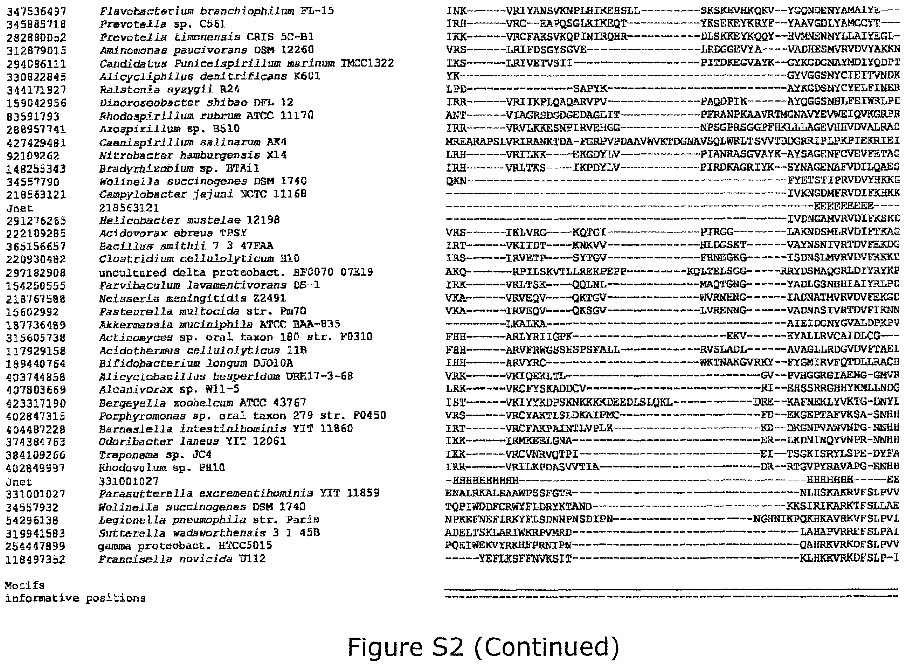

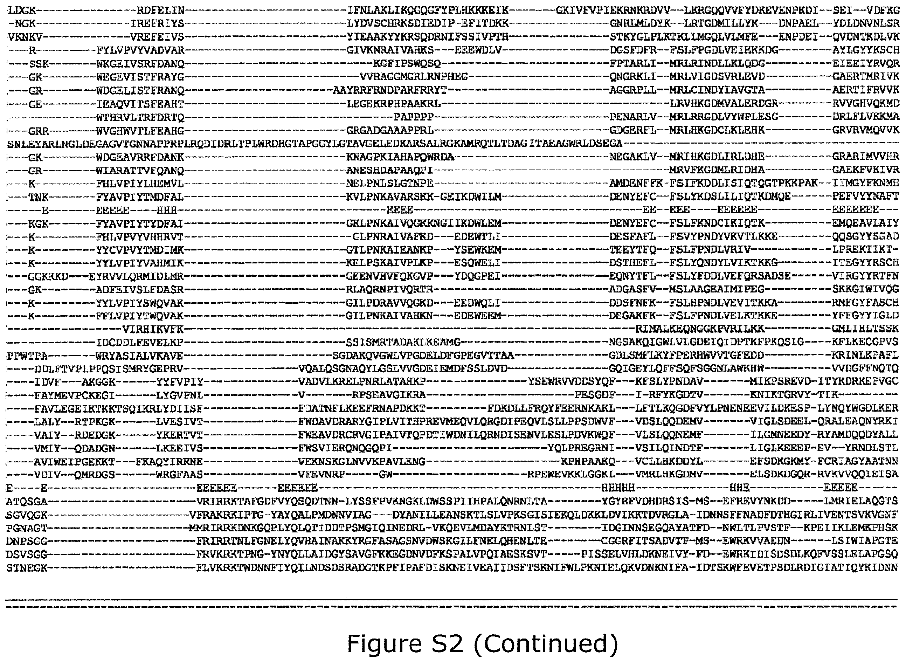

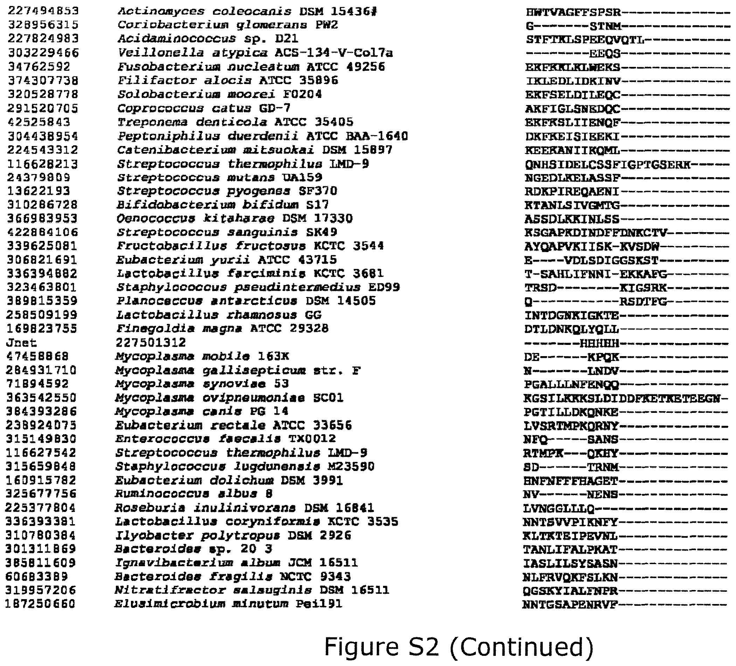

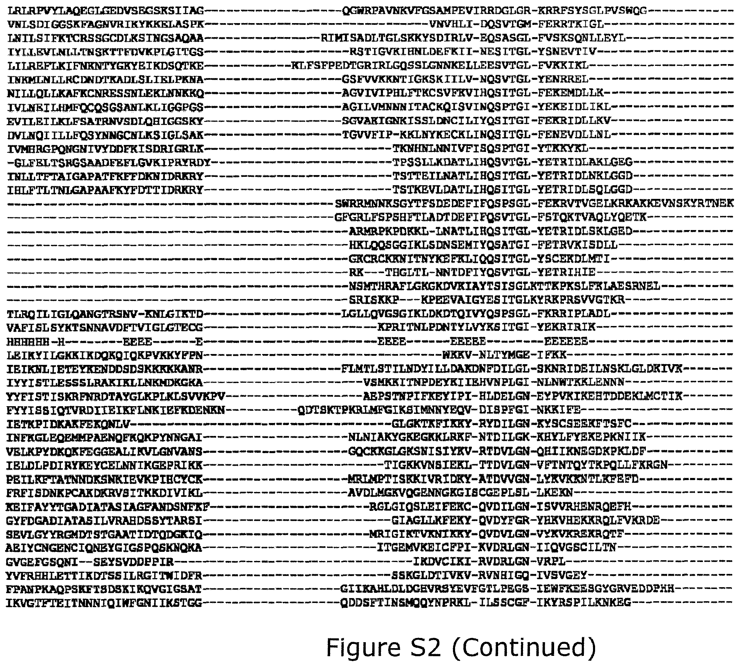

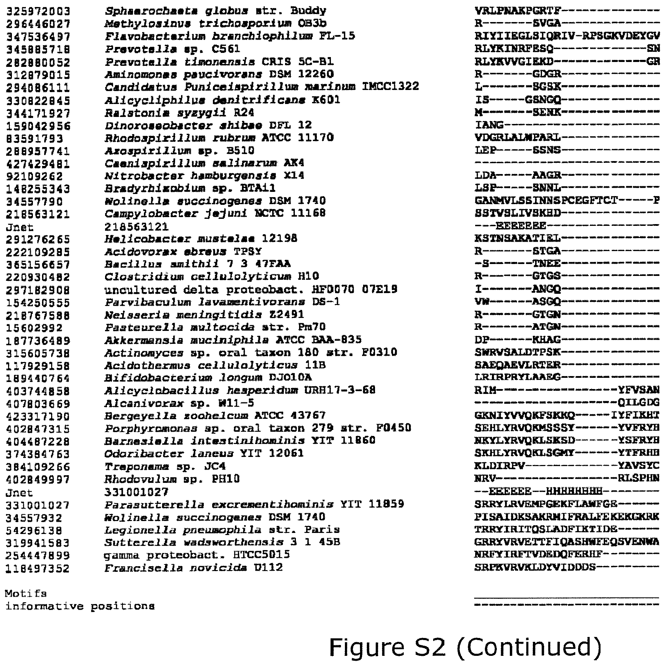

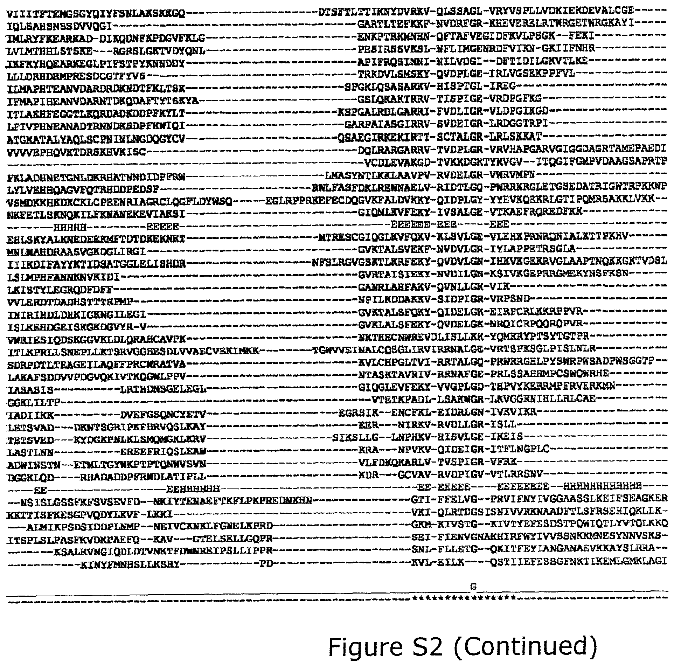

**Figure 2****.** RNase III is a general executioner of tracrRNA:pre-crRNA processing in type II CRISPR-Cas. Northern blot analysis of total RNA from *S*. *pyogenes* WT, Δ*rnc* and Δ*rnc* complemented with rnc orthologs or mutants (truncated *rnc* and inactivated (dead) (D51A) *rnc*) probed for tracrRNA (top) and crRNA repeat (bottom). RNA sizes in nt and schematic representations of tracrRNA (red-black) and crRNA (green-black) are indicated on the right (16). The vertical black arrows indicate the processing sites. tracrRNA-171 nt and tracrRNA-89 nt forms correspond to primary tracrRNA transcripts. The presence of tracrRNA-75 nt and crRNA 39-42 nt forms indicates tracrRNA and pre-crRNA co-processing. *S. pyogenes* tracrRNA and pre-crRNA are co-processed by all analyzed RNase III orthologs. The truncated version and catalytic inactive mutant of *S. pyogenes* RNase III are both deficient in tracrRNA:pre-crRNA processing.

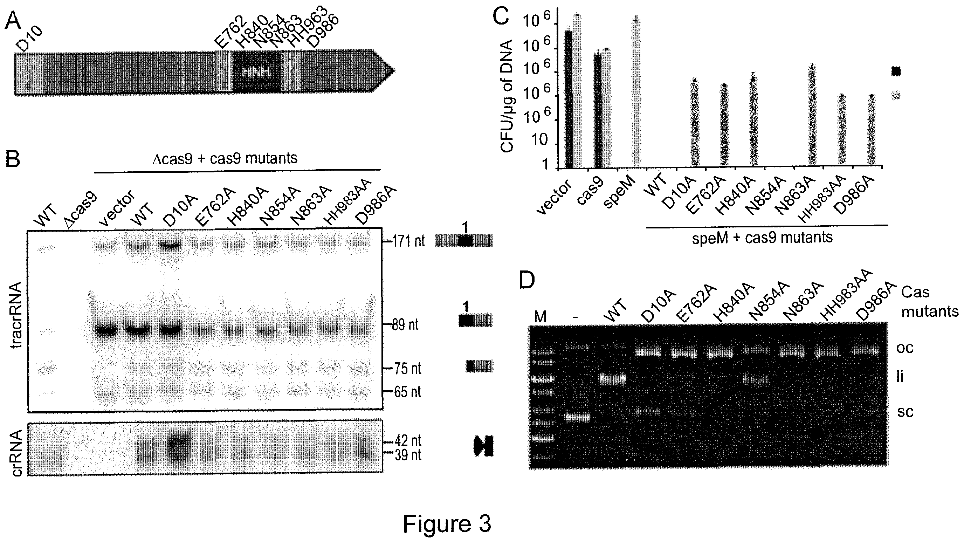

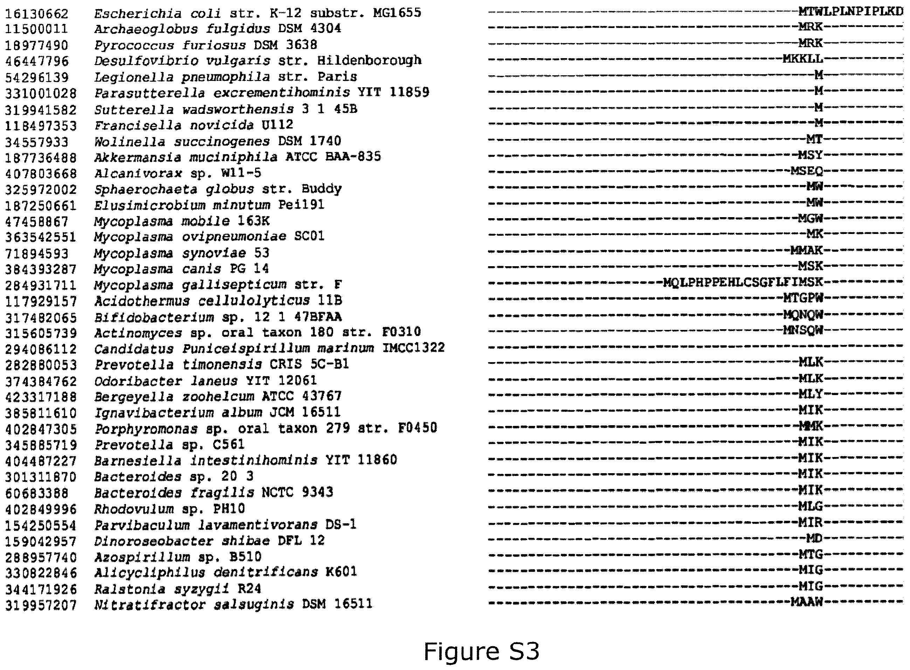

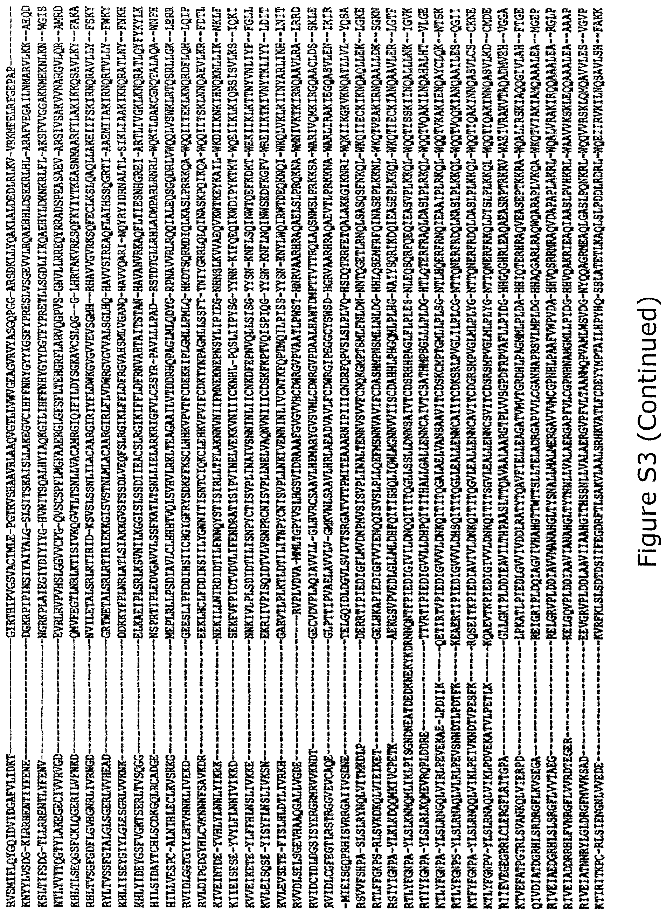

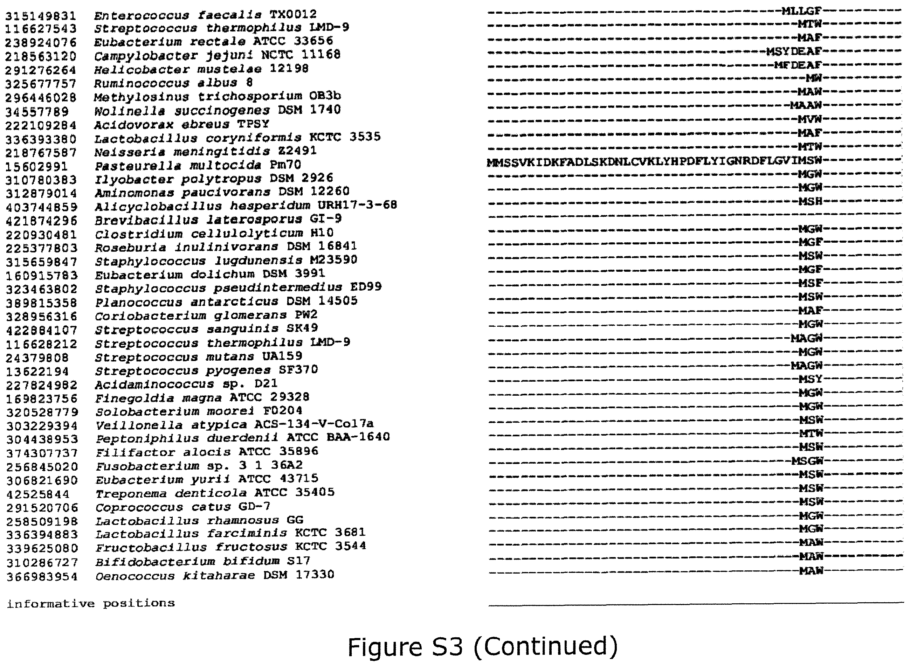

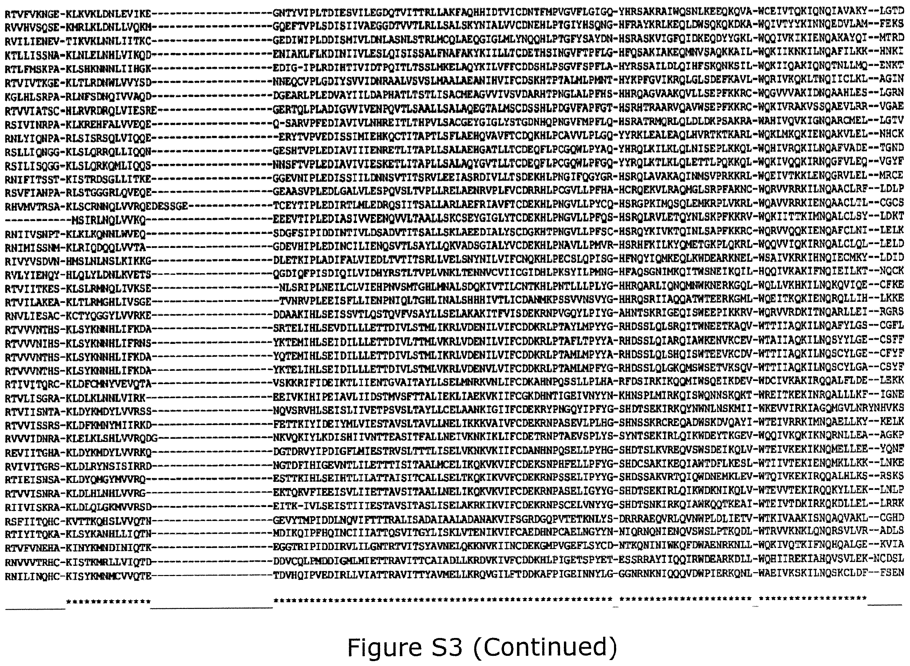

**Figure 3****.** Conserved motifs of Cas9 are required for DNA interference but not for dual-RNA processing by RNase III. (**A**) Schematic representation of *S. pyogenes* Cas9. The conserved HNH and splitted RuvC motifs and analyzed amino acids are indicated. (**B**) Northern blot analysis of total RNA from *S. pyogenes* WT, Δ*cas9* and Δ*cas9* complemented with pEC342 or pEC342 containing *cas9* WT or mutant genes, probed for tracrRNA and crRNA repeat. Maturation of tracrRNA and pre-crRNA generating tracrRNA-75 nt and crRNA-39-42 nt forms is observed in all Δ*cas9* strains complemented with the *cas9* mutants. (**C**) *In vivo* protospacer targeting. Transformation assays of S. *pyogenes* WT and Δ*cas9* with pEC85 (vector), pEC85Ω*cas9* (*cas9*), pEC85Ω*speM* (*speM*), and pEC85ΩtracrRNA-171 nt plasmids containing *speM* and cas9 mutants. The CFUs (colony forming units) per µg of plasmid DNA were determined in at least three independent experiments. The results +/- SD of technical triplicates of one representative experiment are shown. Cas9 N854A is the only mutant that did not tolerate the protospacer plasmid as observed for WT Cas9, indicating that this residue is not involved in DNA interference. (**D**) *In vitro* plasmid cleavage. Agarose gel electrophoresis of plasmid DNA (5 nM) containing *speM* protospacer (pEC287) incubated with 25 nM Cas9 WT or mutants in the presence of equimolar amounts of dual-RNA-*speM* (see Materials and Methods). Cas9 WT and N854A generated linear cleavage products while the other Cas9 mutants created only nicked products. M, 1 kb DNA ladder (Fermentas); oc: open circular, li: linear; sc: supercoiled.

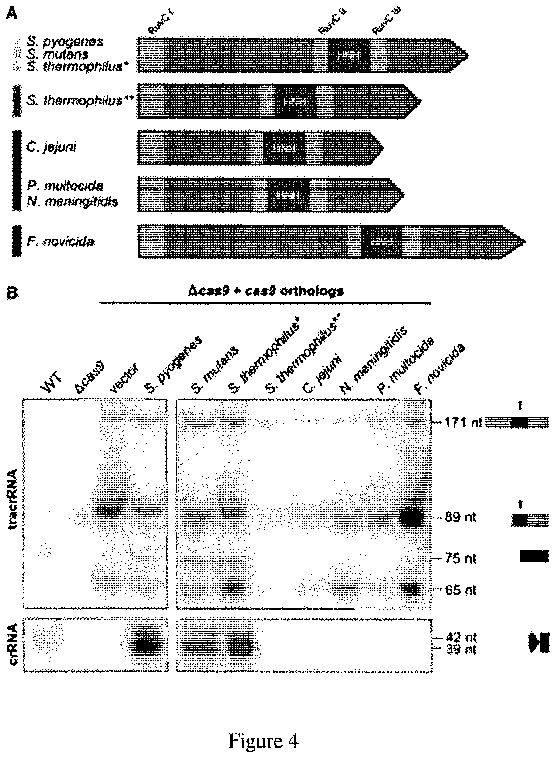

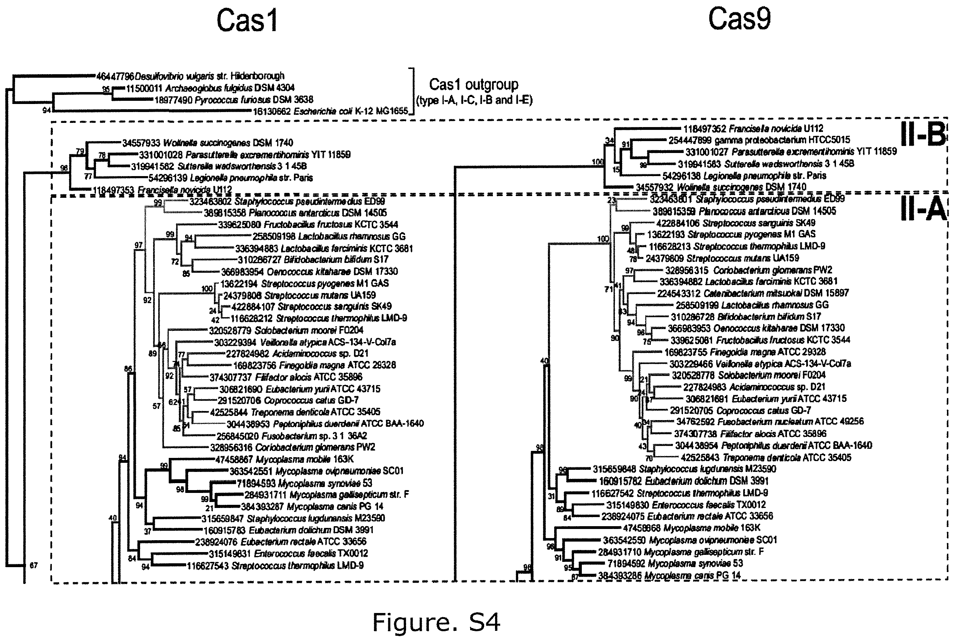

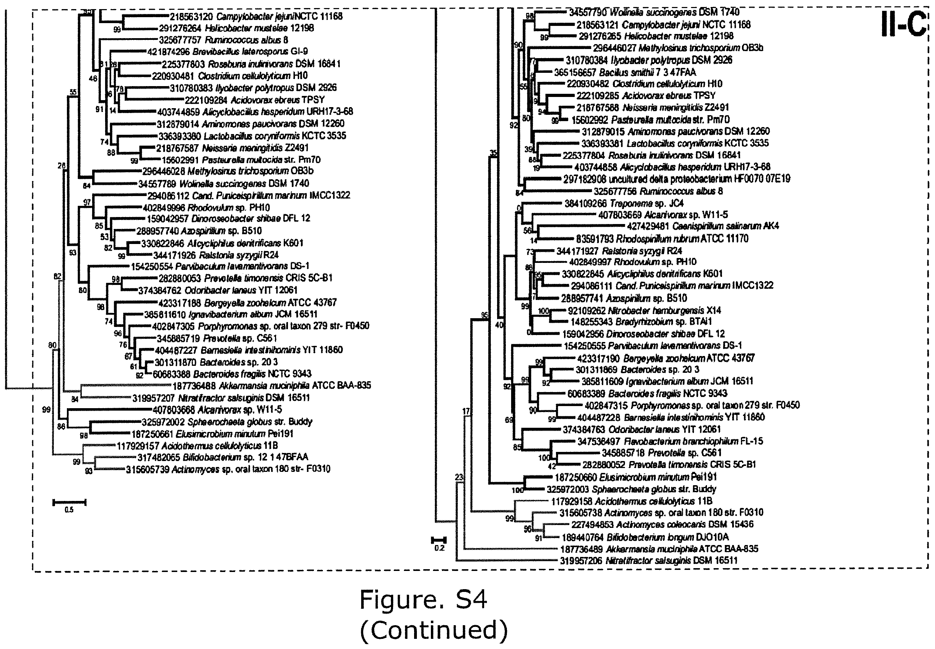

**Figure 4****.** Cas9 from closely related CRISPR-Cas systems can substitute the role of *S*. *pyogenes* Cas9 in RNA processing by RNase III. (**A**) Schematic representation of Cas9 from selected bacterial species. The protein sizes and distances between conserved motifs (RuvC and HNH) are drawn in scale. See Supplementary Figure S1. (**B**) Northern blot analysis of total RNA extracted from *S. pyogenes* WT, Δ*cas9* and Δ*cas9* complemented with pEC342 (backbone vector containing tracrRNA-171 nt and the cas operon promoter from *S. pyogenes*) or pEC342-based plasmids containing cas9 orthologous genes, probed for tracrRNA and crRNA repeat. Mature forms of *S. pyogenes* tracrRNA and pre-crRNA are observed only in the presence of *S. pyogenes* Cas9 WT or closely related Cas9 orthologs from *S*. *mutans* and *S*. *thermophilus**.

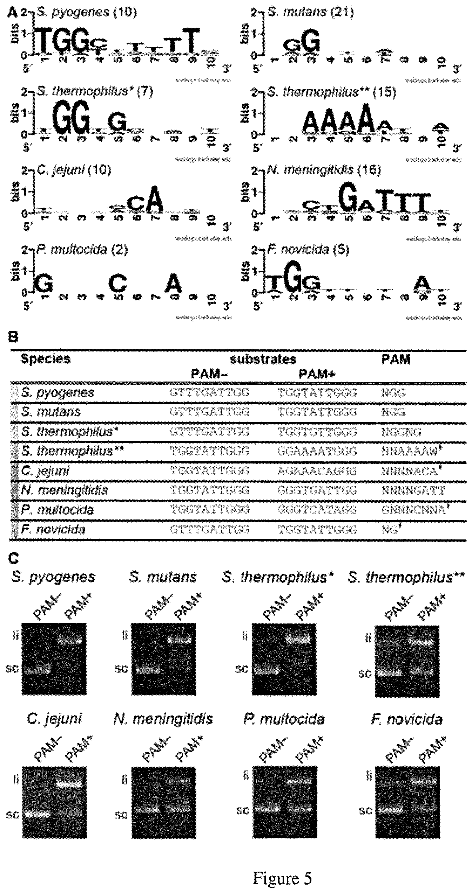

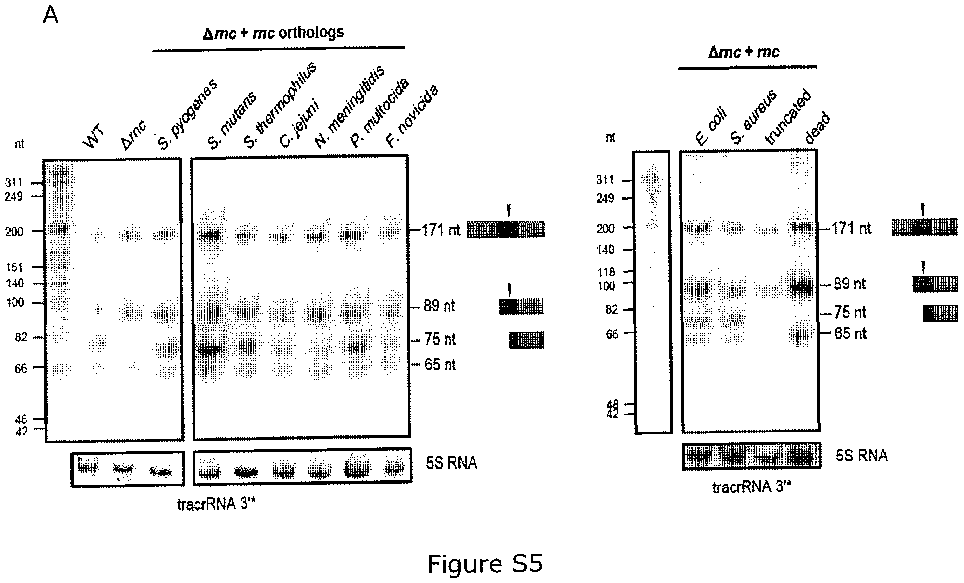

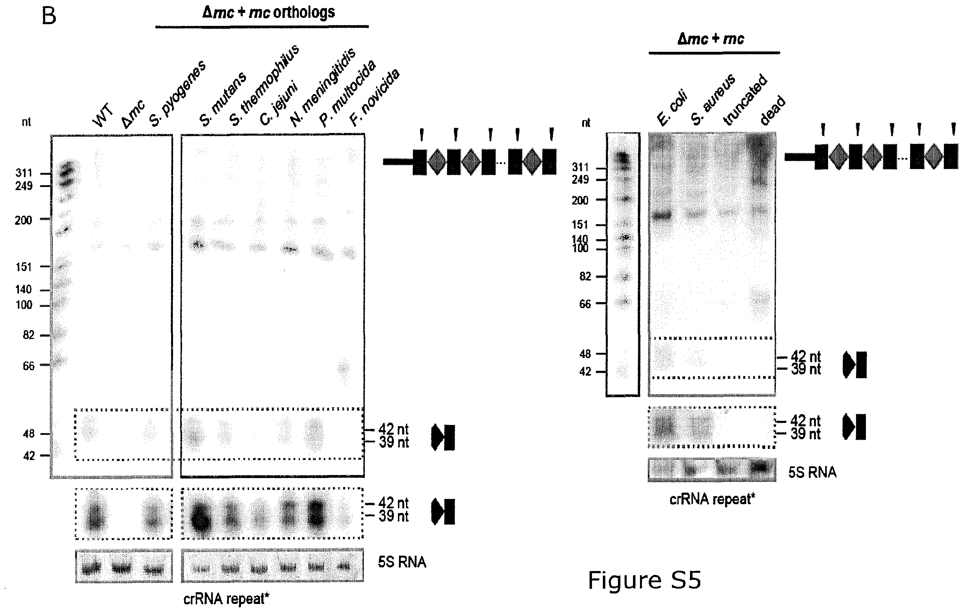

**Figure 5****.** Cas9 orthologs cleave DNA in the presence of their cognate dual-RNA and specific PAM *in vitro.* (**A**) Logo plot of protospacer adjacent sequences derived from BLAST analysis of spacer sequences for selected bacterial species. The logo plot gives graphical representation of most abundant nucleotides downstream of the protospacer sequence. The numbers in brackets correspond to the number of analyzed protospacers. (**B**) DNA substrates designed for specific PAM verification. Based on the logo plot for each species, plasmid DNA substrates were designed to contain the *speM* protospacer and the indicated sequence downstream, either comprising (PAM+) or not (PAM-) the proposed PAM. The predicted PAMs were verified by cleavage assays narrowing down the necessary nucleotides for activity (data not shown); therefore the sequence used differs slightly from the logoplot shown in (**A**). The high abundance of other nucleotides not being part of the PAM can be explained by redundancy of the coding sequences containing the protospacers, and by the limited number of found protospacer targets. The last column shows the PAM sequence for each species, which was already published (no symbol) or derived from this work (^{#}). (**C**) *In vitro* plasmid cleavage assays by dual-RNA:Cas9 orthologs on plasmid DNA with the 10 bp protospacer adjacent sequence (summarized in (**B**)). Each Cas9 ortholog in complex with its cognate dual-RNA cleaves plasmids containing the corresponding species-specific PAM (PAM+). No cleavage is observed with plasmids that did not contain the specific PAM (PAM-). li: linear cleavage product, sc: supercoiled plasmid DNA.

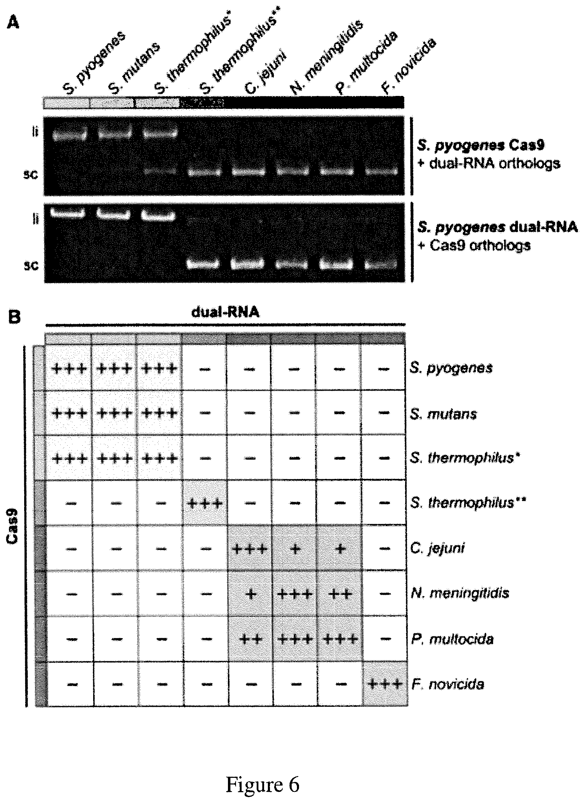

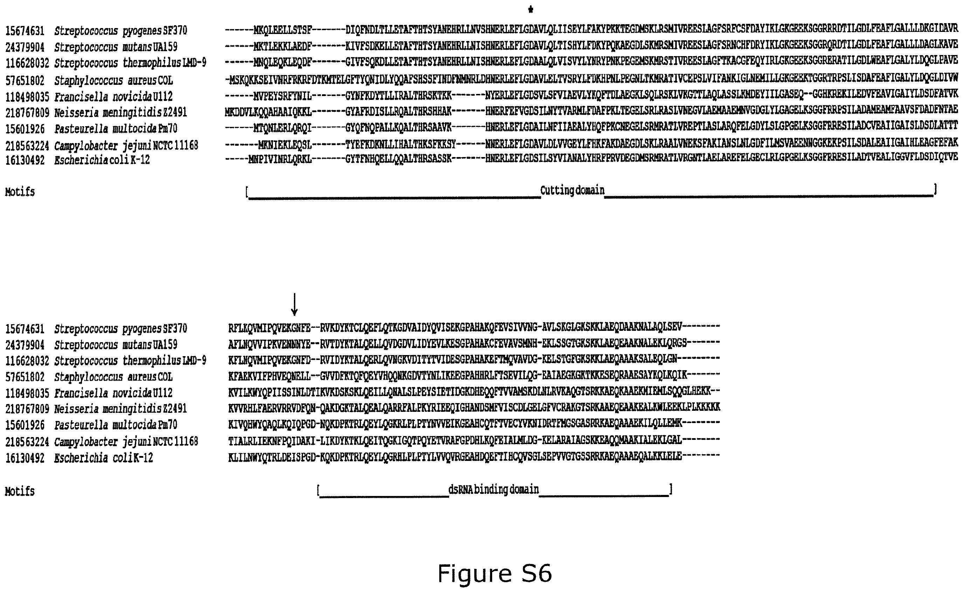

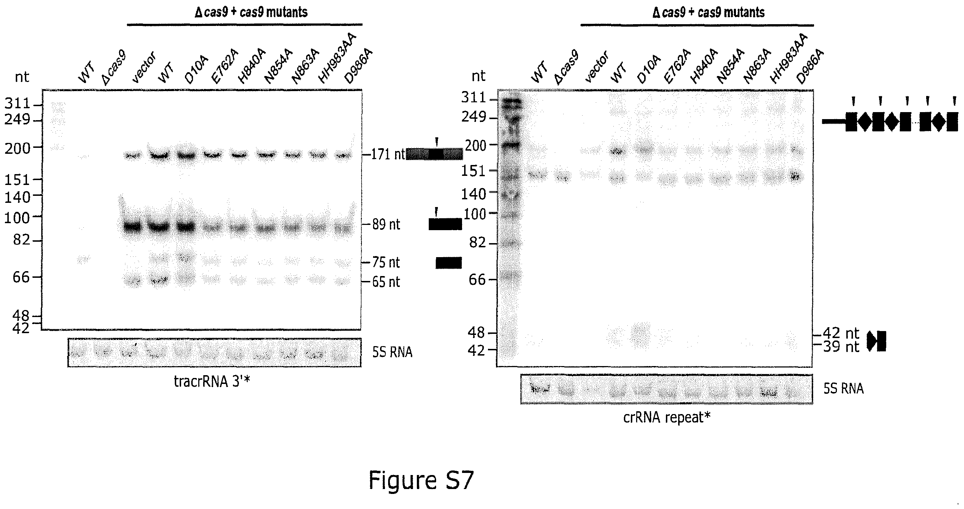

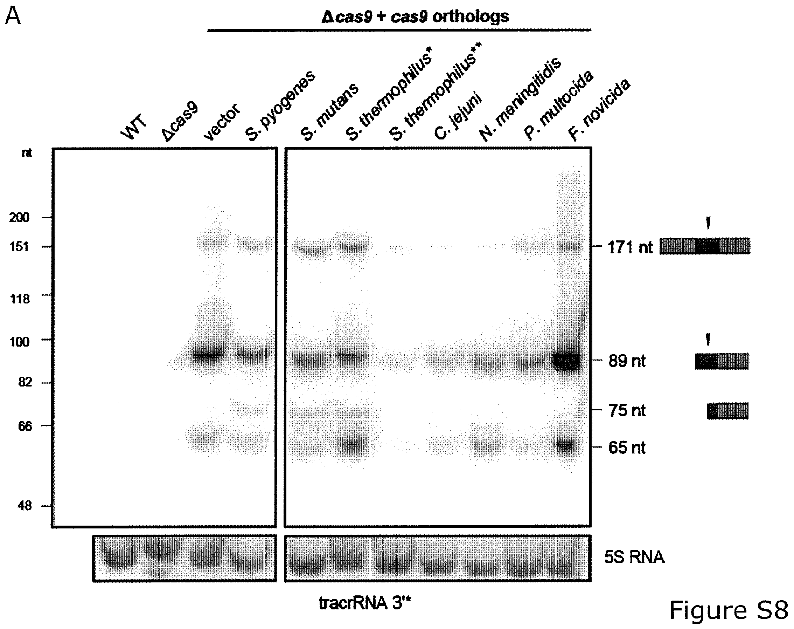

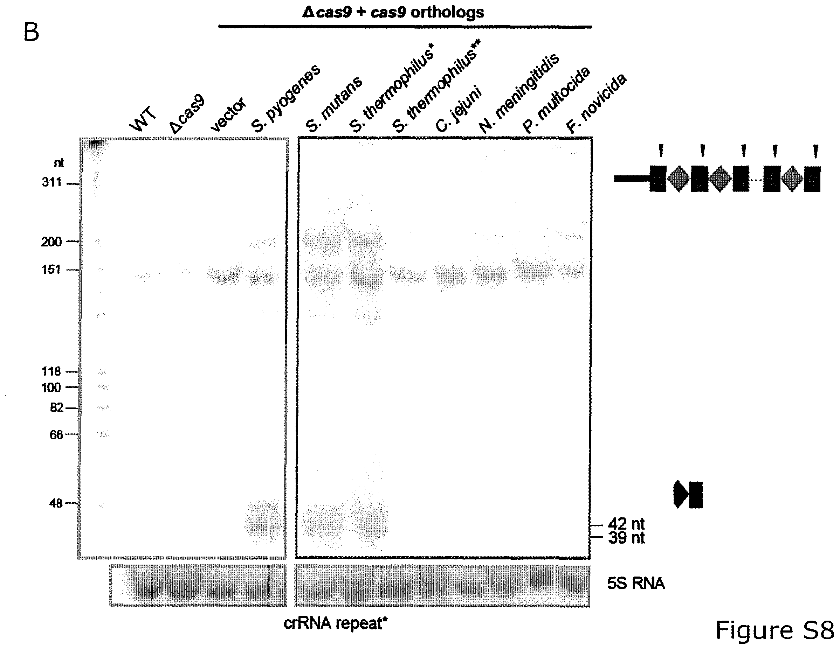

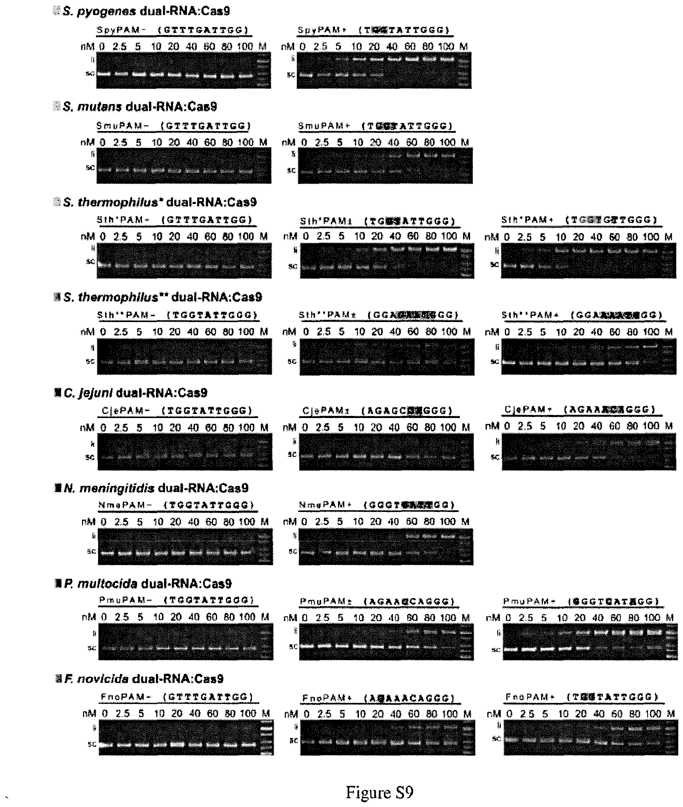

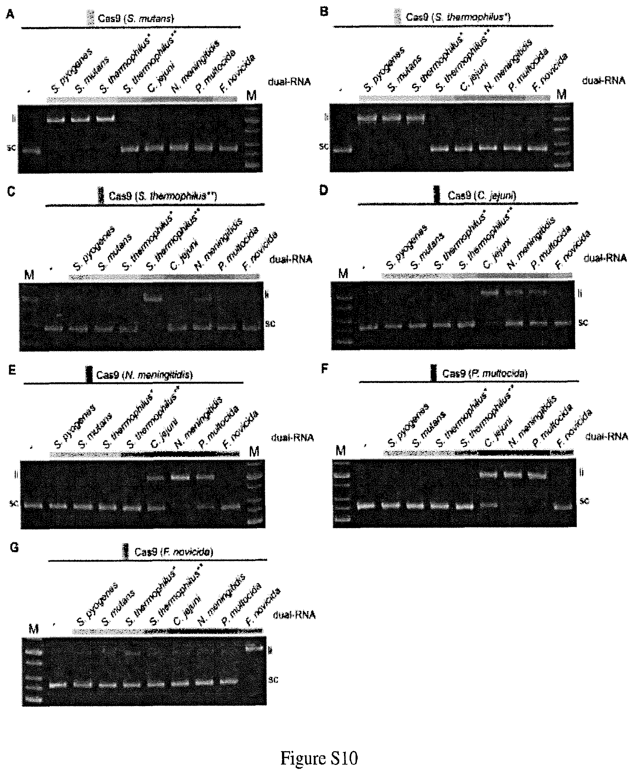

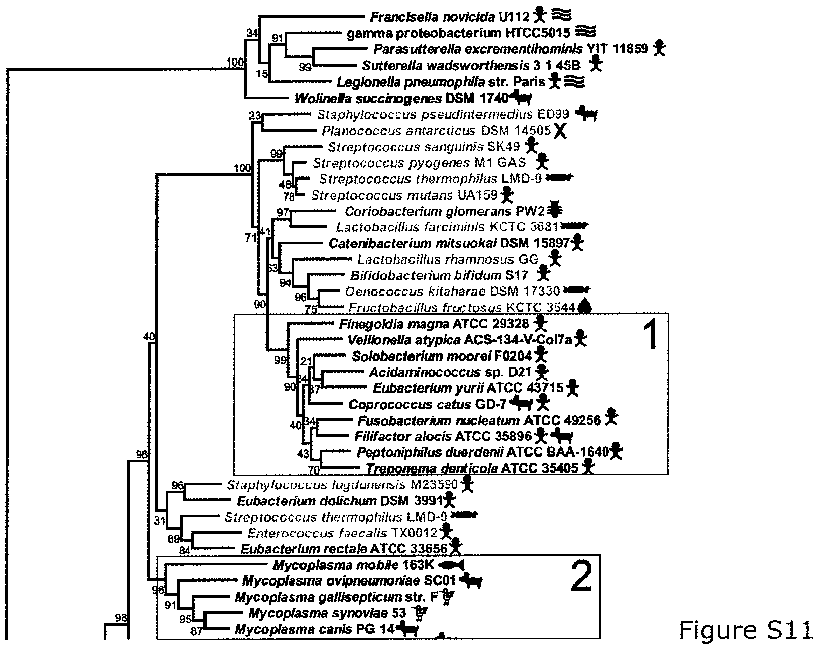

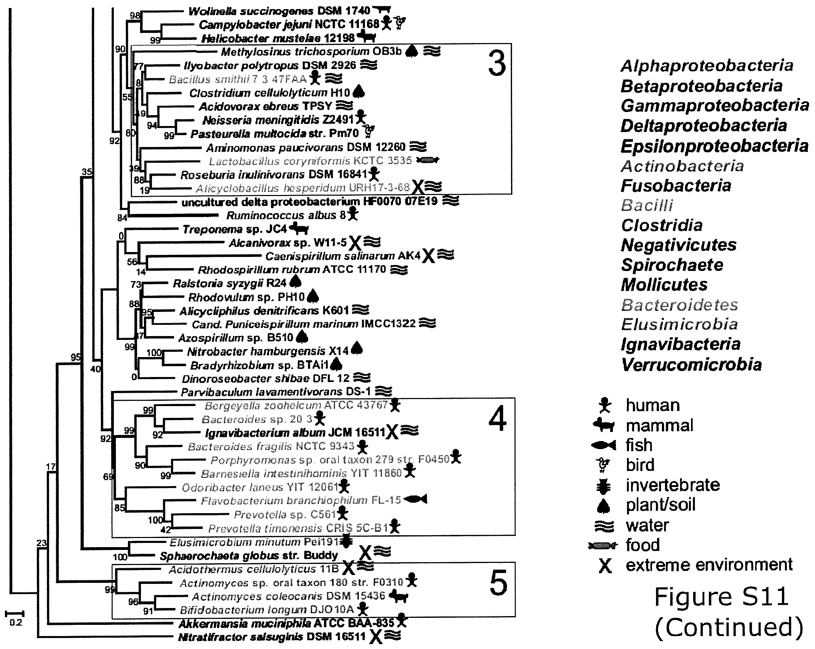

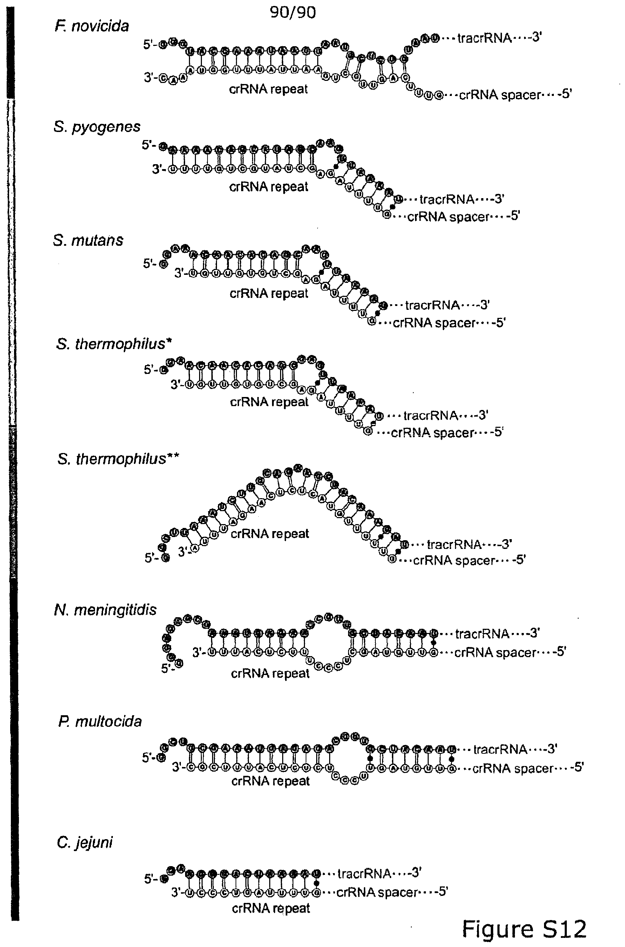

**Figure 6****.** Cas9 and dual-RNA co-evolved. (**A**) *In vitro* plasmid cleavage assays using *S. pyogenes* Cas9 in complex with orthologous dual-RNA (upper panel) and orthologous Cas9 enzymes in complex with *S*. *pyogenes* dual-RNA (lower panel). Plasmid DNA containing protospacer *speM* and *S. pyogenes* PAM (NGG) was incubated with different dual-RNAs in complex with *S. pyogenes* Cas9. tracrRNA and crRNA-repeat sequences of the dual-RNAs are from the indicated bacterial species, with crRNA spacer targeting *speM.* In the lower panel, plasmid DNA containing *speM* protospacer and the specific PAM was incubated with Cas9 orthologs in complex with *S. pyogenes* dual-RNA. *S. pyogenes* Cas9 can cleave plasmid DNA only in the presence of dual-RNA from *S*. *pyogenes, S. mutans* and *S. thermophilus** (yellow). Dual-RNA from *S. pyogenes* can mediate DNA cleavage only with Cas9 from *S. pyogenes, S. mutans* and *S. thermophilus** (yellow). li: linear cleavage product; sc: supercoiled plasmid DNA. (**B**) Summary of Cas9 and dual-RNA orthologs exchangeability. Specific PAM sequences were used according to Figure 5. The color code reflects the type II CRISPR-Cas subgroups (Figure 1). +++: 100 - 75% cleavage activity; ++: 75 - 50% cleavage activity; +: 50 - 25% cleavage activity; -: 25 - 0% cleavage activity observed under the conditions tested. Cas9 and dual-RNA duplexes from the same type II group can be interchanged and still mediate plasmid cleavage providing that the PAM sequence is specific for Cas9. See also Supplementary Figure S10.
**Supplementary Figure S1.** Biochemical characteristics and SDS-PAGE analysis of Cas9 proteins purified in this study. (A) Overview of characteristics of Cas9 orthologous proteins ^{a}Note that the biochemical characteristics of S. *pyogenes* Cas9 WT and mutants are identical; ^{b}GenInfo (GI) Identifier; ^{c}ε, Extinction coefficient. (**B**) SDS PAGE analysis of purified mutants of Cas9 from S. *pyogenes.* (**C**) SDS PAGE analysis of purified Cas9 orthologs. M: PageRulerTM Unstained Protein Ladder (Thermo Scientific).
**Supplementary Figure S2.** Multiple sequence alignment of representative Cas9 sequences (see Supplementary Table S2 and Material and Methods). The rows described as Jnet with following GI identifier of a selected Cas9 sequence provide the predicted secondary structure of Cas9 within the corresponding subgroups (sequences indicated below each Jnet). Conserved motifs are marked below the alignment and the mutated amino acid residues are highlighted. Asterisks indicate informative positions chosen for the Cas9 tree reconstruction.
**Supplementary Figure S3.** Multiple sequence alignment of representative Cas1 sequences (see Supplementary Table S2 and Materials and Methods). Informative positions chosen for the Cas1 tree reconstruction are marked with asterisks at the bottom of the alignment.
**Supplementary Figure S4.** Phylogenetic analysis of representative Cas9 and Cas1 sequences. Phylogenetic trees of Cas1 (left) and Cas9 (right) reconstructed from selected, informative positions of Cas1 and Cas9 multiple sequence alignments are shown (see Figure 1 and Supplementary Figures S2 and S3). The Cas1 tree is rooted to the outgroup of selected Cas1 orthologs of type I CRISPR-Cas systems. The Cas1 and Cas9 orthologs of the types classified as II-A, II-B and II-C are highlighted with shaded boxes. The same branch colors were used for each bacterial strain on both trees. Each protein is represented by the Genlnfo (GI) identifier followed by the bacterial strain name. The bootstrap values are given for each node (see Materials and Methods). The scale bars for the branch length are given as the estimated number of amino acid substitution per site. Note the similarity of the trees topology and monophyletic clusters of subtypes II-A and II-B on both trees supported by high bootstrap values.
**Supplementary Figure S5.** RNase III is a general executioner of tracrRNA:pre-crRNA processing in type II CRISPR-Cas. Northern blot analysis of total RNA from *S*. *pyogenes* WT, Δ*rnc* and Δ*rnc* complemented with *rnc* orthologs or *rnc* mutants probed with (**A**) tracrRNA and (**B**) crRNA repeat (Supplementary Table S1). The dashed-line boxes represented below the Northern blots in (**B**) show the area of the blots with enhanced exposure. All RNAse III orthologs can co-process *S. pyogenes* tracrRNA and pre-crRNA. No mature forms of tracrRNA and crRNAs could be observed in Δ*rnc* complemented with the truncated version or catalytically inactive (dead) mutant of RNase IIII.
**Supplementary Figure S6.** Multiple sequence alignment of bacterial endoribonucleases III used in the study. Domains indicated below the alignment are according to the domains identified in RNase III from *E. coli* (58, 59). The conserved catalytic aspartate residue mutated in the catalytically inactive "*rnc* dead" mutant and the last amino acid of the truncated *rnc* mutant are indicated above the alignment with an asterisk and an arrow, respectively.
**Supplementary Figure S7.** Conserved catalytic amino acid residues of Cas9 are not involved in dual-RNA processing by RNase III. Northern blot analysis of total RNA extracted from *S*. *pyogenes* WT, Δ*cas9* and Δ*cas9* complemented with pEC342 (backbone vector containing tracrRNA-171 nt and the native cas operon promoter from *S*. *pyogenes*) or pEC342-derived plasmids encoding Cas9 WT or mutants, hybridized with (**A**) tracrRNA or (**B**) crRNA repeat probe (Supplementary Table S1). tracrRNA:crRNA co-processing is observed in all strains encoding Cas9 point mutants. Note that in a previous study, we observed low abundance of tracrRNA in the cas9 deletion mutant (16). For this reason, plasmids used in cas9 complementation studies were designed to encode tracrRNA in addition to *cas9.*
**Supplementary Figure S8.** Cas9 and tracrRNA:crRNA co-evolved. Northern blot analysis of total RNA extracted from *S*. *pyogenes* WT, Δ*cas9* and Δ*cas9* complemented with pEC342 or pEC342-derived plasmids encoding Cas9 WT or mutants - hybridized with (**A**) tracrRNA or (**B**) crRNA repeat probe (Supplementary Table S1). Only S. *pyogenes* Cas9 WT and closely related Cas9 orthologs from *S*. *mutans* and *S*. *thermophilus** (CRISPR3) can contribute to coprocessing of *S*. *pyogenes* tracrRNA:pre-crRNA.
**Supplementary Figure S9.** Cas9 orthologs cleave plasmid DNA in the presence of their cognate dual-RNA and specific PAM. Agarose gel electrophoresis analysis of dual-RNA:Cas9 titration (0-100 nM dual-RNA-Cas9 complex) on plasmid DNA (5 nM) containing *speM* protospacer and adjacent WT PAM (PAM+), imperfect PAM (PAM±) or no PAM (PAM-). For *S*. *pyogenes, S. mutans, S. thermophilus**, *S. thermophilus*** and *N. meningitidis,* the PAM sequence has already been published (27,28,53,54). For the other bacterial species, PAMs were predicted based on the downstream sequence of protospacer identified in the investigated or related strains (see Supplementary Table S2 and Materials and Methods). The 10 bp sequence located directly downstream of the crRNA-targeted *speM* protospacer is shown. The nucleotide(s) predicted to belong to the PAM sequence are shaded in grey. li: linear cleavage product, sc: supercoiled plasmid DNA, M: 1 kb DNA ladder.
**Supplementary Figure S10.** Summary of *in vitro* plasmid cleavage assays of Cas9 orthologs in combination with dual-RNAs. Agarose gel electrophoresis of cleavage assays. (**A**) S. *mutans* Cas9 (50 nM), (**B**) *S*. *thermophilus** Cas9 (25 nM), (**C**) *S*. *thermophilus*** Cas9 (100 nM), (D) *C. jejuni* Cas9 (100 nM), (**E**) *N. meningitidis* Cas9 (100 nM), (**F**) *P. multocida* Cas9 (25 nM), (G**)** *F. novicida* Cas9 (100 nM) in complex with equimolar concentrations of each of the dual-RNA orthologs were incubated with plasmid DNA (5 nM) containing *speM* protospacer sequence and the PAM sequence specific to the Cas9 ortholog analyzed. li: linear cleavage product, sc: supercoiled plasmid DNA, M: 1 kb DNA ladder.
**Supplementary Figure S11.** Cas9 tree topology suggests both horizontal and vertical transfer of type II CRISPR-Cas systems. See Figure 1, Supplementary Figure S4 and Supplementary Table S4. The codes for taxonomy (phyla in color) and habitat (symbols) of the bacterial strains harbouring representative Cas9 orthologs are indicated (right panel). The clusters grouping evolutionary distant bacteria (1 and 3) but isolated mainly from similar sources (human for cluster 1 and mostly environmental samples for cluster 3) suggest horizontal transfer of type II systems. Clusters 2, 4 and 5 group closely related bacteria isolated from diverse habitats indicating vertical transfer of the systems.
**Supplementary Figure S12.** tracrRNA:crRNA repeat duplexes form similar secondary structures in loci with closely related Cas9 orthologs. Antirepeat sequence of processed tracrRNA (red) and repeat-derived sequence of mature crRNA (grey) were co-folded for each type II CRISPR-Cas locus studied (see Materials and Methods). Color bars indicated on the left group dual-RNAs from loci with closely related Cas9 (see Figure 1 and Supplementary Figure S4). RNA duplexes belonging to the same groups display structural similarities, suggesting a role of the structure in dual-RNA recognition by Cas9.

### Detailed Description

### Terminology

All technical and scientific terms used herein have the same meaning as commonly understood by one of ordinary skill in the art to which this invention belongs, unless the technical or scientific term is defined differently herein.

The terms "polynucleotide" and "nucleic acid," used interchangeably herein, refer to a polymeric form of nucleotides of any length, either ribonucleotides or deoxyribonucleotides. Thus, this term includes, but is not limited to, single-, double-, or multi-stranded DNA or RNA, genomic DNA, cDNA, DNA-RNA hybrids, or a polymer comprising purine and pyrimidine bases or other natural, chemically or biochemically modified, non-natural, or derivatized nucleotide bases. "Oligonucleotide" generally refers to polynucleotides of between about 5 and about 100 nucleotides of single- or double-stranded DNA. However, for the purposes of this disclosure, there is no upper limit to the length of an oligonucleotide. Oligonucleotides are also known as "oligomers" or "oligos" and may be isolated from genes, or chemically synthesized by methods known in the art. The terms "polynucleotide" and "nucleic acid" should be understood to include, as applicable to the embodiments being described, single-stranded (such as sense or antisense) and double-stranded polynucleotides.

"Genomic DNA" refers to the DNA of a genome of an organism including, but not limited to, the DNA of the genome of a bacterium, fungus, archea, plant or animal.

"Manipulating" DNA encompasses binding, nicking one strand, or cleaving (i.e., cutting) both strands of the DNA, or encompasses modifying the DNA or a polypeptide associated with the DNA (e.g., the modifications of paragraphs [00161] or [00162]). Manipulating DNA can silence, activate, or modulate (either increase or decrease) the expression of an RNA or polypeptide encoded by the DNA.

A "stem-loop structure" refers to a nucleic acid having a secondary structure that includes a region of nucleotides which are known or predicted to form a double strand (stem portion) that is linked on one side by a region of predominantly single-stranded nucleotides (loop portion). The terms "hairpin" and "fold-back" structures are also used herein to refer to stem-loop structures. Such structures are well known in the art and these terms are used consistently with their known meanings in the art. As is known in the art, a stem-loop structure does not require exact base-pairing. Thus, the stem may include one or more base mismatches. Alternatively, the base-pairing may be exact, i.e. not include any mismatches.

By "hybridizable" or "complementary" or "substantially complementary" it is meant that a nucleic acid (e.g. RNA) comprises a sequence of nucleotides that enables it to non-covalently bind, i.e. form Watson-Crick base pairs and/or G/U base pairs, "anneal", or "hybridize," to another nucleic acid in a sequence-specific, antiparallel, manner (i.e., a nucleic acid specifically binds to a complementary nucleic acid) under the appropriate in vitro and/or in vivo conditions of temperature and solution ionic strength. As is known in the art, standard Watson-Crick base-pairing includes: adenine (A) pairing with thymidine (T), adenine (A) pairing with uracil (U), and guanine (G) pairing with cytosine (C) [DNA, RNA]. In addition, it is also known in the art that for hybridization between two RNA molecules (e.g., dsRNA), guanine (G) base pairs with uracil (U). For example, G/U base-pairing is partially responsible for the degeneracy (i.e., redundancy) of the genetic code in the context of tRNA anti-codon base-pairing with codons in mRNA. In the context of this disclosure, a guanine (G) of a protein-binding segment (dsRNA duplex) of a guide RNA molecule is considered complementary to a uracil (U), and vice versa. As such, when a G/U base-pair can be made at a given nucleotide position a protein-binding segment (dsRNA duplex) of a guide RNA molecule, the position is not considered to be non-complementary, but is instead considered to be complementary.

Hybridization and washing conditions are well known and exemplified in Sambrook, J., Fritsch, E. F. and Maniatis, T. Molecular Cloning: A Laboratory Manual, Second Edition, Cold Spring Harbor Laboratory Press, Cold Spring Harbor (1989), particularly Chapter 11 and Table 11.1 therein; and Sambrook, J. and Russell, W., Molecular Cloning: A Laboratory Manual, Third Edition, Cold Spring Harbor Laboratory Press, Cold Spring Harbor (2001). The conditions of temperature and ionic strength determine the "stringency" of the hybridization.

Hybridization requires that the two nucleic acids contain complementary sequences, although mismatches between bases are possible. The conditions appropriate for hybridization between two nucleic acids depend on the length of the nucleic acids and the degree of complementation, variables well known in the art. The greater the degree of complementation between two nucleotide sequences, the greater the value of the melting temperature (Tm) for hybrids of nucleic acids having those sequences. For hybridizations between nucleic acids with short stretches of complementarity (e.g. complementarity over 35 or less, 30 or less, 25 or less, 22 or less, 20 or less, or 18 or less nucleotides) the position of mismatches becomes important (see Sambrook et al., supra, 11.7-11.8). Typically, the length for a hybridizable nucleic acid is at least about 10 nucleotides. Illustrative minimum lengths for a hybridizable nucleic acid are: at least about 15 nucleotides; at least about 20 nucleotides; at least about 22 nucleotides; at least about 25 nucleotides; and at least about 30 nucleotides). Furthermore, the skilled artisan will recognize that the temperature and wash solution salt concentration may be adjusted as necessary according to factors such as length of the region of complementation and the degree of complementation.

It is understood in the art that the sequence of polynucleotide need not be 100% complementary to that of its target nucleic acid to be specifically hybridizable or hybridizable. Moreover, a polynucleotide may hybridize over one or more segments such that intervening or adjacent segments are not involved in the hybridization event (e.g., a loop structure or hairpin structure). A polynucleotide can comprise at least 70%, at least 80%, at least 90%, at least 95%, at least 99%, or 100% sequence complementarity to a target region within the target nucleic acid sequence to which they are targeted. For example, an antisense nucleic acid in which 18 of 20 nucleotides of the antisense compound are complementary to a target region, and would therefore specifically hybridize, would represent 90 percent complementarity. In this example, the remaining noncomplementary nucleotides may be clustered or interspersed with complementary nucleotides and need not be contiguous to each other or to complementary nucleotides. Percent complementarity between particular stretches of nucleic acid sequences within nucleic acids can be determined routinely using BLAST programs (basic local alignment search tools) and PowerBLAST programs known in the art (Altschul et al., J. Mol. Biol., 1990, 215, 403-410; Zhang and Madden, Genome Res., 1997, 7, 649-656) or by using the Gap program (Wisconsin Sequence Analysis Package, Version 8 for Unix, Genetics Computer Group, University Research Park, Madison Wis.), using default settings, which uses the algorithm of Smith and Waterman (Adv. Appl. Math., 1981, 2, 482-489).

The terms "peptide," "polypeptide," and "protein" are used interchangeably herein, and refer to a polymeric form of amino acids of any length, which can include coded and non-coded amino acids, chemically or biochemically modified or derivatized amino acids, and polypeptides having modified peptide backbones.

"Binding" as used herein (e.g. with reference to an RNA-binding domain of a polypeptide) refers to a non-covalent interaction between macromolecules (e.g., between a protein and a nucleic acid). While in a state of non-covalent interaction, the macromolecules are said to be "associated" or "interacting" or "binding" (e.g., when a molecule X is said to interact with a molecule Y, it is meant the molecule X binds to molecule Y in a non-covalent manner). Not all components of a binding interaction need be sequence-specific (e.g., contacts with phosphate residues in a DNA backbone), but some portions of a binding interaction may be sequence-specific. Binding interactions are generally characterized by a dissociation constant (*K*_{d}) of less than 10⁻⁶ M, less than 10⁻⁷ M, less than 10⁻⁸ M, less than 10⁻⁹ M, less than 10⁻¹⁰ M, less than 10⁻¹¹ M, less than 10⁻¹² M, less than 10⁻¹³ M, less than 10⁻¹⁴ M, or less than 10⁻¹⁵ M. "Affinity" refers to the strength of binding, increased binding affinity being correlated with a lower *K*_{d}. By "binding domain" it is meant a protein domain that is able to bind non-covalently to another molecule. A binding domain can bind to, for example, a DNA molecule (a DNA-binding protein), an RNA molecule (an RNA-binding protein) and/or a protein molecule (a protein-binding protein). In the case of a protein domain-binding protein, it can bind to itself (to form homodimers, homotrimers, etc.) and/or it can bind to one or more molecules of a different protein or proteins.

The term "conservative amino acid substitution" refers to the interchangeability in proteins of amino acid residues having similar side chains. For example, a group of amino acids having aliphatic side chains consists of glycine, alanine, valine, leucine, and isoleucine; a group of amino acids having aliphatic-hydroxyl side chains consists of serine and threonine; a group of amino acids having amide containing side chains consisting of asparagine and glutamine; a group of amino acids having aromatic side chains consists of phenylalanine, tyrosine, and tryptophan; a group of amino acids having basic side chains consists of lysine, arginine, and histidine; a group of amino acids having acidic side chains consists of glutamate and aspartate; and a group of amino acids having sulfur containing side chains consists of cysteine and methionine. Exemplary conservative amino acid substitution groups are: valine-leucine-isoleucine, phenylalanine-tyrosine, lysine-arginine, alanine-valine, and asparagine-glutamine.

A polynucleotide or polypeptide has a certain percent "sequence identity" to another polynucleotide or polypeptide, meaning that, when aligned, that percentage of bases or amino acids are the same, and in the same relative position, when comparing the two sequences. Sequence identity can be determined in a number of different manners. To determine sequence identity, sequences can be aligned using various methods and computer programs (e.g., BLAST, T-COFFEE, MUSCLE, MAFFT, etc.), available over the world wide web at sites including ncbi.nlm.nili.gov/BLAST, ebi.ac.uk/Tools/msa/tcoffee/, ebi.ac.uk/Tools/msa/muscle/, mafft.cbrc.jp/alignment/software/. See, e.g., Altschul et al. (1990), J. Mol. Bioi. 215:403-10. Sequence alignments standard in the art are used according to the invention to determine amino acid residues in a Cas9 ortholog that "correspond to" amino acid residues in another Cas9 ortholog. The amino acid residues of Cas9 orthologs that correspond to amino acid residues of other Cas9 orthologs appear at the same position in alignments of the sequences.

A DNA sequence that "encodes" a particular RNA is a DNA nucleic acid sequence that is transcribed into RNA. A DNA polynucleotide may encode an RNA (mRNA) that is translated into protein, or a DNA polynucleotide may encode an RNA that is not translated into protein (e.g. tRNA, rRNA, or a guide RNA; also called "non-coding" RNA or "ncRNA"). A "protein coding sequence" or a sequence that encodes a particular protein or polypeptide, is a nucleic acid sequence that is transcribed into mRNA (in the case of DNA) and is translated (in the case of mRNA) into a polypeptide in vitro or in vivo when placed under the control of appropriate regulatory sequences. The boundaries of the coding sequence are determined by a start codon at the 5' terminus (N-terminus) and a translation stop nonsense codon at the 3' terminus (C-terminus). A coding sequence can include, but is not limited to, cDNA from prokaryotic or eukaryotic mRNA, genomic DNA sequences from prokaryotic or eukaryotic DNA, and synthetic nucleic acids. A transcription termination sequence will usually be located 3' to the coding sequence.

As used herein, a "promoter sequence" is a DNA regulatory region capable of binding RNA polymerase and initiating transcription of a downstream (3' direction) coding or non-coding sequence. For purposes of defining the present invention, the promoter sequence is bounded at its 3' terminus by the transcription initiation site and extends upstream (5' direction) to include the minimum number of bases or elements necessary to initiate transcription at levels detectable above background. Within the promoter sequence will be found a transcription initiation site, as well as protein binding domains responsible for the binding of RNA polymerase. Eukaryotic promoters will often, but not always, contain "TATA" boxes and "CAT" boxes. Various promoters, including inducible promoters, may be used to drive the various vectors of the present invention.

A promoter can be a constitutively active promoter (i.e., a promoter that is constitutively in an active/"ON" state), it may be an inducible promoter (i.e., a promoter whose state, active/"ON" or inactive/"OFF", is controlled by an external stimulus, e.g., the presence of a particular temperature, compound, or protein.), it may be a spatially restricted promoter (i.e., transcriptional control element, enhancer, etc.)(e.g., tissue specific promoter, cell type specific promoter, etc.), and it may be a temporally restricted promoter (i.e., the promoter is in the "ON" state or "OFF" state during specific stages of embryonic development or during specific stages of a biological process, e.g., hair follicle cycle in mice).

Suitable promoters can be derived from viruses and can therefore be referred to as viral promoters, or they can be derived from any organism, including prokaryotic or eukaryotic organisms. Suitable promoters can be used to drive expression by any RNA polymerase (e.g., pol I, pol II, pol III). Exemplary promoters include, but are not limited to the SV40 early promoter, mouse mammary tumor virus long terminal repeat (LTR) promoter; adenovirus major late promoter (Ad MLP); a herpes simplex virus (HSV) promoter, a cytomegalovirus (CMV) promoter such as the CMV immediate early promoter region (CMVIE), a rous sarcoma virus (RSV) promoter, a human U6 small nuclear promoter (U6) (Miyagishi et al. , Nature Biotechnology 20, 497 - 500 (2002)), an enhanced U6 promoter (e.g., Xia et al., Nucleic Acids Res. 2003 Sep 1;31(17)), a human H1 promoter (H1), and the like.

Examples of inducible promoters include, but are not limited toT7 RNA polymerase promoter, T3 RNA polymerase promoter, Isopropyl-beta-D-thiogalactopyranoside (IPTG)-regulated promoter, lactose induced promoter, heat shock promoter, Tetracycline-regulated promoter, Steroid-regulated promoter, Metal-regulated promoter, estrogen receptor-regulated promoter, etc. Inducible promoters can therefore be regulated by molecules including, but not limited to, doxycycline; RNA polymerase, e.g., T7 RNA polymerase; an estrogen receptor; an estrogen receptor fusion; etc.

In some embodiments, the promoter is a spatially restricted promoter (i.e., cell type specific promoter, tissue specific promoter, etc.) such that in a multi-cellular organism, the promoter is active (i.e., "ON") in a subset of specific cells. Spatially restricted promoters may also be referred to as enhancers, transcriptional control elements, control sequences, etc. Any convenient spatially restricted promoter may be used and the choice of suitable promoter (e.g., a brain specific promoter, a promoter that drives expression in a subset of neurons, a promoter that drives expression in the germline, a promoter that drives expression in the lungs, a promoter that drives expression in muscles, a promoter that drives expression in islet cells of the pancreas, etc.) will depend on the organism. For example, various spatially restricted promoters are known for plants, flies, worms, mammals, mice, etc. Thus, a spatially restricted promoter can be used to regulate the expression of a nucleic acid encoding a site-directed modifying polypeptide in a wide variety of different tissues and cell types, depending on the organism. Some spatially restricted promoters are also temporally restricted such that the promoter is in the "ON" state or "OFF" state during specific stages of embryonic development or during specific stages of a biological process (e.g., hair follicle cycle in mice).

For illustration purposes, examples of spatially restricted promoters include, but are not limited to, neuron-specific promoters, adipocyte-specific promoters, cardiomyocyte-specific promoters, smooth muscle-specific promoters, photoreceptor-specific promoters, etc. Neuron-specific spatially restricted promoters include, but are not limited to, a neuron-specific enolase (NSE) promoter (see, e.g., EMBL HSENO2, X51956); an aromatic amino acid decarboxylase (AADC) promoter; a neurofilament promoter (see, e.g., GenBank HUMNFL, L04147); a synapsin promoter (see, e.g., GenBank HUMSYNIB, M55301); a thy-1 promoter (see, e.g., Chen et al. (1987) Cell 51:7-19; and Llewellyn, et al. (2010) Nat. Med. 16(10):1161-1166); a serotonin receptor promoter (see, e.g., GenBank S62283); a tyrosine hydroxylase promoter (TH) (see, e.g., Oh et al. (2009) Gene Ther 16:437; Sasaoka et al. (1992) Mol. Brain Res. 16:274; Boundy et al. (1998) J. Neurosci. 18:9989; and Kaneda et al. (1991) Neuron 6:583-594); a GnRH promoter (see, e.g., Radovick et al. (1991) Proc. Natl. Acad. Sci. USA 88:3402-3406); an L7 promoter (see, e.g., Oberdick et al. (1990) Science 248:223-226); a DNMT promoter (see, e.g., Bartge et al. (1988) Proc. Natl. Acad. Sci. USA 85:3648-3652); an enkephalin promoter (see, e.g., Comb et al. (1988) EMBO J. 17:3793-3805); a myelin basic protein (MBP) promoter; a Ca2+-calmodulin-dependent protein kinase II-alpha (CamKIM) promoter (see, e.g., Mayford et al. (1996) Proc. Natl. Acad. Sci. USA 93:13250; and Casanova et al. (2001) Genesis 31:37); a CMV enhancer/platelet-derived growth factor-p promoter (see, e.g., Liu et al. (2004) Gene Therapy 11:52-60); and the like.

Adipocyte-specific spatially restricted promoters include, but are not limited to aP2 gene promoter/enhancer, e.g., a region from -5.4 kb to +21 bp of a human aP2 gene (see, e.g., Tozzo et al. (1997) Endocrinol. 138:1604; Ross et al. (1990) Proc. Natl. Acad. Sci. USA 87:9590; and Pavjani et al. (2005) Nat. Med. 11:797); a glucose transporter-4 (GLUT4) promoter (see, e.g., Knight et al. (2003) Proc. Natl. Acad. Sci. USA 100:14725); a fatty acid translocase (FAT/CD36) promoter (see, e.g., Kuriki et al. (2002) Biol. Pharm. Bull. 25:1476; and Sato et al. (2002) J. Biol. Chem. 277:15703); a stearoyl-CoA desaturase-1 (SCD1) promoter (Tabor et al. (1999) J. Biol. Chem. 274:20603); a leptin promoter (see, e.g., Mason et al. (1998) Endocrinol. 139:1013; and Chen et al. (1999) Biochem. Biophys. Res. Comm. 262:187); an adiponectin promoter (see, e.g., Kita et al. (2005) Biochem. Biophys. Res. Comm. 331:484; and Chakrabarti (2010) Endocrinol. 151:2408); an adipsin promoter (see, e.g., Platt et al. (1989) Proc. Natl. Acad. Sci. USA 86:7490); a resistin promoter (see, e.g., Seo et al. (2003) Molec. Endocrinol. 17:1522); and the like.

Cardiomyocyte-specific spatially restricted promoters include, but are not limited to control sequences derived from the following genes: myosin light chain-2, a-myosin heavy chain, AE3, cardiac troponin C, cardiac actin, and the like. Franz et al. (1997) Cardiovasc. Res. 35:560-566; Robbins et al. (1995) Ann. N.Y. Acad. Sci. 752:492-505; Linn et al. (1995) Circ. Res. 76:584591; Parmacek et al. (1994) Mol. Cell. Biol. 14:1870-1885; Hunter et al. (1993) Hypertension 22:608-617; and Sartorelli et al. (1992) Proc. Natl. Acad. Sci. USA 89:4047-4051.

Smooth muscle-specific spatially restricted promoters include, but are not limited to an SM22a promoter (see, e.g., Akyiirek et al. (2000) Mol. Med. 6:983; and U.S. Patent No. 7,169,874); a smoothelin promoter (see, e.g., WO 2001/018048); an a-smooth muscle actin promoter; and the like. For example, a 0.4 kb region of the SM22a promoter, within which lie two CArG elements, has been shown to mediate vascular smooth muscle cell-specific expression (see, e.g., Kim, et al. (1997) Mol. Cell. Biol. 17, 2266-2278; Li, et al., (1996) J. Cell Biol. 132, 849-859; and Moessler, et al. (1996) Development 122, 2415-2425).

Photoreceptor-specific spatially restricted promoters include, but are not limited to, a rhodopsin promoter; a rhodopsin kinase promoter (Young et al. (2003) Ophthalmol. Vis. Sci. 44:4076); a beta phosphodiesterase gene promoter (Nicoud et al. (2007) J. Gene Med. 9:1015); a retinitis pigmentosa gene promoter (Nicoud et al. (2007) supra); an interphotoreceptor retinoid-binding protein (IRBP) gene enhancer (Nicoud et al. (2007) supra); an IRBP gene promoter (Yokoyama et al. (1992) Exp Eye Res. 55:225); and the like.

The terms "DNA regulatory sequences," "control elements," and "regulatory elements," used interchangeably herein, refer to transcriptional and translational control sequences, such as promoters, enhancers, polyadenylation signals, terminators, protein degradation signals, and the like, that provide for and/or regulate transcription of a non-coding sequence (e.g., guide RNA) or a coding sequence (e.g., site-directed modifying polypeptide, or Cas9 polypeptide) and/or regulate translation of an encoded polypeptide.

IThe term "naturally-occurring" or "unmodified" as used herein as applied to a nucleic acid, a polypeptide, a cell, or an organism, refers to a nucleic acid, polypeptide, cell, or organism that is found in nature. For example, a polypeptide or polynucleotide sequence that is present in an organism (including viruses) that can be isolated from a source in nature and which has not been intentionally modified by a human in the laboratory is naturally occurring.

The term "chimeric" as used herein as applied to a nucleic acid or polypeptide refers to two components that are defined by structures derived from different sources. For example, where "chimeric" is used in the context of a chimeric polypeptide (e.g., a chimeric Cas9 protein), the chimeric polypeptide includes amino acid sequences that are derived from different polypeptides. A chimeric polypeptide may comprise either modified or naturally-occurring polypeptide sequences (e.g., a first amino acid sequence from a modified or unmodified Cas9 protein; and a second amino acid sequence other than the Cas9 protein). Similarly, "chimeric" in the context of a polynucleotide encoding a chimeric polypeptide includes nucleotide sequences derived from different coding regions (e.g., a first nucleotide sequence encoding a modified or unmodified Cas9 protein; and a second nucleotide sequence encoding a polypeptide other than a Cas9 protein).

The term "chimeric polypeptide" refers to a polypeptide which is not naturally occurring, e.g., is made by the artificial combination (i.e., "fusion") of two otherwise separated segments of amino sequence through human intervention. A polypeptide that comprises a chimeric amino acid sequence is a chimeric polypeptide. Some chimeric polypeptides can be referred to as "fusion variants."

"Heterologous," as used herein, means a nucleotide or peptide that is not found in the native nucleic acid or protein, respectively. For example, in a chimeric Cas9 protein, the RNA-binding domain of a naturally-occurring bacterial Cas9 polypeptide (or a variant thereof) may be fused to a heterologous polypeptide sequence (i.e. a polypeptide sequence from a protein other than Cas9 or a polypeptide sequence from another organism). The heterologous polypeptide may exhibit an activity (e.g., enzymatic activity) that will also be exhibited by the chimeric Cas9 protein (e.g., methyltransferase activity, acetyltransferase activity, kinase activity, ubiquitinating activity, etc.). A heterologous nucleic acid may be linked to a naturally-occurring nucleic acid (or a variant thereof) (e.g., by genetic engineering) to generate a chimeric polynucleotide encoding a chimeric polypeptide. As another example, in a fusion variant Cas9 site-directed polypeptide, a variant Cas9 site-directed polypeptide may be fused to a heterologous polypeptide (i.e. a polypeptide other than Cas9), which exhibits an activity that will also be exhibited by the fusion variant Cas9 site-directed polypeptide. A heterologous nucleic acid may be linked to a variant Cas9 site-directed polypeptide (e.g., by genetic engineering) to generate a polynucleotide encoding a fusion variant Cas9 site-directed polypeptide. "Heterologous," as used herein, additionally means a nucleotide or polypeptide in a cell that is not its native cell.

The term "cognate" refers to two biomolecules that normally interact or co-exist in nature.

"Recombinant," as used herein, means that a particular nucleic acid (DNA or RNA) or vector is the product of various combinations of cloning, restriction, polymerase chain reaction (PCR) and/or ligation steps resulting in a construct having a structural coding or non-coding sequence distinguishable from endogenous nucleic acids found in natural systems. DNA sequences encoding polypeptides can be assembled from cDNA fragments or from a series of synthetic oligonucleotides, to provide a synthetic nucleic acid which is capable of being expressed from a recombinant transcriptional unit contained in a cell or in a cell-free transcription and translation system. Genomic DNA comprising the relevant sequences can also be used in the formation of a recombinant gene or transcriptional unit. Sequences of non-translated DNA may be present 5' or 3' from the open reading frame, where such sequences do not interfere with manipulation or expression of the coding regions, and may indeed act to modulate production of a desired product by various mechanisms (see "DNA regulatory sequences", below). Alternatively, DNA sequences encoding RNA (e.g., guide RNA) that is not translated may also be considered recombinant. Thus, e.g., the term "recombinant" nucleic acid refers to one which is not naturally occurring, e.g., is made by the artificial combination of two otherwise separated segments of sequence through human intervention. This artificial combination is often accomplished by either chemical synthesis means, or by the artificial manipulation of isolated segments of nucleic acids, e.g., by genetic engineering techniques. Such is usually done to replace a codon with a codon encoding the same amino acid, a conservative amino acid, or a non-conservative amino acid. Alternatively, it is performed to join together nucleic acid segments of desired functions to generate a desired combination of functions. This artificial combination is often accomplished by either chemical synthesis means, or by the artificial manipulation of isolated segments of nucleic acids, e.g., by genetic engineering techniques. When a recombinant polynucleotide encodes a polypeptide, the sequence of the encoded polypeptide can be naturally occurring ("wild type") or can be a variant (e.g., a mutant) of the naturally occurring sequence. Thus, the term "recombinant" polypeptide does not necessarily refer to a polypeptide whose sequence does not naturally occur. Instead, a "recombinant" polypeptide is encoded by a recombinant DNA sequence, but the sequence of the polypeptide can be naturally occurring ("wild type") or non-naturally occurring (e.g., a variant, a mutant, etc.). Thus, a "recombinant" polypeptide is the result of human intervention, but may be a naturally occurring amino acid sequence.

A "vector" or "expression vector" is a replicon, such as plasmid, phage, virus, or cosmid, to which another DNA segment, i.e. an "insert", may be attached so as to bring about the replication of the attached segment in a cell.

An "expression cassette" comprises a DNA coding sequence operably linked to a promoter. "Operably linked" refers to a juxtaposition wherein the components so described are in a relationship permitting them to function in their intended manner. For instance, a promoter is operably linked to a coding sequence if the promoter affects its transcription or expression. The terms "recombinant expression vector," or "DNA construct" are used interchangeably herein to refer to a DNA molecule comprising a vector and at least one insert. Recombinant expression vectors are usually generated for the purpose of expressing and/or propagating the insert(s), or for the construction of other recombinant nucleotide sequences. The nucleic acid(s) may or may not be operably linked to a promoter sequence and may or may not be operably linked to DNA regulatory sequences.

A cell has been "genetically modified" or "transformed" or "transfected" by exogenous DNA, e.g. a recombinant expression vector, when such DNA has been introduced inside the cell. The presence of the exogenous DNA results in permanent or transient genetic change. The transforming DNA may or may not be integrated (covalently linked) into the genome of the cell.

In prokaryotes, yeast, and mammalian cells for example, the transforming DNA may be maintained on an episomal element such as a plasmid. With respect to eukaryotic cells, a stably transformed cell is one in which the transforming DNA has become integrated into a chromosome so that it is inherited by daughter cells through chromosome replication. This stability is demonstrated by the ability of the eukaryotic cell to establish cell lines or clones that comprise a population of daughter cells containing the transforming DNA. A "clone" is a population of cells derived from a single cell or common ancestor by mitosis. A "cell line" is a clone of a primary cell that is capable of stable growth *in vitro* for many generations.

Suitable methods of genetic modification (also referred to as "transformation") include e.g., viral or bacteriophage infection, transfection, conjugation, protoplast fusion, lipofection, electroporation, calcium phosphate precipitation, polyethyleneimine (PEI)-mediated transfection, DEAE-dextran mediated transfection, liposome-mediated transfection, particle gun technology, calcium phosphate precipitation, direct micro injection, nanoparticle-mediated nucleic acid delivery (see, e.g., Panyam et., al Adv Drug Deliv Rev. 2012 Sep 13. pii: S0169-409X(12)00283-9. doi: 10.1016/j.addr.2012.09.023), and the like.

The choice of method of genetic modification is generally dependent on the type of cell being transformed and the circumstances under which the transformation is taking place (e.g., in vitro, ex vivo, or in vivo). A general discussion of these methods can be found in Ausubel, et al., Short Protocols in Molecular Biology, 3rd ed., Wiley & Sons, 1995.

A "host cell," as used herein, denotes an in vivo or in vitro eukaryotic cell, a prokaryotic cell (e.g., bacterial or archaeal cell), or a cell from a multicellular organism (e.g., a cell line) cultured as a unicellular entity, which eukaryotic or prokaryotic cells can be, or have been, used as recipients for a nucleic acid, and include the progeny of the original cell which has been transformed by the nucleic acid. It is understood that the progeny of a single cell may not necessarily be completely identical in morphology or in genomic or total DNA complement as the original parent, due to natural, accidental, or deliberate mutation. A "recombinant host cell" (also referred to as a "genetically modified host cell") is a host cell into which has been introduced a heterologous nucleic acid, e.g., an expression vector. For example, a bacterial host cell is a genetically modified bacterial host cell by virtue of introduction into a suitable bacterial host cell of an exogenous nucleic acid (e.g., a plasmid or recombinant expression vector) and a eukaryotic host cell is a genetically modified eukaryotic host cell (e.g., a mammalian germ cell), by virtue of introduction into a suitable eukaryotic host cell of an exogenous nucleic acid.

A "target DNA" as used herein is a DNA polynucleotide that comprises a "target site" or "target sequence." The terms "target site," "target sequence," "target protospacer DNA, "or" protospacer-like sequence" are used interchangeably herein to refer to a nucleic acid sequence present in a target DNA to which a DNA-targeting segment of a guide RNA will bind, provided sufficient conditions for binding exist. For example, the target site (or target sequence) 5'-GAGCATATC-3' within a target DNA is targeted by (or is bound by, or hybridizes with, or is complementary to) the RNA sequence 5'-GAUAUGCUC-3'. Suitable DNA/RNA binding conditions include physiological conditions normally present in a cell. Other suitable DNA/RNA binding conditions (e.g., conditions in a cell-free system) are known in the art; see, e.g., Sambrook, supra. The strand of the target DNA that is complementary to and hybridizes with the guide RNA is referred to as the "complementary strand" and the strand of the target DNA that is complementary to the "complementary strand" (and is therefore not complementary to the guide RNA) is referred to as the "noncomplementary strand" or "non-complementary strand." By "site-directed modifying polypeptide" or "RNA-binding site-directed polypeptide" or "RNA-binding site-directed modifying polypeptide" or "site-directed polypeptide" it is meant a polypeptide that binds RNA and is targeted to a specific DNA sequence. A site-directed modifying polypeptide as described herein is targeted to a specific DNA sequence by the RNA molecule to which it is bound. The RNA molecule comprises a sequence that binds, hybridizes to, or is complementary to a target sequence within the target DNA, thus targeting the bound polypeptide to a specific location within the target DNA (the target sequence). Exemplary target sequences of the invention are set out in SEQ ID NOs: 801-2701. SEQ ID NOs: 801-973 are protospacer-like target sequences 5' to the PAM sequence NNNNACA in the human CCR5 gene. SEQ ID NOs: 974-1078 are protospacer-like sequences 5' to the PAM sequence GNNNCNNA in the human CCR5 gene. SEQ ID NOs: 1079-1222 are protospacer-like target sequences 5' to the PAM sequence NNNNACA in the exons of the human CCR5 gene. SEQ ID NOs: 1223-1312 are protospacer-like sequences 5' to the PAM sequence GNNNCNNA in the exons of the human CCR5 gene. SEQ ID NOs: 1313-1348 are protospacer-like target sequences 5' to the PAM sequence NNNNACA around the 5' end of the human CCR5 gene. SEQ ID NOs: 1349-1371 are protospacer-like sequences 5' to the PAM sequence GNNNCNNA around the 5' end of the human CCR5 gene. SEQ ID NOs: 1372-1415 are protospacer-like target sequences 5' to the PAM sequence NNNNACA around the delta 32 locus in the human CCR5 gene. SEQ ID NOs: 1416-1443 are protospacer-like sequences 5' to the PAM sequence GNNNCNNA around the delta 32 locus in the human CCR5 gene. SEQ ID NOs: 1444-1900 are protospacer-like target sequences 5' to the PAM sequence NNNNACA in the human BCL11A gene. SEQ ID NOs: 1901-2162 are protospacer-like sequences 5' to the PAM sequence GNNNCNNA in the human BCL11A gene. SEQ ID NOs: 2163-2482 are protospacer-like target sequences 5' to the PAM sequence NNNNACA in the exons of the human BCL11A gene. SEQ ID NOs: 2483-2666 are protospacer-like sequences 5' to the PAM sequence GNNNCNNA in the exons of the human BCL11A gene. SEQ ID NOs: 2667-2686 are protospacer-like target sequences 5' to the PAM sequence NNNNACA around the 5' end of the human BCL11A gene. SEQ ID NOs: 2687-2701 are protospacer-like sequences 5' to the PAM sequence GNNNCNNA around the 5' end of the human BCL11A gene. Target sequences at least 80% identical to the sequences set out in SEQ ID NOs: 801-2701 are also contemplated.

By "cleavage" it is meant the breakage of the covalent backbone of a DNA molecule. Cleavage can be initiated by a variety of methods including, but not limited to, enzymatic or chemical hydrolysis of a phosphodiester bond. Both single-stranded cleavage and double-stranded cleavage are possible, and double-stranded cleavage can occur as a result of two distinct single-stranded cleavage events. DNA cleavage can result in the production of either blunt ends or staggered ends. In certain embodiments, a complex comprising a guide RNA and a site-directed modifying polypeptide is used for targeted double-stranded DNA cleavage.

"Nuclease" and "endonuclease" are used interchangeably herein to mean an enzyme which possesses endonucleolytic catalytic activity for DNA cleavage.

By "cleavage domain" or "active domain" or "nuclease domain" of a nuclease it is meant the polypeptide sequence or domain within the nuclease which possesses the catalytic activity for DNA cleavage. A cleavage domain can be contained in a single polypeptide chain or cleavage activity can result from the association of two (or more) polypeptides. A single nuclease domain may consist of more than one isolated stretch of amino acids within a given polypeptide.

By "site-directed polypeptide" or "RNA-binding site-directed polypeptide" or "RNA-binding site-directed polypeptide" it is meant a polypeptide that binds RNA and is targeted to a specific DNA sequence. A site-directed polypeptide as described herein is targeted to a specific DNA sequence by the RNA molecule to which it is bound. The RNA molecule comprises a sequence that is complementary to a target sequence within the target DNA, thus targeting the bound polypeptide to a specific location within the target DNA (the target sequence).

The RNA molecule that binds to the site-directed modifying polypeptide and targets the polypeptide to a specific location within the target DNA is referred to herein as the "guide RNA" or "guide RNA polynucleotide" (also referred to herein as a "guide RNA" or "gRNA"). A guide RNA comprises two segments, a "DNA-targeting segment" and a "protein-binding segment." By "segment" it is meant a segment/section/region of a molecule, e.g., a contiguous stretch of nucleotides in an RNA. A segment can also mean a region/section of a complex such that a segment may comprise regions of more than one molecule. For example, in some cases the protein-binding segment (described below) of a guide RNA is one RNA molecule and the protein-binding segment therefore comprises a region of that RNA molecule. In other cases, the protein-binding segment (described below) of a guide RNA comprises two separate molecules that are hybridized along a region of complementarity. As an illustrative, non-limiting example, a protein-binding segment of a guide RNA that comprises two separate molecules can comprise (i) base pairs 40-75 of a first RNA molecule that is 100 base pairs in length; and (ii) base pairs 10-25 of a second RNA molecule that is 50 base pairs in length. The definition of "segment," unless otherwise specifically defined in a particular context, is not limited to a specific number of total base pairs, is not limited to any particular number of base pairs from a given RNA molecule, is not limited to a particular number of separate molecules within a complex, and may include regions of RNA molecules that are of any total length and may or may not include regions with complementarity to other molecules.

The DNA-targeting segment (or "DNA-targeting sequence") comprises a nucleotide sequence that is complementary to a specific sequence within a target DNA (the complementary strand of the target DNA) designated the "protospacer-like" sequence herein. The protein-binding segment (or "protein-binding sequence") interacts with a site-directed modifying polypeptide. When the site-directed modifying polypeptide is a Cas9 or Cas9 related polypeptide (described in more detail below), site-specific cleavage of the target DNA occurs at locations determined by both (i) base-pairing complementarity between the guide RNA and the target DNA; and (ii) a short motif (referred to as the protospacer adjacent motif (PAM)) in the target DNA.

The protein-binding segment of a guide RNA comprises, in part, two complementary stretches of nucleotides that hybridize to one another to form a double stranded RNA duplex (dsRNA duplex).

In some embodiments, a nucleic acid (e.g., a guide RNA, a nucleic acid comprising a nucleotide sequence encoding a guide RNA; a nucleic acid encoding a site-directed polypeptide; etc.) comprises a modification or sequence that provides for an additional desirable feature (e.g., modified or regulated stability; subcellular targeting; tracking, e.g., a fluorescent label; a binding site for a protein or protein complex; etc.). Non-limiting examples include: a 5' cap (e.g., a 7-methylguanylate cap (m7G)); a 3' polyadenylated tail (i.e., a 3' poly(A) tail); a riboswitch sequence (e.g., to allow for regulated stability and/or regulated accessibility by proteins and/or protein complexes); a stability control sequence; a sequence that forms a dsRNA duplex (i.e., a hairpin)); a modification or sequence that targets the RNA to a subcellular location (e.g., nucleus, mitochondria, chloroplasts, and the like); a modification or sequence that provides for tracking (e.g., direct conjugation to a fluorescent molecule, conjugation to a moiety that facilitates fluorescent detection, a sequence that allows for fluorescent detection, etc.); a modification or sequence that provides a binding site for proteins (e.g., proteins that act on DNA, including transcriptional activators, transcriptional repressors, DNA methyltransferases, DNA demethylases, histone acetyltransferases, histone deacetylases, and the like); and combinations thereof.

In some embodiments, a guide RNA comprises an additional segment at either the 5' or 3' end that provides for any of the features described above. For example, a suitable third segment can comprise a 5' cap (e.g., a 7-methylguanylate cap (m7G)); a 3' polyadenylated tail (i.e., a 3' poly(A) tail); a riboswitch sequence (e.g., to allow for regulated stability and/or regulated accessibility by proteins and protein complexes); a stability control sequence; a sequence that forms a dsRNA duplex (i.e., a hairpin)); a sequence that targets the RNA to a subcellular location (e.g., nucleus, mitochondria, chloroplasts, and the like); a modification or sequence that provides for tracking (e.g., direct conjugation to a fluorescent molecule, conjugation to a moiety that facilitates fluorescent detection, a sequence that allows for fluorescent detection, etc.); a modification or sequence that provides a binding site for proteins (e.g., proteins that act on DNA, including transcriptional activators, transcriptional repressors, DNA methyltransferases, DNA demethylases, histone acetyltransferases, histone deacetylases, and the like); and combinations thereof.

A guide RNA and a site-directed modifying polypeptide (i.e., site-directed polypeptide) form a complex (i.e., bind via non-covalent interactions). The guide RNA provides target specificity to the complex by comprising a nucleotide sequence that is complementary to a sequence of a target DNA. The site-directed modifying polypeptide of the complex provides the site-specific activity. In other words, the site-directed modifying polypeptide is guided to a target DNA sequence (e.g. a target sequence in a chromosomal nucleic acid; a target sequence in an extrachromosomal nucleic acid, e.g. an episomal nucleic acid, a minicircle, etc.; a target sequence in a mitochondrial nucleic acid; a target sequence in a chloroplast nucleic acid; a target sequence in a plasmid; etc.) by virtue of its association with the protein-binding segment of the guide RNA.

In some embodiments, a guide RNA comprises two separate RNA molecules (RNA polynucleotides: an "activator-RNA" and a "targeter-RNA", see below) and is referred to herein as a "double-molecule guide RNA" or a "two-molecule guide RNA." In other embodiments, the guide RNA is a single RNA molecule (single RNA polynucleotide) and is referred to herein as a "single-molecule guide RNA," a "single-guide RNA," or an "sgRNA." The term "guide RNA" or "gRNA" is inclusive, referring both to double-molecule guide RNAs and to single-molecule guide RNAs (i.e., sgRNAs).

A two-molecule guide RNA comprises two separate RNA molecules (a "targeter-RNA" and an "activator-RNA"). Each of the two RNA molecules of a two-molecule guide RNA comprises a stretch of nucleotides that are complementary to one another such that the complementary nucleotides of the two RNA molecules hybridize to form the double stranded RNA duplex of the protein-binding segment.

An exemplary two-molecule guide RNA comprises a crRNA-like ("CRISPR RNA" or "targeter-RNA") molecule (which includes a CRISPR repeat or CRISPR repeat-like sequence) and a corresponding tracrRNA-like ("trans-activating CRISPR RNA" or "activator-RNA" or "tracrRNA") molecule. A crRNA-like molecule (targeter-RNA) comprises both the DNA-targeting segment (single stranded) of the guide RNA and a stretch ("duplex-forming segment") of nucleotides that forms one half of the dsRNA duplex of the protein-binding segment of the guide RNA. A corresponding tracrRNA-like molecule (activator-RNA) comprises a stretch of nucleotides (duplex-forming segment) that forms the other half of the dsRNA duplex of the protein-binding segment of the guide RNA. In other words, a stretch of nucleotides of a crRNA-like molecule are complementary to and hybridize with a stretch of nucleotides of a tracrRNA-like molecule to form the dsRNA duplex of the protein-binding domain of the guide RNA. As such, each crRNA-like molecule can be said to have a corresponding tracrRNA-like molecule. The crRNA-like molecule additionally provides the single stranded DNA-targeting segment. Thus, a crRNA-like and a tracrRNA-like molecule (as a corresponding pair) hybridize to form a guide RNA. A double-molecule guide RNA can comprise any corresponding crRNA and tracrRNA pair.

A two-molecule guide RNA can be designed to allow for controlled (i.e., conditional) binding of a targeter-RNA with an activator-RNA. Because a two-molecule guide RNA is not functional unless both the activator-RNA and the targeter-RNA are bound in a functional complex with Cas9, a two-molecule guide RNA can be inducible (e.g., drug inducible) by rendering the binding between the activator-RNA and the targeter-RNA to be inducible. As one non-limiting example, RNA aptamers can be used to regulate (i.e., control) the binding of the activator-RNA with the targeter-RNA. Accordingly, the activator-RNA and/or the targeter-RNA can comprise an RNA aptamer sequence.

A single-molecule guide RNA comprises two stretches of nucleotides (a targeter-RNA and an activator-RNA) that are complementary to one another, are covalently linked (directly, or by intervening nucleotides), and hybridize to form the double stranded RNA duplex (dsRNA duplex) of the protein-binding segment, thus resulting in a stem-loop structure. The targeter-RNA and the activator-RNA can be covalently linked via the 3' end of the targeter-RNA and the 5' end of the activator-RNA. Alternatively, targeter-RNA and the activator-RNA can be covalently linked via the 5' end of the targeter-RNA and the 3' end of the activator-RNA.

An exemplary single-molecule guide RNA comprises two complementary stretches of nucleotides that hybridize to form a dsRNA duplex. In some embodiments, one of the two complementary stretches of nucleotides of the single-molecule guide RNA (or the DNA encoding the stretch) is at least about 60% Identical to one of the activator-RNA (tracrRNA) sequences set forth in Supplementary Table S5 over a stretch of at least 8 contiguous nucleotides. For example, one of the two complementary stretches of nucleotides of the single-molecule guide RNA (or the DNA encoding the stretch) is at least about 65% identical, at least about 70% identical, at least about 75% identical, at least about 80% identical, at least about 85% identical, at least about 90% identical, at least about 95% identical, at least about 98% identical, at least about 99% identical or 100 % identical to one of the tracrRNA sequences set forth in Supplementary Table S5 over a stretch of at least 8 contiguous , at least 9 contiguous, at least 10 contiguous, at least 11 contiguous, at least 12 contiguous, at least 13 contiguous, at least 14 contiguous or at least 15 contiguous nucleotides. For example, the single-molecule guide RNA may comprise a nucleotide sequence that is at least 70% identical over at least 10 contiguous nucleotides, at least 80% identical over at least 10 contiguous nucleotides, at least 70% identical over at least 11 contiguous nucleotides, at least 80% identical over at least 11 contiguous nucleotides, at least 70% identical over at least 12 contiguous nucleotides, or at least 80% identical over at least 12 contiguous nucleotides of one of the tracrRNA sequences set forth in Supplementary Table S5. It is understood that where a series of percent identities and a series of lengths of nucleotides sequences are set out as options, each and every combination of a percent identity with a length (e.g. 8, 9, 10, 12, 13, 14, 15 nucleotides) of nucleotide sequence is contemplated.

In some embodiments, one of the two complementary stretches of nucleotides of the single-molecule guide RNA (or the DNA encoding the stretch) is at least about 60% identical to one of the targeter-RNA (crRNA/CRISPR repeat) sequences set forth in Supplementary Table S5 over a stretch of at least 8 contiguous nucleotides. For example, one of the two complementary stretches of nucleotides of the single-molecule guide RNA (or the DNA encoding the stretch) is at least about 65% identical, at least about 70% identical, at least about 75% identical, at least about 80% identical, at least about 85% identical, at least about 90% identical, at least about 95% identical, at least about 98% identical, at least about 99% identical or 100 % identical to one of the crRNA/CRISPR repeat sequences set forth in Supplementary Table S5 over a stretch of at least 8 contiguous, at least 9 contiguous, at least 10 contiguous, at least 11 contiguous, at least 12 contiguous, at least 13 contiguous, at least 14 contiguous or at least 15 contiguous nucleotides. For example, the single-molecule guide RNA may comprise a nucleotide sequence that is at least 70% identical over at least 10 contiguous nucleotides, at least 80% identical over at least 10 contiguous nucleotides, at least 70% identical over at least 11 contiguous nucleotides, at least 80% identical over at least 11 contiguous nucleotides, at least 70% identical over at least 12 contiguous nucleotides, or at least 80% identical over at least 12 contiguous nucleotides of one of the CRISPR repeat sequences set forth in Supplementary Table S5. It is understood that where a series of percent identities and a series of lengths of nucleotides sequences are set out as options, each and every combination of a percent identity with a length (e.g. 8, 9, 10, 11, 12, 13, 14, 15 nucleotides) of nucleotide sequence is contemplated.

The term "activator-RNA" is used herein to mean a tracrRNA-like molecule of a double-molecule guide RNA. The term "targeter-RNA" is used herein to mean a crRNA-like molecule of a double-molecule guide RNA. The term "duplex-forming segment" is used herein to mean the stretch of nucleotides of an activator-RNA or a targeter-RNA that contributes to the formation of the dsRNA duplex by hybridizing to a stretch of nucleotides of a corresponding activator-RNA or targeter-RNA molecule. In other words, an activator-RNA comprises a duplex-forming segment that is complementary to the duplex-forming segment of the corresponding targeter-RNA. As such, an activator-RNA comprises a duplex-forming segment while a targeter-RNA comprises both a duplex-forming segment and the DNA-targeting segment of the guide RNA. Therefore, a double-molecule guide RNA can be comprised of any corresponding activator-RNA and targeter-RNA pair.

RNA aptamers are known in the art and are generally a synthetic version of a riboswitch. The terms "RNA aptamer" and "riboswitch" are used interchangeably herein to encompass both synthetic and natural nucleic acid sequences that provide for inducible regulation of the structure (and therefore the availability of specific sequences) of the RNA molecule of which they are part. RNA aptamers usually comprise a sequence that folds into a particular structure (e.g., a hairpin), which specifically binds a particular drug (e.g., a small molecule). Binding of the drug causes a structural change in the folding of the RNA, which changes a feature of the nucleic acid of which the aptamer is a part. As non-limiting examples: (i) an activator-RNA with an aptamer may not be able to bind to the cognate targeter-RNA unless the aptamer is bound by the appropriate drug; (ii) a targeter-RNA with an aptamer may not be able to bind to the cognate activator-RNA unless the aptamer is bound by the appropriate drug; and (iii) a targeter-RNA and an activator-RNA, each comprising a different aptamer that binds a different drug, may not be able to bind to each other unless both drugs are present. As illustrated by these examples, a two-molecule guide RNA can be designed to be inducible.

Examples of aptamers and riboswitches can be found, for example, in: Nakamura et al., Genes Cells. 2012 May;17(5):344-64; Vavalle et al., Future Cardiol. 2012 May;8(3):371-82; Citartan et al., Biosens Bioelectron. 2012 Apr 15;34(1):1-11; and Liberman et al., Wiley Interdiscip Rev RNA. 2012 May-Jun;3(3):369-84; all of which are herein incorporated by reference in their entirety.

The term "stem cell" is used herein to refer to a cell (e.g., plant stem cell, vertebrate stem cell) that has the ability both to self-renew and to generate a differentiated cell type (see Morrison et al. (1997) Cell 88:287-298). In the context of cell ontogeny, the adjective "differentiated", or "differentiating" is a relative term. A "differentiated cell" is a cell that has progressed further down the developmental pathway than the cell it is being compared with. Thus, pluripotent stem cells (described below) can differentiate into lineage-restricted progenitor cells (e.g.,mesodermal stem cells), which in turn can differentiate into cells that are further restricted (e.g., neuron progenitors), which can differentiate into end-stage cells (i.e., terminally differentiated cells, e.g., neurons, cardiomyocytes, etc.), which play a characteristic role in a certain tissue type, and may or may not retain the capacity to proliferate further. Stem cells may be characterized by both the presence of specific markers (e.g., proteins, RNAs, etc.) and the absence of specific markers. Stem cells may also be identified by functional assays both in vitro and in vivo, particularly assays relating to the ability of stem cells to give rise to multiple differentiated progeny.

Stem cells of interest include pluripotent stem cells (PSCs). The term "pluripotent stem cell" or "PSC" is used herein to mean a stem cell capable of producing all cell types of the organism. Therefore, a PSC can give rise to cells of all germ layers of the organism (e.g., the endoderm, mesoderm, and ectoderm of a vertebrate). Pluripotent cells are capable of forming teratomas and of contributing to ectoderm, mesoderm, or endoderm tissues in a living organism. Pluripotent stem cells of plants are capable of giving rise to all cell types of the plant (e.g., cells of the root, stem, leaves, etc.).

PSCs of animals can be derived in a number of different ways. For example, embryonic stem cells (ESCs) are derived from the inner cell mass of an embryo (Thomson et. al, Science. 1998 Nov 6;282(5391):1145-7) whereas induced pluripotent stem cells (iPSCs) are derived from somatic cells (Takahashi et. al, Cell. 2007 Nov 30;131(5):861-72; Takahashi et. al, Nat Protoc. 2007;2(12):3081-9; Yu et. al, Science. 2007 Dec 21;318(5858):1917-20. Epub 2007 Nov 20). Because the term PSC refers to pluripotent stem cells regardless of their derivation, the term PSC encompasses the terms ESC and iPSC, as well as the term embryonic germ stem cells (EGSC), which are another example of a PSC. PSCs may be in the form of an established cell line, they may be obtained directly from primary embryonic tissue, or they may be derived from a somatic cell. PSCs can be target cells of the methods described herein.

By "embryonic stem cell" (ESC) is meant a PSC that was isolated from an embryo, typically from the inner cell mass of the blastocyst. ESC lines are listed in the NIH Human Embryonic Stem Cell Registry, e.g. hESBGN-01, hESBGN-02, hESBGN-03, hESBGN-04 (BresaGen, Inc.); HES-1, HES-2, HES-3, HES-4, HES-5, HES-6 (ES Cell International); Miz-hES1 (MizMedi Hospital-Seoul National University); HSF-1, HSF-6 (University of California at San Francisco); and H1, H7, H9, H13, H14 (Wisconsin Alumni Research Foundation (WiCell Research Institute)). Stem cells of interest also include embryonic stem cells from other primates, such as Rhesus stem cells and marmoset stem cells. The stem cells may be obtained from any mammalian species, e.g. human, equine, bovine, porcine, canine, feline, rodent, e.g. mice, rats, hamster, primate, etc. (Thomson et al. (1998) Science 282:1145; Thomson et al. (1995) Proc. Natl. Acad. Sci USA 92:7844; Thomson et al. (1996) Biol. Reprod. 55:254; Shamblott et al., Proc. Natl. Acad. Sci. USA 95:13726, 1998). In culture, ESCs typically grow as flat colonies with large nucleo-cytoplasmic ratios, defined borders and prominent nucleoli. In addition, ESCs express SSEA-3, SSEA-4, TRA-1-60, TRA-1-81, and Alkaline Phosphatase, but not SSEA-1. Examples of methods of generating and characterizing ESCs may be found in, for example, US Patent No. 7,029,913, US Patent No. 5,843,780, and US Patent No. 6,200,806, the disclosures of which are incorporated herein by reference. Methods for proliferating hESCs in the undifferentiated form are described in WO 99/20741, WO 01/51616, and WO 03/020920. By "embryonic germ stem cell" (EGSC) or "embryonic germ cell" or "EG cell" is meant a PSC that is derived from germ cells and/or germ cell progenitors, e.g. primordial germ cells, i.e. those that would become sperm and eggs. Embryonic germ cells (EG cells) are thought to have properties similar to embryonic stem cells as described above. Examples of methods of generating and characterizing EG cells may be found in, for example, US Patent No. 7,153,684; Matsui, Y., et al., (1992) Cell 70:841; Shamblott, M., et al. (2001) Proc. Natl. Acad. Sci. USA 98: 113; Shamblott, M., et al. (1998) Proc. Natl. Acad. Sci. USA, 95:13726; and Koshimizu, U., et al. (1996) Development, 122:1235, the disclosures of which are incorporated herein by reference.

By "induced pluripotent stem cell" or "iPSC" it is meant a PSC that is derived from a cell that is not a PSC (i.e., from a cell this is differentiated relative to a PSC). iPSCs can be derived from multiple different cell types, including terminally differentiated cells. iPSCs have an ES cell-like morphology, growing as flat colonies with large nucleo-cytoplasmic ratios, defined borders and prominent nuclei. In addition, iPSCs express one or more key pluripotency markers known by one of ordinary skill in the art, including but not limited to Alkaline Phosphatase, SSEA3, SSEA4, Sox2, Oct3/4, Nanog, TRA160, TRA181, TDGF 1, Dnmt3b, FoxD3, GDF3, Cyp26al, TERT, and zfp42. Examples of methods of generating and characterizing iPSCs may be found in, for example, U.S. Patent Publication Nos. US20090047263, US20090068742, US20090191159, US20090227032, US20090246875, and US20090304646, the disclosures of which are incorporated herein by reference. Generally, to generate iPSCs, somatic cells are provided with reprogramming factors (e.g. Oct4, SOX2, KLF4, MYC, Nanog, Lin28, etc.) known in the art to reprogram the somatic cells to become pluripotent stem cells.

By "somatic cell" it is meant any cell in an organism that, in the absence of experimental manipulation, does not ordinarily give rise to all types of cells in an organism. In other words, somatic cells are cells that have differentiated sufficiently that they will not naturally generate cells of all three germ layers of the body, i.e. ectoderm, mesoderm and endoderm. For example, somatic cells would include both neurons and neural progenitors, the latter of which may be able to naturally give rise to all or some cell types of the central nervous system but cannot give rise to cells of the mesoderm or endoderm lineages.

By "mitotic cell" it is meant a cell undergoing mitosis. Mitosis is the process by which a eukaryotic cell separates the chromosomes in its nucleus into two identical sets in two separate nuclei. It is generally followed immediately by cytokinesis, which divides the nuclei, cytoplasm, organelles and cell membrane into two cells containing roughly equal shares of these cellular components.

By "post-mitotic cell" it is meant a cell that has exited from mitosis, i.e., it is "quiescent", i.e. it is no longer undergoing divisions. This quiescent state may be temporary, i.e. reversible, or it may be permanent.

By "meiotic cell" it is meant a cell that is undergoing meiosis. Meiosis is the process by which a cell divides its nuclear material for the purpose of producing gametes or spores. Unlike mitosis, in meiosis, the chromosomes undergo a recombination step which shuffles genetic material between chromosomes. Additionally, the outcome of meiosis is four (genetically unique) haploid cells, as compared with the two (genetically identical) diploid cells produced from mitosis.

By "recombination" it is meant a process of exchange of genetic information between two polynucleotides. As used herein, "homology-directed repair (HDR)" refers to the specialized form DNA repair that takes place, for example, during repair of double-strand breaks in cells. This process requires nucleotide sequence homology, uses a "donor" molecule to template repair of a "target" molecule (i.e., the one that experienced the double-strand break), and leads to the transfer of genetic information from the donor to the target. Homology-directed repair may result in an alteration of the sequence of the target molecule (e.g., insertion, deletion, mutation), if the donor polynucleotide differs from the target molecule and part or all of the sequence of the donor polynucleotide is incorporated into the target DNA. In some embodiments, the donor polynucleotide, a portion of the donor polynucleotide, a copy of the donor polynucleotide, or a portion of a copy of the donor polynucleotide integrates into the target DNA.

By "non-homologous end joining (NHEJ)" it is meant the repair of double-strand breaks in DNA by direct ligation of the break ends to one another without the need for a homologous template (in contrast to homology-directed repair, which requires a homologous sequence to guide repair). NHEJ often results in the loss (deletion) of nucleotide sequence near the site of the double-strand break.

The terms "treatment", "treating" and the like are used herein to generally mean obtaining a desired pharmacologic and/or physiologic effect. The effect may be prophylactic in terms of completely or partially preventing a disease or symptom thereof and/or may be therapeutic in terms of a partial or complete cure for a disease and/or adverse effect attributable to the disease. "Treatment" as used herein covers any treatment of a disease or symptom in a mammal, and includes: (a) preventing the disease or symptom from occurring in a subject which may be predisposed to acquiring the disease or symptom but has not yet been diagnosed as having it; (b) inhibiting the disease or symptom, i.e., arresting its development; or (c) relieving the disease, i.e., causing regression of the disease. The therapeutic agent may be administered before, during or after the onset of disease or injury. The treatment of ongoing disease, where the treatment stabilizes or reduces the undesirable clinical symptoms of the patient, is of particular interest. Such treatment is desirably performed prior to complete loss of function in the affected tissues. The therapy will desirably be administered during the symptomatic stage of the disease, and in some cases after the symptomatic stage of the disease.

The terms "individual," "subject," "host," and "patient," are used interchangeably herein and refer to any mammalian subject for whom diagnosis, treatment, or therapy is desired, particularly humans.

General methods in molecular and cellular biochemistry can be found in such standard textbooks as Molecular Cloning: A Laboratory Manual, 3rd Ed. (Sambrook et al., HaRBor Laboratory Press 2001); Short Protocols in Molecular Biology, 4th Ed. (Ausubel et al. eds., John Wiley & Sons 1999); Protein Methods (Bollag et al., John Wiley & Sons 1996); Nonviral Vectors for Gene Therapy (Wagner et al. eds., Academic Press 1999); Viral Vectors (Kaplift & Loewy eds., Academic Press 1995); Immunology Methods Manual (I. Lefkovits ed., Academic Press 1997); and Cell and Tissue Culture: Laboratory Procedures in Biotechnology (Doyle & Griffiths, John Wiley & Sons 1998), the disclosures of which are incorporated herein by reference.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention.

The phrase "consisting essentially of" is meant herein to exclude anything that is not the specified active component or components of a system, or that is not the specified active portion or portions of a molecule.

Certain ranges are presented herein with numerical values being preceded by the term "about." The term "about" is used herein to provide literal support for the exact number that it precedes, as well as a number that is near to or approximately the number that the term precedes. In determining whether a number is near to or approximately a specifically recited number, the near or approximating unrecited number may be a number which, in the context in which it is presented, provides the substantial equivalent of the specifically recited number.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination. All combinations of the embodiments pertaining to the invention are specifically embraced by the present invention and are disclosed herein just as if each and every combination was individually and explicitly disclosed. In addition, all sub-combinations of the various embodiments and elements thereof are also specifically embraced by the present invention and are disclosed herein just as if each and every such sub-combination was individually and explicitly disclosed herein.

### Aspects of the Disclosure - Part I

### Nucleic Acids

### Guide RNA

The present disclosure provides a guide RNA that directs the activities of an associated polypeptide (e.g., a site-directed modifying polypeptide) to a specific target sequence within a target DNA. A guide RNA comprises: a first segment (also referred to herein as a "DNA-targeting segment" or a "DNA-targeting sequence") and a second segment (also referred to herein as a "protein-binding segment" or a "protein-binding sequence").

### DNA-targeting segment of a guide RNA

The DNA-targeting segment of a guide RNA comprises a nucleotide sequence that is complementary to a sequence in a target DNA. In other words, the DNA-targeting segment of a guide RNA interacts with a target DNA in a sequence-specific manner via hybridization (i.e., base pairing). As such, the nucleotide sequence of the DNA-targeting segment may vary and determines the location within the target DNA that the guide RNA and the target DNA will interact. The DNA-targeting segment of a guide RNA can be modified (e.g., by genetic engineering) to hybridize to any desired sequence within a target DNA.

The DNA-targeting segment can have a length of from about 12 nucleotides to about 100 nucleotides. For example, the DNA-targeting segment can have a length of from about 12 nucleotides (nt) to about 80 nt, from about 12 nt to about 50 nt, from about12 nt to about 40 nt, from about 12 nt to about 30 nt, from about 12 nt to about 25 nt, from about 12 nt to about 20 nt, or from about 12 nt to about 19 nt. For example, the DNA-targeting segment can have a length of from about 19 nt to about 20 nt, from about 19 nt to about 25 nt, from about 19 nt to about 30 nt, from about 19 nt to about 35 nt, from about 19 nt to about 40 nt, from about 19 nt to about 45 nt, from about 19 nt to about 50 nt, from about 19 nt to about 60 nt, from about 19 nt to about 70 nt, from about 19 nt to about 80 nt, from about 19 nt to about 90 nt, from about 19 nt to about 100 nt, from about 20 nt to about 25 nt, from about 20 nt to about 30 nt, from about 20 nt to about 35 nt, from about 20 nt to about 40 nt, from about 20 nt to about 45 nt, from about 20 nt to about 50 nt, from about 20 nt to about 60 nt, from about 20 nt to about 70 nt, from about 20 nt to about 80 nt, from about 20 nt to about 90 nt, or from about 20 nt to about 100 nt. The nucleotide sequence (the DNA-targeting sequence) of the DNA-targeting segment that is complementary to a nucleotide sequence (target sequence) of the target DNA can have a length at least about 12 nt. For example, the DNA-targeting sequence of the DNA-targeting segment that is complementary to a target sequence of the target DNA can have a length at least about 12 nt, at least about 15 nt, at least about 18 nt, at least about 19 nt, at least about 20 nt, at least about 25 nt, at least about 30 nt, at least about 35 nt or at least about 40 nt. For example, the DNA-targeting sequence of the DNA-targeting segment that is complementary to a target sequence of the target DNA can have a length of from about 12 nucleotides (nt) to about 80 nt, from about 12 nt to about 50 nt, from about 12 nt to about 45 nt, from about 12 nt to about 40 nt, from about 12 nt to about 35 nt, from about 12 nt to about 30 nt, from about 12 nt to about 25 nt, from about 12 nt to about 20 nt, from about 12 nt to about 19 nt, from about 19 nt to about 20 nt, from about 19 nt to about 25 nt, from about 19 nt to about 30 nt, from about 19 nt to about 35 nt, from about 19 nt to about 40 nt, from about 19 nt to about 45 nt, from about 19 nt to about 50 nt, from about 19 nt to about 60 nt, from about 20 nt to about 25 nt, from about 20 nt to about 30 nt, from about 20 nt to about 35 nt, from about 20 nt to about 40 nt, from about 20 nt to about 45 nt, from about 20 nt to about 50 nt, or from about 20 nt to about 60 nt. The nucleotide sequence (the DNA-targeting sequence) of the DNA-targeting segment that is complementary to a nucleotide sequence (target sequence) of the target DNA can have a length at least about 12 nt.

In some cases, the DNA-targeting sequence of the DNA-targeting segment that is complementary to a target sequence of the target DNA is 20 nucleotides in length. In some cases, the DNA-targeting sequence of the DNA-targeting segment that is complementary to a target sequence of the target DNA is 16 nucleotides, 17 nucleotides, 18 nucleotides or 19 nucleotides in length.

The percent complementarity between the DNA-targeting sequence of the DNA-targeting segment and the target sequence of the target DNA can be at least 60% (e.g., at least 65%, at least 70%, at least 75%, at least 80%, at least 85%, at least 90%, at least 95%, at least 97%, at least 98%, at least 99%, or 100%). For example, the DNA-targeting sequence may be at least about 80% identical to about 10 contiguous nucleotides, or at least about 80% identical to about 11 contiguous nucleotides, or at least about 80% identical to about 12 contiguous nucleotides, or at least about 80% identical to about 13 contiguous nucleotides, or at least about 80% identical to about 14 contiguous nucleotides, or at least about 80% identical to about 15 contiguous nucleotides, or at least about 80% identical to about 16 contiguous nucleotides, or at least about 80% identical to about 17 contiguous nucleotides of the target sequence. In some cases, the percent complementarity between the DNA-targeting sequence of the DNA-targeting segment and the target sequence of the target DNA is 100% over the seven contiguous 5'-most nucleotides of the target sequence of the complementary strand of the target DNA. In some cases, the percent complementarity between the DNA-targeting sequence of the DNA-targeting segment and the target sequence of the target DNA is at least 60% over about 20 contiguous nucleotides. In some cases, the percent complementarity between the DNA-targeting sequence of the DNA-targeting segment and the target sequence of the target DNA is 100% over the fourteen contiguous 5'-most nucleotides of the target sequence of the complementary strand of the target DNA and as low as 0% over the remainder. In such a case, the DNA-targeting sequence can be considered to be 14 nucleotides in length. In some cases, the percent complementarity between the DNA-targeting sequence of the DNA-targeting segment and the target sequence of the target DNA is 100% over the seven contiguous 5'-most nucleotides of the target sequence of the complementary strand of the target DNA and as low as 0% over the remainder. In such a case, the DNA-targeting sequence can be considered to be 7 nucleotides in length.

### Protein-binding segment of a guide RNA

The protein-binding segment of a guide RNA interacts with a site-directed modifying polypeptide. The guide RNA guides the bound polypeptide to a specific nucleotide sequence within target DNA via the above mentioned DNA-targeting segment. The protein-binding segment of a guide RNA comprises two stretches of nucleotides that are complementary to one another. The complementary nucleotides of the protein-binding segment hybridize to form a double stranded RNA duplex (dsRNA).

A double-molecule guide RNA comprises two separate RNA molecules. Each of the two RNA molecules of a double-molecule guide RNA comprises a stretch of nucleotides that are complementary to one another such that the complementary nucleotides of the two RNA molecules hybridize to form the double-stranded RNA duplex of the protein-binding segment.

In some embodiments, the duplex-forming segment of the activator-RNA is at least about 60% identical to one of the activator-RNA (tracrRNA) molecules set forth in Supplementary Table S5, or a complement thereof, over a stretch of at least 8 contiguous nucleotides. For example, the duplex-forming segment of the activator-RNA (or the DNA encoding the duplex-forming segment of the activator-RNA) is at least about 60% identical, at least about 65% identical, at least about 70% identical, at least about 75% identical, at least about 80% identical, at least about 85% identical, at least about 90% identical, at least about 95% identical, at least about 98% identical, at least about 99% identical, or 100 % identical, to one of the tracrRNA sequences set forth in Supplementary Table S5, or a complement thereof, over a stretch of at least 8 contiguous , at least 9 contiguous, at least 10 contiguous, at least 11 contiguous, at least 12 contiguous, at least 13 contiguous, at least 14 contiguous or at least 15 contiguous nucleotides. For example, the activator-RNA may comprise a nucleotide sequence that is at least 70% identical over at least 10 contiguous nucleotides, at least 80% identical over at least 10 contiguous nucleotides, at least 70% identical over at least 11 contiguous nucleotides, at least 80% identical over at least 11 contiguous nucleotides, at least 70% identical over at least 12 contiguous nucleotides, or at least 80% identical over at least 12 contiguous nucleotides of one of the tracrRNA sequences set forth in Supplementary Table S5.

In some embodiments, the duplex-forming segment of the targeter-RNA is at least about 60% identical to one of the targeter-RNA (crRNA/CRISPR repeat) seqeunces set forth in Supplementary Table S5, or a complement thereof, over a stretch of at least 8 contiguous nucleotides. For example, the duplex-forming segment of the targeter-RNA (or the DNA encoding the duplex-forming segment of the targeter-RNA) is at least about 65% identical, at least about 70% identical, at least about 75% identical, at least about 80% identical, at least about 85% identical, at least about 90% identical, at least about 95% identical, at least about 98% identical, at least about 99% identical or 100 % identical to one of the crRNA/CRISPR repeat sequences set forth in Supplementary Table S5, or a complement thereof, over a stretch of at least 8 contiguous , at least 9 contiguous, at least 10 contiguous, at least 11 contiguous, at least 12 contiguous, at least 13 contiguous, at least 14 contiguous or at least 15 contiguous nucleotides. For example, the targeter-RNA may comprise a nucleotide sequence that is at least 70% identical over at least 10 contiguous nucleotides, at least 80% identical over at least 10 contiguous nucleotides, at least 70% identical over at least 11 contiguous nucleotides, at least 80% identical over at least 11 contiguous nucleotides, at least 70% identical over at least 12 contiguous nucleotides, at least 80% identical over at least 12 contiguous nucleotides, at least 80% identical over at least 13 contiguous nucleotides, at least 80% identical over at least 14 contiguous nucleotides, at least 80% identical over at least 15 contiguous nucleotides, at least 80% identical over at least 16 contiguous nucleotides, or at least 80% identical over at least 17 contiguous nucleotides, to one of the CRISPR repeat sequences set forth in Supplementary Table S5.

A two-molecule guide RNA can be designed to allow for controlled (i.e., conditional) binding of a targeter-RNA with an activator-RNA. Because a two-molecule guide RNA is not functional unless both the activator-RNA and the targeter-RNA are bound in a functional complex with Cas9, a two-molecule guide RNA can be inducible (e.g., drug inducible) by rendering the binding between the activator-RNA and the targeter-RNA to be inducible. As one non-limiting example, RNA aptamers can be used to regulate (i.e., control) the binding of the activator-RNA with the targeter-RNA. Accordingly, the activator-RNA and/or the targeter-RNA can comprise an RNA aptamer sequence.

RNA aptamers are known in the art and are generally a synthetic version of a riboswitch. The terms "RNA aptamer" and "riboswitch" are used interchangeably herein to encompass both synthetic and natural nucleic acid sequences that provide for inducible regulation of the structure (and therefore the availability of specific sequences) of the RNA molecule of which they are part. RNA aptamers usually comprise a sequence that folds into a particular structure (e.g., a hairpin), which specifically binds a particular drug (e.g., a small molecule). Binding of the drug causes a structural change in the folding of the RNA, which changes a feature of the nucleic acid of which the aptamer is a part. As non-limiting examples: (i) an activator-RNA with an aptamer may not be able to bind to the cognate targeter-RNA unless the aptamer is bound by the appropriate drug; (ii) a targeter-RNA with an aptamer may not be able to bind to the cognate activator-RNA unless the aptamer is bound by the appropriate drug; and (iii) a targeter-RNA and an activator-RNA, each comprising a different aptamer that binds a different drug, may not be able to bind to each other unless both drugs are present. As illustrated by these examples, a two-molecule guide RNA can be designed to be inducible.

Examples of aptamers and riboswitches can be found, for example, in: Nakamura et al., Genes Cells. 2012 May;17(5):344-64; Vavalle et al., Future Cardiol. 2012 May;8(3):371-82; Citartan et al., Biosens Bioelectron. 2012 Apr 15;34(1):1-11; and Liberman et al., Wiley Interdiscip Rev RNA. 2012 May-Jun;3(3):369-84; all of which are herein incorporated by reference in their entirety.

Non-limiting examples of nucleotide sequences that can be included in a two-molecule guide RNA include either of the sequences set forth in Supplmentary Table S5, or complements thereof pairing with any sequences set forth in Supplementary Table S5,or complements thereof that can hybridize to form a protein binding segment.

A single-molecule guide RNA comprises two stretches of nucleotides (a targeter-RNA and an activator-RNA) that are complementary to one another, are covalently linked (directly, or by intervening nucleotides referred to as "linkers" or "linker nucleotides"), and hybridize to form the double stranded RNA duplex (dsRNA duplex) of the protein-binding segment, thus resulting in a stem-loop structure. The targeter-RNA and the activator-RNA can be covalently linked via the 3' end of the targeter-RNA and the 5' end of the activator-RNA. Alternatively, targeter-RNA and the activator-RNA can be covalently linked via the 5' end of the targeter-RNA and the 3' end of the activator-RNA.

The linker of a single-molecule guide RNA can have a length of from about 3 nucleotides to about 100 nucleotides. For example, the linker can have a length of from about 3 nucleotides (nt) to about 90 nt, from about 3 nucleotides (nt) to about 80 nt, from about 3 nucleotides (nt) to about 70 nt, from about 3 nucleotides (nt) to about 60 nt, from about 3 nucleotides (nt) to about 50 nt, from about 3 nucleotides (nt) to about 40 nt, from about 3 nucleotides (nt) to about 30 nt, from about 3 nucleotides (nt) to about 20 nt or from about 3 nucleotides (nt) to about 10 nt. For example, the linker can have a length of from about 3 nt to about 5 nt, from about 5 nt to about 10 nt, from about 10 nt to about 15 nt, from about 15 nt to about 20 nt, from about 20 nt to about 25 nt, from about 25 nt to about 30 nt, from about 30 nt to about 35 nt, from about 35 nt to about 40 nt, from about 40 nt to about 50 nt, from about 50 nt to about 60 nt, from about 60 nt to about 70 nt, from about 70 nt to about 80 nt, from about 80 nt to about 90 nt, or from about 90 nt to about 100 nt. In some embodiments, the linker of a single-molecule guide RNA is 4 nt.

An exemplary single-molecule guide RNA comprises two complementary stretches of nucleotides that hybridize to form a dsRNA duplex. In some embodiments, one of the two complementary stretches of nucleotides of the single-molecule guide RNA (or the DNA encoding the stretch) is at least about 60% identical to one of the activator-RNA (tracrRNA) molecules set forth in Supplementary Table S5, or a complement thereof, over a stretch of at least 8 contiguous nucleotides. For example, one of the two complementary stretches of nucleotides of the single-molecule guide RNA (or the DNA encoding the stretch) is at least about 65% identical, at least about 70% identical, at least about 75% identical, at least about 80% identical, at least about 85% identical, at least about 90% identical, at least about 95% identical, at least about 98% identical, at least about 99% identical or 100 % identical to one of the tracrRNA sequences set forth in Supplementary Table S5, or a complement thereof, over a stretch of at least 8 contiguous , at least 9 contiguous, at least 10 contiguous, at least 11 contiguous, at least 12 contiguous, at least 13 contiguous, at least 14 contiguous or at least 15 contiguous nucleotides. For example, the single-molecule guide RNA may comprise a nucleotide sequence that is at least 70% identical over at least 10 contiguous nucleotides, at least 80% identical over at least 10 contiguous nucleotides, at least 70% identical over at least 11 contiguous nucleotides, at least 80% identical over at least 11 contiguous nucleotides, at least 70% identical over at least 12 contiguous nucleotides, or at least 80% identical over at least 12 contiguous nucleotides of one of the tracrRNA sequences set forth in Supplementary Table S5.

In some embodiments, one of the two complementary stretches of nucleotides of the single-molecule guide RNA (or the DNA encoding the stretch) is at least about 60% identical to one of the targeter-RNA (crRNA/CRISPR repeat) sequences set forth in Supplementary Table S5, or a complement thereof, over a stretch of at least 8 contiguous nucleotides. For example, one of the two complementary stretches of nucleotides of the single-molecule guide RNA (or the DNA encoding the stretch) is at least about 65% identical, at least about 70% identical, at least about 75% identical, at least about 80% identical, at least about 85% identical, at least about 90% identical, at least about 95% identical, at least about 98% identical, at least about 99% identical or 100 % identical to one of the crRNA/CRISPR repeat sequences set forth in Supplementary Table S5, or a complement thereof, over a stretch of at least 8 contiguous , at least 9 contiguous, at least 10 contiguous, at least 11 contiguous, at least 12 contiguous, at least 13 contiguous, at least 14 contiguous or at least 15 contiguous nucleotides. For example, the single-molecule guide RNA may comprise a nucleotide sequence that is at least 70% identical over at least 10 contiguous nucleotides, at least 80% identical over at least 10 contiguous nucleotides, at least 70% identical over at least 11 contiguous nucleotides, at least 80% identical over at least 11 contiguous nucleotides, at least 70% identical over at least 12 contiguous nucleotides, or at least 80% identical over at least 12 contiguous nucleotides, or at least about 80% identical to about 13 contiguous nucleotides, or at least about 80% identical to about 14 contiguous nucleotides, or at least about 80% identical to about 15 contiguous nucleotides, or at least about 80% identical to about 16 contiguous nucleotides, or at least about 80% identical to about 17 contiguous nucleotides of one of the CRISPR repeat sequences set forth in Supplementary Table S5.

Appropriate naturally occurring cognate pairs of crRNAs and tracrRNAs can be routinely determined by taking into account the species name and base-pairing (for the dsRNA duplex of the protein-binding domain) when determining appropriate cognate pairs. Non-cognate pairs are also contemplated for use in the invention. In some embodiments of non-cognate pairs, each RNA is from a Cas9 cluster herein wherein the Cas9 endonucleases share 80% identity over 80% of their amino acid sequences.

Artificial sequences that share very little identity (roughly 50% identity, or alternatively about 70% identity over about 50% of the full length protein) with naturally occurring a tracrRNAs and crRNAs can function with Cas9 to cleave target DNA as long as the structure of the protein-binding domain of the guide RNA is conserved. Thus, RNA folding structure of a naturally occuring protein-binding domain of a DNA-trageting RNA can be taken into account in order to design artificial protein-binding domains (either two-molecule or single-molecule versions). As structures can readily be produced by one of ordinary skill in the art for any naturally occurring crRNA:tracrRNA pair from any, an artificial DNA-targeting-RNA can be designed to mimic the natural structure for a given species when using the Cas9 (or a related Cas9) from that species. Thus, a suitable guide RNA can be an artificially designed RNA (non-naturally occurring) comprising a protein-binding domain that was designed to mimic the structure of a protein-binding domain of a naturally occurring guide RNA.

The protein-binding segment can have a length of from about 10 nucleotides to about 100 nucleotides. For example, the protein-binding segment can have a length of from about 15 nucleotides (nt) to about 80 nt, from about 15 nt to about 50 nt, from about 15 nt to about 40 nt, from about 15 nt to about 30 nt or from about 15 nt to about 25 nt.

Also with regard to both a single-molecule guide RNA and to a double-molecule guide RNA, the dsRNA duplex of the protein-binding segment can have a length from about 6 base pairs (bp) to about 50bp. For example, the dsRNA duplex of the protein-binding segment can have a length from about 6 bp to about 40 bp, from about 6 bp to about 30bp, from about 6 bp to about 25 bp, from about 6 bp to about 20 bp, from about 6 bp to about 15 bp, from about 8 bp to about 40 bp, from about 8 bp to about 30bp, from about 8 bp to about 25 bp, from about 8 bp to about 20 bp or from about 8 bp to about 15 bp. For example, the dsRNA duplex of the protein-binding segment can have a length from about from about 8 bp to about 10 bp, from about 10 bp to about 15 bp, from about 15 bp to about 18 bp, from about 18 bp to about 20 bp, from about 20 bp to about 25 bp, from about 25 bp to about 30 bp, from about 30 bp to about 35 bp, from about 35 bp to about 40 bp, or from about 40 bp to about 50 bp. In some embodiments, the dsRNA duplex of the protein-binding segment has a length of 36 base pairs. The percent complementarity between the nucleotide sequences that hybridize to form the dsRNA duplex of the protein-binding segment can be at least about 60%. For example, the percent complementarity between the nucleotide sequences that hybridize to form the dsRNA duplex of the protein-binding segment can be at least about 65%, at least about 70%, at least about 75%, at least about 80%, at least about 85%, at least about 90%, at least about 95%, at least about 98%, or at least about 99% . In some cases, the percent complementarity between the nucleotide sequences that hybridize to form the dsRNA duplex of the protein-binding segment is 100%.

### Site-directed modifying polypeptide

A guide RNA and a site-directed modifying polypeptide form a complex. The guide RNA provides target specificity to the complex by comprising a nucleotide sequence that is complementary to a sequence of a target DNA (as noted above). The site-directed modifying polypeptide is guided to a DNA sequence (e.g. a chromosomal sequence or an extrachromosomal sequence, e.g. an episomal sequence, a minicircle sequence, a mitochondrial sequence, a chloroplast sequence, etc.) by virtue of its association with at least the protein-binding segment of the guide RNA (described above).

A site-directed modifying polypeptide modifies target DNA (e.g., cleavage or methylation of target DNA) and/or a polypeptide associated with target DNA (e.g., methylation or acetylation of a histone tail). A site-directed modifying polypeptide is also referred to herein as a "site-directed polypeptide" or an "RNA binding site-directed modifying polypeptide." In some cases, the site-directed modifying polypeptide is a naturally-occurring modifying polypeptide. In other cases, the site-directed modifying polypeptide is not a naturally-occurring polypeptide (e.g., a chimeric polypeptide as discussed below or a naturally-occurring polypeptide that is modified, e.g., mutation, deletion, insertion).

Naturally-occurring site-directed modifying polypeptides bind a guide RNA, are thereby directed to a specific sequence within a target DNA, and cleave the target DNA to generate a double strand break. The amino acid sequences of exemplary naturally-occurring Cas9 site-directed modifying polypeptide orthologs are set out in SEQ ID NOs: 1-800. The amino acid sequence of the S. pyrogenes Cas9 endonuclease is set out in SEQ ID NO: 8. A site-directed modifying polypeptide comprises two portions, an RNA-binding portion and an activity portion. In some embodiments, a site-directed modifying polypeptide comprises: (i) an RNA-binding portion that interacts with a guide RNA, wherein the guide RNA comprises a nucleotide sequence that is complementary to a sequence in a target DNA; and (ii) an activity portion that exhibits site-directed enzymatic activity (e.g., activity for DNA methylation, activity for DNA cleavage, activity for histone acetylation, activity for histone methylation, etc.), wherein the site of enzymatic activity is determined by the guide RNA.

In other embodiments, a site-directed modifying polypeptide comprises: (i) an RNA-binding portion that interacts with a guide RNA, wherein the guide RNA comprises a nucleotide sequence that is complementary to a sequence in a target DNA; and (ii) an activity portion that modulates transcription within the target DNA (e.g., to increase or decrease transcription), wherein the site of modulated transcription within the target DNA is determined by the guide RNA.

In some cases, a site-directed modifying polypeptide has enzymatic activity that modifies target DNA (e.g., nuclease activity, methyltransferase activity, demethylase activity, DNA repair activity, DNA damage activity, deamination activity, dismutase activity, alkylation activity, depurination activity, oxidation activity, pyrimidine dimer forming activity, integrase activity, transposase activity, recombinase activity, polymerase activity, ligase activity, helicase activity, photolyase activity or glycosylase activity).

In other cases, a site-directed modifying polypeptide has enzymatic activity that modifies a polypeptide (e.g., a histone) associated with target DNA (e.g., methyltransferase activity, demethylase activity, acetyltransferase activity, deacetylase activity, kinase activity, phosphatase activity, ubiquitin ligase activity, deubiquitinating activity, adenylation activity, deadenylation activity, SUMOylating activity, deSUMOylating activity, ribosylation activity, deribosylation activity, myristoylation activity or demyristoylation activity).

### Exemplary site-directed modifying polypeptides

In some cases, the site-directed modifying polypeptide comprises an amino acid sequence having at least about 75%, at least about 80%, at least about 85%, at least about 90%, at least about 95%, at least about 99%, or 100%, amino acid sequence identity to amino acids 7-166 and/or 731-1003 of SEQ ID NO: 8, or to the corresponding portions in any of the amino acid sequences set forth as SEQ ID NOs: 1-800.

### Nucleic acid modifications

In some embodiments, a nucleic acid (e.g., a guide RNA) comprises one or more modifications, e.g., a base modification, a backbone modification, etc, to provide the nucleic acid with a new or enhanced feature (e.g., improved stability). As is known in the art, a nucleoside is a base-sugar combination. The base portion of the nucleoside is normally a heterocyclic base. The two most common classes of such heterocyclic bases are the purines and the pyrimidines. Nucleotides are nucleosides that further include a phosphate group covalently linked to the sugar portion of the nucleoside. For those nucleosides that include a pentofuranosyl sugar, the phosphate group can be linked to the 2', the 3', or the 5' hydroxyl moiety of the sugar. In forming oligonucleotides, the phosphate groups covalently link adjacent nucleosides to one another to form a linear polymeric compound. In turn, the respective ends of this linear polymeric compound can be further joined to form a circular compound, however, linear compounds are generally suitable. In addition, linear compounds may have internal nucleotide base complementarity and may therefore fold in a manner as to produce a fully or partially double-stranded compound. Within oligonucleotides, the phosphate groups are commonly referred to as forming the internucleoside backbone of the oligonucleotide. The normal linkage or backbone of RNA and DNA is a 3' to 5' phosphodiester linkage.

### Modified backbones and modified internucleoside linkages

Examples of suitable nucleic acids containing modifications include nucleic acids containing modified backbones or non-natural internucleoside linkages. Nucleic acids having modified backbones include those that retain a phosphorus atom in the backbone and those that do not have a phosphorus atom in the backbone.

Suitable modified oligonucleotide backbones containing a phosphorus atom therein include, for example, phosphorothioates, chiral phosphorothioates, phosphorodithioates, phosphotriesters, aminoalkylphosphotriesters, methyl and other alkyl phosphonates including 3'-alkylene phosphonates, 5'-alkylene phosphonates and chiral phosphonates, phosphinates, phosphoramidates including 3¹-amino phosphoramidate and aminoalkylphosphoramidates, phosphorodiamidates, thionophosphoramidates, thionoalkylphosphonates, thionoalkylphosphotriesters, selenophosphates and boranophosphates having normal 3'-5' linkages, 2'-5' linked analogs of these, and those having inverted polarity wherein one or more internucleotide linkages is a 3' to 3', 5' to 5' or 2' to 2' linkage. Suitable oligonucleotides having inverted polarity comprise a single 3' to 3' linkage at the 3'-most internucleotide linkage i.e. a single inverted nucleoside residue which may be a basic (the nucleobase is missing or has a hydroxyl group in place thereof). Various salts (such as, for example, potassium or sodium), mixed salts and free acid forms are also included.

In some embodiments, a nucleic acid comprises one or more phosphorothioate and/or heteroatom internucleoside linkages, in particular -CH₂-NH-O-CH₂-, -CH₂-N(CH₃)-O-CH₂- (known as a methylene (methylimino) or MMI backbone), -CH₂-O-N(CH₃)-CH₂-, -CH₂-N(CH₃)-N(CH₃)-CH₂- and -ON(CH₃)-CH₂-CH₂- (wherein the native phosphodiester intemucleotide linkage is represented as -OP(=O)(OH)-O-CH₂-). MMI type internucleoside linkages are disclosed in the above referenced U.S. Pat. No. 5,489,677. Suitable amide internucleoside linkages are disclosed in t U.S. Pat. No. 5,602,240.

Also suitable are nucleic acids having morpholino backbone structures as described in, e.g., U.S. Pat. No. 5,034,506. For example, in some embodiments, a nucleic acid comprises a 6-membered morpholino ring in place of a ribose ring. In some of these embodiments, a phosphorodiamidate or other non-phosphodiester internucleoside linkage replaces a phosphodiester linkage.

Suitable modified polynucleotide backbones that do not include a phosphorus atom therein have backbones that are formed by short chain alkyl or cycloalkyl internucleoside linkages, mixed heteroatom and alkyl or cycloalkyl intemucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; riboacetyl backbones; alkene containing backbones; sulfamate backbones; methyleneimino and methylenehydrazino backbones; sulfonate and sulfonamide backbones; amide backbones; and others having mixed N, 0, S and CH₂ component parts.

### Mimetics

A nucleic acid can be a nucleic acid mimetic. The term "mimetic" as it is applied to polynucleotides is intended to include polynucleotides wherein only the furanose ring or both the furanose ring and the internucleotide linkage are replaced with non-furanose groups, replacement of only the furanose ring is also referred to in the art as being a sugar surrogate. The heterocyclic base moiety or a modified heterocyclic base moiety is maintained for hybridization with an appropriate target nucleic acid. One such nucleic acid, a polynucleotide mimetic that has been shown to have excellent hybridization properties, is referred to as a peptide nucleic acid (PNA). In PNA, the sugar-backbone of a polynucleotide is replaced with an amide containing backbone, in particular an aminoethylglycine backbone. The nucleotides are retained and are bound directly or indirectly to aza nitrogen atoms of the amide portion of the backbone.

One polynucleotide mimetic that has been reported to have excellent hybridization properties is a peptide nucleic acid (PNA). The backbone in PNA compounds is two or more linked aminoethylglycine units which gives PNA an amide containing backbone. The heterocyclic base moieties are bound directly or indirectly to aza nitrogen atoms of the amide portion of the backbone. Representative U.S. patents that describe the preparation of PNA compounds include, but are not limited to: U.S. Pat. Nos. 5,539,082; 5,714,331; and 5,719,262.

Another class of polynucleotide mimetic that has been studied is based on linked morpholino units (morpholino nucleic acid) having heterocyclic bases attached to the morpholino ring. A number of linking groups have been reported that link the morpholino monomeric units in a morpholino nucleic acid. One class of linking groups has been selected to give a non-ionic oligomeric compound. The non-ionic morpholino-based oligomeric compounds are less likely to have undesired interactions with cellular proteins. Morpholino-based polynucleotides are nonionic mimics of oligonucleotides which are less likely to form undesired interactions with cellular proteins (Dwaine A. Braasch and David R. Corey, Biochemistry, 2002, 41(14), 45034510). Morpholino-based polynucleotides are disclosed in U.S. Pat. No. 5,034,506. A variety of compounds within the morpholino class of polynucleotides have been prepared, having a variety of different linking groups joining the monomeric subunits.

A further class of polynucleotide mimetic is referred to as cyclohexenyl nucleic acids (CeNA). The furanose ring normally present in a DNA/RNA molecule is replaced with a cyclohexenyl ring. CeNA DMT protected phosphoramidite monomers have been prepared and used for oligomeric compound synthesis following classical phosphoramidite chemistry. Fully modified CeNA oligomeric compounds and oligonucleotides having specific positions modified with CeNA have been prepared and studied (see Wang et al., J. Am. Chem. Soc., 2000, 122, 85958602). In general the incorporation of CeNA monomers into a DNA chain increases its stability of a DNA/RNA hybrid. CeNA oligoadenylates formed complexes with RNA and DNA complements with similar stability to the native complexes. The study of incorporating CeNA structures into natural nucleic acid structures was shown by NMR and circular dichroism to proceed with easy conformational adaptation.

A further modification includes Locked Nucleic Acids (LNAs) in which the 2'-hydroxyl group is linked to the 4' carbon atom of the sugar ring thereby forming a 2'-C,4'-C-oxymethylene linkage thereby forming a bicyclic sugar moiety. The linkage can be a methylene (-CH₂-), group bridging the 2' oxygen atom and the 4' carbon atom wherein n is 1 or 2 (Singh et al., Chem. Commun., 1998, 4, 455-456). LNA and LNA analogs display very high duplex thermal stabilities with complementary DNA and RNA (Tm=+3 to +10° C), stability towards 3'-exonucleolytic degradation and good solubility properties. Potent and nontoxic antisense oligonucleotides containing LNAs have been described (Wahlestedt et al., Proc. Natl. Acad. Sci. U.S.A., 2000, 97, 5633-5638).

The synthesis and preparation of the LNA monomers adenine, cytosine, guanine, 5-methylcytosine, thymine and uracil, along with their oligomerization, and nucleic acid recognition properties have been described (Koshkin et al., Tetrahedron, 1998, 54, 3607-3630). LNAs and preparation thereof are also described in WO 98/39352 and WO 99/14226.

### Modified sugar moieties

A nucleic acid can also include one or more substituted sugar moieties. Suitable polynucleotides comprise a sugar substituent group selected from: OH; F; O-, S-, or N-alkyl; O-, S-, or N-alkenyl; O-, S- or N-alkynyl; or O-alkyl-O-alkyl, wherein the alkyl, alkenyl and alkynyl may be substituted or unsubstituted C.sub.1 to C₁₀ alkyl or C₂ to C₁₀ alkenyl and alkynyl. Particularly suitable are O((CH₂)ₙO)ₘCH₃, O(CH₂)ₙOCH₃, O(CH₂)ₙNH₂, O(CH₂)CH₃, O(CH₂)ₙONH₂, and O(CH₂)ₙON((CH₂)ₙCH₃)₂, where n and m are from 1 to about 10. Other suitable polynucleotides comprise a sugar substituent group selected from: C₁ to C₁₀ lower alkyl, substituted lower alkyl, alkenyl, alkynyl, alkaryl, aralkyl, O-alkaryl or O-aralkyl, SH, SCH₃, OCN, Cl, Br, CN, CF₃, OCF₃, SOCH₃, SO₂CH₃, ONO₂, NO₂, N₃, NH₂, heterocycloalkyl, heterocycloalkaryl, aminoalkylamino, polyalkylamino, substituted silyl, an RNA cleaving group, a reporter group, an intercalator, a group for improving the pharmacokinetic properties of an oligonucleotide, or a group for improving the pharmacodynamic properties of an oligonucleotide, and other substituents having similar properties. A suitable modification includes 2'-methoxyethoxy 2'-O-CH₂ CH₂OCH₃, also known as -2'-O-(2-methoxyethyl) or 2'-MOE) (Martin et al., Hely. Chim. Acta, 1995, 78, 486-504) i.e., an alkoxyalkoxy group. A further suitable modification includes 2'-dimethylaminooxyethoxy, i.e., a O(CH₂)₂ON(CH₃)₂ group, also known as 2'-DMAOE, as described in examples hereinbelow, and 2'-dimethylaminoethoxyethoxy (also known in the art as 2'-O-dimethyl-amino-ethoxy-ethyl or 2'-DMAEOE), i.e., 2'-O-CH₂-O-CH₂-N(CH₃)₂.

Other suitable sugar substituent groups include methoxy (-O-CH₃), aminopropoxy (--O-CH₂CH₂ CH₂NH₂), allyl (-CH₂-CH=CH₂), -O-allyl (--O--CH₂-CH=CH₂) and fluoro (F). 2'-sugar substituent groups may be in the arabino (up) position or ribo (down) position. A suitable 2'-arabino modification is 2'-F. Similar modifications may also be made at other positions on the oligomeric compound, particularly the 3' position of the sugar on the 3' terminal nucleoside or in 2'-5' linked oligonucleotides and the 5' position of 5' terminal nucleotide. Oligomeric compounds may also have sugar mimetics such as cyclobutyl moieties in place of the pentofuranosyl sugar.

### Base modifications and substitutions

A nucleic acid may also include nucleobase (often referred to in the art simply as "base") modifications or substitutions. As used herein, "unmodified" or "natural" nucleobases include the purine bases adenine (A) and guanine (G), and the pyrimidine bases thymine (T), cytosine (C) and uracil (U). Modified nucleobases include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl (-C=C-CH₃) uracil and cytosine and other alkynyl derivatives of pyrimidine bases, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 2-F-adenine, 2-amino-adenine, 8-azaguanine and 8-azaadenine, 7-deazaguanine and 7-deazaadenine and 3-deazaguanine and 3-deazaadenine. Further modified nucleobases include tricyclic pyrimidines such as phenoxazine cytidine(1H-pyrimido(5,4-b)(1,4)benzoxazin-2(3H)-one), phenothiazine cytidine (1H-pyrimido(5,4-b)(1,4)benzothiazin-2(3H)-one), G-clamps such as a substituted phenoxazine cytidine (e.g. 9-(2-aminoethoxy)-H-pyrimido(5,4-(b) (1,4)benzoxazin-2(3H)-one), carbazole cytidine (2H-pyrimido(4,5-b)indol-2-one), pyridoindole cytidine (H-pyrido(3',2':4,5)pyrrolo(2,3-d)pyrimidin-2-one).

Heterocyclic base moieties may also include those in which the purine or pyrimidine base is replaced with other heterocycles, for example 7-deaza-adenine, 7-deazaguanosine, 2-aminopyridine and 2-pyridone. Further nucleobases include those disclosed in U.S. Pat. No. 3,687,808, those disclosed in The Concise Encyclopedia Of Polymer Science And Engineering, pages 858-859, Kroschwitz, J. I., ed. John Wiley & Sons, 1990, those disclosed by Englisch et al., Angewandte Chemie, International Edition, 1991, 30, 613, and those disclosed by Sanghvi, Y. S., Chapter 15, Antisense Research and Applications, pages 289-302, Crooke, S. T. and Lebleu, B., ed., CRC Press, 1993. Certain of these nucleobases are useful for increasing the binding affinity of an oligomeric compound. These include 5-substituted pyrimidines, 6-azapyrimidines and N-2, N-6 and O-6 substituted purines, including 2-aminopropyladenine, 5-propynyluracil and 5-propynylcytosine. 5-methylcytosine substitutions have been shown to increase nucleic acid duplex stability by 0.6-1.2° C. (Sanghvi et al., eds., Antisense Research and Applications, CRC Press, Boca Raton, 1993, pp. 276-278) and are suitable base substitutions, e.g., when combined with 2'-O-methoxyethyl sugar modifications.

"Complementary" refers to the capacity for pairing, through base stacking and specific hydrogen bonding, between two sequences comprising naturally or non-naturally occurring (e.g., modified as described above) bases (nucleosides) or analogs thereof. For example, if a base at one position of a nucleic acid is capable of hydrogen bonding with a base at the corresponding position of a target, then the bases are considered to be complementary to each other at that position. Nucleic acids can comprise universal bases, or inert abasic spacers that provide no positive or negative contribution to hydrogen bonding. Base pairings may include both canonical Watson-Crick base pairing and non-Watson-Crick base pairing (e.g., Wobble base pairing and Hoogsteen base pairing). It is understood that for complementary base pairings, adenosine-type bases (A) are complementary to thymidine-type bases (T) or uracil-type bases (U), that cytosine-type bases (C) are complementary to guanosine-type bases (G), and that universal bases such as such as 3-nitropyrrole or 5-nitroindole can hybridize to and are considered complementary to any A, C, U, or T. Nichols et al., Nature, 1994;369:492-493 and Loakes et al., Nucleic Acids Res., 1994;22:4039-4043. Inosine (I) has also been considered in the art to be a universal base and is considered complementary to any A, C, U, or T. See Watkins and SantaLucia, Nucl. Acids Research, 2005; 33 (19): 6258-6267.

### Conjugates

Another possible modification of a nucleic acid involves chemically linking to the polynucleotide one or more moieties or conjugates which enhance the activity, cellular distribution or cellular uptake of the oligonucleotide. These moieties or conjugates can include conjugate groups covalently bound to functional groups such as primary or secondary hydroxyl groups. Conjugate groups include, but are not limited to, intercalators, reporter molecules, polyamines, polyamides, polyethylene glycols, polyethers, groups that enhance the pharmacodynamic properties of oligomers, and groups that enhance the pharmacokinetic properties of oligomers. Suitable conjugate groups include, but are not limited to, cholesterols, lipids, phospholipids, biotin, phenazine, folate, phenanthridine, anthraquinone, acridine, fluoresceins, rhodamines, coumarins, and dyes. Groups that enhance the pharmacodynamic properties include groups that improve uptake, enhance resistance to degradation, and/or strengthen sequence-specific hybridization with the target nucleic acid. Groups that enhance the pharmacokinetic properties include groups that improve uptake, distribution, metabolism or excretion of a nucleic acid.

Conjugate moieties include but are not limited to lipid moieties such as a cholesterol moiety (Letsinger et al., Proc. Natl. Acad. Sci. USA, 1989, 86, 6553-6556), cholic acid (Manoharan et al., Bioorg. Med. Chem. Let., 1994, 4, 1053-1060), a thioether, e.g., hexyl-S-tritylthiol (Manoharan et al., Ann. N.Y. Acad. Sci., 1992, 660, 306-309; Manoharan et al., Bioorg. Med. Chem. Let., 1993, 3, 2765-2770), a thiocholesterol (Oberhauser et al., Nucl. Acids Res., 1992, 20, 533-538), an aliphatic chain, e.g., dodecandiol or undecyl residues (Saison-Behmoaras et al., EMBO J., 1991, 10, 1111-1118; Kabanov et al., FEBS Lett., 1990, 259, 327-330; Svinarchuk et al., Biochimie, 1993, 75, 49-54), a phospholipid, e.g., di-hexadecyl-rac-glycerol or triethylammonium 1,2-di-O-hexadecyl-rac-glycero-3-H-phosphonate (Manoharan et al., Tetrahedron Lett., 1995, 36, 3651-3654; Shea et al., Nucl. Acids Res., 1990, 18, 3777-3783), a polyamine or a polyethylene glycol chain (Manoharan et al., Nucleosides & Nucleotides, 1995, 14, 969-973), or adamantane acetic acid (Manoharan et al., Tetrahedron Lett., 1995, 36, 36513654), a palmityl moiety (Mishra et al., Biochim. Biophys. Acta, 1995, 1264, 229-237), or an octadecylamine or hexylamino-carbonyl-oxycholesterol moiety (Crooke et al., J. Pharmacol. Exp. Ther., 1996, 277, 923-937.\

A conjugate may include a "Protein Transduction Domain" or PTD (also known as a CPP - cell penetrating peptide), which may refer to a polypeptide, polynucleotide, carbohydrate, or organic or inorganic compound that facilitates traversing a lipid bilayer, micelle, cell membrane, organelle membrane, or vesicle membrane. A PTD attached to another molecule, which can range from a small polar molecule to a large macromolecule and/or a nanoparticle, facilitates the molecule traversing a membrane, for example going from extracellular space to intracellular space, or cytosol to within an organelle. In some embodiments, a PTD is covalently linked to the amino terminus of an exogenous polypeptide (e.g., a site-directed modifying polypeptide). In some embodiments, a PTD is covalently linked to the carboxyl terminus of an exogenous polypeptide (e.g., a site-directed modifying polypeptide). In some embodiments, a PTD is covalently linked to a nucleic acid (e.g., a guide RNA, a polynucleotide encoding a guide RNA, a polynucleotide encoding a site-directed modifying polypeptide, etc.). Exemplary PTDs include but are not limited to a minimal undecapeptide protein transduction domain (corresponding to residues 47-57 of HIV-1 TAT comprising YGRKKRRQRRR; a polyarginine sequence comprising a number of arginines sufficient to direct entry into a cell (e.g., 3, 4, 5, 6, 7, 8, 9, 10, or 10-50 arginines); a VP22 domain (Zender et al. (2002) Cancer Gene Ther. 9(6):489-96); an Drosophila Antennapedia protein transduction domain (Noguchi et al. (2003) Diabetes 52(7):1732-1737); a truncated human calcitonin peptide (Trehin et al. (2004) Pharm. Research 21:1248-1256); polylysine (Wender et al. (2000) Proc. Natl. Acad. Sci. USA 97:13003-13008); RRQRRTSKLMKR; Transport= GWTLNSAGYLLGKINLKALAALAKKIL; KALAWEAKLAKALAKALAKHLAKALAKALKCEA; and RQIKIWFQNRRMKWKK. Exemplary PTDs include but are not limited to, YGRKKRRQRRR; RKKRRQRRR; an arginine homopolymer of from 3 arginine residues to 50 arginine residues; Exemplary PTD domain amino acid sequences include, but are not limited to, any of the following: YGRKKRRQRRR; RKKRRQRR; YARAAARQARA; THRLPRRRRRR; and GGRRARRRRRR. In some embodiments, the PTD is an activatable CPP (ACPP) (Aguilera et al. (2009) Integr Biol (Camb) June; 1(5-6): 371-381). ACPPs comprise a polycationic CPP (e.g., Arg9 or "R9") connected via a cleavable linker to a matching polyanion (e.g., Glu9 or "E9"), which reduces the net charge to nearly zero and thereby inhibits adhesion and uptake into cells. Upon cleavage of the linker, the polyanion is released, locally unmasking the polyarginine and its inherent adhesiveness, thus "activating" the ACPP to traverse the membrane.

### Exemplary guide RNAs

In some embodiments, a guide RNA comprises two separate RNA polynucleotide molecules. The first of the two separate RNA polynucleotide molecules (the activator-RNA) comprises a nucleotide sequence having at least about 60%, at least about 65%, at least about 70%, at least about 75%, at least about 80%, at least about 85%, at least about 90%, at least about 95%, at least about 98%, at least about 99%, or 100% nucleotide sequence identity over a stretch of at least 8 contiguous, at least 9 contiguous, at least 10 contiguous, at least 11 contiguous, at least 12 contiguous, at least 13 contiguous, at least 14 contiguous or at least 15 contiguous nucleotides to any one of the tracrRNA nucleotide sequences set forth in Supplementary Table S5, or complements thereof. The second of the two separate RNA polynucleotide molecules (the targeter-RNA) comprises a nucleotide sequence having at least about 60%, at least about 65%, at least about 70%, at least about 75%, at least about 80%, at least about 85%, at least about 90%, at least about 95%, at least about 98%, at least about 99%, or 100% nucleotide sequence identity over a stretch of at least 8 contiguous, at least 9 contiguous, at least 10 contiguous, at least 11 contiguous, at least 12 contiguous, at least 13 contiguous, at least 14 contiguous or at least 15 contiguous nucleotides to the cognate CRISPR repeat nucleotide sequence set forth in Supplementary Table S5, or complements thereof. In some embodiments, a suitable guide RNA is a single-molecule RNA polynucleotide and comprises a first nucleotide sequence having at least about 60%, at least about 65%, at least about 70%, at least about 75%, at least about 80%, at least about 85%, at least about 90%, at least about 95%, at least about 98%, at least about 99%, or 100% nucleotide sequence identity over a stretch of at least 8 contiguous, at least 9 contiguous, at least 10 contiguous, at least 11 contiguous, at least 12 contiguous, at least 13 contiguous, at least 14 contiguous or at least 15 contiguous nucleotides to any one of the tracrRNA nucleotide sequences set forth in Supplementary Table S5 and a second nucleotide sequence having at least about 60%, at least about 65%, at least about 70%, at least about 75%, at least about 80%, at least about 85%, at least about 90%, at least about 95%, at least about 98%, at least about 99%, or 100% nucleotide sequence identity over a stretch of at least 8 contiguous, at least 9 contiguous, at least 10 contiguous, at least 11 contiguous, at least 12 contiguous, at least 13 contiguous, at least 14 contiguous or at least 15contiguous nucleotides to the cognate CRISPR repeat nucleotide sequence set forth in Supplementary Table S5, or complements thereof.

In some embodiments, the single-molecule guide RNAs comprise a DNA-targeting segment and a protein-binding segment complementary thereto, wherein the protein-binding segment comprises a tracrRNA set out in Supplementary Table S5 or wherein the protein-binding segment comprises a tracrRNA at least 80% identical over at least 20 nucleotides to a tracrRNA set out in Supplementary Table S5, or at least about 60%, at least about 65%, at least about 70%, at least about 75%, at least about 80%, at least about 85%, at least about 90%, at least about 95%, at least about 98%, at least about 99%, or 100% nucleotide sequence identity over a stretch of at least 8 contiguous, at least 9 contiguous, at least 10 contiguous, at least 11 contiguous, at least 12 contiguous, at least 13 contiguous, at least 14 contiguous or at least 15 contiguous nucleotides of any one of the tracrRNA nucleotide sequences set forth in Supplementary Table S5. For example, the protein-binding segment may comprise a tracrRNA at least 70% identical over at least 10 contiguous nucleotides, at least 80% identical over at least 10 contiguous nucleotides, at least 70% identical over at least 11 contiguous nucleotides, at least 80% identical over at least 11 contiguous nucleotides, at least 70% identical over at least 12 contiguous nucleotides, or at least 80% identical over at least 12 contiguous nucleotides.

In some embodiments, the single-molecule guide RNAs comprise a DNA-targeting segment and a protein-binding segment, wherein the protein-binding segment comprises a tracrRNA set out in Supplementary Table S5 or wherein the protein-binding segment comprises a tracrRNA at least 80% identical over at least 20 nucleotides to a tracrRNA set out in Supplementary Table S5. In some embodiments, the protein-binding segment comprises a CRISPR repeat set out in Supplementary Table S5 that is the CRISPR repeat cognate to the tracrRNA of the protein-binding segment. In some embodiments, the DNA-targeting segment comprises RNA complementary to a protospacer-like sequence in a target DNA 5' to a PAM sequence. In some embodiments, the tracrRNA and CRISPR repeat are respectively the C. jejuni tracrRNA and its cognate CRISPR repeat set out in Supplementary Table S5 and the PAM sequence is NNNNACA. In some embodiments, the tracrRNA and CRISPR repeat are respectively at least 80% identical to the C. jejuni tracrRNA and its cognate CRISPR repeat set out in Supplementary Table S5 and the PAM sequence is NNNNACA. In some embodiments, the single-molecule guide RNA comprises a sequence that hybridizes to a protospacer-like sequence set out in one of SEQ ID NOs: 801-2701.

In some embodiments, the double-molecule guide RNAs comprise a targeter-RNA and an activator-RNA complementary thereto, wherein the activator-RNA comprises a tracrRNA set out in Supplementary Table S5 or wherein the activator-RNA comprises a tracrRNA at least 80% identical over at least 20 nucleotides to a tracrRNA set out in Supplementary Table S5. In some embodiments, the double-molecule guide RNA comprises a modified backbone, a non-natural internucleoside linkage, a nucleic acid mimetic, a modified sugar moiety, a base modification, a modification or sequence that provides for modified or regulated stability, a modification or sequence that provides for subcellular tracking, a modification or sequence that provides for tracking, or a modification or sequence that provides for a binding site for a protein or protein complex. In some embodiments, the targeter-RNA comprises a CRISPR repeat set out in Supplementary Table S5. In some embodiments, the targeter-RNA comprises a CRISPR repeat set out in Supplementary Table S5 that is the cognate CRISPR repeat of the tracrRNA of the activator-RNA. In some embodiments, the targeter-RNA further comprises RNA complementary to a protospacer-like sequence in a target DNA 5' to a PAM sequence. In some embodiments, the tracrRNA and CRISPR repeat are respectively the C. jejuni tracrRNA and its cognate CRISPR repeat set out in Supplementary Table S5 and the PAM sequence is NNNNACA. In some embodiments, the tracrRNA and CRISPR repeat are at least 80% identical to respectively the C. jejuni tracrRNA and its cognate CRISPR repeat set out in Supplementary Table S5 and the PAM sequence is NNNNACA. In some embodiments, the double-molecule guide RNA comprises a sequence that hybridizes to a protospacer-like sequence set out in one of SEQ ID NOs: 801-2701.

### Nucleic acids encoding a guide RNA and/or a site-directed modifying polypeptide

The present disclosure provides a nucleic acid comprising a nucleotide sequence encoding a guide RNA and/or a site-directed modifying polypeptide. In some embodiments, a guide RNA-encoding nucleic acid is an expression vector, e.g., a recombinant expression vector.

In some embodiments, a method involves contacting a target DNA or introducing into a cell (or a population of cells) one or more nucleic acids comprising nucleotide sequences encoding a guide RNA and/or a site-directed modifying polypeptide. In some embodiments a cell comprising a target DNA is *in vitro.* In some embodiments a cell comprising a target DNA is *in vivo.* Suitable nucleic acids comprising nucleotide sequences encoding a guide RNA and/or a site-directed modifying polypeptide include expression vectors, where an expression vector comprising a nucleotide sequence encoding a guide RNA and/or a site-directed modifying polypeptide is a "recombinant expression vector."

In some embodiments, the recombinant expression vector is a viral construct, e.g., a recombinant adeno-associated virus construct (see, e.g., U.S. Patent No. 7,078,387), a recombinant adenoviral construct, a recombinant lentiviral construct, a recombinant retroviral construct, etc.

Suitable expression vectors include, but are not limited to, viral vectors (e.g. viral vectors based on vaccinia virus; poliovirus; adenovirus (see, e.g., Li et al., Invest Opthalmol Vis Sci 35:2543 2549, 1994; Borras et al., Gene Ther 6:515 524, 1999; Li and Davidson, PNAS 92:7700 7704, 1995; Sakamoto et al., H Gene Ther 5:1088 1097, 1999; WO 94/12649, WO 93/03769; WO 93/19191; WO 94/28938; WO 95/11984 and WO 95/00655); adeno-associated virus (see, e.g., Ali et al., Hum Gene Ther 9:81 86, 1998, Flannery et al., PNAS 94:6916 6921, 1997; Bennett et al., Invest Opthalmol Vis Sci 38:2857 2863, 1997; Jomary et al., Gene Ther 4:683 690, 1997, Rolling et al., Hum Gene Ther 10:641 648, 1999; Ali et al., Hum Mol Genet 5:591 594, 1996; Srivastava in WO 93/09239, Samulski et al., J. Vir. (1989) 63:3822-3828; Mendelson et al., Virol. (1988) 166:154-165; and Flotte et al., PNAS (1993) 90:10613-10617); SV40; herpes simplex virus; human immunodeficiency virus (see, e.g., Miyoshi et al., PNAS 94:10319 23, 1997; Takahashi et al., J Virol 73:7812 7816, 1999); a retroviral vector (e.g., Murine Leukemia Virus, spleen necrosis virus, and vectors derived from retroviruses such as Rous Sarcoma Virus, Harvey Sarcoma Virus, avian leukosis virus, a lentivirus, human immunodeficiency virus, myeloproliferative sarcoma virus, and mammary tumor virus); and the like.

Numerous suitable expression vectors are known to those of skill in the art, and many are commercially available. The following vectors are provided by way of example; for eukaryotic host cells: pXT1, pSG5 (Stratagene), pSVK3, pBPV, pMSG, and pSVLSV40 (Pharmacia). However, any other vector may be used so long as it is compatible with the host cell. Depending on the host/vector system utilized, any of a number of suitable transcription and translation control elements, including constitutive and inducible promoters, transcription enhancer elements, transcription terminators, etc. may be used in the expression vector (see e.g., Bitter et al. (1987) Methods in Enzymology, 153:516-544).

In some embodiments, a nucleotide sequence encoding a guide RNA and/or a site-directed modifying polypeptide is operably linked to a control element, e.g., a transcriptional control element, such as a promoter. The transcriptional control element may be functional in either a eukaryotic cell, e.g., a mammalian cell; or a prokaryotic cell (e.g., bacterial or archaeal cell). In some embodiments, a nucleotide sequence encoding a guide RNA and/or a site-directed modifying polypeptide is operably linked to multiple control elements that allow expression of the nucleotide sequence encoding a guide RNA and/or a site-directed modifying polypeptide in both prokaryotic and eukaryotic cells.

Non-limiting examples of suitable eukaryotic promoters (promoters functional in a eukaryotic cell) include those from cytomegalovirus (CMV) immediate early, herpes simplex virus (HSV) thymidine kinase, early and late SV40, long terminal repeats (LTRs) from retrovirus, and mouse metallothionein-I. Selection of the appropriate vector and promoter is well within the level of ordinary skill in the art. The expression vector may also contain a ribosome binding site for translation initiation and a transcription terminator. The expression vector may also include appropriate sequences for amplifying expression. The expression vector may also include nucleotide sequences encoding protein tags (e.g., 6xHis tag, hemagglutinin tag, green fluorescent protein, etc.) that are fused to the site-directed modifying polypeptide, thus resulting in a chimeric polypeptide.

In some embodiments, a nucleotide sequence encoding a guide RNA and/or a site-directed modifying polypeptide is operably linked to an inducible promoter. In some embodiments, a nucleotide sequence encoding a guide RNA and/or a site-directed modifying polypeptide is operably linked to a constitutive promoter.

Methods of introducing a nucleic acid into a host cell are known in the art, and any known method can be used to introduce a nucleic acid (e.g., an expression construct) into a cell. Suitable methods include e.g., viral or bacteriophage infection, transfection, conjugation, protoplast fusion, lipofection, electroporation, calcium phosphate precipitation, polyethyleneimine (PEI)-mediated transfection, DEAE-dextran mediated transfection, liposome-mediated transfection, particle gun technology, calcium phosphate precipitation, direct micro injection, nanoparticle-mediated nucleic acid delivery (see, e.g., Panyam et., al Adv Drug Deliv Rev. 2012 Sep 13. pii: S0169-409X(12)00283-9. doi: 10.1016/j.addr.2012.09.023), and the like.

### Chimeric Polypeptides

The present disclosure provides a chimeric site-directed modifying polypeptide. A chimeric site-directed modifying polypeptide interacts with (e.g., binds to) a guide RNA (described above). The guide RNA guides the chimeric site-directed modifying polypeptide to a target sequence within target DNA (e.g. a chromosomal sequence or an extrachromosomal sequence, e.g. an episomal sequence, a minicircle sequence, a mitochondrial sequence, a chloroplast sequence, etc.). A chimeric site-directed modifying polypeptide modifies target DNA (e.g., cleavage or methylation of target DNA) and/or a polypeptide associated with target DNA (e.g., methylation or acetylation of a histone tail).

A chimeric site-directed modifying polypeptide modifies target DNA (e.g., cleavage or methylation of target DNA) and/or a polypeptide associated with target DNA (e.g., methylation or acetylation of a histone tail). A chimeric site-directed modifying polypeptide is also referred to herein as a "chimeric site-directed polypeptide" or a "chimeric RNA binding site-directed modifying polypeptide."

A chimeric site-directed modifying polypeptide comprises two portions, an RNA-binding portion and an activity portion. A chimeric site-directed modifying polypeptide comprises amino acid sequences that are derived from at least two different polypeptides. A chimeric site-directed modifying polypeptide can comprise modified and/or naturally-occurring polypeptide sequences (e.g., a first amino acid sequence from a modified or unmodified Cas9 protein; and a second amino acid sequence other than the Cas9 protein).

### RNA-binding portion

In some cases, the RNA-binding portion of a chimeric site-directed modifying polypeptide is a naturally-occurring polypeptide. In other cases, the RNA-binding portion of a chimeric site-directed modifying polypeptide is not a naturally-occurring molecule (modified, e.g., mutation, deletion, insertion). Naturally-occurring RNA-binding portions of interest are derived from site-directed modifying polypeptides known in the art. For example, SEQ ID NOs: 1-800 provide a non-limiting set of naturally occurring Cas9 endonucleases that can be used as site-directed modifying polypeptides. In some cases, the RNA-binding portion of a chimeric site-directed modifying polypeptide comprises an amino acid sequence having at least about 75%, at least about 80%, at least about 85%, at least about 90%, at least about 95%, at least about 98%, at least about 99%, or 100%, amino acid sequence identity to the RNA-binding portion of a polypeptide set forth in SEQ ID NOs: 1-800.

In some cases, the site-directed modifying polypeptide comprises an amino acid sequence having at least about 75%, at least about 80%, at least about 85%, at least about 90%, at least about 95%, at least about 99%, or 100%, amino acid sequence identity to amino acids 7-166 and/or 731-1003 of SEQ ID NO: 8, or to the corresponding portions in any of the amino acid sequences set forth as SEQ ID NOs: 1-800.

### Activity portion

In addition to the RNA-binding portion, the chimeric site-directed modifying polypeptide comprises an "activity portion." In some embodiments, the activity portion of a chimeric site-directed modifying polypeptide comprises the naturally-occurring activity portion of a site-directed modifying polypeptide (e.g., Cas9 endonuclease). In other embodiments, the activity portion of a subject chimeric site-directed modifying polypeptide comprises a modified amino acid sequence (e.g., substitution, deletion, insertion) of a naturally-occurring activity portion of a site-directed modifying polypeptide. Naturally-occurring activity portions of interest are derived from site-directed modifying polypeptides known in the art. For example, SEQ ID NOs: 1-800 are a non-limiting set of naturally occurring Cas9 endonucleases that can be used as site-directed modifying polypeptides. The activity portion of a chimeric site-directed modifying polypeptide is variable and may comprise any heterologous polypeptide sequence that may be useful in the methods disclosed herein. In some embodiments, the activity portion of a site-directed modifying polypeptide comprises a portion of a Cas9 ortholog (including, but not limited to, the Cas9 orthologs set out in one of SEQ ID NOs: 1-800) that is at least 90% identical to amino acids 7-166 of SEQ ID NO: 8 and/or at least 90% identical to amino acids 731-1003 of SEQ ID NO: 8. In some embodiments, a chimeric site-directed modifying polypeptide comprises: (i) an RNA-binding portion that interacts with a guide RNA, wherein the guide RNA comprises a nucleotide sequence that is complementary to a sequence in a target DNA; and (ii) an activity portion that exhibits site-directed enzymatic activity (e.g., activity for DNA methylation, activity for DNA cleavage, activity for histone acetylation, activity for histone methylation, etc.), wherein the site of enzymatic activity is determined by the guide RNA.

In other embodiments, a chimeric site-directed modifying polypeptide comprises: (i) an RNA-binding portion that interacts with a guide RNA, wherein the guide RNA comprises a nucleotide sequence that is complementary to a sequence in a target DNA; and (ii) an activity portion that modulates transcription within the target DNA (e.g., to increase or decrease transcription), wherein the site of modulated transcription within the target DNA is determined by the guide RNA.

In some cases, the activity portion of a chimeric site-directed modifying polypeptide has enzymatic activity that modifies target DNA (e.g., nuclease activity, methyltransferase activity, demethylase activity, DNA repair activity, DNA damage activity, deamination activity, dismutase activity, alkylation activity, depurination activity, oxidation activity, pyrimidine dimer forming activity, integrase activity, transposase activity, recombinase activity, polymerase activity, ligase activity, helicase activity, photolyase activity or glycosylase activity).

In other cases, the activity portion of a chimeric site-directed modifying polypeptide has enzymatic activity (e.g., methyltransferase activity, demethylase activity, acetyltransferase activity, deacetylase activity, kinase activity, phosphatase activity, ubiquitin ligase activity, deubiquitinating activity, adenylation activity, deadenylation activity, SUMOylating activity, deSUMOylating activity, ribosylation activity, deribosylation activity, myristoylation activity or demyristoylation activity) that modifies a polypeptide associated with target DNA (e.g., a histone).

In some cases, the activity portion of a chimeric site-directed modifying polypeptide exhibits enzymatic activity (described above). In other cases, the activity portion of a chimeric site-directed modifying polypeptide modulates transcription of the target DNA (described above). The activity portion of a chimeric site-directed modifying polypeptide is variable and may comprise any heterologous polypeptide sequence that may be useful in the methods disclosed herein.

### Exemplary chimeric site-directed modifying polypeptides

In some embodiments, the activity portion of the chimeric site-directed modifying polypeptide comprises a modified form of the Cas9 protein, including modified forms of any of the Cas9 orthologs described herein, such as SEQ ID NOs: 1-800). In some instances, the modified form of the Cas9 protein comprises an amino acid change (e.g., deletion, insertion, or substitution) that reduces the naturally-occurring nuclease activity of the Cas9 protein. For example, in some instances, the modified form of the Cas9 protein has less than 50%, less than 40%, less than 30%, less than 20%, less than 10%, less than 5%, or less than 1% of the nuclease activity of the corresponding wild-type Cas9 polypeptide. In some cases, the modified form of the Cas9 polypeptide has no substantial nuclease activity.

In some embodiments, the modified form of the Cas9 polypeptide is a D10A (aspartate to alanine at amino acid position 10 of SEQ ID NO:8) mutation (or the corresponding mutation of any of the proteins presented in SEQ ID NOs: 1-800) that can cleave the complementary strand of the target DNA but has reduced ability to cleave the non-complementary strand of the target DNA. In some embodiments, the modified form of the SEQ ID NO: 8 Cas9 polypeptide is a H840A (histidine to alanine at amino acid position 840) mutation (or the corresponding mutation of any of the proteins set forth as SEQ ID NOs: 1-800) that can cleave the non-complementary strand of the target DNA but has reduced ability to cleave the complementary strand of the target DNA. In some embodiments, the modified form of the SEQ ID NO: 8 Cas9 polypeptide harbors both the D10A and the H840A mutations (or the corresponding mutations of any of the proteins set forth as SEQ ID NOs: 1-800) such that the polypeptide has a reduced ability to cleave both the complementary and the non-complementary strands of the target DNA. Other residues can be mutated to achieve the above effects (i.e. inactivate one or the other nuclease portions). As non-limiting examples, S. pyogenes Cas9 residues D10, G12, G17, E762, H840, N863, H982, H983, A984, D986, and/or A987 of SEQ ID NO: 8 (or the corresponding mutations of any of the proteins set forth as SEQ ID NOs: 1-800) can be altered (i.e., substituted). Also, mutations other than alanine substitutions are contemplated.

In some some embodiments, a modified Cas9 endonuclease comprises one or more mutations corresponding to S. pyogenes Cas9 mutation E762A, HH983AA or D986A in SEQ ID NO: 8. In some embodiments, the modified Cas 9 endonuclease further comprises one or more mutations corresponding to S. pyogenes Cas9 mutation D10A, H840A, G12A, G17A, N854A, N863A, N982A or A984A in SEQ ID NO: 8. For example, the modified Cas9 endonuclease may comprise a variant at least about 75% identical to any of SEQ ID NOs: 1-800 that comprises one or more mutations corresponding to a mutation E762A, HH983AA or D986A in SEQ ID NO: 8; and/or one or more mutations corresponding to a mutation D10A, H840A, G12A, G17A, N854A, N863A, N982A or A984A in SEQ ID NO: 8. In some embodiments, such a variant comprises a region at least about 75%, at least about 80%, at least about 85%, at least about 90%, at least about 95%, at least about 99% or 100% amino acid sequence identity to the regions corresponding to amino acids 7-166 and/or 731-1003 of SEQ ID NO: 8.

**Table 1. Table 1 lists four motifs that are present in Cas9 sequences from various species. The amino acids listed here are from the Cas9 from S. pyogenes (SEQ ID NO:8).**

| | **Motif** | **Amino acids (residue #s)** | Highly conserved |
|---|---|---|---|
| | RuvC-like I | IGLDIGTNSVGWAVI (7-21) | D10, G12, G17 |
| | RuvC-like II | IVIEMARE (759-766) | E762 |
| | HNH-motif | DVDHIVPQSFLKDDSIDNKVLTRSDKN (837-863) | H840, N854, N863 |
| | RuvC-like II | HHAHDAYL (982-989) | H982, H983, A984, D986, A987 |

In some cases, the chimeric site-directed modifying polypeptide comprises an amino acid sequence having at least about 75%, at least about 80%, at least about 85%, at least about 90%, at least about 95%, at least about 99% or 100% amino acid sequence identity to amino acids 7-166 and/or 731 - 1003 of SEQ ID NO: 8, or to the corresponding portions in any of the amino acid sequences set forth as SEQ ID NOs: 1-800. In some cases, the chimeric site-directed modifying polypeptide comprises 4 motifs (as listed in Table 1), each with amino acid sequences having at least about 75%, at least about 80%, at least about 85%, at least about 90%, at least about 95%, at least about 99% or 100% amino acid sequence identity to each of the 4 motifs listed in Table 1, or to the corresponding portions in any of the amino acid sequences set forth as SEQ ID NOs: 1-800. In some cases, the chimeric site-directed modifying polypeptide comprises amino acid sequences having at least about 75%, at least about 80%, at least about 85%, at least about 90%, at least about 95%, at least about 99% or 100% amino acid sequence identity to amino acids 7-166 and/or 731-1003 of SEQ ID NO: 8, or to the corresponding portions in any of the amino acid sequences set forth as SEQ ID NOs: 1-800.

In some embodiments, the activity portion of the site-directed modifying polypeptide comprises a heterologous polypeptide that has DNA-modifying activity and/or transcription factor activity and/or DNA-associated polypeptide-modifying activity. In some cases, a heterologous polypeptide replaces a portion of the Cas9 polypeptide that provides nuclease activity. In other embodiments, a site-directed modifying polypeptide comprises both a portion of the Cas9 polypeptide that normally provides nuclease activity (and that portion can be fully active or can instead be modified to have less than 100% of the corresponding wild-type activity) and a heterologous polypeptide. In other words, in some cases, a chimeric site-directed modifying polypeptide is a fusion polypeptide comprising both the portion of the Cas9 polypeptide that normally provides nuclease activity and the heterologous polypeptide. In other cases, a chimeric site-directed modifying polypeptide is a fusion polypeptide comprising a modified variant of the activity portion of the Cas9 polypeptide (e.g., amino acid change, deletion, insertion) and a heterologous polypeptide. In yet other cases, a chimeric site-directed modifying polypeptide is a fusion polypeptide comprising a heterologous polypeptide and the RNA-binding portion of a naturally-occurring or a modified site-directed modifying polypeptide.

For example, in a chimeric Cas9 protein, a naturally-occurring (or modified, e.g., mutation, deletion, insertion) bacterial Cas9 polypeptide may be fused to a heterologous polypeptide sequence (i.e. a polypeptide sequence from a protein other than Cas9 or a polypeptide sequence from another organism). The heterologous polypeptide sequence may exhibit an activity (e.g., enzymatic activity) that will also be exhibited by the chimeric Cas9 protein (e.g., methyltransferase activity, acetyltransferase activity, kinase activity, ubiquitinating activity, etc.). A heterologous nucleic acid sequence may be linked to another nucleic acid sequence (e.g., by genetic engineering) to generate a chimeric nucleotide sequence encoding a chimeric polypeptide. In some embodiments, a chimeric Cas9 polypeptide is generated by fusing a Cas9 polypeptide (e.g., wild type Cas9 or a Cas9 variant, e.g., a Cas9 with reduced or inactivated nuclease activity) with a heterologous sequence that provides for subcellular localization (e.g., a nuclear localization signal (NLS) for targeting to the nucleus; a mitochondrial localization signal for targeting to the mitochondria; a chloroplast localization signal for targeting to a chloroplast; an ER retention signal; and the like). In some embodiments, the heterologous sequence can provide a tag for ease of tracking or purification (e.g., a fluorescent protein, e.g., green fluorescent protein (GFP), YFP, RFP, CFP, mCherry, tdTomato, and the like; a HIS tag, e.g., a 6XHis tag; a hemagglutinin (HA) tag; a FLAG tag; a Myc tag; and the like). In some embodiments, the heterologous sequence can provide for increased or decreased stability. In some embodiments, the heterologous sequence can provide a binding domain (e.g., to provide the ability of a chimeric Cas9 polypeptide to bind to another protein of interest, e.g., a DNA or histone modifying protein, a transcription factor or transcription repressor, a recruiting protein, etc.).

### Nucleic acid encoding a chimeric site-directed modifying polypeptide

The present disclosure provides a nucleic acid comprising a nucleotide sequence encoding a chimeric site-directed modifying polypeptide. In some embodiments, the nucleic acid comprising a nucleotide sequence encoding a chimeric site-directed modifying polypeptide is an expression vector, e.g., a recombinant expression vector.

In some embodiments, a method involves contacting a target DNA or introducing into a cell (or a population of cells) one or more nucleic acids comprising a chimeric site-directed modifying polypeptide. Suitable nucleic acids comprising nucleotide sequences encoding a chimeric site-directed modifying polypeptide include expression vectors, where an expression vector comprising a nucleotide sequence encoding a chimeric site-directed modifying polypeptide is a "recombinant expression vector."

In some embodiments, the recombinant expression vector is a viral construct, e.g., a recombinant adeno-associated virus construct (see, e.g., U.S. Patent No. 7,078,387), a recombinant adenoviral construct, a recombinant lentiviral construct, etc.

Suitable expression vectors include, but are not limited to, viral vectors (e.g. viral vectors based on vaccinia virus; poliovirus; adenovirus (see, e.g., Li et al., Invest Opthalmol Vis Sci 35:2543 2549, 1994; Borras et al., Gene Ther 6:515 524, 1999; Li and Davidson, PNAS 92:7700 7704, 1995; Sakamoto et al., H Gene Ther 5:1088 1097, 1999; WO 94/12649, WO 93/03769; WO 93/19191; WO 94/28938; WO 95/11984 and WO 95/00655); adeno-associated virus (see, e.g., Ali et al., Hum Gene Ther 9:81 86, 1998, Flannery et al., PNAS 94:6916 6921, 1997; Bennett et al., Invest Opthalmol Vis Sci 38:2857 2863, 1997; Jomary et al., Gene Ther 4:683 690, 1997, Rolling et al., Hum Gene Ther 10:641 648, 1999; Ali et al., Hum Mol Genet 5:591 594, 1996; Srivastava in WO 93/09239, Samulski et al., J. Vir. (1989) 63:3822-3828; Mendelson et al., Virol. (1988) 166:154-165; and Flotte et al., PNAS (1993) 90:10613-10617); SV40; herpes simplex virus; human immunodeficiency virus (see, e.g., Miyoshi et a1., PNAS 94:10319 23, 1997; Takahashi et al., J Virol 73:7812 7816, 1999); a retroviral vector (e.g., Murine Leukemia Virus, spleen necrosis virus, and vectors derived from retroviruses such as Rous Sarcoma Virus, Harvey Sarcoma Virus, avian leukosis virus, a lentivirus, human immunodeficiency virus, myeloproliferative sarcoma virus, and mammary tumor virus); and the like.

Numerous suitable expression vectors are known to those of skill in the art, and many are commercially available. The following vectors are provided by way of example; for eukaryotic host cells: pXT1, pSG5 (Stratagene), pSVK3, pBPV, pMSG, and pSVLSV40 (Pharmacia). However, any other vector may be used so long as it is compatible with the host cell.

Depending on the host/vector system utilized, any of a number of suitable transcription and translation control elements, including constitutive and inducible promoters, transcription enhancer elements, transcription terminators, etc. may be used in the expression vector (see e.g., Bitter et al. (1987) Methods in Enzymology, 153:516-544).

In some embodiments, a nucleotide sequence encoding a chimeric site-directed modifying polypeptide is operably linked to a control element, e.g., a transcriptional control element, such as a promoter. The transcriptional control element may be functional in either a eukaryotic cell, e.g., a mammalian cell; or a prokaryotic cell (e.g., bacterial or archaeal cell). In some embodiments, a nucleotide sequence encoding a chimeric site-directed modifying polypeptide is operably linked to multiple control elements that allow expression of the nucleotide sequence encoding a chimeric site-directed modifying polypeptide in both prokaryotic and eukaryotic cells.

Non-limiting examples of suitable eukaryotic promoters (promoters functional in a eukaryotic cell) include those from cytomegalovirus (CMV) immediate early, herpes simplex virus (HSV) thymidine kinase, early and late SV40, long terminal repeats (LTRs) from retrovirus, and mouse metallothionein-I. Selection of the appropriate vector and promoter is well within the level of ordinary skill in the art. The expression vector may also contain a ribosome binding site for translation initiation and a transcription terminator. The expression vector may also include appropriate sequences for amplifying expression. The expression vector may also include nucleotide sequences encoding protein tags (e.g., 6xHis tag, hemagglutinin (HA) tag, a fluorescent protein (e.g., a green fluorescent protein; a yellow fluorescent protein, etc.), etc.) that are fused to the chimeric site-directed modifying polypeptide.

In some embodiments, a nucleotide sequence encoding a chimeric site-directed modifying polypeptide is operably linked to an inducible promoter (e.g., heat shock promoter, Tetracycline-regulated promoter, Steroid-regulated promoter, Metal-regulated promoter, estrogen receptor-regulated promoter, etc.). In some embodiments, a nucleotide sequence encoding a chimeric site-directed modifying polypeptide is operably linked to a spatially restricted and/or temporally restricted promoter (e.g., a tissue specific promoter, a cell type specific promoter, etc.). In some embodiments, a nucleotide sequence encoding a chimeric site-directed modifying polypeptide is operably linked to a constitutive promoter.

Methods of introducing a nucleic acid into a host cell are known in the art, and any known method can be used to introduce a nucleic acid (e.g., an expression construct) into a stem cell or progenitor cell. Suitable methods include, include e.g., viral or bacteriophage infection, transfection, conjugation, protoplast fusion, lipofection, electroporation, calcium phosphate precipitation, polyethyleneimine (PEI)-mediated transfection, DEAE-dextran mediated transfection, liposome-mediated transfection, particle gun technology, calcium phosphate precipitation, direct micro injection, nanoparticle-mediated nucleic acid delivery (see, e.g., Panyam et., al Adv Drug Deliv Rev. 2012 Sep 13. pii: 50169-409X(12)00283-9. doi: 10.1016/j.addr.2012.09.023), and the like.

### Methods

The present disclosure provides methods for modifying a target DNA and/or a target DNA-associated polypeptide. Generally, a method involves contacting a target DNA with a complex (a "targeting complex"), which complex comprises a guide RNA and a site-directed modifying polypeptide.

As discussed above, a guide RNA and a site-directed modifying polypeptide form a complex. The guide RNA provides target specificity to the complex by comprising a nucleotide sequence that is complementary to a sequence of a target DNA. The site-directed modifying polypeptide of the complex provides the site-specific activity. In some embodiments, a complex modifies a target DNA, leading to, for example, DNA cleavage, DNA methylation, DNA damage, DNA repair, etc. In other embodiments, a complex modifies a target polypeptide associated with target DNA (e.g., a histone, a DNA-binding protein, etc.), leading to, for example, histone methylation, histone acetylation, histone ubiquitination, and the like. The target DNA may be, for example, naked DNA *in vitro,* chromosomal DNA in cells *in vitro,* chromosomal DNA in cells *in vivo,* etc.

In some cases, the site-directed modifying polypeptide exhibits nuclease activity that cleaves target DNA at a target DNA sequence defined by the region of complementarity between the guide RNA and the target DNA. In some cases, when the site-directed modifying polypeptide is a Cas9 or Cas9 related polypeptide, site-specific cleavage of the target DNA occurs at locations determined by both (i) base-pairing complementarity between the guide RNA and the target DNA; and (ii) a short motif [referred to as the protospacer adjacent motif (PAM)] in the target DNA. In some embodiments (e.g., when Cas9 from S. pyogenes is used), the PAM sequence of the non-complementary strand is 5'-XGG-3', where X is any DNA nucleotide and X is immediately 3' of the target sequence of the non-complementary strand of the target DNA. As such, the PAM sequence of the complementary strand is 5'-CCY-3', where Y is any DNA nucleotide and Y is immediately 5' of the target sequence of the complementary strand of the target DNA (where the PAM of the non-complementary strand is 5'-GGG-3' and the PAM of the complementary strand is 5'-CCC-3'). In some such embodiments, X and Y can be complementary and the X-Y base pair can be any basepair (e.g., X=C and Y=G; X=G and Y=C; X=A and Y=T, X=T and Y=A).

In some cases, different Cas9 proteins (i.e., Cas9 proteins from various species) may be advantageous to use in the various provided methods in order to capitalize on various enzymatic characteristics of the different Cas9 proteins (e.g., for different PAM sequence preferences; for increased or decreased enzymatic activity; for an increased or decreased level of cellular toxicity; to change the balance between NHEJ, homology-directed repair, single strand breaks, double strand breaks, etc.).

Cas9 proteins from various species (see SEQ ID NOs: 1-800) may require different PAM sequences in the target DNA. Thus, for a particular Cas9 protein of choice, the PAM sequence requirement may be different than the 5'-XGG-3' sequence described above. The present disclosure, for example, provides a C. jejuni PAM sequence NNNNACA; P. multocida PAM sequences GNNNCNNA or NNNNC; an F. novicida PAM sequence NG; an S. thermophilus** PAM sequence NNAAAAW; an L. innocua PAM sequence NGG; and an S. dysgalactiae PAM sequence NGG.

Exemplary methods provided that take advantage of characteristics of Cas9 orthologs include the following.

A method for manipulating DNA in a cell, comprising contacting the DNA with a Cas9 ortholog-guideRNA complex, wherein the complex comprises: (a) a cognate guide RNA for a first Cas9 endonuclease from a cluster in Supplementary Table S2 and (b) a second Cas9 endonuclease from the cluster that is exchangeable with preserved high cleavage efficiency with the first endonuclease and shares at least 80% identity with the first endonuclease over 80% of their length. In some embodiments, the guide is a single-molecule guide RNA. In some embodiments, the guide RNA is a double-molecule guide RNA.In some embodiments, the first Cas9 endonuclease is from S. pyogenes and the second Cas9 endonuclease is from S. mutans. In some embodiments, the first Cas9 endonuclease is from S. theromophilus* and the second Cas9 endonuclease is from S. mutans. In some embodiments, the first Cas9 endonuclease is from N. meningitidis and the second Cas9 endonuclease is from P. multocida.

A method for manipulating DNA in a cell, comprising contacting the DNA with a Cas9 ortholog-guideRNA complex, wherein the complex comprises: (a) a cognate guide RNA of a first Cas9 endonuclease from a cluster in Supplementary Table S6 and (b) an Cas9 endonuclease from a cluster in Supplementary Table S6 that is exchangeable with lowered cleavage efficiency with the first endonuclease and shares at least 50% amino acid sequence identity with the first endonuclease over 70% of their length. In some embodiments, the guide is a single-molecule guide RNA. In some embodiments, the guide RNA is a double-molecule guide RNA.In some embodiments, the first Cas9 endonuclease is from C. Jejuni and the second Cas9 endonuclease is from P. multocida. In some embodiments, the first Cas9 endonuclease is from N. meningitidis and the second Cas9 endonuclease is from P. multocida.

A method for manipulating DNA in a cell, comprising contacting the DNA with two or more Cas9-guideRNA complexes, wherein each Cas9-guideRNA complex comprises: (a) a Cas9 endonuclease from a different cluster in Supplementary Table S6 exhibiting less than 50% amino acid sequence identity with the other endonucleases of the method over 70% of their length, and (b) a guide RNA specifically complexed with each Cas9 endonuclease. In some embodiments, the guide is a single-molecule guide RNA. In some embodiments, the guide RNA is a double-molecule guide RNA.In some embodiments, the Cas9 endonucleases are from F. novicida and S. pyogenes. In some embodiments, the Cas9 endonucleases are from N. meningitidis and S. mutans. In some embodiments, the S. thermophilus* and S. thermophilus** Cas9 endonucleases.

Many Cas9 orthologs from a wide variety of species have been identified herein. All identified Cas9 orthologs have the same domain architecture with a central HNH endonuclease domain and a split RuvC/RNaseH domain. Cas9 proteins share four key motifs with a conserved architecture. Motifs 1, 2, and 4 are RuvC like motifs while motif 3 is an HNH-motif. In some cases, a suitable site-directed modifying polypeptide comprises an amino acid sequence having four motifs, each of motifs 1-4 having at least about 75%, at least about 80%, at least about 85%, at least about 90%, at least about 95%, at least about 99% or 100% amino acid sequence identity to the motifs 1-4 of the Cas9 amino acid sequence depicted in Table 1), or to the corresponding portions in any of the amino acid sequences set forth in SEQ ID NOs: 1-800. In some cases, a suitable site-directed modifying polypeptide comprises an amino acid sequence having at least about 75%, at least about 80%, at least about 85%, at least about 90%, at least about 95%, at least about 99% or 100% amino acid sequence identity to amino acids 7-166 and/or 731-1003 of SEQ ID NO: 8, or to the corresponding portions in any of the amino acid sequences set forth as SEQ ID NOs: 1-800.

The nuclease activity cleaves target DNA to produce double strand breaks. These breaks are then repaired by the cell in one of two ways: non-homologous end joining, and homology-directed repair. In non-homologous end joining (NHEJ), the double-strand breaks are repaired by direct ligation of the break ends to one another. As such, no new nucleic acid material is inserted into the site, although some nucleic acid material may be lost, resulting in a deletion. In homology-directed repair, a donor polynucleotide with homology to the cleaved target DNA sequence is used as a template for repair of the cleaved target DNA sequence, resulting in the transfer of genetic information from the donor polynucleotide to the target DNA. As such, new nucleic acid material may be inserted/copied into the site. In some cases, a target DNA is contacted with a donor polynucleotide. In some cases, a donor polynucleotide is introduced into a cell. The modifications of the target DNA due to NHEJ and/or homology-directed repair lead to, for example, gene correction, gene replacement, gene tagging, transgene insertion, nucleotide deletion, gene disruption, gene mutation, sequence replacement, etc. Accordingly, cleavage of DNA by a site-directed modifying polypeptide may be used to delete nucleic acid material from a target DNA sequence (e.g., to disrupt a gene that makes cells susceptible to infection (e.g. the CCRS or CXCR4 gene, which makes T cells susceptible to HIV infection), to remove disease-causing trinucleotide repeat sequences in neurons, to create gene knockouts and mutations as disease models in research, etc.) by cleaving the target DNA sequence and allowing the cell to repair the sequence in the absence of an exogenously provided donor polynucleotide. Thus, the methods can be used to knock out a gene (resulting in complete lack of transcription or altered transcription) or to knock in genetic material into a locus of choice in the target DNA.

Alternatively, if a guide RNA and a site-directed modifying polypeptide are coadministered to cells with a donor polynucleotide sequence that includes at least a segment with homology to the target DNA sequence, the subject methods may be used to add, i.e. insert or replace, nucleic acid material to a target DNA sequence (e.g. to "knock in" a nucleic acid that encodes for a protein, an siRNA, an miRNA, etc.), to add a tag (e.g., 6xHis, a fluorescent protein (e.g., a green fluorescent protein; a yellow fluorescent protein, etc.), hemagglutinin (HA), FLAG, etc.), to add a regulatory sequence to a gene (e.g. promoter, polyadenylation signal, internal ribosome entry sequence (IRES), 2A peptide, start codon, stop codon, splice signal, localization signal, etc.), to modify a nucleic acid sequence (e.g., introduce a mutation), and the like. As such, a complex comprising a guide RNA and a site-directed modifying polypeptide is useful in any *in vitro* or *in vivo* application in which it is desirable to modify DNA in a site-specific, i.e. "targeted", way, for example gene knock-out, gene knock-in, gene editing, gene tagging, sequence replacement, etc., as used in, for example, gene therapy, e.g. to treat a disease or as an antiviral, antipathogenic, or anticancer therapeutic, the production of genetically modified organisms in agriculture, the large scale production of proteins by cells for therapeutic, diagnostic, or research purposes, the induction of iPS cells, biological research, the targeting of genes of pathogens for deletion or replacement, etc.

In some embodiments, the site-directed modifying polypeptide comprises a modified form of the Cas9 protein. In some instances, the modified form of the Cas9 protein comprises an amino acid change (e.g., deletion, insertion, or substitution) that reduces the naturally-occurring nuclease activity of the Cas9 protein. For example, in some instances, the modified form of the Cas9 protein has less than 50%, less than 40%, less than 30%, less than 20%, less than 10%, less than 5%, or less than 1% of the nuclease activity of the corresponding wild-type Cas9 polypeptide. In some cases, the modified form of the Cas9 polypeptide has no substantial nuclease activity. When a site-directed modifying polypeptide is a modified form of the Cas9 polypeptide that has no substantial nuclease activity, it can be referred to as "dCas9."

In some embodiments, the modified form of the Cas9 polypeptide is a D10A (aspartate to alanine at amino acid position 10 of SEQ ID NO:8) mutation (or the corresponding mutation of any of the proteins set forth as SEQ ID NOs: 1-800) that can cleave the complementary strand of the target DNA but has reduced ability to cleave the non-complementary strand of the target DNA (thus resulting in a single strand break (SSB) instead of a DSB). In some embodiments, the modified form of the Cas9 polypeptide is a H840A (histidine to alanine at amino acid position 840 of SEQ ID NO:8) mutation (or the corresponding mutation of any of the proteins set forth as SEQ ID NOs: 1-800) that can cleave the non-complementary strand of the target DNA but has reduced ability to cleave the complementary strand of the target DNA (thus resulting in a single strand break (SSB) instead of a DSB). The use of the D10A or H840A variant of SEQ ID NO: 8 Cas9 (or the corresponding mutations in any of the proteins set forth as SEQ ID NOs: 1-800) can alter the expected biological outcome because the non-homologous end joining (NHEJ) is much more likely to occur when DSBs are present as opposed to SSBs. Thus, in some cases where one wishes to reduce the likelihood of DSB (and therefore reduce the likelihood of NHEJ), a D10A or H840A variant of Cas9 can be used. Other residues can be mutated to achieve the same effect (i.e. inactivate one or the other nuclease portions). As non-limiting examples, SEQ ID NO: 8 S. pyogenes Cas9 residues D10, G12, G17, E762, H840, N863, H982, H983, A984, D986, and/or A987 (or the corresponding mutations of any of the proteins set forth as SEQ ID NOs: 1-800) can be altered (i.e., substituted). Also, mutations other than alanine substitutions are contemplated. In some embodiments when a site-directed polypeptide (e.g., site-directred modifying polypeptide) has reduced catalytic activity (e.g., when a SEQ ID NO: 8 Cas9 protein has a D10, G12, G17, E762, H840, N863, H982, H983, A984, D986, and/or a A987 mutation, e.g., D 10A, G12A, G17A, E762A, H840A, N863A, H982A, H983A, A984A, and/or D986A), the polypeptide can still bind to target DNA in a site-specific manner (because it is still guided to a target DNA sequence by a guide RNA) as long as it retains the ability to interact with the guide RNA.

In some embodiments, the modified form of the SEQ ID NO: 8 Cas9 polypeptide harbors both the D10A and the H840A mutations (or the corresponding mutations of any of the proteins set forth as SEQ ID NOs: 1-800) such that the polypeptide has a reduced ability to cleave both the complementary and the non-complementary strands of the target DNA (i.e., the variant can have no substantial nuclease activity). Other residues can be mutated to achieve the same effect (i.e. inactivate one or the other nuclease portions). As non-limiting examples, SEQ ID NO: 8 residues D10, G12, G17, E762, H840, N863, H982, H983, A984, D986, and/or A987 (or the corresponding mutations of any of the proteins set forth as SEQ ID NOs: 1-800) can be altered (i.e., substituted). Also, mutations other than alanine substitutions are contemplated.

In some embodiments, the site-directed modifying polypeptide comprises a heterologous sequence (e.g., a fusion). In some embodiments, a heterologous sequence can provide for subcellular localization of the site-directed modifying polypeptide (e.g., a nuclear localization signal (NLS) for targeting to the nucleus; a mitochondrial localization signal for targeting to the mitochondria; a chloroplast localization signal for targeting to a chloroplast; a ER retention signal; and the like). In some embodiments, a heterologous sequence can provide a tag for ease of tracking or purification (e.g., a fluorescent protein, e.g., green fluorescent protein (GFP), YFP, RFP, CFP, mCherry, tdTomato, and the like; a his tag, e.g., a 6XHis tag; a hemagglutinin (HA) tag; a FLAG tag; a Myc tag; and the like). In some embodiments, the heterologous sequence can provide for increased or decreased stability.

In some embodiments, a site-directed modifying polypeptide can be codon-optimized. This type of optimization is known in the art and entails the mutation of foreign-derived DNA to mimic the codon preferences of the intended host organism or cell while encoding the same protein. Thus, the codons are changed, but the encoded protein remains unchanged. For example, if the intended target cell was a human cell, a human codon-optimized Cas9 (or variant, e.g., enzymatically inactive variant) would be a suitable site-directed modifying polypeptide. Any suitable site-directed modifying polypeptide (e.g., any Cas9 such as any of the sequences set forth in SEQ ID NOs: 1-800) can be codon optimized. As another non-limiting example, if the intended host cell were a mouse cell, than a mouse codon-optimized Cas9 (or variant, e.g., enzymatically inactive variant) would be a suitable site-directed modifying polypeptide. While codon optimization is not required, it is acceptable and may be preferable in certain cases.

Polyadenylation signals can also be chosen to optimize expression in the intended host.

In some embodiments, a guide RNA and a site-directed modifying polypeptide are used as an inducible system for shutting off gene expression in bacterial cells. In some cases, nucleic acids encoding an appropriate guide RNA and/or an appropriate site-directed polypeptide are incorporated into the chromosome of a target cell and are under control of an inducible promoter. When the guide RNA and/or the site-directed polypeptide are induced, the target DNA is cleaved (or otherwise modified) at the location of interest (e.g., a target gene on a separate plasmid), when both the guide RNA and the site-directed modifying polypeptide are present and form a complex. As such, in some cases, bacterial expression strains are engineered to include nucleic acid sequences encoding an appropriate site-directed modifying polypeptide in the bacterial genome and/or an appropriate guide RNA on a plasmid (e.g., under control of an inducible promoter), allowing experiments in which the expression of any targeted gene (expressed from a separate plasmid introduced into the strain) could be controlled by inducing expression of the guide RNA and the site-directed polypeptide.

In some cases, the site-directed modifying polypeptide has enzymatic activity that modifies target DNA in ways other than introducing double strand breaks. Enzymatic activity of interest that may be used to modify target DNA (e.g., by fusing a heterologous polypeptide with enzymatic activity to a site-directed modifying polypeptide, thereby generating a chimeric site-directed modifying polypeptide) includes, but is not limited methyltransferase activity, demethylase activity, DNA repair activity, DNA damage activity, deamination activity, dismutase activity, alkylation activity, depurination activity, oxidation activity, pyrimidine dimer forming activity, integrase activity, transposase activity, recombinase activity, polymerase activity, ligase activity, helicase activity, photolyase activity or glycosylase activity). Methylation and demethylation is recognized in the art as an important mode of epigenetic gene regulation while DNA damage and repair activity is essential for cell survival and for proper genome maintenance in response to environmental stresses.

As such, the methods herein find use in the epigenetic modification of target DNA and may be employed to control epigenetic modification of target DNA at any location in a target DNA by genetically engineering the desired complementary nucleic acid sequence into the DNA-targeting segment of a guide RNA. The methods herein also find use in the intentional and controlled damage of DNA at any desired location within the target DNA. The methods herein also find use in the sequence-specific and controlled repair of DNA at any desired location within the target DNA. Methods to target DNA-modifying enzymatic activities to specific locations in target DNA find use in both research and clinical applications.

In some cases, the site-directed modifying polypeptide has activity that modulates the transcription of target DNA (e.g., in the case of a chimeric site-directed modifying polypeptide, etc.). In some cases, a chimeric site-directed modifying polypeptides comprising a heterologous polypeptide that exhibits the ability to increase or decrease transcription (e.g., transcriptional activator or transcription repressor polypeptides) is used to increase or decrease the transcription of target DNA at a specific location in a target DNA, which is guided by the DNA-targeting segment of the guide RNA. Examples of source polypeptides for providing a chimeric site-directed modifying polypeptide with transcription modulatory activity include, but are not limited to light-inducible transcription regulators, small molecule/drug-responsive transcription regulators, transcription factors, transcription repressors, etc. In some cases, the method is used to control the expression of a targeted coding-RNA (protein-encoding gene) and/or a targeted non-coding RNA (e.g., tRNA, rRNA, snoRNA, siRNA, miRNA, long ncRNA, etc.). In some cases, the site-directed modifying polypeptide has enzymatic activity that modifies a polypeptide associated with DNA (e.g. histone). In some embodiments, the enzymatic activity is methyltransferase activity, demethylase activity, acetyltransferase activity, deacetylase activity, kinase activity, phosphatase activity, ubiquitin ligase activity (i.e., ubiquitination activity), deubiquitinating activity, adenylation activity, deadenylation activity, SUMOylating activity, deSUMOylating activity, ribosylation activity, deribosylation activity, myristoylation activity, demyristoylation activity glycosylation activity (e.g., from GlcNAc transferase) or deglycosylation activity. The enzymatic activities listed herein catalyze covalent modifications to proteins. Such modifications are known in the art to alter the stability or activity of the target protein (e.g., phosphorylation due to kinase activity can stimulate or silence protein activity depending on the target protein). Of particular interest as protein targets are histones. Histone proteins are known in the art to bind DNA and form complexes known as nucleosomes. Histones can be modified (e.g., by methylation, acetylation, ubuitination, phosphorylation) to elicit structural changes in the surrounding DNA, thus controlling the accessibility of potentially large portions of DNA to interacting factors such as transcription factors, polymerases and the like. A single histone can be modified in many different ways and in many different combinations (e.g., trimethylation of lysine 27 of histone 3, H3K27, is associated with DNA regions of repressed transcription while trimethylation of lysine 4 of histone 3, H3K4, is associated with DNA regions of active transcription). Thus, a site-directed modifying polypeptide with histone-modifying activity finds use in the site specific control of DNA structure and can be used to alter the histone modification pattern in a selected region of target DNA. Such methods find use in both research and clinical applications.

In some embodiments, multiple guide RNAs are used simultaneously to simultaneously modify different locations on the same target DNA or on different target DNAs. In some embodiments, two or more guide RNAs target the same gene or transcript or locus. In some embodiments, two or more guide RNAs target different unrelated loci. In some embodiments, two or more guide RNAs target different, but related loci.

In some cases, the site-directed modifying polypeptide is provided directly as a protein. As one non-limiting example, fungi (e.g., yeast) can be transformed with exogenous protein and/or nucleic acid using spheroplast transformation (see Kawai et al., Bioeng Bugs. 2010 Nov-Dec; 1(6):395-403 : "Transformation of Saccharomyces cerevisiae and other fungi: methods and possible underlying mechanism"; and Tanka et al., Nature. 2004 Mar 18;428(6980):323-8: "Conformational variations in an infectious protein determine prion strain differences"; both of which are herein incorporated by reference in their entirety). Thus, a site-directed modifying polypeptide (e.g., Cas9) can be incorporated into a spheroplast (with or without nucleic acid encoding a guide RNA and with or without a donor polynucleotide) and the spheroplast can be used to introduce the content into a yeast cell. A site-directed modifying polypeptide can be introduced into a cell (provided to the cell) by any convenient method; such methods are known to those of ordinary skill in the art. As another non-limiting example, a site-directed modifying polypeptide can be injected directly into a cell (e.g., with or without nucleic acid encoding a guide RNA and with or without a donor polynucleotide), e.g., a cell of a zebrafish embryo, the pronucleus of a fertilized mouse oocyte, etc.

### Target cells of interest

In some of the above applications, the methods may be employed to induce DNA cleavage, DNA modification, and/or transcriptional modulation in mitotic or post-mitotic cells *in vivo* and/or *ex vivo* and/or *in vitro* (e.g., to produce genetically modified cells that can be reintroduced into an individual). Because the guide RNA provide specificity by hybridizing to target DNA, a mitotic and/or post-mitotic cell of interest in the disclosed methods may include a cell from any organism (e.g. a bacterial cell, an archaeal cell, a cell of a single-cell eukaryotic organism, a plant cell, an algal cell, e.g., *Botryococcus braunii, Chlamydomonas reinhardtii, Nannochloropsis gaditana, Chlorella pyrenoidosa, Sargassum patens C. Agardh,* and the like, a fungal cell (e.g., a yeast cell), an animal cell, a cell from an invertebrate animal (e.g. fruit fly, cnidarian, echinoderm, nematode, etc.), a cell from a vertebrate animal (e.g., fish, amphibian, reptile, bird, mammal), a cell from a mammal, a cell from a rodent, a cell from a primate, a cell from a human, etc.).

Any type of cell may be of interest (e.g. a stem cell, e.g. an embryonic stem (ES) cell, an induced pluripotent stem (iPS) cell, a germ cell; a somatic cell, e.g. a fibroblast, a hematopoietic cell, a neuron, a muscle cell, a bone cell, a hepatocyte, a pancreatic cell; an in vitro or in vivo embryonic cell of an embryo at any stage, e.g., a 1-cell, 2-cell, 4-cell, 8-cell, etc. stage zebrafish embryo; etc.). Cells may be from established cell lines or they may be primary cells, where "primary cells", "primary cell lines", and "primary cultures" are used interchangeably herein to refer to cells and cells cultures that have been derived from a and allowed to grow in vitro for a limited number of passages, i.e. splittings, of the culture. For example, primary cultures are cultures that may have been passaged 0 times, 1 time, 2 times, 4 times, 5 times, 10 times, or 15 times, but not enough times go through the crisis stage. Typically, the primary cell lines of the present invention are maintained for fewer than 10 passages in vitro. Target cells are in many embodiments unicellular organisms, or are grown in culture.

If the cells are primary cells, they may be harvest from an individual by any convenient method. For example, leukocytes may be conveniently harvested by apheresis, leukocytapheresis, density gradient separation, etc., while cells from tissues such as skin, muscle, bone marrow, spleen, liver, pancreas, lung, intestine, stomach, etc. are most conveniently harvested by biopsy. An appropriate solution may be used for dispersion or suspension of the harvested cells. Such solution will generally be a balanced salt solution, e.g. normal saline, phosphate-buffered saline (PBS), Hank's balanced salt solution, etc., conveniently supplemented with fetal calf serum or other naturally occurring factors, in conjunction with an acceptable buffer at low concentration, generally from 5-25 mM. Convenient buffers include HEPES, phosphate buffers, lactate buffers, etc. The cells may be used immediately, or they may be stored, frozen, for long periods of time, being thawed and capable of being reused. In such cases, the cells will usually be frozen in 10% DMSO, 50% serum, 40% buffered medium, or some other such solution as is commonly used in the art to preserve cells at such freezing temperatures, and thawed in a manner as commonly known in the art for thawing frozen cultured cells.

### Nucleic acids encoding a guide RNA and/or a site-directed modifying polypeptide

In some embodiments, a method involves contacting a target DNA or introducing into a cell (or a population of cells) one or more nucleic acids comprising nucleotide sequences encoding a guide RNA and/or a site-directed modifying polypeptide and/or a donor polynucleotide. Suitable nucleic acids comprising nucleotide sequences encoding a guide RNA and/or a site-directed modifying polypeptide include expression vectors, where an expression vector comprising a nucleotide sequence encoding a guide RNA and/or a site-directed modifying polypeptide is a "recombinant expression vector."

In some embodiments, the recombinant expression vector is a viral construct, e.g., a recombinant adeno-associated virus construct (see, e.g., U.S. Patent No. 7,078,387), a recombinant adenoviral construct, a recombinant lentiviral construct, etc.

Suitable expression vectors include, but are not limited to, viral vectors (e.g. viral vectors based on vaccinia virus; poliovirus; adenovirus (see, e.g., Li et al., Invest Opthalmol Vis Sci 35:2543 2549, 1994; Borras et al., Gene Ther 6:515 524, 1999; Li and Davidson, PNAS 92:7700 7704, 1995; Sakamoto et al., H Gene Ther 5:1088 1097, 1999; WO 94/12649, WO 93/03769; WO 93/19191; WO 94/28938; WO 95/11984 and WO 95/00655); adeno-associated virus (see, e.g., Ali et al., Hum Gene Ther 9:81 86, 1998, Flannery et al., PNAS 94:6916 6921, 1997; Bennett et al., Invest Opthalmol Vis Sci 38:2857 2863, 1997; Jomary et al., Gene Ther 4:683 690, 1997, Rolling et al., Hum Gene Ther 10:641 648, 1999; Ali et al., Hum Mol Genet 5:591 594, 1996; Srivastava in WO 93/09239, Samulski et al., J. Vir. (1989) 63:3822-3828; Mendelson et al., Virol. (1988) 166:154-165; and Flotte et al., PNAS (1993) 90:10613-10617); SV40; herpes simplex virus; human immunodeficiency virus (see, e.g., Miyoshi et al., PNAS 94:10319 23, 1997; Takahashi et al., J Virol 73:7812 7816, 1999); a retroviral vector (e.g., Murine Leukemia Virus, spleen necrosis virus, and vectors derived from retroviruses such as Rous Sarcoma Virus, Harvey Sarcoma Virus, avian leukosis virus, a lentivirus, human immunodeficiency virus, myeloproliferative sarcoma virus, and mammary tumor virus); and the like.

Numerous suitable expression vectors are known to those of skill in the art, and many are commercially available. The following vectors are provided by way of example; for eukaryotic host cells: pXT1, pSG5 (Stratagene), pSVK3, pBPV, pMSG, and pSVLSV40 (Pharmacia). However, any other vector may be used so long as it is compatible with the host cell.

In some embodiments, a nucleotide sequence encoding a guide RNA and/or a site-directed modifying polypeptide is operably linked to a control element, e.g., a transcriptional control element, such as a promoter. The transcriptional control element may be functional in either a eukaryotic cell, e.g., a mammalian cell, or a prokaryotic cell (e.g., bacterial or archaeal cell). In some embodiments, a nucleotide sequence encoding a guide RNA and/or a site-directed modifying polypeptide is operably linked to multiple control elements that allow expression of the nucleotide sequence encoding a guide RNA and/or a site-directed modifying polypeptide in both prokaryotic and eukaryotic cells.

Depending on the host/vector system utilized, any of a number of suitable transcription and translation control elements, including constitutive and inducible promoters, transcription enhancer elements, transcription terminators, etc. may be used in the expression vector (e.g., U6 promoter, H1 promoter, etc.; see above) (see e.g., Bitter et al. (1987) Methods in Enzymology, 153:516-544).

In some embodiments, a guide RNA and/or a site-directed modifying polypeptide can be provided as RNA. In such cases, the guide RNA and/or the RNA encoding the site-directed modifying polypeptide can be produced by direct chemical synthesis or may be transcribed *in vitro* from a DNA encoding the guide RNA. Methods of synthesizing RNA from a DNA template are well known in the art. In some cases, the guide RNA and/or the RNA encoding the site-directed modifying polypeptide will be synthesized *in vitro* using an RNA polymerase enzyme (e.g., T7 polymerase, T3 polymerase, SP6 polymerase, etc.). Once synthesized, the RNA may directly contact a target DNA or may be introduced into a cell by any of the well-known techniques for introducing nucleic acids into cells (e.g., microinjection, electroporation, transfection, etc).

Nucleotides encoding a guide RNA (introduced either as DNA or RNA) and/or a site-directed modifying polypeptide (introduced as DNA or RNA) and/or a donor polynucleotide may be provided to the cells using well-developed transfection techniques; see, e.g. Angel and Yanik (2010) PLoS ONE 5(7): e 11756, and the commercially available TransMessenger® reagents from Qiagen, StemfectTM RNA Transfection Kit from Stemgent, and TransIT®-mRNA Transfection Kit from Mims Bio LLC. See also Beumer et al. (2008) Efficient gene targeting in Drosophila by direct embryo injection with zinc-finger nucleases. PNAS 105(50):19821-19826. Alternatively, nucleic acids encoding a guide RNA and/or a site-directed modifying polypeptide and/or a chimeric site-directed modifying polypeptide and/or a donor polynucleotide may be provided on DNA vectors. Many vectors, e.g. plasmids, cosmids, minicircles, phage, viruses, etc., useful for transferring nucleic acids into target cells are available. The vectors comprising the nucleic acid(s) may be maintained episomally, e.g. as plasmids, minicircle DNAs, viruses such cytomegalovirus, adenovirus, etc., or they may be integrated into the target cell genome, through homologous recombination or random integration, e.g. retrovirus-derived vectors such as MMLV, HIV-1, ALV, etc.

Vectors may be provided directly to the cells. In other words, the cells are contacted with vectors comprising the nucleic acid encoding guide RNA and/or a site-directed modifying polypeptide and/or a chimeric site-directed modifying polypeptide and/or a donor polynucleotide such that the vectors are taken up by the cells. Methods for contacting cells with nucleic acid vectors that are plasmids, including electroporation, calcium chloride transfection, microinjection, and lipofection are well known in the art. For viral vector delivery, the cells are contacted with viral particles comprising the nucleic acid encoding a guide RNA and/or a site-directed modifying polypeptide and/or a chimeric site-directed modifying polypeptide and/or a donor polynucleotide. Retroviruses, for example, lentiviruses, are particularly suitable to the method of the invention. Commonly used retroviral vectors are "defective", i.e. unable to produce viral proteins required for productive infection. Rather, replication of the vector requires growth in a packaging cell line. To generate viral particles comprising nucleic acids of interest, the retroviral nucleic acids comprising the nucleic acid are packaged into viral capsids by a packaging cell line. Different packaging cell lines provide a different envelope protein (ecotropic, amphotropic or xenotropic) to be incorporated into the capsid, this envelope protein determining the specificity of the viral particle for the cells (ecotropic for murine and rat; amphotropic for most mammalian cell types including human, dog and mouse; and xenotropic for most mammalian cell types except murine cells). The appropriate packaging cell line may be used to ensure that the cells are targeted by the packaged viral particles. Methods of introducing the retroviral vectors comprising the nucleic acid encoding the reprogramming factors into packaging cell lines and of collecting the viral particles that are generated by the packaging lines are well known in the art. Nucleic acids can also introduced by direct micro-injection (e.g., injection of RNA into a zebrafish embryo).

Vectors used for providing the nucleic acids encoding guide RNA and/or a site-directed modifying polypeptide and/or a chimeric site-directed modifying polypeptide and/or a donor polynucleotide to the cells will typically comprise suitable promoters for driving the expression, that is, transcriptional activation, of the nucleic acid of interest. In other words, the nucleic acid of interest will be operably linked to a promoter. This may include ubiquitously acting promoters, for example, the CMV-13-actin promoter, or inducible promoters, such as promoters that are active in particular cell populations or that respond to the presence of drugs such as tetracycline. By transcriptional activation, it is intended that transcription will be increased above basal levels in the target cell by at least about 10 fold, by at least about 100 fold, more usually by at least about 1000 fold. In addition, vectors used for providing a guide RNA and/or a site-directed modifying polypeptide and/or a chimeric site-directed modifying polypeptide and/or a donor polynucleotide to the cells may include nucleic acid sequences that encode for selectable markers in the target cells, so as to identify cells that have taken up the guide RNA and/or a site-directed modifying polypeptide and/or a chimeric site-directed modifying polypeptide and/or a donor polynucleotide.

A guide RNA and/or a site-directed modifying polypeptide and/or a chimeric site-directed modifying polypeptide may instead be used to contact DNA or introduced into cells as RNA. Methods of introducing RNA into cells are known in the art and may include, for example, direct injection, transfection, or any other method used for the introduction of DNA. A site-directed modifying polypeptide may instead be provided to cells as a polypeptide. Such a polypeptide may optionally be fused to a polypeptide domain that increases solubility of the product. The domain may be linked to the polypeptide through a defined protease cleavage site, e.g. a TEV sequence, which is cleaved by TEV protease. The linker may also include one or more flexible sequences, e.g. from 1 to 10 glycine residues. In some embodiments, the cleavage of the fusion protein is performed in a buffer that maintains solubility of the product, e.g. in the presence of from 0.5 to 2 M urea, in the presence of polypeptides and/or polynucleotides that increase solubility, and the like. Domains of interest include endosomolytic domains, e.g. influenza HA domain; and other polypeptides that aid in production, e.g. IF2 domain, GST domain, GRPE domain, and the like. The polypeptide may be formulated for improved stability. For example, the peptides may be PEGylated, where the polyethyleneoxy group provides for enhanced lifetime in the blood stream.

Additionally or alternatively, the site-directed modifying polypeptide may be fused to a polypeptide permeant domain to promote uptake by the cell. A number of permeant domains are known in the art and may be used in the non-integrating polypeptides of the present invention, including peptides, peptidomimetics, and non-peptide carriers. For example, a permeant peptide may be derived from the third alpha helix of Drosophila melanogaster transcription factor Antennapaedia, referred to as penetratin, which comprises the amino acid sequence RQIKIWFQNRRMKWKK. As another example, the permeant peptide comprises the HIV-1 tat basic region amino acid sequence, which may include, for example, amino acids 49-57 of naturally-occurring tat protein. Other permeant domains include poly-arginine motifs, for example, the region of amino acids 34-56 of HIV-1 rev protein, nona-arginine, octa-arginine, and the like. (See, for example, Futaki et al. (2003) Curr Protein Pept Sci. 2003 Apr; 4(2): 87-9 and 446; and Wender et al. (2000) Proc. Natl. Acad. Sci. U.S.A 2000 Nov. 21; 97(24):13003-8; published U.S. Patent applications 20030220334; 20030083256; 20030032593; and 20030022831, herein specifically incorporated by reference for the teachings of translocation peptides and peptoids). The nona-arginine (R9) sequence is one of the more efficient PTDs that have been characterized (Wender et al. 2000; Uemura et al. 2002). The site at which the fusion is made may be selected in order to optimize the biological activity, secretion or binding characteristics of the polypeptide. The optimal site will be determined by routine experimentation.

A site-directed modifying polypeptide may be produced *in* vitro or by eukaryotic cells or by prokaryotic cells, and it may be further processed by unfolding, e.g. heat denaturation, DTT reduction, etc. and may be further refolded, using methods known in the art.

Modifications of interest that do not alter primary sequence include chemical derivatization of polypeptides, e.g., acylation, acetylation, carboxylation, amidation, etc. Also included are modifications of glycosylation, e.g. those made by modifying the glycosylation patterns of a polypeptide during its synthesis and processing or in further processing steps; e.g. by exposing the polypeptide to enzymes which affect glycosylation, such as mammalian glycosylating or deglycosylating enzymes. Also embraced are sequences that have phosphorylated amino acid residues, e.g. phosphotyrosine, phosphoserine, or phosphothreonine.

Also included in the invention are guide RNAs and site-directed modifying polypeptides that have been modified using ordinary molecular biological techniques and synthetic chemistry so as to improve their resistance to proteolytic degradation, to change the target sequence specificity, to optimize solubility properties, to alter protein activity (e.g., transcription modulatory activity, enzymatic activity, etc) or to render them more suitable as a therapeutic agent. Analogs of such polypeptides include those containing residues other than naturally occurring L-amino acids, e.g. D-amino acids or non-naturally occurring synthetic amino acids. D-amino acids may be substituted for some or all of the amino acid residues. The site-directed modifying polypeptides may be prepared by in vitro synthesis, using conventional methods as known in the art. Various commercial synthetic apparatuses are available, for example, automated synthesizers by Applied Biosystems, Inc., Beckman, etc. By using synthesizers, naturally occurring amino acids may be substituted with unnatural amino acids. The particular sequence and the manner of preparation will be determined by convenience, economics, purity required, and the like.

If desired, various groups may be introduced into the peptide during synthesis or during expression, which allow for linking to other molecules or to a surface. Thus cysteines can be used to make thioethers, histidines for linking to a metal ion complex, carboxyl groups for forming amides or esters, amino groups for forming amides, and the like.

The site-directed modifying polypeptides may also be isolated and purified in accordance with conventional methods of recombinant synthesis. A lysate may be prepared of the expression host and the lysate purified using HPLC, exclusion chromatography, gel electrophoresis, affinity chromatography, or other purification technique. For the most part, the compositions which are used will comprise at least 20% by weight of the desired product, more usually at least about 75% by weight, preferably at least about 95% by weight, and for therapeutic purposes, usually at least about 99.5% by weight, in relation to contaminants related to the method of preparation of the product and its purification. Usually, the percentages will be based upon total protein. To induce DNA cleavage and recombination, or any desired modification to a target DNA, or any desired modification to a polypeptide associated with target DNA, the guide RNA and/or the site-directed modifying polypeptide and/or the donor polynucleotide, whether they be introduced as nucleic acids or polypeptides, are provided to the cells for about 30 minutes to about 24 hours, e.g., 1 hour, 1.5 hours, 2 hours, 2.5 hours, 3 hours, 3.5 hours 4 hours, 5 hours, 6 hours, 7 hours, 8 hours, 12 hours, 16 hours, 18 hours, 20 hours, or any other period from about 30 minutes to about 24 hours, which may be repeated with a frequency of about every day to about every 4 days, e.g., every 1.5 days, every 2 days, every 3 days, or any other frequency from about every day to about every four days. The agent(s) may be provided to the cells one or more times, e.g. one time, twice, three times, or more than three times, and the cells allowed to incubate with the agent(s) for some amount of time following each contacting event e.g. 16-24 hours, after which time the media is replaced with fresh media and the cells are cultured further. In cases in which two or more different targeting complexes are provided to the cell (e.g., two different guide RNAs that are complementary to different sequences within the same or different target DNA), the complexes may be provided simultaneously (e.g. as two polypeptides and/or nucleic acids), or delivered simultaneously. Alternatively, they may be provided consecutively, e.g. the targeting complex being provided first, followed by the second targeting complex, etc. or vice versa.

Typically, an effective amount of the guide RNA and/or site-directed modifying polypeptide and/or donor polynucleotide is provided to the target DNA or cells to induce target modification. An effective amount of the guide RNA and/or site-directed modifying polypeptide and/or donor polynucleotide is the amount to induce a 2-fold increase or more in the amount of target modification observed between two homologous sequences relative to a negative control, e.g. a cell contacted with an empty vector or irrelevant polypeptide. That is to say, an effective amount or dose of the guide RNA and/or site-directed modifying polypeptide and/or donor polynucleotide will induce a 2-fold increase, a 3-fold increase, a 4-fold increase or more in the amount of target modification observed at a target DNA region, in some instances a 5-fold increase, a 6-fold increase or more, sometimes a 7-fold or 8-fold increase or more in the amount of recombination observed, e.g. an increase of 10-fold, 50-fold, or 100-fold or more, in some instances, an increase of 200-fold, 500-fold, 700-fold, or 1000-fold or more, e.g. a 5000-fold, or 10,000-fold increase in the amount of recombination observed. The amount of target modification may be measured by any convenient method. For example, a silent reporter construct comprising complementary sequence to the targeting segment (targeting sequence) of the guide RNA flanked by repeat sequences that, when recombined, will reconstitute a nucleic acid encoding an active reporter may be cotransfected into the cells, and the amount of reporter protein assessed after contact with the guide RNA and/or site-directed modifying polypeptide and/or donor polynucleotide, e.g. 2 hours, 4 hours, 8 hours, 12 hours, 24 hours, 36 hours, 48 hours, 72 hours or more after contact with the guide RNA and/or site-directed modifying polypeptide and/or donor polynucleotide. As another, more sensitivity assay, for example, the extent of recombination at a genomic DNA region of interest comprising target DNA sequences may be assessed by PCR or Southern hybridization of the region after contact with a guide RNA and/or site-directed modifying polypeptide and/or donor polynucleotide, e.g. 2 hours, 4 hours, 8 hours, 12 hours, 24 hours, 36 hours, 48 hours, 72 hours or more after contact with the guide RNA and/or site-directed modifying polypeptide and/or donor polynucleotide.

Contacting the cells with a guide RNA and/or site-directed modifying polypeptide and/or donor polynucleotide may occur in any culture media and under any culture conditions that promote the survival of the cells. For example, cells may be suspended in any appropriate nutrient medium that is convenient, such as Iscove's modified DMEM or RPMI 1640, supplemented with fetal calf serum or heat inactivated goat serum (about 5-10%), L-glutamine, a thiol, particularly 2-mercaptoethanol, and antibiotics, e.g. penicillin and streptomycin. The culture may contain growth factors to which the cells are responsive. Growth factors, as defined herein, are molecules capable of promoting survival, growth and/or differentiation of cells, either in culture or in the intact tissue, through specific effects on a transmembrane receptor. Growth factors include polypeptides and non-polypeptide factors. Conditions that promote the survival of cells are typically permissive of nonhomologous end joining and homology-directed repair. In applications in which it is desirable to insert a polynucleotide sequence into a target DNA sequence, a polynucleotide comprising a donor sequence to be inserted is also provided to the cell. By a "donor sequence" or "donor polynucleotide" it is meant a nucleic acid sequence to be inserted at the cleavage site induced by a site-directed modifying polypeptide. The donor polynucleotide will contain sufficient homology to a genomic sequence at the cleavage site, e.g. 70%, 80%, 85%, 90%, 95%, or 100% homology with the nucleotide sequences flanking the cleavage site, e.g. within about 50 bases or less of the cleavage site, e.g. within about 30 bases, within about 15 bases, within about 10 bases, within about 5 bases, or immediately flanking the cleavage site, to support homology-directed repair between it and the genomic sequence to which it bears homology. Approximately 25, 50, 100, or 200 nucleotides, or more than 200 nucleotides, of sequence homology between a donor and a genomic sequence (or any integral value between 10 and 200 nucleotides, or more) will support homology-directed repair. Donor sequences can be of any length, e.g. 10 nucleotides or more, 50 nucleotides or more, 100 nucleotides or more, 250 nucleotides or more, 500 nucleotides or more, 1000 nucleotides or more, 5000 nucleotides or more, etc.

The donor sequence is typically not identical to the genomic sequence that it replaces. Rather, the donor sequence may contain at least one or more single base changes, insertions, deletions, inversions or rearrangements with respect to the genomic sequence, so long as sufficient homology is present to support homology-directed repair. In some embodiments, the donor sequence comprises a non-homologous sequence flanked by two regions of homology, such that homology-directed repair between the target DNA region and the two flanking sequences results in insertion of the non-homologous sequence at the target region. Donor sequences may also comprise a vector backbone containing sequences that are not homologous to the DNA region of interest and that are not intended for insertion into the DNA region of interest. Generally, the homologous region(s) of a donor sequence will have at least 50% sequence identity to a genomic sequence with which recombination is desired. In certain embodiments, 60%, 70%, 80%, 90%, 95%, 98%, 99%, or 99.9% sequence identity is present. Any value between 1% and 100% sequence identity can be present, depending upon the length of the donor polynucleotide. The donor sequence may comprise certain sequence differences as compared to the genomic sequence, e.g. restriction sites, nucleotide polymorphisms, selectable markers (e.g., drug resistance genes, fluorescent proteins, enzymes etc.), etc., which may be used to assess for successful insertion of the donor sequence at the cleavage site or in some cases may be used for other purposes (e.g., to signify expression at the targeted genomic locus). In some cases, if located in a coding region, such nucleotide sequence differences will not change the amino acid sequence, or will make silent amino acid changes (i.e., changes which do not affect the structure or function of the protein). Alternatively, these sequences differences may include flanking recombination sequences such as FLPs, IoxP sequences, or the like, that can be activated at a later time for removal of the marker sequence.

The donor sequence may be provided to the cell as single-stranded DNA, single-stranded RNA, double-stranded DNA, or double-stranded RNA. It may be introduced into a cell in linear or circular form. If introduced in linear form, the ends of the donor sequence may be protected (e.g., from exonucleolytic degradation) by methods known to those of skill in the art. For example, one or more dideoxynucleotide residues are added to the 3' terminus of a linear molecule and/or self-complementary oligonucleotides are ligated to one or both ends. See, for example, Chang et al. (1987) Proc. Natl. Acad Sci USA 84:4959-4963; Nehls et al. (1996) Science 272:886-889. Additional methods for protecting exogenous polynucleotides from degradation include, but are not limited to, addition of terminal amino group(s) and the use of modified internucleotide linkages such as, for example, phosphorothioates, phosphoramidates, and 0-methyl ribose or deoxyribose residues. As an alternative to protecting the termini of a linear donor sequence, additional lengths of sequence may be included outside of the regions of homology that can be degraded without impacting recombination. A donor sequence can be introduced into a cell as part of a vector molecule having additional sequences such as, for example, replication origins, promoters and genes encoding antibiotic resistance. Moreover, donor sequences can be introduced as naked nucleic acid, as nucleic acid complexed with an agent such as a liposome or poloxamer, or can be delivered by viruses (e.g., adenovirus, AAV), as described above for nucleic acids encoding a guide RNA and/or site-directed modifying polypeptide and/or donor polynucleotide.

Following the methods described above, a DNA region of interest may be cleaved and modified, i.e. "genetically modified", ex vivo. In some embodiments, as when a selectable marker has been inserted into the DNA region of interest, the population of cells may be enriched for those comprising the genetic modification by separating the genetically modified cells from the remaining population. Prior to enriching, the "genetically modified" cells may make up only about 1% or more (e.g., 2% or more, 3% or more, 4% or more, 5% or more, 6% or more, 7% or more, 8% or more, 9% or more, 10% or more, 15% or more, or 20% or more) of the cellular population. Separation of "genetically modified" cells may be achieved by any convenient separation technique appropriate for the selectable marker used. For example, if a fluorescent marker has been inserted, cells may be separated by fluorescence activated cell sorting, whereas if a cell surface marker has been inserted, cells may be separated from the heterogeneous population by affinity separation techniques, e.g. magnetic separation, affinity chromatography, "panning" with an affinity reagent attached to a solid matrix, or other convenient technique. Techniques providing accurate separation include fluorescence activated cell sorters, which can have varying degrees of sophistication, such as multiple color channels, low angle and obtuse light scattering detecting channels, impedance channels, etc. The cells may be selected against dead cells by employing dyes associated with dead cells (e.g. propidium iodide). Any technique may be employed which is not unduly detrimental to the viability of the genetically modified cells. Cell compositions that are highly enriched for cells comprising modified DNA are achieved in this manner. By "highly enriched", it is meant that the genetically modified cells will be 70% or more, 75% or more, 80% or more, 85% or more, 90% or more of the cell composition, for example, about 95% or more, or 98% or more of the cell composition. In other words, the composition may be a substantially pure composition of genetically modified cells.

Genetically modified cells produced by the methods described herein may be used immediately. Alternatively, the cells may be frozen at liquid nitrogen temperatures and stored for long periods of time, being thawed and capable of being reused. In such cases, the cells will usually be frozen in 10% dimethylsulfoxide (DMSO), 50% serum, 40% buffered medium, or some other such solution as is commonly used in the art to preserve cells at such freezing temperatures, and thawed in a manner as commonly known in the art for thawing frozen cultured cells.

The genetically modified cells may be cultured in vitro under various culture conditions. The cells may be expanded in culture, i.e. grown under conditions that promote their proliferation. Culture medium may be liquid or semi-solid, e.g. containing agar, methylcellulose, etc. The cell population may be suspended in an appropriate nutrient medium, such as Iscove's modified DMEM or RPMI 1640, normally supplemented with fetal calf serum (about 5-10%), L-glutamine, a thiol, particularly 2-mercaptoethanol, and antibiotics, e.g. penicillin and streptomycin. The culture may contain growth factors to which the regulatory T cells are responsive. Growth factors, as defined herein, are molecules capable of promoting survival, growth and/or differentiation of cells, either in culture or in the intact tissue, through specific effects on a transmembrane receptor. Growth factors include polypeptides and non-polypeptide factors.

Cells that have been genetically modified in this way may be transplanted to a subject for purposes such as gene therapy, e.g. to treat a disease or as an antiviral, antipathogenic, or anticancer therapeutic, for the production of genetically modified organisms in agriculture, or for biological research. The subject may be a neonate, a juvenile, or an adult. Of particular interest are mammalian subjects. Mammalian species that may be treated with the present methods include canines and felines; equines; bovines; ovines; etc. and primates, particularly humans. Animal models, particularly small mammals (e.g. mouse, rat, guinea pig, hamster, lagomorpha (e.g., rabbit), etc.) may be used for experimental investigations.

Cells may be provided to the subject alone or with a suitable substrate or matrix, e.g. to support their growth and/or organization in the tissue to which they are being transplanted. Usually, at least 1x10³ cells will be administered, for example 5x10³ cells, 1x10⁴ cells, 5x10⁴ cells, 1x10⁵ cells, 1 x 10⁶ cells or more. The cells may be introduced to the subject via any of the following routes: parenteral, subcutaneous, intravenous, intracranial, intraspinal, intraocular, or into spinal fluid. The cells may be introduced by injection, catheter, or the like. Examples of methods for local delivery, that is, delivery to the site of injury, include, e.g. through an Ommaya reservoir, e.g. for intrathecal delivery (see e.g. US Patent Nos. 5,222,982 and 5385582, incorporated herein by reference); by bolus injection, e.g. by a syringe, e.g. into a joint; by continuous infusion, e.g. by cannulation, e.g. with convection (see e.g. US Application No. 20070254842, incorporated herein by reference); or by implanting a device upon which the cells have been reversably affixed (see e.g. US Application Nos. 20080081064 and 20090196903, incorporated herein by reference). Cells may also be introduced into an embryo (e.g., a blastocyst) for the purpose of generating a transgenic animal (e.g., a transgenic mouse).

The number of administrations of treatment to a subject may vary. Introducing the genetically modified cells into the subject may be a one-time event; but in certain situations, such treatment may elicit improvement for a limited period of time and require an on-going series of repeated treatments. In other situations, multiple administrations of the genetically modified cells may be required before an effect is observed. The exact protocols depend upon the disease or condition, the stage of the disease and parameters of the individual subject being treated.

In other aspects of the disclosure, the guide RNA and/or site-directed modifying polypeptide and/or donor polynucleotide are employed to modify cellular DNA in vivo, again for purposes such as gene therapy, e.g. to treat a disease or as an antiviral, antipathogenic, or anticancer therapeutic, for the production of genetically modified organisms in agriculture, or for biological research. In these in vivo embodiments, a guide RNA and/or site-directed modifying polypeptide and/or donor polynucleotide are administered directly to the individual. A guide RNA and/or site-directed modifying polypeptide and/or donor polynucleotide may be administered by any of a number of well-known methods in the art for the administration of peptides, small molecules and nucleic acids to a subject. A guide RNA and/or site-directed modifying polypeptide and/or donor polynucleotide can be incorporated into a variety of formulations. More particularly, a guide RNA and/or site-directed modifying polypeptide and/or donor polynucleotide of the present invention can be formulated into pharmaceutical compositions by combination with appropriate pharmaceutically acceptable carriers or diluents.

Pharmaceutical preparations are compositions that include one or more a guide RNA and/or site-directed modifying polypeptide and/or donor polynucleotide present in a pharmaceutically acceptable vehicle. "Pharmaceutically acceptable vehicles" may be vehicles approved by a regulatory agency of the Federal or a state government or listed in the U.S. Pharmacopeia or other generally recognized pharmacopeia for use in mammals, such as humans. The term "vehicle" refers to a diluent, adjuvant, excipient, or carrier with which a compound of the invention is formulated for administration to a mammal. Such pharmaceutical vehicles can be lipids, e.g. liposomes, e.g. liposome dendrimers; liquids, such as water and oils, including those of petroleum, animal, vegetable or synthetic origin, such as peanut oil, soybean oil, mineral oil, sesame oil and the like, saline; gum acacia, gelatin, starch paste, talc, keratin, colloidal silica, urea, and the like. In addition, auxiliary, stabilizing, thickening, lubricating and coloring agents may be used. Pharmaceutical compositions may be formulated into preparations in solid, semi-solid, liquid or gaseous forms, such as tablets, capsules, powders, granules, ointments, solutions, suppositories, injections, inhalants, gels, microspheres, and aerosols. As such, administration of the a guide RNA and/or site-directed modifying polypeptide and/or donor polynucleotide can be achieved in various ways, including oral, buccal, rectal, parenteral, intraperitoneal, intradermal, transdermal, intratracheal, intraocular, etc., administration. The active agent may be systemic after administration or may be localized by the use of regional administration, intramural administration, or use of an implant that acts to retain the active dose at the site of implantation. The active agent may be formulated for immediate activity or it may be formulated for sustained release.

For some conditions, particularly central nervous system conditions, it may be necessary to formulate agents to cross the blood-brain barrier (BBB). One strategy for drug delivery through the BBB entails disruption of the BBB, either by osmotic means such as mannitol or leukotrienes, or biochemically by the use of vasoactive substances such as bradykinin. The potential for using BBB opening to target specific agents to brain tumors is also an option. A BBB disrupting agent can be co-administered with the therapeutic compositions of the invention when the compositions are administered by intravascular injection. Other strategies to go through the BBB may entail the use of endogenous transport systems, including Caveolin-1 mediated transcytosis, carrier-mediated transporters such as glucose and amino acid carriers, receptor-mediated transcytosis for insulin or transferrin, and active efflux transporters such as p-glycoprotein. Active transport moieties may also be conjugated to the therapeutic compounds for use in the invention to facilitate transport across the endothelial wall of the blood vessel. Alternatively, drug delivery of therapeutics agents behind the BBB may be by local delivery, for example by intrathecal delivery, e.g. through an Ommaya reservoir (see e.g. US Patent Nos. 5,222,982 and 5385582, incorporated herein by reference); by bolus injection, e.g. by a syringe, e.g. intravitreally or intracranially; by continuous infusion, e.g. by cannulation, e.g. with convection (see e.g. US Application No. 20070254842, incorporated here by reference); or by implanting a device upon which the agent has been reversably affixed (see e.g. US Application Nos. 20080081064 and 20090196903, incorporated herein by reference).

Typically, an effective amount of a guide RNA and/or site-directed modifying polypeptide and/or donor polynucleotide are provided. As discussed above with regard to ex vivo methods, an effective amount or effective dose of a guide RNA and/or site-directed modifying polypeptide and/or donor polynucleotide in vivo is the amount to induce a 2 fold increase or more in the amount of recombination observed between two homologous sequences relative to a negative control, e.g. a cell contacted with an empty vector or irrelevant polypeptide. The amount of recombination may be measured by any convenient method, e.g. as described above and known in the art. The calculation of the effective amount or effective dose of a guide RNA and/or site-directed modifying polypeptide and/or donor polynucleotide to be administered is within the skill of one of ordinary skill in the art, and will be routine to those persons skilled in the art. The final amount to be administered will be dependent upon the route of administration and upon the nature of the disorder or condition that is to be treated.

The effective amount given to a particular patient will depend on a variety of factors, several of which will differ from patient to patient. A competent clinician will be able to determine an effective amount of a therapeutic agent to administer to a patient to halt or reverse the progression the disease condition as required. Utilizing LD50 animal data, and other information available for the agent, a clinician can determine the maximum safe dose for an individual, depending on the route of administration. For instance, an intravenously administered dose may be more than an intrathecally administered dose, given the greater body of fluid into which the therapeutic composition is being administered. Similarly, compositions which are rapidly cleared from the body may be administered at higher doses, or in repeated doses, in order to maintain a therapeutic concentration. Utilizing ordinary skill, the competent clinician will be able to optimize the dosage of a particular therapeutic in the course of routine clinical trials.

For inclusion in a medicament, a guide RNA and/or site-directed modifying polypeptide and/or donor polynucleotide may be obtained from a suitable commercial source. As a general proposition, the total pharmaceutically effective amount of the a guide RNA and/or site-directed modifying polypeptide and/or donor polynucleotide administered parenterally per dose will be in a range that can be measured by a dose response curve.

Therapies based on a guide RNA and/or site-directed modifying polypeptide and/or donor polynucleotides, i.e. preparations of a guide RNA and/or site-directed modifying polypeptide and/or donor polynucleotide to be used for therapeutic administration, must be sterile. Sterility is readily accomplished by filtration through sterile filtration membranes (e.g., 0.2 µm membranes). Therapeutic compositions generally are placed into a container having a sterile access port, for example, an intravenous solution bag or vial having a stopper pierceable by a hypodermic injection needle. The therapies based on a guide RNA and/or site-directed modifying polypeptide and/or donor polynucleotide may be stored in unit or multi-dose containers, for example, sealed ampules or vials, as an aqueous solution or as a lyophilized formulation for reconstitution. As an example of a lyophilized formulation, 10-ml vials are filled with 5 ml of sterile-filtered 1% (w/v) aqueous solution of compound, and the resulting mixture is lyophilized. The infusion solution is prepared by reconstituting the lyophilized compound using bacteriostatic Water-for-Injection.

Pharmaceutical compositions can include, depending on the formulation desired, pharmaceutically-acceptable, non-toxic carriers of diluents, which are defined as vehicles commonly used to formulate pharmaceutical compositions for animal or human administration. The diluent is selected so as not to affect the biological activity of the combination. Examples of such diluents are distilled water, buffered water, physiological saline, PBS, Ringer's solution, dextrose solution, and Hank's solution. In addition, the pharmaceutical composition or formulation can include other carriers, adjuvants, or non-toxic, nontherapeutic, nonimmunogenic stabilizers, excipients and the like. The compositions can also include additional substances to approximate physiological conditions, such as pH adjusting and buffering agents, toxicity adjusting agents, wetting agents and detergents.

The composition can also include any of a variety of stabilizing agents, such as an antioxidant for example. When the pharmaceutical composition includes a polypeptide, the polypeptide can be complexed with various well-known compounds that enhance the in vivo stability of the polypeptide, or otherwise enhance its pharmacological properties (e.g., increase the half-life of the polypeptide, reduce its toxicity, enhance solubility or uptake). Examples of such modifications or complexing agents include sulfate, gluconate, citrate and phosphate. The nucleic acids or polypeptides of a composition can also be complexed with molecules that enhance their in vivo attributes. Such molecules include, for example, carbohydrates, polyamines, amino acids, other peptides, ions (e.g., sodium, potassium, calcium, magnesium, manganese), and lipids.

Further guidance regarding formulations that are suitable for various types of administration can be found in Remington's Pharmaceutical Sciences, Mace Publishing Company, Philadelphia, Pa., 17th ed. (1985). For a brief review of methods for drug delivery, see, Langer, Science 249:1527-1533 (1990).

The pharmaceutical compositions can be administered for prophylactic and/or therapeutic treatments. Toxicity and therapeutic efficacy of the active ingredient can be determined according to standard pharmaceutical procedures in cell cultures and/or experimental animals, including, for example, determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Therapies that exhibit large therapeutic indices are preferred.

The data obtained from cell culture and/or animal studies can be used in formulating a range of dosages for humans. The dosage of the active ingredient typically lines within a range of circulating concentrations that include the ED50 with low toxicity. The dosage can vary within this range depending upon the dosage form employed and the route of administration utilized. The components used to formulate the pharmaceutical compositions are preferably of high purity and are substantially free of potentially harmful contaminants (e.g., at least National Food (NF) grade, generally at least analytical grade, and more typically at least pharmaceutical grade). Moreover, compositions intended for in vivo use are usually sterile. To the extent that a given compound must be synthesized prior to use, the resulting product is typically substantially free of any potentially toxic agents, particularly any endotoxins, which may be present during the synthesis or purification process. Compositions for parental administration are also sterile, substantially isotonic and made under GMP conditions.

The effective amount of a therapeutic composition to be given to a particular patient will depend on a variety of factors, several of which will differ from patient to patient. A competent clinician will be able to determine an effective amount of a therapeutic agent to administer to a patient to halt or reverse the progression the disease condition as required. Utilizing LD50 animal data, and other information available for the agent, a clinician can determine the maximum safe dose for an individual, depending on the route of administration. For instance, an intravenously administered dose may be more than an intrathecally administered dose, given the greater body of fluid into which the therapeutic composition is being administered. Similarly, compositions which are rapidly cleared from the body may be administered at higher doses, or in repeated doses, in order to maintain a therapeutic concentration. Utilizing ordinary skill, the competent clinician will be able to optimize the dosage of a particular therapeutic in the course of routine clinical trials.

### Genetically Modified Host Cells

The present disclosure provides genetically modified host cells, including isolated genetically modified host cells, where a genetically modified host cell comprises (has been genetically modified with: 1) an exogenous guide RNA; 2) an exogenous nucleic acid comprising a nucleotide sequence encoding a guide RNA; 3) an exogenous site-directed modifying polypeptide (e.g., a naturally occurring Cas9; a modified, i.e., mutated or variant, Cas9; a chimeric Cas9; etc.); 4) an exogenous nucleic acid comprising a nucleotide sequence encoding a site-directed modifying polypeptide; or 5) any combination of the above. A genetically modified cell is generated by genetically modifying a host cell with, for example: 1) an exogenous guide RNA; 2) an exogenous nucleic acid comprising a nucleotide sequence encoding a guide RNA; 3) an exogenous site-directed modifying polypeptide; 4) an exogenous nucleic acid comprising a nucleotide sequence encoding a site-directed modifying polypeptide; or 5) any combination of the above.).

All cells suitable to be a target cell are also suitable to be a genetically modified host cell. For example, a genetically modified host cells of interest can be a cell from any organism (e.g. a bacterial cell, an archaeal cell, a cell of a single-cell eukaryotic organism, a plant cell, an algal cell, e.g., *Botryococcus braunii, Chlamydomonas reinhardtii, Nannochloropsis gaditana, Chlorella pyrenoidosa, Sargassum patens C*. *Agardh,* and the like, a fungal cell (e.g., a yeast cell), an animal cell, a cell from an invertebrate animal (e.g. fruit fly, cnidarian, echinoderm, nematode, etc.), a cell from a vertebrate animal (e.g., fish, amphibian, reptile, bird, mammal), a cell from a mammal (e.g., a pig, a cow, a goat, a sheep, a rodent, a rat, a mouse, a non-human primate, a human, etc.), etc.

In some embodiments, a genetically modified host cell has been genetically modified with an exogenous nucleic acid comprising a nucleotide sequence encoding a site-directed modifying polypeptide (e.g., a naturally occurring Cas9; a modified, i.e., mutated or variant, Cas9; a chimeric Cas9; etc.). The DNA of a genetically modified host cell can be targeted for modification by introducing into the cell a guide RNA (or a DNA encoding a guide RNA, which determines the genomic location/sequence to be modified) and optionally a donor nucleic acid. In some embodiments, the nucleotide sequence encoding a site-directed modifying polypeptide is operably linked to an inducible promoter (e.g., heat shock promoter, Tetracycline-regulated promoter, Steroid-regulated promoter, Metal-regulated promoter, estrogen receptor-regulated promoter, etc.). In some embodiments, the nucleotide sequence encoding a site-directed modifying polypeptide is operably linked to a spatially restricted and/or temporally restricted promoter (e.g., a tissue specific promoter, a cell type specific promoter, etc.). In some embodiments, the nucleotide sequence encoding a site-directed modifying polypeptide is operably linked to a constitutive promoter.

In some embodiments, a genetically modified host cell is *in vitro.* In some embodiments, a genetically modified host cell is *in vivo.* In some embodiments, a genetically modified host cell is a prokaryotic cell or is derived from a prokaryotic cell. In some embodiments, a genetically modified host cell is a bacterial cell or is derived from a bacterial cell. In some embodiments, a genetically modified host cell is an archaeal cell or is derived from an archaeal cell. In some embodiments, a genetically modified host cell is a eukaryotic cell or is derived from a eukaryotic cell. In some embodiments, a genetically modified host cell is a plant cell or is derived from a plant cell. In some embodiments, a genetically modified host cell is an animal cell or is derived from an animal cell. In some embodiments, a genetically modified host cell is an invertebrate cell or is derived from an invertebrate cell. In some embodiments, a genetically modified host cell is a vertebrate cell or is derived from a vertebrate cell. In some embodiments, a genetically modified host cell is a mammalian cell or is derived from a mammalian cell. In some embodiments, a genetically modified host cell is a rodent cell or is derived from a rodent cell. In some embodiments, a genetically modified host cell is a human cell or is derived from a human cell.

The present disclosure further provides progeny of a genetically modified cell, where the progeny can comprise the same exogenous nucleic acid or polypeptide as the genetically modified cell from which it was derived. The present disclosure further provides a composition comprising a genetically modified host cell.

### Genetically modified stem cells and genetically modified progenitor cells

In some embodiments, a genetically modified host cell is a genetically modified stem cell or progenitor cell. Suitable host cells include, e.g., stem cells (adult stem cells, embryonic stem cells, iPS cells, etc.) and progenitor cells (e.g., cardiac progenitor cells, neural progenitor cells, etc.). Suitable host cells include mammalian stem cells and progenitor cells, including, e.g., rodent stem cells, rodent progenitor cells, human stem cells, human progenitor cells, etc. Suitable host cells include *in vitro* host cells, e.g., isolated host cells.

In some embodiments, a genetically modified host cell comprises an exogenous guide RNA nucleic acid. In some embodiments, a genetically modified host cell comprises an exogenous nucleic acid comprising a nucleotide sequence encoding a guide RNA. In some embodiments, a genetically modified host cell comprises an exogenous site-directed modifying polypeptide (e.g., a naturally occurring Cas9; a modified, i.e., mutated or variant, Cas9; a chimeric Cas9; etc.). In some embodiments, a genetically modified host cell comprises an exogenous nucleic acid comprising a nucleotide sequence encoding a site-directed modifying polypeptide. In some embodiments, a genetically modified host cell comprises exogenous nucleic acid comprising a nucleotide sequence encoding 1) a guide RNA and 2) a site-directed modifying polypeptide.

In some cases, the site-directed modifying polypeptide comprises an amino acid sequence having at least about 75%, at least about 80%, at least about 85%, at least about 90%, at least about 95%, at least about 99%, or 100%, amino acid sequence identity to amino acids 7-166 and/or 731-1003 of SEQ ID NO: 8, or to the corresponding portions in any of the amino acid sequences set forth as SEQ ID NOs: 1-800.

### Compositions

The present disclosure provides a composition comprising a guide RNA and/or a site-directed modifying polypeptide. In some cases, the site-directed modifying polypeptide is a chimeric polypeptide. A composition is useful for carrying out a method of the present disclosure, e.g., a method for site-specific modification of a target DNA; a method for site-specific modification of a polypeptide associated with a target DNA; etc.

### Compositions comprising a guide RNA

The present disclosure provides a composition comprising a guide RNA. The composition can comprise, in addition to the guide RNA, one or more of: a salt, e.g., NaCl, MgCl₂, KCI, MgSO₄, etc.; a buffering agent, e.g., a Tris buffer, N-(2-Hydroxyethyl)piperazine-N'-(2-ethanesulfonic acid) (HEPES), 2-(N-Morpholino)ethanesulfonic acid (MES), MES sodium salt, 3-(N-Morpholino)propanesulfonic acid (MOPS), N-tris[Hydroxymethyl]methy1-3-aminopropanesulfonic acid (TAPS), etc.; a solubilizing agent; a detergent, e.g., a non-ionic detergent such as Tween-20, etc.; a nuclease inhibitor; and the like. For example, in some cases, a composition comprises a guide RNA and a buffer for stabilizing nucleic acids.

In some embodiments, a guide RNA present in a composition is pure, e.g., at least about 75%, at least about 80%, at least about 85%, at least about 90%, at least about 95%, at least about 98%, at least about 99%, or more than 99% pure, where "% purity" means that guide RNA is the recited percent free from other macromolecules, or contaminants that may be present during the production of the guide RNA.

### Compositions comprising a chimeric polypeptide

The present disclosure provides a composition a chimeric polypeptide. The composition can comprise, in addition to the guide RNA, one or more of: a salt, e.g., NaCl, MgCl₂, KCI, MgSO₄, etc.; a buffering agent, e.g., a Tris buffer, HEPES, MES, MES sodium salt, MOPS, TAPS, etc.; a solubilizing agent; a detergent, e.g., a non-ionic detergent such as Tween-20, etc.; a protease inhibitor; a reducing agent (e.g., dithiothreitol); and the like.

In some embodiments, a chimeric polypeptide present in a composition is pure, e.g., at least about 75%, at least about 80%, at least about 85%, at least about 90%, at least about 95%, at least about 98%, at least about 99%, or more than 99% pure, where "% purity" means that the site-directed modifying polypeptide is the recited percent free from other proteins, other macromolecules, or contaminants that may be present during the production of the chimeric polypeptide.

### Compositions comprising a guide RNA and a site-directed modifying polypeptide

The present disclosure provides a composition comprising: (i) a guide RNA or a DNA polynucleotide encoding the same; and ii) a site-directed modifying polypeptide, or a polynucleotide encoding the same. In some cases, the site-directed modifying polypeptide is a chimeric site-directed modifying polypeptide. In other cases, the site-directed modifying polypeptide is a naturally-occurring site-directed modifying polypeptide. In some instances, the site-directed modifying polypeptide exhibits enzymatic activity that modifies a target DNA. In other cases, the site-directed modifying polypeptide exhibits enzymatic activity that modifies a polypeptide that is associated with a target DNA. In still other cases, the site-directed modifying polypeptide modulates transcription of the target DNA.

The present disclosure provides a composition comprising: (i) a guide RNA, as described above, or a DNA polynucleotide encoding the same, the guide RNA comprising: (a) a first segment comprising a nucleotide sequence that is complementary to a sequence in a target DNA; and (b) a second segment that interacts with a site-directed modifying polypeptide; and (ii) the site-directed modifying polypeptide, or a polynucleotide encoding the same, the site-directed modifying polypeptide comprising: (a) an RNA-binding portion that interacts with the guide RNA; and (b) an activity portion that exhibits site-directed enzymatic activity, wherein the site of enzymatic activity is determined by the guide RNA.

In some instances, a composition comprises: a composition comprising: (i) a guide RNA, the guide RNA comprising: (a) a first segment comprising a nucleotide sequence that is complementary to a sequence in a target DNA; and (b) a second segment that interacts with a site-directed modifying polypeptide; and (ii) the site-directed modifying polypeptide, the site-directed modifying polypeptide comprising: (a) an RNA-binding portion that interacts with the guide RNA; and (b) an activity portion that exhibits site-directed enzymatic activity, wherein the site of enzymatic activity is determined by the guide RNA.

In other embodiments, a composition comprises: (i) a polynucleotide encoding a guide RNA, the guide RNA comprising: (a) a first segment comprising a nucleotide sequence that is complementary to a sequence in a target DNA; and (b) a second segment that interacts with a site-directed modifying polypeptide; and (ii) a polynucleotide encoding the site-directed modifying polypeptide, the site-directed modifying polypeptide comprising: (a) an RNA-binding portion that interacts with the guide RNA; and (b) an activity portion that exhibits site-directed enzymatic activity, wherein the site of enzymatic activity is determined by the guide RNA.

In some embodiments, a composition includes both RNA molecules of a double-molecule guide RNA. As such, in some embodiments, a composition includes an activator-RNA that comprises a duplex-forming segment that is complementary to the duplex-forming segment of a targeter-. The duplex-forming segments of the activator-RNA and the targeter-RNA hybridize to form the dsRNA duplex of the protein-binding segment of the guide RNA. The targeter-RNA further provides the DNA-targeting segment (single stranded) of the guide RNA and therefore targets the guide RNA to a specific sequence within the target DNA. As one non-limiting example, the duplex-forming segment of the activator-RNA comprises a nucleotide sequence that has at least about 70%, at least about 80%, at least about 90%, at least about 95%, at least about 98%, or 100% identity with a tracrRNA sequence set out in Supplementary Table S5. As another non-limiting example, the duplex-forming segment of the targeter-RNA comprises a nucleotide sequence that has at least about 70%, at least about 80%, at least about 90%, at least about 95%, at least about 98%, or 100% identity with a CRISPR repeat sequence set out in Supplementary Table S5.

The present disclosure provides a composition comprising: (i) a guide RNA, or a DNA polynucleotide encoding the same, the guide RNA comprising: (a) a first segment comprising a nucleotide sequence that is complementary to a sequence in a target DNA; and (b) a second segment that interacts with a site-directed modifying polypeptide; and (ii) the site-directed modifying polypeptide, or a polynucleotide encoding the same, the site-directed modifying polypeptide comprising: (a) an RNA-binding portion that interacts with the guide RNA; and (b) an activity portion that modulates transcription within the target DNA, wherein the site of modulated transcription within the target DNA is determined by the guide RNA.

For example, in some cases, a composition comprises: (i) a guide RNA, the guide RNA comprising: (a) a first segment comprising a nucleotide sequence that is complementary to a sequence in a target DNA; and (b) a second segment that interacts with a site-directed modifying polypeptide; and (ii) the site-directed modifying polypeptide, the site-directed modifying polypeptide comprising: (a) an RNA-binding portion that interacts with the guide RNA; and (b) an activity portion that modulates transcription within the target DNA, wherein the site of modulated transcription within the target DNA is determined by the guide RNA.

As another example, in some cases, a composition comprises: (i) a DNA polynucleotide encoding a guide RNA, the guide RNA comprising: (a) a first segment comprising a nucleotide sequence that is complementary to a sequence in a target DNA; and (b) a second segment that interacts with a site-directed modifying polypeptide; and (ii) a polynucleotide encoding the site-directed modifying polypeptide, the site-directed modifying polypeptide comprising: (a) an RNA-binding portion that interacts with the guide RNA; and (b) an activity portion that modulates transcription within the target DNA, wherein the site of modulated transcription within the target DNA is determined by the guide RNA. A composition can comprise, in addition to i) a guide RNA, or a DNA polynucleotide encoding the same; and ii) a site-directed modifying polypeptide, or a polynucleotide encoding the same, one or more of: a salt, e.g., NaCl, MgCl₂, KCl, MgSO₄, etc.; a buffering agent, e.g., a Tris buffer, HEPES, MES, MES sodium salt, MOPS, TAPS, etc.; a solubilizing agent; a detergent, e.g., a non-ionic detergent such as Tween-20, etc.; a protease inhibitor; a reducing agent (e.g., dithiothreitol); and the like.

In some cases, the components of the composition are individually pure, e.g., each of the components is at least about 75%, at least about 80%, at least about 90%, at least about 95%, at least about 98%, at least about 99%, or at least 99%, pure. In some cases, the individual components of a composition are pure before being added to the composition.

For example, in some embodiments, a site-directed modifying polypeptide present in a composition is pure, e.g., at least about 75%, at least about 80%, at least about 85%, at least about 90%, at least about 95%, at least about 98%, at least about 99%, or more than 99% pure, where "% purity" means that the site-directed modifying polypeptide is the recited percent free from other proteins (e.g., proteins other than the site-directed modifying polypeptide), other macromolecules, or contaminants that may be present during the production of the site-directed modifying polypeptide.

### Kits

The present disclosure provides kits for carrying out a method. A kit can include one or more of: a site-directed modifying polypeptide; a nucleic acid comprising a nucleotide encoding a site-directed modifying polypeptide; a guide RNA; a nucleic acid comprising a nucleotide sequence encoding a guide RNA; an activator-RNA; a nucleic acid comprising a nucleotide sequence encoding an activator-RNA; a targeter-RNA; and a nucleic acid comprising a nucleotide sequence encoding a targeter-RNA. A site-directed modifying polypeptide; a nucleic acid comprising a nucleotide encoding a site-directed modifying polypeptide; a guide RNA; a nucleic acid comprising a nucleotide sequence encoding a guide RNA; an activator-RNA; a nucleic acid comprising a nucleotide sequence encoding an activator-RNA; a targeter-RNA; and a nucleic acid comprising a nucleotide sequence encoding a targeter-RNA, are described in detail above. A kit may comprise a complex that comprises two or more of: a site-directed modifying polypeptide; a nucleic acid comprising a nucleotide encoding a site-directed modifying polypeptide; a guide RNA; a nucleic acid comprising a nucleotide sequence encoding a guide RNA; an activator-RNA; a nucleic acid comprising a nucleotide sequence encoding an activator-RNA; a targeter-RNA; and a nucleic acid comprising a nucleotide sequence encoding a targeter-RNA. In some embodiments, a kit comprises a site-directed modifying polypeptide, or a polynucleotide encoding the same. In some embodiments, the site-directed modifying polypeptide comprises: (a) an RNA-binding portion that interacts with the guide RNA; and (b) an activity portion that modulates transcription within the target DNA, wherein the site of modulated transcription within the target DNA is determined by the guide RNA. In some cases, the activity portion of the site-directed modifying polypeptide exhibits reduced or inactivated nuclease activity. In some cases, the site-directed modifying polypeptide is a chimeric site-directed modifying polypeptide.

In some embodiments, a kit comprises: a site-directed modifying polypeptide, or a polynucleotide encoding the same, and a reagent for reconstituting and/or diluting the site-directed modifying polypeptide. In other embodiments, a kit comprises a nucleic acid (e.g., DNA, RNA) comprising a nucleotide encoding a site-directed modifying polypeptide. In some embodiments, a kit comprises: a nucleic acid (e.g., DNA, RNA) comprising a nucleotide encoding a site-directed modifying polypeptide; and a reagent for reconstituting and/or diluting the site-directed modifying polypeptide.

A kit comprising a site-directed modifying polypeptide, or a polynucleotide encoding the same, can further include one or more additional reagents, where such additional reagents can be selected from: a buffer for introducing the site-directed modifying polypeptide into a cell; a wash buffer; a control reagent; a control expression vector or RNA polynucleotide; a reagent for *in vitro* production of the site-directed modifying polypeptide from DNA, and the like. In some cases, the site-directed modifying polypeptide included in a kit is a chimeric site-directed modifying polypeptide, as described above.

In some embodiments, a kit comprises a guide RNA, or a DNA polynucleotide encoding the same, the guide RNA comprising: (a) a first segment comprising a nucleotide sequence that is complementary to a sequence in a target DNA; and (b) a second segment that interacts with a site-directed modifying polypeptide. In some embodiments, the guide RNA further comprises a third segment (as described above). In some embodiments, a kit comprises: (i) a guide RNA, or a DNA polynucleotide encoding the same, the guide RNA comprising: (a) a first segment comprising a nucleotide sequence that is complementary to a sequence in a target DNA; and (b) a second segment that interacts with a site-directed modifying polypeptide; and (ii) a site-directed modifying polypeptide, or a polynucleotide encoding the same, the site-directed modifying polypeptide comprising: (a) an RNA-binding portion that interacts with the guide RNA; and (b) an activity portion that exhibits site-directed enzymatic activity, wherein the site of enzymatic activity is determined by the guide RNA. In some embodiments, the activity portion of the site-directed modifying polypeptide does not exhibit enzymatic activity (comprises an inactivated nuclease, e.g., via mutation). In some cases, the kit comprises a guide RNA and a site-directed modifying polypeptide. In other cases, the kit comprises: (i) a nucleic acid comprising a nucleotide sequence encoding a guide RNA; and (ii) a nucleic acid comprising a nucleotide sequence encoding site-directed modifying polypeptide. As another example, a kit can include: (i) a guide RNA, or a DNA polynucleotide encoding the same, comprising: (a) a first segment comprising a nucleotide sequence that is complementary to a sequence in a target DNA; and (b) a second segment that interacts with a site-directed modifying polypeptide; and (ii) the site-directed modifying polypeptide, or a polynucleotide encoding the same, comprising: (a) an RNA-binding portion that interacts with the guide RNA; and (b) an activity portion that that modulates transcription within the target DNA, wherein the site of modulated transcription within the target DNA is determined by the guide RNA In some cases, the kit comprises: (i) a guide RNA; and a site-directed modifying polypeptide. In other cases, the kit comprises: (i) a nucleic acid comprising a nucleotide sequence encoding a guide RNA; and (ii) a nucleic acid comprising a nucleotide sequence encoding site-directed modifying polypeptide. The present disclosure provides a kit comprising: (1) a recombinant expression vector comprising (i) a nucleotide sequence encoding a guide RNA, wherein the guide RNA comprises: (a) a first segment comprising a nucleotide sequence that is complementary to a sequence in a target DNA; and (b) a second segment that interacts with a site-directed modifying polypeptide; and (ii) a nucleotide sequence encoding the site-directed modifying polypeptide, wherein the site-directed modifying polypeptide comprises: (a) an RNA-binding portion that interacts with the guide RNA; and (b) an activity portion that exhibits site-directed enzymatic activity, wherein the site of enzymatic activity is determined by the guide RNA.; and (2) a reagent for reconstitution and/or dilution of the expression vector.

The present disclosure provides a kit comprising: (1) a recombinant expression vector comprising: (i) a nucleotide sequence encoding a guide RNA, wherein the guide RNA comprises: (a) a first segment comprising a nucleotide sequence that is complementary to a sequence in a target DNA; and (b) a second segment that interacts with a site-directed modifying polypeptide; and (ii) a nucleotide sequence encoding the site-directed modifying polypeptide, wherein the site-directed modifying polypeptide comprises: (a) an RNA-binding portion that interacts with the guide RNA; and (b) an activity portion that modulates transcription within the target DNA, wherein the site of modulated transcription within the target DNA is determined by the guide RNA; and (2) a reagent for reconstitution and/or dilution of the recombinant expression vector.

The present disclosure provides a kit comprising: (1) a recombinant expression vector comprising a nucleic acid comprising a nucleotide sequence that encodes a DNA targeting RNA comprising: (i) a first segment comprising a nucleotide sequence that is complementary to a sequence in a target DNA; and (ii) a second segment that interacts with a site-directed modifying polypeptide; and (2) a reagent for reconstitution and/or dilution of the recombinant expression vector. In some embodiments of this kit, the kit comprises: a recombinant expression vector comprising a nucleotide sequence that encodes a site-directed modifying polypeptide, wherein the site-directed modifying polypeptide comprises: (a) an RNA-binding portion that interacts with the guide RNA; and (b) an activity portion that exhibits site-directed enzymatic activity, wherein the site of enzymatic activity is determined by the guide RNA. In other embodiments of this kit, the kit comprises: a recombinant expression vector comprising a nucleotide sequence that encodes a site-directed modifying polypeptide, wherein the site-directed modifying polypeptide comprises: (a) an RNA-binding portion that interacts with the guide RNA; and (b) an activity portion that modulates transcription within the target DNA, wherein the site of modulated transcription within the target DNA is determined by the guide RNA.

In some embodiments of any of the above kits, the kit comprises an activator-RNA or a targeter-RNA. In some embodiments of any of the above kits, the kit comprises a single-molecule guide RNA. In some embodiments of any of the above kits, the kit comprises two or more double-molecule or single-molecule guide RNAs. In some embodiments of any of the above kits, a guide RNA (e.g., including two or more guide RNAs) can be provided as an array (e.g., an array of RNA molecules, an array of DNA molecules encoding the guide RNA(s), etc.). Such kits can be useful, for example, for use in conjunction with the above described genetically modified host cells that comprise a site-directed modifying polypeptide. In some embodiments of any of the above kits, the kit further comprises a donor polynucleotide to effect the desired genetic modification. Components of a kit can be in separate containers; or can be combined in a single container.

In some cases, a kit further comprises one or more variant Cas9 site-directed polypeptides that exhibits reduced endodeoxyribonuclease activity relative to wild-type Cas9.

In some cases, a kit further comprises one or more nucleic acids comprising a nucleotide sequence encoding a variant Cas9 site-directed polypeptide that exhibits reduced endodeoxyribonuclease activity relative to wild-type Cas9.

Any of the above-described kits can further include one or more additional reagents, where such additional reagents can be selected from: a dilution buffer; a reconstitution solution; a wash buffer; a control reagent; a control expression vector or RNA polynucleotide; a reagent for *in vitro* production of the site-directed modifying polypeptide from DNA, and the like.

In addition to above-mentioned components, a kit can further include instructions for using the components of the kit to practice the methods. The instructions for practicing the methods are generally recorded on a suitable recording medium. For example, the instructions may be printed on a substrate, such as paper or plastic, etc. As such, the instructions may be present in the kits as a package insert, in the labeling of the container of the kit or components thereof (i.e., associated with the packaging or subpackaging) etc. In other embodiments, the instructions are present as an electronic storage data file present on a suitable computer readable storage medium, e.g. CD-ROM, diskette, flash drive, etc. In yet other embodiments, the actual instructions are not present in the kit, but means for obtaining the instructions from a remote source, e.g. via the internet, are provided. An example of this embodiment is a kit that includes a web address where the instructions can be viewed and/or from which the instructions can be downloaded. As with the instructions, this means for obtaining the instructions is recorded on a suitable substrate.

### Non-Human Genetically Modified Organisms

In some embodiments, a genetically modified host cell has been genetically modified with an exogenous nucleic acid comprising a nucleotide sequence encoding a site-directed modifying polypeptide (e.g., a naturally occurring Cas9; a modified, i.e., mutated or variant, Cas9; a chimeric Cas9; etc.). If such a cell is a eukaryotic single-cell organism, then the modified cell can be considered a genetically modified organism. In some embodiments, the non-human genetically modified organism is a Cas9 transgenic multicellular organism.

In some embodiments, a genetically modified non-human host cell (e.g., a cell that has been genetically modified with an exogenous nucleic acid comprising a nucleotide sequence encoding a site-directed modifying polypeptide, e.g., a naturally occurring Cas9; a modified, i.e., mutated or variant, Cas9; a chimeric Cas9; etc.) can generate a genetically modified nonhuman organism (e.g., a mouse, a fish, a frog, a fly, a worm, etc.). For example, if the genetically modified host cell is a pluripotent stem cell (i.e., PSC) or a germ cell (e.g., sperm, oocyte, etc.), an entire genetically modified organism can be derived from the genetically modified host cell. In some embodiments, the genetically modified host cell is a pluripotent stem cell (e.g., ESC, iPSC, pluripotent plant stem cell, etc.) or a germ cell (e.g., sperm cell, oocyte, etc.), either in vivo or in vitro, that can give rise to a genetically modified organism. In some embodiments the genetically modified host cell is a vertebrate PSC (e.g., ESC, iPSC, etc.) and is used to generate a genetically modified organism (e.g. by injecting a PSC into a blastocyst to produce a chimeric/mosaic animal, which could then be mated to generate non-chimeric/non-mosaic genetically modified organisms; grafting in the case of plants; etc.). Any convenient method/protocol for producing a genetically modified organism, including the methods described herein, is suitable for producing a genetically modified host cell comprising an exogenous nucleic acid comprising a nucleotide sequence encoding a site-directed modifying polypeptide (e.g., a naturally occurring Cas9; a modified, i.e., mutated or variant, Cas9; a chimeric Cas9; etc.). Methods of producing genetically modified organisms are known in the art. For example, see Cho et al., Curr Protoc Cell Biol. 2009 Mar;Chapter 19:Unit 19.11: Generation of transgenic mice; Gama et al., Brain Struct Funct. 2010 Mar;214(2-3):91-109. Epub 2009 Nov 25: Animal transgenesis: an overview; Husaini et al., GM Crops. 2011 Jun-Dec;2(3): 150-62. Epub 2011 Jun 1: Approaches for gene targeting and targeted gene expression in plants.

In some embodiments, a genetically modified organism comprises a target cell for methods of the invention, and thus can be considered a source for target cells. For example, if a genetically modified cell comprising an exogenous nucleic acid comprising a nucleotide sequence encoding a site-directed modifying polypeptide (e.g., a naturally occurring Cas9; a modified, i.e., mutated or variant, Cas9; a chimeric Cas9; etc.) is used to generate a genetically modified organism, then the cells of the genetically modified organism comprise the exogenous nucleic acid comprising a nucleotide sequence encoding a site-directed modifying polypeptide (e.g., a naturally occurring Cas9; a modified, i.e., mutated or variant, Cas9; a chimeric Cas9; etc.). In some such embodiments, the DNA of a cell or cells of the genetically modified organism can be targeted for modification by introducing into the cell or cells a guide RNA (or a DNA encoding a guide RNA) and optionally a donor nucleic acid. For example, the introduction of a guide RNA (or a DNA encoding a guide RNA) into a subset of cells (e.g., brain cells, intestinal cells, kidney cells, lung cells, blood cells, etc.) of the genetically modified organism can target the DNA of such cells for modification, the genomic location of which will depend on the DNA-targeting sequence of the introduced guide RNA.

In some embodiments, a genetically modified organism is a source of target cells for methods of the invention. For example, a genetically modified organism comprising cells that are genetically modified with an exogenous nucleic acid comprising a nucleotide sequence encoding a site-directed modifying polypeptide (e.g., a naturally occurring Cas9; a modified, i.e., mutated or variant, Cas9; a chimeric Cas9; etc.) can provide a source of genetically modified cells, for example PSCs (e.g., ESCs, iPSCs, sperm, oocytes, etc.), neurons, progenitor cells, cardiomyocytes, etc.

In some embodiments, a genetically modified cell is a PSC comprising an exogenous nucleic acid comprising a nucleotide sequence encoding a site-directed modifying polypeptide (e.g., a naturally occurring Cas9; a modified, i.e., mutated or variant, Cas9; a chimeric Cas9; etc.). As such, the PSC can be a target cell such that the DNA of the PSC can be targeted for modification by introducing into the PSC a guide RNA (or a DNA encoding a guide RNA) and optionally a donor nucleic acid, and the genomic location of the modification will depend on the DNA-targeting sequence of the introduced guide RNA. Thus, in some embodiments, the methods described herein can be used to modify the DNA (e.g., delete and/or replace any desired genomic location) of PSCs derived from a genetically modified organism. Such modified PSCs can then be used to generate organisms having both (i) an exogenous nucleic acid comprising a nucleotide sequence encoding a site-directed modifying polypeptide (e.g., a naturally occurring Cas9; a modified, i.e., mutated or variant, Cas9; a chimeric Cas9; etc.) and (ii) a DNA modification that was introduced into the PSC.

An exogenous nucleic acid comprising a nucleotide sequence encoding a site-directed modifying polypeptide (e.g., a naturally occurring Cas9; a modified, i.e., mutated or variant, Cas9; a chimeric Cas9; etc.) can be under the control of (i.e., operably linked to) an unknown promoter (e.g., when the nucleic acid randomly integrates into a host cell genome) or can be under the control of (i.e., operably linked to) a known promoter. Suitable known promoters can be any known promoter and include constitutively active promoters (e.g., CMV promoter), inducible promoters (e.g., heat shock promoter, Tetracycline-regulated promoter, Steroid-regulated promoter, Metal-regulated promoter, estrogen receptor-regulated promoter, etc.), spatially restricted and/or temporally restricted promoters (e.g., a tissue specific promoter, a cell type specific promoter, etc.), etc.

A genetically modified organism (e.g. an organism whose cells comprise a nucleotide sequence encoding a site-directed modifying polypeptide, e.g., a naturally occurring Cas9; a modified, i.e., mutated or variant, Cas9; a chimeric Cas9; etc.) can be any organism including for example, a plant; algae; an invertebrate (e.g., a cnidarian, an echinoderm, a worm, a fly, etc.); a vertebrate (e.g., a fish (e.g., zebrafish, puffer fish, gold fish, etc.), an amphibian (e.g., salamander, frog, etc.), a reptile, a bird, a mammal, etc.); an ungulate (e.g., a goat, a pig, a sheep, a cow, etc.); a rodent (e.g., a mouse, a rat, a hamster, a guinea pig); a lagomorpha (e.g., a rabbit); etc.

In some cases, the site-directed modifying polypeptide comprises an amino acid sequence having at least about 75%, at least about 80%, at least about 85%, at least about 90%, at least about 95%, at least about 99%, or 100%, amino acid sequence identity to amino acids 7-166 and/or 731-1003 of SEQ ID NO: 8, or to the corresponding portions in any of the amino acid sequences set forth as SEQ ID NOs: 1-800.

### Transgenic non-human animals

As described above, in some embodiments, a nucleic acid (e.g., a nucleotide sequence encoding a site-directed modifying polypeptide, e.g., a naturally occurring Cas9; a modified, i.e., mutated or variant, Cas9; a chimeric Cas9; etc.) or a recombinant expression vector is used as a transgene to generate a transgenic animal that produces a site-directed modifying polypeptide. Thus, the present disclosure further provides a transgenic non-human animal, which animal comprises a transgene comprising a nucleic acid comprising a nucleotide sequence encoding a site-directed modifying polypeptide, e.g., a naturally occurring Cas9; a modified, i.e., mutated or variant, Cas9; a chimeric Cas9; etc., as described above. In some embodiments, the genome of the transgenic non-human animal comprises a nucleotide sequence encoding a site-directed modifying polypeptide. In some embodiments, the transgenic non-human animal is homozygous for the genetic modification. In some embodiments, the transgenic non-human animal is heterozygous for the genetic modification. In some embodiments, the transgenic non-human animal is a vertebrate, for example, a fish (e.g., zebra fish, gold fish, puffer fish, cave fish, etc.), an amphibian (frog, salamander, etc.), a bird (e.g., chicken, turkey, etc.), a reptile (e.g., snake, lizard, etc.), a mammal (e.g., an ungulate, e.g., a pig, a cow, a goat, a sheep, etc.; a lagomorph (e.g., a rabbit); a rodent (e.g., a rat, a mouse); a nonhuman primate; etc.), etc.

An exogenous nucleic acid comprising a nucleotide sequence encoding a site-directed modifying polypeptide (e.g., a naturally occurring Cas9; a modified, i.e., mutated or variant, Cas9; a chimeric Cas9; etc.) can be under the control of (i.e., operably linked to) an unknown promoter (e.g., when the nucleic acid randomly integrates into a host cell genome) or can be under the control of (i.e., operably linked to) a known promoter. Suitable known promoters can be any known promoter and include constitutively active promoters (e.g., CMV promoter), inducible promoters (e.g., heat shock promoter, Tetracycline-regulated promoter, Steroid-regulated promoter, Metal-regulated promoter, estrogen receptor-regulated promoter, etc.), spatially restricted and/or temporally restricted promoters (e.g., a tissue specific promoter, a cell type specific promoter, etc.), etc.

In some cases, the site-directed modifying polypeptide comprises an amino acid sequence having at least about 75%, at least about 80%, at least about 85%, at least about 90%, at least about 95%, at least about 99%, or 100%, amino acid sequence identity to amino acids 7-166 and/or 731-1003 of SEQ ID NO: 8, or to the corresponding portions in any of the amino acid sequences set forth as SEQ ID NOs: 1-800.

### Transgenic plants

As described above, in some embodiments, a nucleic acid (e.g., a nucleotide sequence encoding a site-directed modifying polypeptide, e.g., a naturally occurring Cas9; a modified, i.e., mutated or variant, Cas9; a chimeric Cas9; etc.) or a recombinant expression vector is used as a transgene to generate a transgenic plant that produces a site-directed modifying polypeptide. Thus, the present disclosure further provides a transgenic plant, which plant comprises a transgene comprising a nucleic acid comprising a nucleotide sequence encoding site-directed modifying polypeptide, e.g., a naturally occurring Cas9; a modified, i.e., mutated or variant, Cas9; a chimeric Cas9; etc., as described above. In some embodiments, the genome of the transgenic plant comprises a nucleic acid. In some embodiments, the transgenic plant is homozygous for the genetic modification. In some embodiments, the transgenic plant is heterozygous for the genetic modification.

Methods of introducing exogenous nucleic acids into plant cells are well known in the art. Such plant cells are considered "transformed," as defined above. Suitable methods include viral infection (such as double stranded DNA viruses), transfection, conjugation, protoplast fusion, electroporation, particle gun technology, calcium phosphate precipitation, direct microinjection, silicon carbide whiskers technology, *Agrobacterium-mediated* transformation and the like. The choice of method is generally dependent on the type of cell being transformed and the circumstances under which the transformation is taking place (i.e. *in vitro, ex vivo,* or *in vivo).* Transformation methods based upon the soil bacterium *Agrobacterium tumefaciens* are particularly useful for introducing an exogenous nucleic acid molecule into a vascular plant. The wild type form of *Agrobacterium* contains a Ti (tumor-inducing) plasmid that directs production of tumorigenic crown gall growth on host plants. Transfer of the tumor-inducing T-DNA region of the Ti plasmid to a plant genome requires the Ti plasmid-encoded virulence genes as well as T-DNA borders, which are a set of direct DNA repeats that delineate the region to be transferred. An *Agrobacterium-based* vector is a modified form of a Ti plasmid, in which the tumor inducing functions are replaced by the nucleic acid sequence of interest to be introduced into the plant host.

*Agrobacterium-mediated* transformation generally employs cointegrate vectors or binary vector systems, in which the components of the Ti plasmid are divided between a helper vector, which resides permanently in the *Agrobacterium* host and carries the virulence genes, and a shuttle vector, which contains the gene of interest bounded by T-DNA sequences. A variety of binary vectors are well known in the art and are commercially available, for example, from Clontech (Palo Alto, Calif.). Methods of coculturing *Agrobacterium* with cultured plant cells or wounded tissue such as leaf tissue, root explants, hypocotyledons, stem pieces or tubers, for example, also are well known in the art. See., e.g., Glick and Thompson, (eds.), Methods in Plant Molecular Biology and Biotechnology, Boca Raton, Fla.: CRC Press (1993).

Microprojectile-mediated transformation also can be used to produce a transgenic plant. This method, first described by Klein et al. (Nature 327:70-73 (1987)), relies on microprojectiles such as gold or tungsten that are coated with the desired nucleic acid molecule by precipitation with calcium chloride, spermidine or polyethylene glycol. The microprojectile particles are accelerated at high speed into an angiosperm tissue using a device such as the BIOLISTIC PD-1000 (Biorad; Hercules Calif.).

A nucleic acid may be introduced into a plant in a manner such that the nucleic acid is able to enter a plant cell(s), e.g., via an *in vivo* or *ex vivo* protocol. By *"in vivo,"* it is meant in the nucleic acid is administered to a living body of a plant e.g. infiltration. By *"ex vivo"* it is meant that cells or explants are modified outside of the plant, and then such cells or organs are regenerated to a plant. A number of vectors suitable for stable transformation of plant cells or for the establishment of transgenic plants have been described, including those described in Weissbach and Weissbach, (1989) Methods for Plant Molecular Biology Academic Press, and Gelvin et al., (1990) Plant Molecular Biology Manual, Kluwer Academic Publishers. Specific examples include those derived from a Ti plasmid of *Agrobacterium tumefaciens,* as well as those disclosed by Herrera-Estrella et al. (1983) Nature 303: 209, Bevan (1984) Nucl Acid Res. 12: 8711-8721, Klee (1985) Bio/Technolo 3: 637-642. Alternatively, non-Ti vectors can be used to transfer the DNA into plants and cells by using free DNA delivery techniques. By using these methods transgenic plants such as wheat, rice (Christou (1991) Bio/Technology 9:957-9 and 4462) and corn (Gordon-Kamm (1990) Plant Cell 2: 603-618) can be produced. An immature embryo can also be a good target tissue for monocots for direct DNA delivery techniques by using the particle gun (Weeks et al. (1993) Plant Physiol 102: 1077-1084; Vasil (1993) Bio/Technolo 10: 667-674; Wan and Lemeaux (1994) Plant Physiol 104: 37-48 and for Agrobacterium-mediated DNA transfer (Ishida et al. (1996) Nature Biotech 14: 745-750). Exemplary methods for introduction of DNA into chloroplasts are biolistic bombardment, polyethylene glycol transformation of protoplasts, and microinjection (Daniell et al Nat. Biotechnol 16:345-348, 1998; Staub et al Nat. Biotechnol 18: 333-338, 2000; O'Neill et al Plant J. 3:729-738, 1993; Knoblauch et al Nat. Biotechnol 17: 906-909; U.S. Pat. Nos. 5,451,513, 5,545,817, 5,545,818, and 5,576,198; in Intl. Application No. WO 95/16783; and in Boynton et al., Methods in Enzymology 217: 510-536 (1993), Svab et al., Proc. Natl. Acad. Sci. USA 90: 913-917 (1993), and McBride et al., Proc. Nati. Acad. Sci. USA 91: 7301-7305 (1994)). Any vector suitable for the methods of biolistic bombardment, polyethylene glycol transformation of protoplasts and microinjection will be suitable as a targeting vector for chloroplast transformation. Any double stranded DNA vector may be used as a transformation vector, especially when the method of introduction does not utilize *Agrobacterium.*

Plants which can be genetically modified include grains, forage crops, fruits, vegetables, oil seed crops, palms, forestry, and vines. Specific examples of plants which can be modified follow: maize, banana, peanut, field peas, sunflower, tomato, canola, tobacco, wheat, barley, oats, potato, soybeans, cotton, carnations, sorghum, lupin and rice.

Also provided by the disclosure are transformed plant cells, tissues, plants and products that contain the transformed plant cells. A feature of the transformed cells, and tissues and products that include the same is the presence of a nucleic acid integrated into the genome, and production by plant cells of a site-directed modifying polypeptide, e.g., a naturally occurring Cas9; a modified, i.e., mutated or variant, Cas9; a chimeric Cas9; etc. Recombinant plant cells of the present invention are useful as populations of recombinant cells, or as a tissue, seed, whole plant, stem, fruit, leaf, root, flower, stem, tuber, grain, animal feed, a field of plants, and the like.

A nucleic acid comprising a nucleotide sequence encoding a site-directed modifying polypeptide (e.g., a naturally occurring Cas9; a modified, i.e., mutated or variant, Cas9; a chimeric Cas9; etc.) can be under the control of (i.e., operably linked to) an unknown promoter (e.g., when the nucleic acid randomly integrates into a host cell genome) or can be under the control of (i.e., operably linked to) a known promoter. Suitable known promoters can be any known promoter and include constitutively active promoters, inducible promoters, spatially restricted and/or temporally restricted promoters, etc.

In some cases, the site-directed modifying polypeptide comprises an amino acid sequence having at least about 75%, at least about 80%, at least about 85%, at least about 90%, at least about 95%, at least about 99%, or 100%, amino acid sequence identity to amino acids 7-166 and/or 731-1003 of SEQ ID NO: 8, or to the corresponding portions in any of the amino acid sequences set forth as SEQ ID NOs: 1-800. Also provided by the disclosure is reproductive material of a transgenic plant, where reproductive material includes seeds, progeny plants and clonal material.

### Detailed Description - Part II

The present disclosure provides methods of modulating transcription of a target nucleic acid in a host cell. The methods generally involve contacting the target nucleic acid with an enzymatically inactive Cas9 polypeptide and a single-guide RNA. The methods are useful in a variety of applications, which are also provided.

A transcriptional modulation method of the present disclosure overcomes some of the drawbacks of methods involving RNAi. A transcriptional modulation method of the present disclosure finds use in a wide variety of applications, including research applications, drug discovery (e.g., high throughput screening), target validation, industrial applications (e.g., crop engineering; microbial engineering, etc.), diagnostic applications, therapeutic applications, and imaging techniques.

### Methods of Modulating Transcription

The present disclosure provides a method of selectively modulating transcription of a target DNA in a host cell. The method generally involves: a) introducing into the host cell: i) a guide RNA, or a nucleic acid comprising a nucleotide sequence encoding the guide RNA; and ii) a variant Cas9 site-directed polypeptide ("variant Cas9 polypeptide"), or a nucleic acid comprising a nucleotide sequence encoding the variant Cas9 polypeptide, where the variant Cas9 polypeptide exhibits reduced endodeoxyribonuclease activity.

The guide RNA (also referred to herein as "guide RNA"; or "gRNA") comprises: i) a first segment comprising a nucleotide sequence that is complementary to a target sequence in a target DNA; ii) a second segment that interacts with a site-directed polypeptide; and iii) a transcriptional terminator. The first segment, comprising a nucleotide sequence that is complementary to a target sequence in a target DNA, is referred to herein as a "targeting segment". The second segment, which interacts with a site-directed polypeptide, is also referred to herein as a "protein-binding sequence" or "dCas9-binding hairpin," or "dCas9 handle." By "segment" it is meant a segment/section/region of a molecule, e.g., a contiguous stretch of nucleotides in an RNA. The definition of "segment," unless otherwise specifically defined in a particular context, is not limited to a specific number of total base pairs, and may include regions of RNA molecules that are of any total length and may or may not include regions with complementarity to other molecules. As described above, guide RNA according to the present disclosure can be a single-molecule guide RNA or a two-moleculte guide RNA. The term "guide RNA" or "gRNA" is inclusive, referring both to two-molecule guide RNAs and to single-molecule guide RNAs (i.e., sgRNAs).

The variant Cas9 site-directed polypeptide comprises: i) an RNA-binding portion that interacts with the guide RNA; and an activity portion that exhibits reduced endodeoxyribonuclease activity.

The guide RNA and the variant Cas9 polypeptide form a complex in the host cell; the complex selectively modulates transcription of a target DNA in the host cell.

In some cases, a transcription modulation method of the present disclosure provides for selective modulation (e.g., reduction or increase) of a target nucleic acid in a host cell. For example, "selective" reduction of transcription of a target nucleic acid reduces transcription of the target nucleic acid by at least about 10%, at least about 20%, at least about 30%, at least about 40%, at least about 50%, at least about 60%, at least about 70%, at least about 80%, at least about 90%, or greater than 90%, compared to the level of transcription of the target nucleic acid in the absence of a guide RNA/variant Cas9 polypeptide complex. Selective reduction of transcription of a target nucleic acid reduces transcription of the target nucleic acid, but does not substantially reduce transcription of a non-target nucleic acid, e.g., transcription of a non-target nucleic acid is reduced, if at all, by less than 10% compared to the level of transcription of the non-target nucleic acid in the absence of the guide RNA/variant Cas9 polypeptide complex.

### Increased transcription

"Selective" increased transcription of a target DNA can increase transcription of the target DNA by at least about 1.1 fold (e.g., at least about 1.2 fold, at least about 1.3 fold, at least about 1.4 fold, at least about 1.5 fold, at least about 1.6 fold, at least about 1.7 fold, at least about 1.8 fold, at least about 1.9 fold, at least about 2 fold, at least about 2.5 fold, at least about 3 fold, at least about 3.5 fold, at least about 4 fold, at least about 4.5 fold, at least about 5 fold, at least about 6 fold, at least about 7 fold, at least about 8 fold, at least about 9 fold, at least about 10 fold, at least about 12 fold, at least about 15 fold, or at least about 20-fold) compared to the level of transcription of the target DNA in the absence of a guide RNA/variant Cas9 polypeptide complex. Selective increase of transcription of a target DNA increases transcription of the target DNA, but does not substantially increase transcription of a non-target DNA, e.g., transcription of a non-target DNA is increased, if at all, by less than about 5-fold (e.g., less than about 4-fold, less than about 3-fold, less than about 2-fold, less than about 1.8-fold, less than about 1.6-fold, less than about 1.4-fold, less than about 1.2-fold, or less than about 1.1 -fold) compared to the level of transcription of the non-targeted DNA in the absence of the guide RNA/variant Cas9 polypeptide complex.

As a non-limiting example, increased transcription can be achieved by fusing dCas9 to a heterologous sequence. Suitable fusion partners include, but are not limited to, a polypeptide that provides an activity that indirectly increases transcription by acting directly on the target DNA or on a polypeptide (e.g., a histone or other DNA-binding protein) associated with the target DNA. Suitable fusion partners include, but are not limited to, a polypeptide that provides for methyltransferase activity, demethylase activity, acetyltransferase activity, deacetylase activity, kinase activity, phosphatase activity, ubiquitin ligase activity, deubiquitinating activity, adenylation activity, deadenylation activity, SUMOylating activity, deSUMOylating activity, ribosylation activity, deribosylation activity, myristoylation activity, or demyristoylation activity.

Additional suitable fusion partners include, but are not limited to, a polypeptide that directly provides for increased transcription of the target nucleic acid (e.g., a transcription activator or a fragment thereof, a protein or fragment thereof that recruits a transcription activator, a small molecule/drug-responsive transcription regulator, etc.).

A non-limiting example of a method using a dCas9 fusion protein to increase transcription in a prokaryote includes a modification of the bacterial one-hybrid (B1H) or two-hybrid (B2H) system. In the B1H system, a DNA binding domain (BD) is fused to a bacterial transcription activation domain (AD, e.g., the alpha subunit of the *Escherichia coli* RNA polymerase (RNAPa)). Thus, a dCas9 can be fused to a heterologous sequence comprising an AD. When the dCas9 fusion protein arrives at the upstream region of a promoter (targeted there by the guide RNA) the AD (e.g., RNAPa) of the dCas9 fusion protein recruits the RNAP holoenzyme, leading to transcription activation. In the B2H system, the BD is not directly fused to the AD; instead, their interaction is mediated by a protein-protein interaction (e.g., GAL11P - GAL4 interaction). To modify such a system for use in the methods, dCas9 can be fused to a first protein sequence that provides for protein-protein interaction (e.g., the yeast GAL11P and/or GAL4 protein) and RNAa can be fused to a second protein sequence that completes the protein-protein interaction (e.g., GAL4 if GAL11P is fused to dCas9, GAL11P if GAL4 is fused to dCas9, etc.). The binding affinity between GAL11P and GAL4 increases the efficiency of binding and transcription firing rate.

A non-limiting example of a method using a dCas9 fusion protein to increase transcription in a eukaryotes includes fusion of dCas9 to an activation domain (AD) (e.g., GAL4, herpesvirus activation protein VP16 or VP64, human nuclear factor NF-κB p65 subunit, etc.). To render the system inducible, expression of the dCas9 fusion protein can be controlled by an inducible promoter (e.g., Tet-ON, Tet-OFF, etc.). The guide RNA can be design to target known transcription response elements (e.g., promoters, enhancers, etc.), known upstream activating sequences (UAS), sequences of unknown or known function that are suspected of being able to control expression of the target DNA, etc.

### Additional fusion partners

Non-limiting examples of fusion partners to accomplish increased or decreased transcription include, but are not limited to, transcription activator and transcription repressor domains (e.g., the Kriippel associated box (KRAB or SKD); the Mad mSIN3 interaction domain (SID); the ERF repressor domain (ERD), etc). In some such cases, the dCas9 fusion protein is targeted by the guide RNA to a specific location (i.e., sequence) in the target DNA and exerts locus-specific regulation such as blocking RNA polymerase binding to a promoter (which selectively inhibits transcription activator function), and/or modifying the local chromatin status (e.g., when a fusion sequence is used that modifies the target DNA or modifies a polypeptide associated with the target DNA). In some cases, the changes are transient (e.g., transcription repression or activation). In some cases, the changes are inheritable (e.g., when epigenetic modifications are made to the target DNA or to proteins associated with the target DNA, e.g., nucleosomal histones).

In some embodiments, the heterologous sequence can be fused to the C-terminus of the dCas9 polypeptide. In some embodiments, the heterologous sequence can be fused to the N-terminus of the dCas9 polypeptide. In some embodiments, the heterologous sequence can be fused to an internal portion (i.e., a portion other than the N- or C- terminus) of the dCas9 polypeptide.

The biological effects of a method using a dCas9 fusion protein can be detected by any convenient method (e.g., gene expression assays; chromatin-based assays, e.g., Chromatin immunoPrecipitation (ChiP), Chromatin in vivo Assay (CiA), etc.; and the like).

In some cases, a method involves use of two or more different guide RNAs. For example, two different guide RNAs can be used in a single host cell, where the two different guide RNAs target two different target sequences in the same target nucleic acid.

Thus, for example, a transcriptional modulation method can further comprise introducing into the host cell a second guide RNA, or a nucleic acid comprising a nucleotide sequence encoding the second guide RNA, where the second guide RNA comprises: i) a first segment comprising a nucleotide sequence that is complementary to a second target sequence in the target DNA; ii) a second segment that interacts with the site-directed polypeptide; and iii) a transcriptional terminator. In some cases, use of two different guide RNAs targeting two different targeting sequences in the same target nucleic acid provides for increased modulation (e.g., reduction or increase) in transcription of the target nucleic acid.

As another example, two different guide RNAs can be used in a single host cell, where the two different guide RNAs target two different target nucleic acids. Thus, for example, a transcriptional modulation method can further comprise introducing into the host cell a second guide RNA, or a nucleic acid comprising a nucleotide sequence encoding the second guide RNA, where the second guide RNA comprises: i) a first segment comprising a nucleotide sequence that is complementary to a target sequence in at least a second target DNA; ii) a second segment that interacts with the site-directed polypeptide; and iii) a transcriptional terminator.

In some embodiments, a nucleic acid (e.g., a guide RNA, e.g., a single-molecule guide RNA, an activator-RNA, a targeter-RNA, etc.; a donor polynucleotide; a nucleic acid encoding a site-directed modifying polypeptide; etc.) comprises a modification or sequence that provides for an additional desirable feature (e.g., modified or regulated stability; subcellular targeting; tracking, e.g., a fluorescent label; a binding site for a protein or protein complex; etc.). Non-limiting examples include: a 5' cap (e.g., a 7-methylguanylate cap (m⁷G)); a 3' polyadenylated tail (i.e., a 3' poly(A) tail); a riboswitch sequence or an aptamer sequence (e.g., to allow for regulated stability and/or regulated accessibility by proteins and/or protein complexes); a terminator sequence; a sequence that forms a dsRNA duplex (i.e., a hairpin)); a modification or sequence that targets the RNA to a subcellular location (e.g., nucleus, mitochondria, chloroplasts, and the like); a modification or sequence that provides for tracking (e.g., direct conjugation to a fluorescent molecule, conjugation to a moiety that facilitates fluorescent detection, a sequence that allows for fluorescent detection, etc.); a modification or sequence that provides a binding site for proteins (e.g., proteins that act on DNA, including transcriptional activators, transcriptional repressors, DNA methyltransferases, DNA demethylases, histone acetyltransferases, histone deacetylases, and the like); and combinations thereof.

### DNA-targeting segment

The DNA-targeting segment (or "DNA-targeting sequence") of a guide RNA comprises a nucleotide sequence that is complementary to a specific sequence within a target DNA (the complementary strand of the target DNA).

In other words, the DNA-targeting segment of a guide RNA interacts with a target DNA in a sequence-specific manner via hybridization (i.e., base pairing). As such, the nucleotide sequence of the DNA-targeting segment may vary and determines the location within the target DNA that the guide RNA and the target DNA will interact. The DNA-targeting segment of a guide RNA can be modified (e.g., by genetic engineering) to hybridize to any desired sequence within a target DNA.

### Stability control sequence (e.g., transcriptional terminator segment)

A stability control sequence influences the stability of an RNA (e.g., a guide RNA, a targeter-RNA, an activator-RNA, etc.). One example of a suitable stability control sequence is a transcriptional terminator segment (i.e., a transcription termination sequence). A transcriptional terminator segment of a guide RNA can have a total length of from about 10 nucleotides to about 100 nucleotides, e.g., from about 10 nucleotides (nt) to about 20 nt, from about 20 nt to about 30 nt, from about 30 nt to about 40 nt, from about 40 nt to about 50 nt, from about 50 nt to about 60 nt, from about 60 nt to about 70 nt, from about 70 nt to about 80 nt, from about 80 nt to about 90 nt, or from about 90 nt to about 100 nt. For example, the transcriptional terminator segment can have a length of from about 15 nucleotides (nt) to about 80 nt, from about 15 nt to about 50 nt, from about 15 nt to about 40 nt, from about 15 nt to about 30 nt or from about 15 nt to about 25 nt.

In some cases, the transcription termination sequence is one that is functional in a eukaryotic cell. In some cases, the transcription termination sequence is one that is functional in a prokaryotic cell.

Nucleotide sequences that can be included in a stability control sequence (e.g., transcriptional termination segment, or in any segment of the guide RNA to provide for increased stability) include, for example, 5'-UAAUCCCACAGCCGCCAGUUCCGCUGGCGGCAUUUU-5' (a Rho-independent *trp* termination site).

### Additional sequences

In some embodiments, a guide RNA comprises at least one additional segment at either the 5' or 3' end. For example, a suitable additional segment can comprise a 5' cap (e.g., a 7-methylguanylate cap (m⁷G)); a 3' polyadenylated tail (i.e., a 3' poly(A) tail); a riboswitch sequence (e.g., to allow for regulated stability and/or regulated accessibility by proteins and protein complexes); a sequence that forms a dsRNA duplex (i.e., a hairpin)); a sequence that targets the RNA to a subcellular location (e.g., nucleus, mitochondria, chloroplasts, and the like); a modification or sequence that provides for tracking (e.g., direct conjugation to a fluorescent molecule, conjugation to a moiety that facilitates fluorescent detection, a sequence that allows for fluorescent detection, etc.); a modification or sequence that provides a binding site for proteins (e.g., proteins that act on DNA, including transcriptional activators, transcriptional repressors, DNA methyltransferases, DNA demethylases, histone acetyltransferases, histone deacetylases, and the like) a modification or sequence that provides for increased, decreased, and/or controllable stability; and combinations thereof.

### Multiple simultaneous guide RNAs

In some embodiments, multiple guide RNAs are used simultaneously in the same cell to simultaneously modulate transcription at different locations on the same target DNA or on different target DNAs. In some embodiments, two or more guide RNAs target the same gene or transcript or locus. In some embodiments, two or more guide RNAs target different unrelated loci. In some embodiments, two or more guide RNAs target different, but related loci.

Because the guide RNAs are small and robust they can be simultaneously present on the same expression vector and can even be under the same transcriptional control if so desired. In some embodiments, two or more (e.g., 3 or more, 4 or more, 5 or more, 10 or more, 15 or more, 20 or more, 25 or more, 30 or more, 35 or more, 40 or more, 45 or more, or 50 or more) guide RNAs are simultaneously expressed in a target cell (from the same or different vectors). The expressed guide RNAs can be differently recognized by Cas9 proteins from different bacteria, such as *S*. *pyogenes, S. thermophilus, L. innocua, and N. meningitidis.*

In some cases, multiple guide RNAs can be encoded in an array mimicking naturally occurring CRISPR arrays of targeter RNAs and corresponding tracrRNAs (activator RNAs). The targeting segments are encoded as approximately 30 nucleotide long sequences (can be about 16 to about 100 nt) and are separated by CRISPR repeat sequences. In some cases, the array and tracrRNAs are introduced to a cell by DNAs encoding the RNAs. In some cases, they are introduced to the cell as RNAs.

To express multiple guide RNAs, an artificial RNA processing system mediated by the Csy4 endoribonuclease can be used. Multiple guide RNAs can be concatenated into a tandem array on a precursor transcript (e.g., expressed from a U6 promoter), and separated by Csy4-specific RNA sequence. Co-expressed Csy4 protein cleaves the precursor transcript into multiple guide RNAs. Advantages for using an RNA processing system include: first, there is no need to use multiple promoters; second, since all guide RNAs are processed from a precursor transcript, their concentrations are normalized for similar dCas9-binding.

Csy4 is a small endoribonuclease (RNase) protein derived from bacteria *Pseudomonas aeruginosa.* Csy4 specifically recognizes a minimal 17-bp RNA hairpin, and exhibits rapid (<1 min) and highly efficient (>99.9%) RNA cleavage. Unlike most RNases, the cleaved RNA fragment remains stable and functionally active. The Csy4-based RNA cleavage can be repurposed into an artificial RNA processing system. In this system, the 17-bp RNA hairpins are inserted between multiple RNA fragments that are transcribed as a precursor transcript from a single promoter. Co-expression of Csy4 is effective in generating individual RNA fragments.

### Site-directed polypeptide

As noted above, a guide RNA and a variant Cas9 site-directed polypeptide form a complex. The guide RNA provides target specificity to the complex by comprising a nucleotide sequence that is complementary to a sequence of a target DNA. The variant Cas9 site-directed polypeptide has reduced endodeoxyribonuclease activity. For example, a variant Cas9 site-directed polypeptide suitable for use in a transcription modulation method of the present disclosure exhibits less than about 20%, less than about 15%, less than about 10%, less than about 5%, less than about 1%, or less than about 0.1%, of the endodeoxyribonuclease activity of a wild-type Cas9 polypeptide, e.g., a wild-type Cas9 polypeptide comprising an amino acid sequence set out in SEQ ID NO:8. In some embodiments, the variant Cas9 site-directed polypeptide has substantially no detectable endodeoxyribonuclease activity. In some embodiments when a site-directed polypeptide has reduced catalytic activity (e.g., when a SEQ ID NO: 8 S. pyogenes Cas9 protein has a D10, G12, G17, E762, H840, N863, H982, H983, A984, D986, and/or a A987 mutation, e.g., D10A, G12A, G17A, E762A, H840A, N863A, H982A, H983A, A984A, and/or D986A), the polypeptide can still bind to target DNA in a site-specific manner (because it is still guided to a target DNA sequence by a guide RNA) as long as it retains the ability to interact with the guide RNA.

In some cases, a suitable variant Cas9 site-directed polypeptide comprises an amino acid sequence having at least about 75%, at least about 80%, at least about 85%, at least about 90%, at least about 95%, at least about 99% or 100% amino acid sequence identity to amino acids 7-166 and/or 731-1003 of SEQ ID NO: 8, or to the corresponding portions in any one of the amino acid sequences SEQ ID NOs: 1-800.

In some cases, the variant Cas9 site-directed polypeptide is a nickase that can cleave the complementary strand of the target DNA but has reduced ability to cleave the non-complementary strand of the target DNA. For example, the variant Cas9 site-directed polypeptide can have a mutation (amino acid substitution) that reduces the function of the RuvC domain. As a non-limiting example, in some cases, the variant Cas9 site-directed polypeptide is a D10A (aspartate to alanine) mutation of SEQ ID NO: 8 (or the corresponding mutation of any of the amino acid sequences set forth in SEQ ID NOs: 1-800).

In some cases, the variant Cas9 site-directed polypeptide in a nickase that can cleave the non-complementary strand of the target DNA but has reduced ability to cleave the complementary strand of the target DNA. For example, the variant Cas9 site-directed polypeptide can have a mutation (amino acid substitution) that reduces the function of the HNH domain (RuvC/HNH/RuvC domain motifs, "domain 2"). As a non-limiting example, in some cases, the variant Cas9 site-directed polypeptide is a H840A (histidine to alanine at amino acid position 840 of SEQ ID NO:8) or the corresponding mutation of any of the amino acid sequences set forth in SEQ ID NOs: 1-800).

In some cases, the variant Cas9 site-directed polypeptide has a reduced ability to cleave both the complementary and the non-complementary strands of the target DNA. As a non-limiting example, in some cases, the variant Cas9 site-directed polypeptide harbors both D10A and H840A mutations of SEQ ID NO: 8 (or the corresponding mutations of any of the amino acid sequences set forth in SEQ ID NOs: 1-800). Other residues can be mutated to achieve the same effect (i.e. inactivate one or the other nuclease portions). As non-limiting examples, S. pyogenes Cas9 residues D10, G12, G17, E762, H840, N863, H982, H983, A984, D986, and/or A987 of SEQ ID NO: 8 (or the corresponding mutations of any of the proteins set forth as SEQ ID NOs: 1-800) can be altered (i.e., substituted) (see Table 1 for examples of the conservation of Cas9 amino acid residues). Also, mutations other than alanine substitutions are contemplated.

In some embodiments, a variant Cas9 endonuclease comprises one or more mutations corresponding to a S. pyogenes Cas9 mutation E762A, HH983AA or D986A in SEQ ID NO: 8. In some embodiments, the modified Cas 9 endonuclease further comprises one or more mutations corresponding to a S. pyogenes Cas9 mutation D10A, H840A, G12A, G17A, N854A, N863A, N982A or A984A in SEQ ID NO: 8.

In some cases, the variant Cas9 site-directed polypeptide is a fusion polypeptide (a "variant Cas9 fusion polypeptide"), i.e., a fusion polypeptide comprising: i) a variant Cas9 site-directed polypeptide; and ii) a covalently linked heterologous polypeptide (also referred to as a "fusion partner").

The heterologous polypeptide may exhibit an activity (e.g., enzymatic activity) that will also be exhibited by the variant Cas9 fusion polypeptide (e.g., methyltransferase activity, acetyltransferase activity, kinase activity, ubiquitinating activity, etc.). A heterologous nucleic acid sequence may be linked to another nucleic acid sequence (e.g., by genetic engineering) to generate a chimeric nucleotide sequence encoding a chimeric polypeptide. In some embodiments, a variant Cas9 fusion polypeptide is generated by fusing a variant Cas9 polypeptide with a heterologous sequence that provides for subcellular localization (i.e., the heterologous sequence is a subcellular localization sequence, e.g., a nuclear localization signal (NLS) for targeting to the nucleus; a mitochondrial localization signal for targeting to the mitochondria; a chloroplast localization signal for targeting to a chloroplast; an ER retention signal; and the like). In some embodiments, the heterologous sequence can provide a tag (i.e., the heterologous sequence is a detectable label) for ease of tracking and/or purification (e.g., a fluorescent protein, e.g., green fluorescent protein (GFP), YFP, RFP, CFP, mCherry, tdTomato, and the like; a histidine tag, e.g., a 6XHis tag; a hemagglutinin (HA) tag; a FLAG tag; a Myc tag; and the like). In some embodiments, the heterologous sequence can provide for increased or decreased stability (i.e., the heterologous sequence is a stability control peptide, e.g., a degron, which in some cases is controllable (e.g., a temperature sensitive or drug controllable degron sequence, see below). In some embodiments, the heterologous sequence can provide for increased or decreased transcription from the target DNA (i.e., the heterologous sequence is a transcription modulation sequence, e.g., a transcription factor/activator or a fragment thereof, a protein or fragment thereof that recruits a transcription factor/activator, a transcription repressor or a fragment thereof, a protein or fragment thereof that recruits a transcription repressor, a small molecule/drug-responsive transcription regulator, etc.). In some embodiments, the heterologous sequence can provide a binding domain (i.e., the heterologous sequence is a protein binding sequence, e.g., to provide the ability of a chimeric dCas9 polypeptide to bind to another protein of interest, e.g., a DNA or histone modifying protein, a transcription factor or transcription repressor, a recruiting protein, etc.).

Suitable fusion partners that provide for increased or decreased stability include, but are not limited to degron sequences. Degrons are readily understood by one of ordinary skill in the art to be amino acid sequences that control the stability of the protein of which they are part. For example, the stability of a protein comprising a degron sequence is controlled at least in part by the degron sequence. In some cases, a suitable degron is constitutive such that the degron exerts its influence on protein stability independent of experimental control (i.e., the degron is not drug inducible, temperature inducible, etc.) In some cases, the degron provides the variant Cas9 polypeptide with controllable stability such that the variant Cas9 polypeptide can be turned "on" (i.e., stable) or "off' (i.e., unstable, degraded) depending on the desired conditions. For example, if the degron is a temperature sensitive degron, the variant Cas9 polypeptide may be functional (i.e., "on", stable) below a threshold temperature (e.g., 42°C, 41°C, 40°C, 39°C, 38°C, 37°C, 36°C, 35°C, 34°C, 33°C, 32°C, 31°C, 30°C, etc.) but non-functional (i.e., "off, degraded) above the threshold temperature. As another example, if the degron is a drug inducible degron, the presence or absence of drug can switch the protein from an "off (i.e., unstable) state to an "on" (i.e., stable) state or vice versa. An exemplary drug inducible degron is derived from the FKBP12 protein. The stability of the degron is controlled by the presence or absence of a small molecule that binds to the degron.

Examples of suitable degrons include, but are not limited to those degrons controlled by Shield-1, DHFR, auxins, and/or temperature. Non-limiting examples of suitable degrons are known in the art (e.g., Dohmen et al., Science, 1994. 263(5151): p. 1273-1276: Heat-inducible degron: a method for constructing temperature-sensitive mutants; Schoeber et al., Am J Physiol Renal Physiol. 2009 Jan;296(1):F204-11 : Conditional fast expression and function of multimeric TRPV5 channels using Shield-1 ; Chu et al., Bioorg Med Chem Lett. 2008 Nov 15;18(22):5941-4: Recent progress with FKBP-derived destabilizing domains ; Kanemaki, Pflugers Arch. 2012 Dec 28: Frontiers of protein expression control with conditional degrons; Yang et al., Mol Cell. 2012 Nov 30;48(4):487-8: Titivated for destruction: the methyl degron; Barbour et al., Biosci Rep. 2013 Jan 18;33(1).: Characterization of the bipartite degron that regulates ubiquitin-independent degradation of thymidylate synthase; and Greussing et al., J Vis Exp. 2012 Nov 10;(69): Monitoring of ubiquitin-proteasome activity in living cells using a Degron (dgn)-destabilized green fluorescent protein (GFP)-based reporter protein; all of which are hereby incorporated in their entirety by reference).

Exemplary degron sequences have been well-characterized and tested in both cells and animals. Thus, fusing Cas9 to a degron sequence produces a "tunable" and "inducible" Cas9 polypeptide. Any of the fusion partners described herein can be used in any desirable combination. As one non-limiting example to illustrate this point, a Cas9 fusion protein can comprise a YFP sequence for detection, a degron sequence for stability, and transcription activator sequence to increase transcription of the target DNA. Furthermore, the number of fusion partners that can be used in a Cas9 fusion protein is unlimited. In some cases, a Cas9 fusion protein comprises one or more (e.g. two or more, three or more, four or more, or five or more) heterologous sequences.

Suitable fusion partners include, but are not limited to, a polypeptide that provides for methyltransferase activity, demethylase activity, acetyltransferase activity, deacetylase activity, kinase activity, phosphatase activity, ubiquitin ligase activity, deubiquitinating activity, adenylation activity, deadenylation activity, SUMOylating activity, deSUMOylating activity, ribosylation activity, deribosylation activity, myristoylation activity, or demyristoylation activity, any of which can be directed at modifying the DNA directly (e.g., methylation of DNA) or at modifying a DNA-associated polypeptide (e.g., a histone or DNA binding protein). Further suitable fusion partners include, but are not limited to boundary elements (e.g., CTCF), proteins and fragments thereof that provide periphery recruitment (e.g., Lamin A, Lamin B, etc.), and protein docking elements (e.g., FKBP/FRB, Pil 1/Aby 1, etc.).

In some embodiments, a site-directed modifying polypeptide can be codon-optimized. This type of optimization is known in the art and entails the mutation of foreign-derived DNA to mimic the codon preferences of the intended host organism or cell while encoding the same protein. Thus, the codons are changed, but the encoded protein remains unchanged. For example, if the intended target cell was a human cell, a human codon-optimized dCas9 (or dCas9 variant) would be a suitable site-directed modifying polypeptide. As another non-limiting example, if the intended host cell were a mouse cell, than a mouse codon-optimized Cas9 (or variant, e.g., enzymatically inactive variant) would be a suitable Cas9 site-directed polypeptide. While codon optimization is not required, it is acceptable and may be preferable in certain cases.

Polyadenylation signals can also be chosen to optimize expression in the intended host.

### Host cells

A method of the present disclosure to modulate transcription may be employed to induce transcriptional modulation in mitotic or post-mitotic cells *in vivo* and/or *ex vivo* and/or *in vitro.* Because the guide RNA provides specificity by hybridizing to target DNA, a mitotic and/or post-mitotic cell can be any of a variety of host cell, where suitable host cells include, but are not limited to, a bacterial cell; an archaeal cell; a single-celled eukaryotic organism; a plant cell; an algal cell, e.g., *Botryococcus braunii, Chlamydomonas reinhardtii, Nannochloropsis gaditana, Chlorella pyrenoidosa, Sargassum patens, C. agardh,* and the like; a fungal cell; an animal cell; a cell from an invertebrate animal (e.g., an insect, a cnidarian, an echinoderm, a nematode, etc.); a eukaryotic parasite (e.g., a malarial parasite, e.g., *Plasmodium fakiparum;* a helminth; etc.); a cell from a vertebrate animal (e.g., fish, amphibian, reptile, bird, mammal); a mammalian cell, e.g., a rodent cell, a human cell, a non-human primate cell, etc. Suitable host cells include naturally-occurring cells; genetically modified cells (e.g., cells genetically modified in a laboratory, e.g., by the "hand of man"); and cells manipulated *in vitro* in any way. In some cases, a host cell is isolated.

Any type of cell may be of interest (e.g. a stem cell, e.g. an embryonic stem (ES) cell, an induced pluripotent stem (iPS) cell, a germ cell; a somatic cell, e.g. a fibroblast, a hematopoietic cell, a neuron, a muscle cell, a bone cell, a hepatocyte, a pancreatic cell; an in vitro or in vivo embryonic cell of an embryo at any stage, e.g., a 1-cell, 2-cell, 4-cell, 8-cell, etc. stage zebrafish embryo; etc.). Cells may be from established cell lines or they may be primary cells, where "primary cells", "primary cell lines", and "primary cultures" are used interchangeably herein to refer to cells and cells cultures that have been derived from a subject and allowed to grow in vitro for a limited number of passages, i.e. splittings, of the culture. For example, primary cultures include cultures that may have been passaged 0 times, 1 time, 2 times, 4 times, 5 times, 10 times, or 15 times, but not enough times go through the crisis stage. Primary cell lines can be are maintained for fewer than 10 passages *in vitro.* Target cells are in many embodiments unicellular organisms, or are grown in culture.

If the cells are primary cells, such cells may be harvest from an individual by any convenient method. For example, leukocytes may be conveniently harvested by apheresis, leukocytapheresis, density gradient separation, etc., while cells from tissues such as skin, muscle, bone marrow, spleen, liver, pancreas, lung, intestine, stomach, etc. are most conveniently harvested by biopsy. An appropriate solution may be used for dispersion or suspension of the harvested cells. Such solution will generally be a balanced salt solution, e.g. normal saline, phosphate-buffered saline (PBS), Hank's balanced salt solution, etc., conveniently supplemented with fetal calf serum or other naturally occurring factors, in conjunction with an acceptable buffer at low concentration, e.g., from 5-25 mM. Convenient buffers include HEPES, phosphate buffers, lactate buffers, etc. The cells may be used immediately, or they may be stored, frozen, for long periods of time, being thawed and capable of being reused. In such cases, the cells will usually be frozen in 10% dimethyl sulfoxide (DMSO), 50% serum, 40% buffered medium, or some other such solution as is commonly used in the art to preserve cells at such freezing temperatures, and thawed in a manner as commonly known in the art for thawing frozen cultured cells.

### Introducing nucleic acid into a host cell

A guide RNA, or a nucleic acid comprising a nucleotide sequence encoding same, can be introduced into a host cell by any of a variety of well-known methods. Similarly, where a method involves introducing into a host cell a nucleic acid comprising a nucleotide sequence encoding a variant Cas9 site-directed polypeptide, such a nucleic acid can be introduced into a host cell by any of a variety of well-known methods.

Methods of introducing a nucleic acid into a host cell are known in the art, and any known method can be used to introduce a nucleic acid (e.g., an expression construct) into a stem cell or progenitor cell. Suitable methods include, include e.g., viral or bacteriophage infection, transfection, conjugation, protoplast fusion, lipofection, electroporation, calcium phosphate precipitation, polyethyleneimine (PEI)-mediated transfection, DEAE-dextran mediated transfection, liposome-mediated transfection, particle gun technology, calcium phosphate precipitation, direct micro injection, nanoparticle-mediated nucleic acid delivery (see, e.g., Panyam et., al Adv Drug Deliv Rev. 2012 Sep 13. pii: 50169-409X(12)00283-9. doi: 10.1016/j.addr.2012.09.023), and the like.

### Nucleic Acids

The present disclosure provides an isolated nucleic acid comprising a nucleotide sequence encoding a guide RNA. In some cases, a nucleic acid also comprises a nucleotide sequence encoding a variant Cas9 site-directed polypeptide.

In some embodiments, a method involves introducing into a host cell (or a population of host cells) one or more nucleic acids comprising nucleotide sequences encoding a guide RNA and/or a variant Cas9 site-directed polypeptide. In some embodiments a cell comprising a target DNA is *in vitro.* In some embodiments a cell comprising a target DNA is *in vivo.* Suitable nucleic acids comprising nucleotide sequences encoding a guide RNA and/or a site-directed polypeptide include expression vectors, where an expression vector comprising a nucleotide sequence encoding a guide RNA and/or a site-directed polypeptide is a "recombinant expression vector."

In some embodiments, the recombinant expression vector is a viral construct, e.g., a recombinant adeno-associated virus construct (see, e.g., U.S. Patent No. 7,078,387), a recombinant adenoviral construct, a recombinant lentiviral construct, a recombinant retroviral construct, etc.
Suitable expression vectors include, but are not limited to, viral vectors (e.g. viral vectors based on vaccinia virus; poliovirus; adenovirus (see, e.g., Li et al., Invest Opthalmol Vis Sci 35:2543 2549, 1994; Borras et al., Gene Ther 6:515 524, 1999; Li and Davidson, PNAS 92:7700 7704, 1995; Sakamoto et al., H Gene Ther 5:1088 1097, 1999; WO 94/12649, WO 93/03769; WO 93/19191; WO 94/28938; WO 95/11984 and WO 95/00655); adeno-associated virus (see, e.g., Ali et al., Hum Gene Ther 9:81 86, 1998, Flannery et al., PNAS 94:6916 6921, 1997; Bennett et al., Invest Opthalmol Vis Sci 38:2857 2863, 1997; Jomary et al., Gene Ther 4:683-690, 1997, Rolling et al., Hum Gene Ther 10:641 648, 1999; Ali et al., Hum Mol Genet 5:591 594, 1996; Srivastava in WO 93/09239, Samulski et al., J. Vir. (1989) 63:3822-3828; Mendelson et al., Virol. (1988) 166:154-165; and Flotte et al., PNAS (1993) 90:10613-10617); SV40; herpes simplex virus; human immunodeficiency virus (see, e.g., Miyoshi et al., PNAS 94:10319 23, 1997; Takahashi et al., J Virol 73:7812 7816, 1999); a retroviral vector (e.g., Murine Leukemia Virus, spleen necrosis virus, and vectors derived from retroviruses such as Rous Sarcoma Virus, Harvey Sarcoma Virus, avian leukosis virus, a lentivirus, human immunodeficiency virus, myeloproliferative sarcoma virus, and mammary tumor virus); and the like.

Numerous suitable expression vectors are known to those of skill in the art, and many are commercially available. The following vectors are provided by way of example; for eukaryotic host cells: pXT1, pSG5 (Stratagene), pSVK3, pBPV, pMSG, and pSVLSV40 (Pharmacia). However, any other vector may be used so long as it is compatible with the host cell.

Depending on the host/vector system utilized, any of a number of suitable transcription and translation control elements, including constitutive and inducible promoters, transcription enhancer elements, transcription terminators, etc. may be used in the expression vector (see e.g., Bitter et al. (1987) Methods in Enzymology, 153:516-544).

In some embodiments, a nucleotide sequence encoding a guide RNA and/or a variant Cas9 site-directed polypeptide is operably linked to a control element, e.g., a transcriptional control element, such as a promoter. The transcriptional control element may be functional in either a eukaryotic cell, e.g., a mammalian cell; or a prokaryotic cell (e.g., bacterial or archaeal cell). In some embodiments, a nucleotide sequence encoding a guide RNA and/or a variant Cas9 site-directed polypeptide is operably linked to multiple control elements that allow expression of the nucleotide sequence encoding a guide RNA and/or a variant Cas9 site-directed polypeptide in both prokaryotic and eukaryotic cells.

A promoter can be a constitutively active promoter (i.e., a promoter that is constitutively in an active/"ON" state), it may be an inducible promoter (i.e., a promoter whose state, active/"ON" or inactive/"OFF", is controlled by an external stimulus, e.g., the presence of a particular temperature, compound, or protein.), it may be a spatially restricted promoter (i.e., transcriptional control element, enhancer, etc.)(e.g., tissue specific promoter, cell type specific promoter, etc.), and it may be a temporally restricted promoter (i.e., the promoter is in the "ON" state or "OFF" state during specific stages of embryonic development or during specific stages of a biological process, e.g., hair follicle cycle in mice).

Suitable promoters can be derived from viruses and can therefore be referred to as viral promoters, or they can be derived from any organism, including prokaryotic or eukaryotic organisms. Suitable promoters can be used to drive expression by any RNA polymerase (e.g., pol I, pol II, pol III). Exemplary promoters include, but are not limited to the SV40 early promoter, mouse mammary tumor virus long terminal repeat (LTR) promoter; adenovirus major late promoter (Ad MLP); a herpes simplex virus (HSV) promoter, a cytomegalovirus (CMV) promoter such as the CMV immediate early promoter region (CMVIE), a rous sarcoma virus (RSV) promoter, a human U6 small nuclear promoter (U6) (Miyagishi et al. , Nature Biotechnology 20, 497 - 500 (2002)), an enhanced U6 promoter (e.g., Xia et al., Nucleic Acids Res. 2003 Sep 1;31(17)), a human H1 promoter (H1), and the like.

Examples of inducible promoters include, but are not limited toT7 RNA polymerase promoter, T3 RNA polymerase promoter, Isopropyl-beta-D-thiogalactopyranoside (IPTG)-regulated promoter, lactose induced promoter, heat shock promoter, Tetracycline-regulated promoter (e.g., Tet-ON, Tet-OFF, etc.), Steroid-regulated promoter, Metal-regulated promoter, estrogen receptor-regulated promoter, etc. Inducible promoters can therefore be regulated by molecules including, but not limited to, doxycycline; RNA polymerase, e.g., T7 RNA polymerase; an estrogen receptor; an estrogen receptor fusion; etc.

In some embodiments, the promoter is a spatially restricted promoter (i.e., cell type specific promoter, tissue specific promoter, etc.) such that in a multi-cellular organism, the promoter is active (i.e., "ON") in a subset of specific cells. Spatially restricted promoters may also be referred to as enhancers, transcriptional control elements, control sequences, etc. Any convenient spatially restricted promoter may be used and the choice of suitable promoter (e.g., a brain specific promoter, a promoter that drives expression in a subset of neurons, a promoter that drives expression in the germline, a promoter that drives expression in the lungs, a promoter that drives expression in muscles, a promoter that drives expression in islet cells of the pancreas, etc.) will depend on the organism. For example, various spatially restricted promoters are known for plants, flies, worms, mammals, mice, etc. Thus, a spatially restricted promoter can be used to regulate the expression of a nucleic acid encoding a site-directed polypeptide in a wide variety of different tissues and cell types, depending on the organism. Some spatially restricted promoters are also temporally restricted such that the promoter is in the "ON" state or "OFF' state during specific stages of embryonic development or during specific stages of a biological process (e.g., hair follicle cycle in mice).

For illustration purposes, examples of spatially restricted promoters include, but are not limited to, neuron-specific promoters, adipocyte-specific promoters, cardiomyocyte-specific promoters, smooth muscle-specific promoters, photoreceptor-specific promoters, etc. Neuron-specific spatially restricted promoters include, but are not limited to, a neuron-specific enolase (NSE) promoter (see, e.g., EMBL HSENO2, X51956); an aromatic amino acid decarboxylase (AADC) promoter; a neurofilament promoter (see, e.g., GenBank HUMNFL, L04147); a synapsin promoter (see, e.g., GenBank HUMSYNIB, M55301); a thy-1 promoter (see, e.g., Chen et al. (1987) Cell 51:7-19; and Llewellyn, et al. (2010) Nat. Med. 16(10):1161-1166); a serotonin receptor promoter (see, e.g., GenBank S62283); a tyrosine hydroxylase promoter (TH) (see, e.g., Oh et al. (2009) Gene Ther 16:437; Sasaoka et al. (1992) Mol. Brain Res. 16:274; Boundy et al. (1998) J. Neurosci. 18:9989; and Kaneda et al. (1991) Neuron 6:583-594); a GnRH promoter (see, e.g., Radovick et al. (1991) Proc. Natl. Acad. Sci. USA 88:3402-3406); an L7 promoter (see, e.g., Oberdick et al. (1990) Science 248:223-226); a DNMT promoter (see, e.g., Bartge et al. (1988) Proc. Natl. Acad. Sci. USA 85:3648-3652); an enkephalin promoter (see, e.g., Comb et al. (1988) EMBO J. 17:3793-3805); a myelin basic protein (MBP) promoter; a Ca²⁺-calmodulin-dependent protein kinase II-alpha (CamKlla) promoter (see, e.g., Mayford et al. (1996) Proc. Natl. Acad. Sci. USA 93:13250; and Casanova et al. (2001) Genesis 31:37); a CMV enhancer/platelet-derived growth factor-0 promoter (see, e.g., Liu et al. (2004) Gene Therapy 11:52-60); and the like.

Adipocyte-specific spatially restricted promoters include, but are not limited to aP2 gene promoter/enhancer, e.g., a region from -5.4 kb to +21 bp of a human aP2 gene (see, e.g., Tozzo et al. (1997) Endocrinol. 138:1604; Ross et al. (1990) Proc. Natl. Acad. Sci. USA 87:9590; and Pavjani et al. (2005) Nat. Med. 11:797); a glucose transporter-4 (GLUT4) promoter (see, e.g., Knight et al. (2003) Proc. Natl. Acad. Sci. USA 100:14725); a fatty acid translocase (FAT/CD36) promoter (see, e.g., Kuriki et al. (2002) Biol. Pharm. Bull. 25:1476; and Sato et al. (2002) J. Biol. Chem. 277:15703); a stearoyl-CoA desaturase-1 (SCD1) promoter (Tabor et al. (1999) J. Biol. Chem. 274:20603); a leptin promoter (see, e.g., Mason et al. (1998) Endocrinol. 139:1013; and Chen et al. (1999) Biochem. Biophys. Res. Comm. 262:187); an adiponectin promoter (see, e.g., Kita et al. (2005) Biochem. Biophys. Res. Comm. 331:484; and Chakrabarti (2010) Endocrinol. 151:2408); an adipsin promoter (see, e.g., Platt et al. (1989) Proc. Natl. Acad. Sci. USA 86:7490); a resistin promoter (see, e.g., Seo et al. (2003) Molec. Endocrinol. 17:1522); and the like.

Cardiomyocyte-specific spatially restricted promoters include, but are not limited to control sequences derived from the following genes: myosin light chain-2, a-myosin heavy chain, AE3, cardiac troponin C, cardiac actin, and the like. Franz et al. (1997) Cardiovasc. Res. 35:560-566; Robbins et al. (1995) Ann. N.Y. Acad. Sci. 752:492-505; Linn et al. (1995) Circ. Res. 76:584591; Parmacek et al. (1994) Mol. Cell. Biol. 14:1870-1885; Hunter et al. (1993) Hypertension 22:608-617; and Sartorelli et al. (1992) Proc. Natl. Acad. Sci. USA 89:4047-4051.

Smooth muscle-specific spatially restricted promoters include, but are not limited to an SM22a promoter (see, e.g., Akyilrek et al. (2000) Mol. Med. 6:983; and U.S. Patent No. 7,169,874); a smoothelin promoter (see, e.g., WO 2001/018048); an a-smooth muscle actin promoter; and the like. For example, a 0.4 kb region of the SM22a promoter, within which lie two CArG elements, has been shown to mediate vascular smooth muscle cell-specific expression (see, e.g., Kim, et al. (1997) Mol. Cell. Biol. 17, 2266-2278; Li, et al., (1996) J. Cell Biol. 132, 849-859; and Moessler, et al. (1996) Development 122, 2415-2425).

Photoreceptor-specific spatially restricted promoters include, but are not limited to, a rhodopsin promoter; a rhodopsin kinase promoter (Young et al. (2003) Ophthalmol. Vis. Sci. 44:4076); a beta phosphodiesterase gene promoter (Nicoud et al. (2007) J. Gene Med. 9:1015); a retinitis pigmentosa gene promoter (Nicoud et al. (2007) *supra);* an interphotoreceptor retinoid-binding protein (IRBP) gene enhancer (Nicoud et al. (2007) *supra);* an IRBP gene promoter (Yokoyama et al. (1992) Exp Eye Res. 55:225); and the like.

### Libraries

The present disclosure provides a library of guide RNAs. The present disclosure provides a library of nucleic acids comprising nucleotides encoding guide RNAs. A library of nucleic acids comprising nucleotides encoding guide RNAs can comprises a library of recombinant expression vectors comprising nucleotides encoding the guide RNAs.

A library can comprise from about 10 individual members to about 10¹² individual members; e.g., a library can comprise from about 10 individual members to about 10² individual members, from about 10² individual members to about 10³ individual members, from about 10³ individual members to about 10⁵ individual members, from about 10⁵ individual members to about 10⁷ individual members, from about 10⁷ individual members to about 10⁹ individual members, or from about 10⁹ individual members to about 10¹² individual members.

An "individual member" of a library differs from other members of the library in the nucleotide sequence of the DNA targeting segment of the guide RNA. Thus, e.g., each individual member of a library can comprise the same or substantially the same nucleotide sequence of the protein-binding segment as all other members of the library; and can comprise the same or substantially the same nucleotide sequence of the transcriptional termination segment as all other members of the library; but differs from other members of the library in the nucleotide sequence of the DNA targeting segment of the guide RNA. In this way, the library can comprise members that bind to different target nucleic acids.

### Uses

A method for modulating transcription according to the present disclosure finds use in a variety of applications, which are also provided. Applications include research applications; diagnostic applications; industrial applications; and treatment applications.

Research applications include, e.g., determining the effect of reducing or increasing transcription of a target nucleic acid on, e.g., development, metabolism, expression of a downstream gene, and the like.

High through-put genomic analysis can be carried out using a transcription modulation method, in which only the DNA-targeting segment of the guide RNA needs to be varied, while the protein-binding segment and the transcription termination segment can (in some cases) be held constant. A library (e.g., a library) comprising a plurality of nucleic acids used in the genomic analysis would include: a promoter operably linked to a guide RNA-encoding nucleotide sequence, where each nucleic acid would include a different DNA-targeting segment, a common protein-binding segment, and a common transcription termination segment. A chip could contain over 5 x 10⁴ unique guide RNAs. Applications would include large-scale phenotyping, gene-to-function mapping, and meta-genomic analysis.

The methods disclosed herein find use in the field of metabolic engineering. Because transcription levels can be efficiently and predictably controlled by designing an appropriate guide RNA, as disclosed herein, the activity of metabolic pathways (e.g., biosynthetic pathways) can be precisely controlled and tuned by controlling the level of specific enzymes (e.g., via increased or decreased transcription) within a metabolic pathway of interest. Metabolic pathways of interest include those used for chemical (fine chemicals, fuel, antibiotics, toxins, agonists, antagonists, etc.) and/or drug production.

Biosynthetic pathways of interest include but are not limited to (1) the mevalonate pathway (e.g., HMG-CoA reductase pathway) (converts acetyl-CoA to dimethylallyl pyrophosphate (DMAPP) and isopentenyl pyrophosphate (IPP), which are used for the biosynthesis of a wide variety of biomolecules including terpenoids/isoprenoids), (2) the non-mevalonate pathway (i.e., the "2-C-methyl-D-erythritol 4-phosphate/1-deoxy-D-xylulose 5-phosphate pathway" or "MEP/DOXP pathway" or "DXP pathway")(also produces DMAPP and IPP, instead by converting pyruvate and glyceraldehyde 3-phosphate into DMAPP and IPP via an alternative pathway to the mevalonate pathway), (3) the polyketide synthesis pathway (produces a variety of polyketides via a variety of polyketide synthase enzymes. Polyketides include naturally occurring small molecules used for chemotherapy (e. g., tetracyclin, and macrolides) and industrially important polyketides include rapamycin (immunosuppressant), erythromycin (antibiotic), lovastatin (anticholesterol drug), and epothilone B (anticancer drug)), (4) fatty acid synthesis pathways, (5) the DAHP (3-deoxy-D-arabino-heptulosonate 7-phosphate) synthesis pathway, (6) pathways that produce potential biofuels (such as short-chain alcohols and alkane, fatty acid methyl esters and fatty alcohols, isoprenoids, etc.), etc.

### Networks and Cascades

The methods disclosed herein can be used to design integrated networks (i.e., a cascade or cascades) of control. For example, a guide RNA / variant Cas9 site-directed polypeptide may be used to control (i.e., modulate, e.g., increase, decrease) the expression of another DNA-tageting RNA or another variant Cas9 site-directed polypeptide. For example, a first guide RNA may be designed to target the modulation of transcription of a second chimeric dCas9 polypeptide with a function that is different than the first variant Cas9 site-directed polypeptide (e.g., methyltransferase activity, demethylase activity, acetyltansferase activity, deacetylase activity, etc.). In addition, because different dCas9 proteins (e.g., derived from different species) may require a different Cas9 handle (i.e., protein binding segment), the second chimeric dCas9 polypeptide can be derived from a different species than the first dCas9 polypeptide above. Thus, in some cases, the second chimeric dCas9 polypeptide can be selected such that it may not interact with the first guide RNA. In other cases, the second chimeric dCas9 polypeptide can be selected such that it does interact with the first guide RNA. In some such cases, the activities of the two (or more) dCas9 proteins may compete (e.g., if the polypeptides have opposing activities) or may synergize (e.g., if the polypeptides have similar or synergistic activities). Likewise, as noted above, any of the complexes (i.e., guide RNA / dCas9 polypeptide) in the network can be designed to control other guide RNAs or dCas9 polypeptides. Because a guide RNA and variant Cas9 site-directed polypeptide can be targeted to any desired DNA sequence, the methods described herein can be used to control and regulate the expression of any desired target. The integrated networks (i.e., cascades of interactions) that can be designed range from very simple to very complex, and are without limit.

In a network wherein two or more components (e.g., guide RNAs, activator-RNAs, targeter-RNAs, or dCas9 polypeptides) are each under regulatory control of another guide RNA/dCas9 polypeptide complex, the level of expression of one component of the network may affect the level of expression (e.g., may increase or decrease the expression) of another component of the network. Through this mechanism, the expression of one component may affect the expression of a different component in the same network, and the network may include a mix of components that increase the expression of other components, as well as components that decrease the expression of other components. As would be readily understood by one of skill in the art, the above examples whereby the level of expression of one component may affect the level of expression of one or more different component(s) are for illustrative purposes, and are not limiting. An additional layer of complexity may be optionally introduced into a network when one or more components are modified (as described above) to be manipulable (i.e., under experimental control, e.g., temperature control; drug control, i.e., drug inducible control; light control; etc.).

As one non-limiting example, a first guide RNA can bind to the promoter of a second guide RNA, which controls the expression of a target therapeutic/metabolic gene. In such a case, conditional expression of the first guide RNA indirectly activates the therapeutic/metabolic gene. RNA cascades of this type are useful, for example, for easily converting a repressor into an activator, and can be used to control the logics or dynamics of expression of a target gene.

A transcription modulation method can also be used for drug discovery and target validation.

### Examples

Various aspects of the invention make use of the following materials and methods and are illustrated by the following non-limiting examples, wherein Example 1 relates to Cas9 orthologs, Example 2 elates to exchangeability of bacterial RNase III enzymes, Example 3 relates to the Cas9 HNH and RuvC domains, Example 4 relates to exchangeability of Cas9 endonucleases in tracrRNA-directed pre-crRNA maturation by RNase III, Example 5 relates to PAMs of Cas9 orthologs and Example 6 relates to exchangeability of guide RNA and Cas9 endonucleases.

### Materials and Methods

### Bacterial strains and culture conditions

Supplementary Table S1 lists bacterial strains used in this study. *S. pyogenes, Streptococcus mutans, Campylobacter jejuni, N. meningitidis, Escherichia coli* and *Francisella novicida* were grown as previously described (15,16). BHI (Brain Heart Infusion, Becton Dickinson) agar and BHI broth medium supplemented with 1% glucose and 1% lactose were used to culture *S. thermophilus* at 42°C in a 5% CO₂ environment (16). *Pasteurella multocida* and *Staphylococcus aureus* were grown at 37°C on BHI agar plates and in BHI broth with shaking. Cell growth was monitored by measuring the optical density of cultures at 620 nm (OD₆₂₀) using a microplate reader (BioTek PowerWave).

### Bacterial transformation

*E. coli* was transformed with plasmid DNA according to standard protocols (35). Transformation of *S. pyogenes* was performed as previously described (36) with some modifications. *S. pyogenes* pre-cultures were diluted 1:100 in fresh THY medium and grown at 37°C, 5% CO₂ until OD₆₂₀ reached 0.3. Glycine was added to the medium to 10% final concentration and growth was maintained for an additional hour. Cells were spun down at 4°C at 2500 x g and washed three times with electroporation buffer (5 mM KH₂PO₄, 0.4 M D-sorbitol, 10% glycerol, pH 4.5), finally suspended in the same buffer and equalized to the same OD₆₂₀. For electroporation, 1 µg of plasmid was incubated with the competent cells on ice for 10 min. The conditions were 25 µF, 600 Ω and 1.5 V using 1 mm electroporation cuvettes (Biorad). After a regeneration time of 3 h, bacteria were spread on agar medium supplemented with kanamycin (300 µg/ml). Transformation assays were performed at least three times independently with technical triplicates. The efficiencies were calculated as CFU (colony-forming units) per µg of plasmid DNA. Positive and negative control transformations were done with backbone plasmid pEC85 and sterile H₂O, respectively.

### DNA manipulations

DNA manipulations including DNA preparation (QIAprep Spin MiniPrep Kit, Qiagen), PCR (*Phusion*® High-Fidelity DNA *Polymerase,* Finnzyme), DNA digestion (restriction enzymes, Fermentas), DNA ligation (T4 DNA ligase, Fermentas), DNA purification (QIAquick PCR Purification Kit, Qiagen) and agarose gel electrophoresis were performed according to standard techniques or manufacturers' protocols with some modifications (35). Site-directed mutagenesis was done using QuikChange II XL kit (Stratagene) or PCR-based mutagenesis (37). Synthetic oligonucleotides (Sigma-Aldrich & Biomers) and plasmids used and generated in this study are listed in Supplementary Table S1. The integrity of all constructed plasmids was verified by enzymatic digestion and sequencing at LGC Genomics.

### Construction of plasmids for complementation studies in S. pyogenes

The backbone shuttle vector pEC85 was used for complementation study (38,39). The RNase-III encoding genes (*rnc* genes) of *S*. *pyogenes, S. mutans, S. thermophilus, C. jejuni, N. meningitidis, P. multocida, F. novicida, E. coli* and *S. aureus,* and the genes encoding truncated and inactive RNase III variants (truncated and inactive (D51A) *rnc* mutants) of *S. pyogenes* were cloned in pEC483 (pEC85 containing the native promoter of *S. pyogenes rnc*) using Ncol and EcoRI restriction sites (Supplementary Table S1, Supplementary Figure S6). The ortholog and mutant cas9 genes were cloned in pEC342 (pEC85 containing a sequence encoding tracrRNA-171 nt (16) and the native promoter of the *S. pyogenes cas* operon) using Sall and Smal restriction sites (Supplementary Table S1). Note that in a previous study, we observed low abundance of tracrRNA in the cas9 deletion mutant. For this reason, plasmids used in cas9 complementation studies were designed to encode tracrRNA in addition to *cas9* (16). The generated *rnc* and *cas9* recombinant plasmids were introduced in *S. pyogenes* Δ*rnc* and Δ*cas9* deletion strains, respectively (Supplementary Table S1). Plasmid integrity in all complemented strains was checked by plasmid DNA extraction and digestion.

### Construction of plasmids for transformation studies in S. pyogenes

Plasmid pEC85 was used as backbone vector for transformation studies. A DNA fragment containing WT *speM* protospacer sequence was cloned in the PstI site of plasmids containing coding sequences of WT or mutated cas9 from *S. pyogenes* (Supplementary Table S1).

### Construction of plasmids for protein purification

The overexpression vector pET16b (Novagen) was modified by inserting three additional restriction sites (SalI, SacI, NotI) into the Ndel restriction site, generating pEC621. The genes coding for the orthologous Cas9 proteins were PCR amplified from genomic DNA of the corresponding strains using primers containing a SalI and a NotI restriction site (Supplementary Table S1). The *S. pyogenes cas9* mutant genes were PCR amplified from the complementation plasmids mentioned above. All orthologous and mutant *cas9* genes were cloned into the SalI and NotI sites of pEC621.

### Construction of substrate plasmids for in vitro cleavage assays

Plasmid pEC287 that contains the *speM* protospacer sequence was used as a vector to construct all substrate plasmids. The PAM sequence located in 3' just next to the crRNA-targeted sequence of the *speM* protospacer (GGG on this plasmid) was modified by PCR-mediated site-directed mutagenesis (37) using one standard oligonucleotide (OLEC 3140 or OLEC3194) that either introduced or removed a XbaI restriction site for screening purposes, and a second mutagenic oligonucleotide to exchange the protospacer adjacent sequence (Supplementary Table S1).

### RNA preparation

Total RNA from *S. pyogenes* SF370 WT, deletion mutants and complemented strains was prepared from culture samples collected at the mid-logarithmic phase of growth using TRIzol (Sigma-Aldrich). The total RNA samples were treated with DNase I (Fermentas) according to the manufacturer's instructions. The concentration of RNA in each sample was measured using NanoDrop.

### Northern blot analysis

Northern blot analysis was carried out essentially as described previously (40-42). Total RNA was separated on 10% polyacrylamide 8 M urea gels and further processed for blotting on nylon membranes (Hybond™ N+, GE healthcare; Trans-Blot® SD semi-dry transfer apparatus, Biorad; 1X TBE, 2 h at 10 V/cm), chemical crosslinking with EDC (1-Ethyl-3-(3-dimethylaminopropyl) carbodiimide hydrochloride) (41) and prehybridization (Rapid-hyb buffer, GE healthcare; 1 h at 42°C). Oligonucleotide probes (40 pmol) were labeled with ³²P (20 µCi) using the T4-polynucleotide kinase (10 U, Fermentas) and purified using G-25 columns (GE Healthcare) prior use. Visualization of the radioactive signal was done using a phosphorimager. 5S rRNA served as loading control.

### Protein purification

*E. coli* Rosetta2(DE3) and *E. coli* NiCo21(DE3) (New England Biolabs) were transformed with overexpression plasmids coding for *S. pyogenes* WT and mutant or orthologous Cas9, respectively. Cells were grown at 37°C to reach an OD₆₀₀ of 0.7 - 0.8, protein expression was induced by adding IPTG to a final concentration of 0.5 mM and cultures were further grown at 13°C overnight. The cells were harvested by centrifugation and the pellet was resuspended in lysis-buffer (20 mM HEPES pH 7.5, 500 mM KCl [1 M for *S. thermophilus** Cas9], 0.1% Triton X-100, 25 mM imidazole) and lysed by sonication. The lysate was cleared by centrifugation (> 20 000 x g) and incubated with Ni-NTA (Qiagen) for 1 h at 4°C. After washing the Ni-NTA with lysis-buffer and wash-buffer (20 mM HEPES pH 7.5, 300 mM KCl, 0.1% Triton X-100, 25 mM imidazole), the recombinant protein was eluted with elution-buffer (20 mM HEPES pH 7.5, 150 mM KCl, 0.1 mM DTT, 250 mM imidazole, 1 mM EDTA) and the fractions were analyzed by SDS-PAGE. In the case of *S. pyogenes* Cas9 WT and mutants, the protein containing eluates were pooled and further purified via HiTrap SP FF (GE Healthcare) cation-exchange chromatography. Briefly, the protein was loaded on the column equilibrated with buffer A (20 mM HEPES pH 7.5, 100 mM KCl) using an FPLC system (Äkta, GE Healthcare). Cas9 was eluted with a gradient of buffer B (20 mM HEPES pH 7.5, 1 M KCl) over 12 ml. 1 ml fractions were collected and analyzed by SDS-PAGE. The protein containing fractions were pooled and dialyzed overnight (20 mM HEPES pH 7.5, 150 mM KCl, 50% glycerol). For Cas9 orthologs, the eluates from Ni-NTA purification were checked for purity by SDS-PAGE. In case of contaminants, a second purification over chitin beads was performed as described in the manual for NiCo21(DE3) cells from New England Biolabs. Briefly, 1 ml chitin beads (New England Biolabs) equilibrated with buffer A was incubated with the Ni²⁺-IMAC eluates for 1 h at 4°C. Afterwards the beads were added onto a column and the Cas9 containing flowthroughs were collected and again checked for purity by SDS-PAGE (Supplementary Figure S1). The purified proteins were dialyzed overnight. The protein concentration was calculated by measuring the OD₂₈₀ using the extinction coefficient. The detailed characteristics of purified proteins are summarized in Supplementary Figure S1A.

### In vitro transcription

RNA for *in vitro* DNA cleavage assays was generated by *in vitro* transcription using the AmpliScribe™ T7-*Flash*™ Transcription Kit (Epicentre) according to the manufacturer's instructions. PCR products or synthetic oligonucleotides used as templates are listed in Supplementary Table S1. The synthesized tracrRNA and repeat region of crRNA from each bacterial species correspond to the mature forms of RNAs as determined by deep RNA sequencing (15) or bioinformatics predictions. The spacer region of all crRNAs used in this study targets the *speM* protospacer (encoding superantigen; targeted by spacer 2 of *S. pyogenes* SF370 CRISPR array, Spyo1h_002 (16)). Transcribed RNAs were precipitated and further purified from 10% polyacrylamide 8 M urea denaturing gel. The RNA concentration was determined by measuring the OD₂₆₀ and the molarity was calculated. Equimolar amounts of crRNA and tracrRNA were mixed in 5X RNA annealing buffer (1 M NaCl, 100 mM HEPES pH 7.5), heated up to 95°C for 5 min and slowly cooled to room temperature before use.

### In vitro DNA cleavage assays

For the cleavage assays using Cas9 mutant proteins, 25 nM of Cas9 were incubated with equimolar amounts of prehybridized *S. pyogenes* dual-RNA in cleavage buffer (20 mM HEPES pH 7.5, 150 mM KCl, 10 mM MgCl₂, 0.5 mM DTT, 0.1 mM EDTA) for 15 min at 37°C. Plasmid DNA (5 nM) containing *speM* (NGG PAM) was added and further incubated for 1 h at 37°C. The reaction was stopped by addition of 5X loading buffer (250 mM EDTA, 30% glycerol, 1.2% SDS, 0.1% (w/v) bromophenol blue) and analyzed by 1% agarose gel electrophoresis in 1X TAE. Cleavage products were visualized by ethidium bromide staining. All other cleavage assays were carried out using the same conditions with the following modifications: KGB (43) (100 mM potassium glutamate, 25 mM Tris/acetate pH 7.5, 10 mM Mg-acetate, 0.5 mM 2-mercaptoethanol, 10 µg/ml BSA) was used as cleavage buffer and different concentrations of dual-RNA:Cas9 complex were analyzed. The concentration of plasmid DNA was kept constant in all experiments, i.e. 5 nM.

### Search for PAM motifs

Spacer sequences of the selected bacterial species were extracted from the CRISPRdatabase (http://crispr.u-psud.fr/crispr/) and used to find cognate protospacer candidates using megaBLAST (http://blast.ncbi.nih.gov/Blast). Protospacer candidates were defined as containing a sequence with ≥90% similarity to the crRNA spacer sequence and originating from phage, plasmid or genomic DNA related to the bacterial species of the targeting CRISPR-Cas. For the investigated CRISPR-Cas loci, the orientation of transcription was determined previously by RNA sequencing or Northern blot analysis (15,16). It was also shown before that in type II CRISPR-Cas, the PAM sequence is located in 3' of the protospacer, juxtaposed to the sequence targeted by cognate crRNA on the non-target strand (14,18,23,44). To identify possible PAMs in each bacterial species, 10 nt sequences on the non-target strand directly downstream of each protospacer sequence were aligned. A logo plot (http://weblogo.berkeley.edu/) showing the most abundant nucleotides was created and PAM sequences were predicted. In the cases of CRISPR-Cas loci for which no suitable protospacer sequences could be identified (*S. mutans* UA159, *C. jejuni* NCTC 11168, *P. multocida* Pm70, *F. novicida* U112), closely related strains of the same species were selected (Supplementary Table S2). The spacer contents of the type II CRISPR arrays in selected strains were analyzed (http://crispr.u-psud.fr/Server/). The spacer sequences were then used to select cognate protospacer sequences as described above.

### Protein sequence analysis

Position-Specific Iterated (PSI)-BLAST program (45) was used to retrieve orthologs of the Cas9 family in the NCBI nr database. Sequences shorter than 800 amino acids were discarded. The BLASTClust program (46) set up with a length coverage cutoff of 0.8 and a score coverage threshold (bit score divided by alignment length) of 0.8 was used to cluster the remaining sequences (Supplementary Table S2). This procedure produced 82 clusters. In the case of sequences reported in this study, one or several representatives from each cluster were selected and aligned using the MUSCLE program (47) with default parameters, followed by a manual correction on the basis of local alignments obtained using PSI-BLAST (45) and HHpred programs (48). The confidently aligned blocks (Supplementary Figure S2) with 285 informative positions were used for maximum likelihood tree reconstruction using the FastTree program (49) with the default parameters: JTT evolutionary model, discrete gamma model with 20 rate categories. The same program was used to calculate the bootstrap values. Cas1 sequences were selected from the corresponding cas operons (Supplementary Table S2). A few incomplete sequences were substituted by other Cas1 sequences from the same Cas9 cluster (Supplementary Table S2). Several Cas1 proteins from subtypes I-A, B, C and E were included as an outgroup. Cas1 sequences were aligned using the same approach described above and 252 informative positions (Supplementary Figure S3) were used for maximum likelihood tree reconstruction using the FastTree program. RNase III multiple sequence alignment was prepared using the MUSCLE program.

### RNA sequence and structure analysis

RNA duplex secondary structures were predicted using RNAcofold of the Vienna RNA package (50,51) and RNAhybrid (http://bibiserv.techfak.uni-bielefeld.de/rnahybrid/). The structure predictions were then visualized using VARNA (52).

**Supplementary Table S1. Strains, plasmids and primers used in the study.**

| **Strain** | **Relevant characteristics** | **Source** |
|---|---|---|
| ***Streptococcus pyogenes*** | | |
| **WT** | | |
| EC904 | SF370 (M1 serotype) (WT) | ATCC 700294 |

| Δ***cas9*** | | |
|---|---|---|
| EC1788 | EC904Δ*cas9* | (16) |

| Δ***rnc*** | | |
|---|---|---|
| EC1636 | EC904Δ*rnc* | (16) |

| Δ***cas9* in SF370 + *cas9* complementations *in trans*** | | |
|---|---|---|
| EC2121 | EC1788 + pEC714 (P*cas9*(Spy)-*cas9*(Spy)-CtHis) | This study |
| EC2127 | EC1788 + pEC710 (171 tracrRNA-P*cas9*(Spy)-CtHis) | This study |
| EC2150 | EC1788 + pEC553 (P*cas9*(Spy)-*cas9-*HH983AA(Spy)-CtHis) | This study |
| EC2151 | EC1788 + pEC554 (P*cas9*(Spy)-*cas9*-D10A(Spy)-CtHis) | This study |
| EC2152 | EC1788 + pEC555 (P*cas9*(Spy)-*cas9*-H840A(Spy)-CtHis) | This study |
| EC2153 | EC1788 + pEC556 (P*cas9*(Spy)-*cas9*-N854A(Spy)-CtHis | This study |
| EC2154 | EC1788 + pEC557 (P*cas9*(Spy)-*cas9*-N863A(Spy)-CtHis) | This study |
| EC2155 | EC1788 + pEC558 (P*cas9*(Spy)-*cas9*-D986A(Spy)-CtHis) | This study |
| EC2156 | EC1788 + pEC559 (P*cas9*(Spy)-*cas9*-E762A(Spy)-CtHis) | This study |
| EC2118 | EC1788 + pEC518 (P*cas9*(Spy)-*cas9*(Cje)-CtHis) | This study |
| EC2128 | EC1788 + pEC538 (P*cas9*(Spy)-*cas9*(Fno)-CtHis) | This study |
| EC2199 | EC1788 + pEC544 (P*cas9*(Spy)-*cas9*(Nme)-CtHis) | This study |
| EC2119 | EC1788 + pEC520 (P*cas9*(Spy)-*cas9*(Pmu)-CtHis) | This study |
| EC2111 | EC1788 + pEC519 (P*cas9*(Spy)-*cas9*(Smu)-CtHis) | This study |
| EC2112 | EC1788 + pEC521 (P*cas9*(Spy)-*cas9*(Sth*)-CtHis) | This study |

| Δ***rnc* in SF370** + ***rnc* complementations *in trans*** | | |
|---|---|---|
| EC2120 | EC1788 + pEC522 (P*cas9*(Spy)-*cas9*(Sth**)-CtHis) | This study |
| EC2076 | EC1636 + pEC484 (P*rnc*(Spy)-*rnc*(Spy)) | This study |
| EC2084 | EC1636 + pEC505 (P*rnc*(Spy)-*rnc*-catalytically inactive(Spy)) | This study |
| EC2083 | EC1636 + pEC504 (P*rnc*(Spy)-*rnc*-RNA binding inactive(Spy)) | This study |
| EC2078 | EC1636 + pEC486 (P*rnc*(Spy)-*rnc*(Cje)) | This study |
| EC2080 | EC1636 + pEC492 (P*rnc*(Spy)-*rnc*(Eco)) | This study |
| EC2126 | EC1636 + pEC537 (P*rnc*(Spy)-*rnc*(Fno)) | This study |
| EC2085 | EC1636 + pEC506 (P*rnc*(Spy)-*rnc*(Nme)) | This study |
| EC2077 | EC1636 + pEC485 (P*rnc*(Spy)-*rnc*(Pmu)) | This study |
| EC2086 | EC1636 + pEC507 (P*rnc*(Spy)-*rnc*(Sau)) | This study |
| EC2082 | EC1636 + pEC494 (P*rnc*(Spy)-*rnc*(Smu)) | This study |
| EC2131 | EC1636 + pEC534 (P*rnc*(Spy)-*rnc*(Sth)) | This study |

| ***Campylobacter jejuni*** | | |
|---|---|---|
| EC437 | NCTC 11168; ATCC 700819 (WT), CIP 107370 | Pasteur Institute |

| ***Francisella novicida*** | | |
|---|---|---|
| EC1041 | U112 (WT) | Anders Sjöstedt |

| ***Neisseria meningitidis*** | | |
|---|---|---|
| EC438 | CIP 107858 | Pasteur Institute |

| ***Pasteurella multocida*** | | |
|---|---|---|
| EC439 | Pm70 (WT), ATCC BAA-1113 | Pasteur Institute |

| ***Staphylococcus aureus*** | | |
|---|---|---|
| EC36 | COL (WT) | Lab strain collection |

| ***Streptococcus mutans*** | | |
|---|---|---|
| EC1293 | UA159 (WT) | (16) |

| ***Streptococcus thermophilus*** | | |
|---|---|---|
| EC810 | LMD-9 (WT) | (16) |

| ***E. coli*** | | |
|---|---|---|
| RDN204 | TOP10, host for cloning | Invitrogen |
| EC1265 | Rosetta | Novagen |

| | | |
|---|---|---|
| ^{a} Cje: *Campylobacter jejuni* NCTC 11168; Eco: *Escherichia coli* TOP10; Fno: *Francisella novicida* U112; Nme: *Neisseria meningitidis* A Z2491; Pmu: *Pasteurella multocida* Pm70; Sau: *Staphylococcus aureus* COL; Smu: *Streptococcus mutans* UA159; Spy: *Streptococcus pyogenes* SF370; Sth: *Streptococcus thermophilus* LMD-9. | | |

| **Plasmid** | **Relevant characteristics** | **Source** |
|---|---|---|
| **Vectors for *S.* *pyogenes*** | | |
| pEC85 | *repDEG-*pAMβ1, pJH1-*aphIII*, ColE1 | Bernhard Roppenser |

| **Plasmids for *cas9* domain functional and co-evolution analysis in *S. pyogenes* SF370** | | |
|---|---|---|
| pEC268 | pEC85Ω171 tracrRNA (171 nt form) | (16) |
| pEC309 | pEC85Ω P*cas9*(Spy)-*cas9*(Spy) | (16) |
| pEC368 | pEC85Ω171 tracrRNA-P*cas9*(Spy)-*cas9*(Spy) | (16) |
| pEC710 | pEC85Ω171 tracrRNA-P*cas9*(Spy)-CtHis | This study |
| pEC714 | pEC710Ω*cas9*(Spy) | This study |
| pEC553 | pEC710Ω*cas9*-HH983AA(Spy)-CtHis | This study |
| pEC615 | pEC553Ω*speM* | This study |
| pEC554 | pEC710Ω*cas9*-D10A(Spy)-CtHis | This study |
| pEC659 | pEC554ΩspeM | This study |
| pEC555 | pEC710Ω*cas9*-H840A(Spy)-CtHis | This study |
| pEC660 | pEC555ΩspeM | This study |
| pEC556 | pEC710Ω*cas9*-N854A(Spy)-CtHis | This study |
| pEC661 | pEC556ΩspeM | This study |
| pEC557 | pEC710Ω*cas9*-N863A(Spy)-CtHis | This study |
| pEC618 | pEC557Ω*speM* | This study |
| pEC558 | pEC710Ω*cas9*-D986A(Spy)-CtHis | This study |
| pEC662 | pEC558ΩspeM | This study |
| pEC559 | pEC710Ω*cas9*-E762A(Spy)-CtHis | This study |
| pEC619 | pEC559ΩspeM | This study |
| pEC518 | pEC710Ω*cas9*(Cje)-CtHis | This study |
| pEC538 | pEC710Ω*cas9*(Fno)-CtHis | This study |
| pEC544 | pEC710Ω*cas9*(Nme)-CtHis | This study |
| pEC520 | pEC710Ωcas9(Pmu)-CtHis | This study |
| pEC519 | pEC710Ω*cas9*(Smu)-CtHis | This study |
| pEC521 | pEC710Ωcas9(Sth*)-CtHis | This study |
| pEC522 | pEC710Ωcas9(Sth**)-CtHis | This study |

| **Plasmids for *rnc* co-evolution analysis in *S.* *pyogenes* SF370** | | |
|---|---|---|
| pEC483 | pEC85ΩP*rnc*(Spy) | This study |
| pEC484 | pEC85ΩP*rnc*(Spy)-*rnc*(Spy) | This study |
| pEC505 | pEC85ΩP*rnc*(Spy)-*rnc*-catalytically inactive(Spy) | This study |
| pEC504 | pEC85ΩP*rnc*(Spy)-*rnc*-RNA binding inactive(Spy) | This study |
| pEC486 | pEC85ΩP*rnc*(Spy)-*rnc*(Cje) | This study |
| pEC492 | pEC85ΩP*rnc*(Spy)-*rnc*(Eco) | This study |
| pEC537 | pEC85ΩP*rnc*(Spy)-rnc(Fno) | This study |
| pEC506 | pEC85ΩP*rnc*(Spy)-rnc(Nme) | This study |
| pEC485 | pEC85ΩP*rnc*(Spy)-*rnc*(Pmu) | This study |
| pEC507 | pEC85ΩP*rnc*(Spy)-*rnc*(Sau) | This study |
| pEC494 | pEC85ΩP*rnc*(Spy)-*rnc*(Smu) | This study |
| pEC534 | pEC85ΩP*rnc*(Spy)-*rnc*(Sth) | This study |

| **Plasmids for protospacer study *in vitro*** | | |
|---|---|---|
| pEC287 | pEC85ΩP*speM*-*speM* (10 bp downstream protospacer: GGGTATTGGG) | Lab plasmid collection |
| pEC691 | pEC287 (10 bp downstream protospacer: TGGTATTGGG) | This study |
| pEC692 | pEC287 (10 bp downstream protospacer: TGGTGTTGGG) | This study |
| pEC693 | pEC287 (10 bp downstream protospacer: GGGTGATTGG) | This study |
| pEC694 | pEC287 (10 bp downstream protospacer: GGAGAATGGG) | This study |
| pEC696 | pEC287 (10 bp downstream protospacer: GGGTCATAGG) | This study |
| pEC697 | pEC287 (10 bp downstream protospacer: AGAAACAGGG) | This study |
| pEC698 | pEC287 (10 bp downstream protospacer: AGAACCAGGG) | This study |
| pEC701 | pEC287 (10 bp downstream protospacer: GTTTGATTGG) | This study |
| pEC706 | pEC287 (10 bp downstream protospacer: GGAAAATGGG) | This study |

| **Plasmids for Cas9 overexpression** | | |
|---|---|---|
| pEC225 | pET16b | Novagen |
| pEC621 | pEC225 inserted with cassette harboring Notl, SacI, SalI site | This study |
| pEC626 | pEC621Ω*cas9*(Spy) | This study |
| pEC627 | pEC621Ω*cas9*-D10A(Spy) | This study |
| pEC628 | pEC621Ω*cas9*-E762A(Spy) | This study |
| pEC629 | pEC621Ωcas9-H840A(Spy) | This study |
| pEC630 | pEC621Ωcas9-N854A(Spy) | This study |
| pEC631 | pEC621Ωcas9-HH983AA(Spy) | This study |
| pEC632 | pEC621Ω*cas9*(Cje) | This study |
| pEC633 | pEC621Ω*cas9*(Pmu) | This study |
| pEC634 | pEC621Ω*cas9*(Nme) | This study |
| pEC635 | pEC621Ω*cas9*(Smu) | This study |
| pEC638 | pEC621Ω*cas9*-N863A(Spy) | This study |
| pEC639 | pEC621Ω*cas9*-D986A(Spy) | This study |
| pEC640 | pEC621Ω*cas9*(Sth*) | This study |
| pEC641 | pEC621Ω*cas9*(Sth**) | This study |
| pEC657 | pEC621Ω*cas9*(Fno) | This study |

| **Purpose** | **Primer** | **Sequence 5'-3'^{a}** | **F/R^{b}** | **Usage^{c}** |
|---|---|---|---|---|
| **tracrRNA expression in *S*. *pyogenes* SF370** | | | | |
| tracrRNA | OLEC101 4 | *GGACTAGCCTTATTTTAACTTG* | R | NB (3' probe) |

| **crRNA (CRISPR01 (type II-A) expression in *S*. *pyogenes* SF370** | | | | |
|---|---|---|---|---|
| crRNA | OLEC104 9 | *GGACCATTCAAAACAGCATAGCTCTAAAAC* | R | NB (repeat) |

| **Loading controls for Northern blots** | | | | |
|---|---|---|---|---|
| 5S rRNA | OLEC288 | *CTAAGCGACTACCTTATCTCA* | R | NB |

| **His-tagged *cas9* constructs (pEC85-based)** | | | | |
|---|---|---|---|---|
| pEC710 | OLEC215 1 | GCAGGAATTCATCAGTGATGGTGATGGTGATGC CCGGGTTTGTCGACCTCCTAAAATAAAAAGTTTA AATTAAATC | F | Cloning |
| | OLEC206 6 | GGTGGTCTGCAGGTTTGCAGTCAGAGTAGAATA GAAG | R | |
| pEC714 | OLEC209 6 | ATGCAGGTCGACATGGATAAGAAATACTCAATAG GC | F | Expression *cas9*(Spy) |
| | OLEC209 7 | ATGCAGCCCGGGGTCACCTCCTAGCTGACTCAA ATC | R | |
| *speM* | OLEC286 7 | ATGCAGCCTGCAGGGTGACAGAGAGAAACTTGA TTCAAC | F | Cloning of *speM* in other plasmids |
| | OLEC286 8 | ATGCAGCCTGCAGGCTTCGTTTAAGTAAACATCA AAGTG | R | |
| pEC518 | OLEC210 4 | ATGCAGGTCGAC*GTGGCAAGAATTTTGGCATTTG* | F | Cloning *cas9*(Cje) |
| | OLEC210 5 | ATGCAGCCCGGG*TTTTTTAAAATCTTCTCTTTGTC* | R | |
| pEC538 | OLEC284 0 | ATTAGTCGAC*ATGAATTTCAAAATATTGCCAATAG* | F | Cloning *cas9*(Fno) |
| | OLEC284 1 | ATTACCCGGGA*TTATTAGATGTTTCATTATAAATAC* | R | |
| pEC544 | OLEC209 2 | ATGCAGGTCGACATGGCTGCCTTCAAACCTAATC C | F | Cloning *cas9*(Nme) |
| | OLEC209 3 | ATGCAGCCCGGGACGGACAGGCGGGCGTTTTTT CAG | R | |
| pEC520 | OLEC210 0 | ATGCAGGTCGACATGCAAACAACAAATTTAAGTT A | F | Cloning *cas9*(Pmu) |
| | OLEC210 1 | ATGCAGCCCGGGACGCACAGGTTGTCTTTGCTG AG | R | |
| pEC519 | OLEC209 0 | ATGCAGGTCGACATGAAAAAACCTTACTCTATTG GAC | F | Cloning *cas9*(Smu) |
| | OLEC209 1 | ATGCAGCCCGGGGTCTCCTCCTAACTTATTGAGA TC | R | |
| pEC521 | OLEC209 8 | ATGCAGGTCGACATGACTAAGCCATACTCAATTG G | F | Cloning *cas9*(Sth*) |
| | OLEC209 9 | ATGCAGCCCGGGACCCTCTCCTAGTTTGGCAAG GTC | R | |
| pEC522 | OLEC210 2 | ATGCAGGTCGACATGAGTGACTTAGTTTTAGGAC TTG | F | Cloning *cas9*(Sth**) |
| | OLEC210 3 | ATGCAGCCCGGGAAAATCTAGCTTAGGCTTATCA CC | R | |
| pEC553 | OLEC222 9 | GTACGTGAGATTAACAATTACGCTGCTGCCCATG ATGCGTATCTA | F | Mutagenesis *cas9-*HH983AA(Spy) |
| | OLEC223 0 | TAGATACGCATCATGGGCAGCAGCGTAATTGTTA ATCTCACGTAC | R | |
| pEC554 | OLEC212 8 | GAAATACTCAATAGGCTTAGCTATCGGCACAAAT AGCGTCG | F | Mutagenesis *cas9*-D10A(Spy) |
| | OLEC212 9 | CGACGCTATTTGTGCCGATAGCTAAGCCTATTGA GTATTTC | R | |
| pEC555 | OLEC222 3 | TTTAAGTGATTATGATGTCGATGCCATTGTTCCAC AAAGTTTCCT | F | Mutagenesis *cas9*-H840A(Spy) |
| | OLEC222 4 | AGGAAACTTTGTGGAACAATGGCATCGACATCAT AATCACTTAAA | R | |
| pEC556 | OLEC222 5 | CCTTAAAGACGATTCAATAGACGCTAAGGTCTTA ACGCGTTCTGA | F | Mutagenesis *cas9*-N854A(Spy) |
| | OLEC222 6 | TCAGAACGCGTTAAGACCTTAGCGTCTATTGAAT CGTCTTTAAGG | R | |
| pEC557 | OLEC222 7 | GGTCTTAACGCGTTCTGATAAAGCTCGTGGTAAA TCGGATAACGT | F | Mutagenesis *cas9*-N863A(Spy) |
| | OLEC222 8 | ACGTTATCCGATTTACCACGAGCTTTATCAGAAC GCGTTAAGACC | R | |
| pEC558 | OLEC223 1 | GTAACAATTACCATCATGCCCATGCTGCGTATCT AAATGCCGTCG | F | Mutagenesis *cas9*-D986A(Spy) |
| | OLEC223 2 | CGACGGCATTTAGATACGCAGCATGGGCATGAT GGTAATTGTTAC | R | |
| pEC559 | OLEC222 1 | CAGAAAATATCGTTATTGCAATGGCACGTGAAAA TCAGACA | F | Mutagenesis *cas9*-E762A(Spy) |
| | OLEC222 2 | TGTCTGATTTTCACGTGCCATTGCAATAACGATAT TTTCTG | R | |

| ***rnc* constructs (pEC85-based)** | | | | |
|---|---|---|---|---|
| pEC483 | OLEC214 9 | ATGCAGGCATGCCCTGTAGTTTTGGCTTGTCTGA TC | F | Cloning in pEC85 |
| | OLEC327 4 | ATGCAGAGCTCCATGGAAAATCCCTTTCATATTT GTCAGTAGACC | R | |
| pEC484 | OLEC210 9 | ATGCAGCCATGGAACAGCTTGAAGAGTTACTCTC AAC | F | Cloning *rnc*(Spy), SEQ |
| | OLEC166 8 | CTTTTAAAAACATCTAAACCTCAC | R | |
| pEC504 | OLEC210 9 | ATGCAGCCATGGAACAGCTTGAAGAGTTACTCTC AAC | F | Cloning of *rnc* RNA binding inactive(Spy) |
| | OLEC265 6 | ATGCAGGAATTCCTACCCTTTTTCCACCTGAGGA ATC | R | |
| pEC505 | OLEC214 2 | GAACGCTTGGAATTTTTAGGAGCCGCTGTTCTAC AATTGATTATT | F | Mutagenesis of *rnc* catalytically inactive(Spy) |
| | OLEC214 3 | AATAATCAATTGTAGAACAGCGGCTCCTAAAAATT CCAAGCGTTC | R | |
| pEC486 | OLEC211 6 | ATGCAGCCATGGAAAACATTGAAAAGCTAGAGCA GAG | F | Cloning *rnc*(Cje), SEQ |
| | OLEC211 7 | ATGCAGGAATTCCTATAAAGCTCCTAATTTCTCAA G | R | |
| pEC492 | OLEC212 4 | ATGCAGCCATGGACCCCATCGTAATTAATCGGCT TC | F | Cloning *rnc*(Eco), SEQ |
| | OLEC212 5 | ATGCAGGAATTCTCATTCCAGCTCCAGTTTTTCA ACG | R | |
| pEC537 | OLEC284 2 | ATTACCATGG*TTCCTGAATATTCACGATTTTATAAC* | F | Cloning *rnc*(Fno), SEQ |
| | OLEC284 3 | ATTGAATTCCTATTTTTTTTCATGTAAGCCTTGTTG TG | R | |
| pEC506 | OLEC211 8 | ATGCAGCCATGGAAGAC GATGTTTTGAAACAGCA GG | F | Cloning *rnc*(Nme), SEQ |
| | OLEC211 9 | ATGCAGGAATTCTCATTTCTTTTTCTTCTTCAGCG GC | R | |
| pEC485 | OLEC211 | ATGCAGCCATGGCTCAAAATTTAGAACGTTTACA | F | Cloning *rnc*(Pmu), |
| | 4 | ACG | | SEQ |
| | OLEC211 5 | ATGCAGGAATTCTCATTTCATTTCCAATAATTGT | R | |
| pEC507 | OLEC212 6 | ATGCAGCCATGGCTAAACAAAAGAAAAGTGAGAT AG | F | Cloning *rnc*(Sau), SEQ |
| | OLEC212 7 | ATGCAGGAATTCCTATTTAATTTGTTTTAATTGCT TATAGG | R | |
| pEC494 | OLEC211 0 | ATGCAGCCATGGAAACATTAGAAAAAAAACTGGC AG | F | Cloning *rnc*(Smu), SEQ |
| | OLEC211 1 | ATGCAGGAATTCTTAAGAACCTCGTTGAAGTTTTT C | R | |
| pEC534 | OLEC284 9 | ATTACCATGGATCAACTTGAACAAAAACTTGAACA GGACTTTGG | F | Cloning *rnc*(Sth), SEQ |
| | OLEC285 0 | ATTAGAATTCTTAATTACCTAGTTGTTCAAGGGCA GACTTCGC | R | |

| **Cas9 overexpression (pEC621 based)** | | | | |
|---|---|---|---|---|
| pEC621 | OLEC297 8 | TAGCGGCCGCGAGCTCGTCGACGC | F | Cassette inserting NotI, SacI, SalI site in pEC225 |
| | OLEC297 9 | TAGCGTCGACGAGCTCGCGGCCGC | R | |
| pEC626, 627, 628, 629, 630, 631, 638, 639 | OLEC209 7 | ATGCAGGTCGACATGGATAAGAAATACTCAATAG GC | F | Cloning *cas9*(Spy and all mutants) |
| | OLEC298 3 | AGCTAGCGGCCGCTCAGTCACCTCCTAGCTGAC TCAAATC | R | |
| pEC632 | OLEC210 4 | ATGCAGGTCGAC*GTGGCAAGAATTTTGGCATTTG* | F | Cloning *cas9*(Cje) |
| | OLEC298 6 | ATGCAGCGGCCGCTCATTTTTAAAATCTTCTCTT TGTC | R | |
| pEC633 | OLEC210 0 | ATGCAGGTCGACATGCAAACAACAAATTTAAGTT A | F | Cloning *cas9*(Pmu) |
| | OLEC217 3 | ATGACGCGGCCGCTTAACGCACAGGTTGTCTTT GCTG | R | |
| pEC634 | OLEC209 | ATGCAGGTCGACATGGCTGCCTTCAAACCTAATC | F | Cloning *cas9*(Nme) |
| | 2 | C | | |
| | OLEC298 2 | ATGACGCGGCCGCTTAACGGACAGGCGGGCGTT TTTTCAG | R | |
| pEC635 | OLEC209 0 | ATGCAGGTCGACATGAAAAAACCTTACTCTATTG GAC | F | Cloning *cas9*(Smu) |
| | OLEC298 1 | ATGACGCGGCCGCTTAGTCTCCTCCTAACTTATT GAG | R | |
| pEC640 | OLEC209 8 | ATGCAGGTCGACATGACTAAGCCATACTCAATTG G | F | Cloning *cas9*(Sth*) |
| | OLEC298 4 | ATGACGCGGCCGCTTAACCCTCTCCTAGTTTGGC AAG | R | |
| pEC641 | OLEC210 2 | ATGCAGGTCGACATGAGTGACTTAGTTTTAGGAC TTG | F | Cloning *cas9*(Sth**) |
| | OLEC298 5 | ATGACGCGGCCGCTTAAAAATCTAGCTTAGGCTT ATCAC | R | |
| pEC657 | OLEC284 0 | ATTAGTCGAC*ATGAATTTCAAAATATTGCCAATAG* | F | Cloning *cas9*(Fno) |
| | OLEC298 7 | ATGCAGCGGCCGCCTAATTATTAGATGTTTCATT ATAAATAC | R | |

| **Mutagenesis 10 bp downstream of *speM* protospacer** | | | | |
|---|---|---|---|---|
| pEC691 | OLEC314 0 | *CAACCACTAATTT*C*TAGAAAAATCTTCG* | R | I Mutagenesis on pEC287 |
| | OLEC314 1 | *CAATTTGTAAAAAATGGTATTGGGGAATTC* | F | |
| pEC692 | OLEC314 0 | *CAACCACTAATTT*C*TAGAAAAATCTTCG* | R | Mutagenesis on pEC287 |
| | OLEC314 2 | *CAATTTGTAAAAAA*T*GGT*G*TTGGGGAATTC* | F | |
| pEC693 | OLEC314 0 | *CAACCACTAATTT*C*TAGAAAAATCTTCG* | R | Mutagenesis on pEC287 |
| | OLEC314 4 | *CAATTTGTAAAAAAGGGT*G*AT*T*GGGAATTC* | F | |
| pEC694 | OLEC314 0 | *CAACCACTAATTT*C*TAGAAAAATCTTCG* | R | Mutagenesis on pEC287 |
| | OLEC314 3 | *CAATTTGTAAAAAAGG*AG*A*A*TGGGGAATTC* | F | |
| pEC696 | OLEC319 4 | *CAACCACTAATTT*T*TAGAAAAATCTTCG* | R | Mutagenesis on pEC693 |
| | OLEC319 7 | *CAATTTGTAAAAAAGGGT*C*AT*A*GGGAATTC* | F | |
| pEC697 | OLEC319 4 | *CAACCACTAATTT*T*TAGAAAAATCTTCG* | R | Mutagenesis on pEC694 |
| | OLEC319 8 | *CAATTTGTAAAAAAAGA*A*A*CA*GGGGAATTC* | F | |
| pEC698 | OLEC319 4 | *CAACCACTAATTT*T*TAGAAAAATCTTCG* | R | Mutagenesis on pEC694 |
| | OLEC319 9 | *CAATTTGTAAAAAA*A*GA*ACCA*GGGGAATTC* | F | |
| pEC701 | OLEC319 4 | *CAACCACTAATTT*T*TAGAAAAATCTTCG* | R | Mutagenesis on pEC693 |
| | OLEC320 4 | *CAATTTGTAAAAAAG*TT*TGATTGGGAATTC* | F | |
| pEC706 | OLEC319 4 | *CAACCACTAATTT*T*TAGAAAAATCTTCG* | R | Mutagenesis on pEC694 |
| | OLEC320 8 | *CAATTTGTAAAAAAGG*AA*A*A*TGGGGAATTC* | F | |

| ***In vitro* tracrRNA and crRNA of *Streptococcus* *pyogenes* SF370 (*speM* spacer underlined)** | | | | |
|---|---|---|---|---|
| T7-tracrRNA | OLEC152 1 | GAAATTAATACGACTCACTATAGAAAACAGCATA GCAAGTTAAAATAA | F | T7-tracrRNA 5' |
| | OLEC152 2 | *AAAAAAAGCACCGACTCGGTGCCAC* | R | T7-tracrRNA 3' |
| T7-crRNA (template) | OLEC217 7 | GAAATTAATACGACTCACTATAGGATAACTCAAT TTGTAAAAAAGTTTTAGAGCTATGCTGTTTTG | F | crRNA speM 5' |
| | OLEC217 9 | CAAAACAGCATAGCTCTAAAACTTTTTACAAATT GAGTTATCCTATAGTGAGTCGTATTAATTTC | R | crRNA speM 3' |
| ***In vitro* tracrRNA and crRNA of *Neisseria* *meningitidis* A Z2491 (*speM* spacer underlined)** | | | | |
| T7-tracrRNA | OLEC308 3 | GAAATTAATACGACTCACTATAGGGAGAGCGAA ATGAGAACCGTTGCTACAATAAGGCGTCTGAAAA GATGTGCCGCAACGCTCTGCCCCTTAAAGCTTCT | F | T7-tracrRNA 5' |
| (template) | | GCTTTAAGGGGCATCGTTTATT | | |
| | OLEC308 4 | AATAAACGATGCCCCTTAAAGCAGAAGCTTTAAG GGGCAGAGCGTTGCGGCACATCTTTTCAGACGC CTTATTGTAGCAACGGTTCTCATTTCGCTCTCCCT ATAGTGAGTCGTATTAATTTC | R | T7-tracrRNA 3' |
| T7-crR NA (template) | OLEC220 9 | GAAATTAATACGACTCACTATAGATGATAACTCA ATTTGTAAAAAAGTTGTAGCTCCCTTTCTCATTT | F | crRNA speM 5' |
| | OLEC221 4 | AAATGAGAAAGGGAGCTACAACTTTTTTACAAATT GAGTTATCATCTATAGTGAGTCGTATTAATTTC | R | crRNA speM 3' |

| ***In vitro* tracrRNA and crRNA of *Streptococcus mutans* UA159 (*speM* spacer underlined)** | | | | |
|---|---|---|---|---|
| T7-tracrRNA | OLEC309 8 | GAAATTAATACGACTCACTATAGGAAACAACACA GCAAGTTAAAATAAG | F | T7-tracrRNA 5' |
| | OLEC309 9 | *AAATAAAAAAGCACCGAATCGG* | R | T7-tracrRNA 3' |
| T7 -crRNA (template) | OLEC308 5 | GAAATTAATACGACTCACTATAGGATAACTCAAT TTGTAAAAAAGTTTTAGAGCTGTGTTGT | F | crRNA speM 5' |
| | OLEC308 6 | ACAACACAGCTCTAAAACTTTTTTACAAATTGAGT TATCCTATAGTGAGTCGTATTAATTTC | R | crRNA speM 3' |

| ***In vitro* tracrRNA and crRNA of *Campylobacter* *jejuni* NCTC 11168 (*speM* spacer underlined)** | | | | |
|---|---|---|---|---|
| T7-tracrRNA (template) | OLEC312 8 | GAAATTAATACGACTCACTATAGGAAGGACTA AAATAAAGAGTTTGCGGGACTCTGCGGGGTTACA ATCCCCTAAAACCGC | F | T7-tracrRNA 5' |
| | OLEC312 9 | GCGGTTTTAGGGGATTGTAACCCCGCAGAGTCC CGCAAACTCTTTATTTTAGTCCCTTCCTATAGTGA GTCGTATTAATTTC | R | T7-tracrRNA 3' |
| T7-crRNA (template) | OLEC308 7 | GAAATTAATACGACTCACTATAGGATAACTCAAT TTGTAAAAAAGTTTTAGTCCCT | F | crRNA speM 5' |
| | OLEC308 8 | AGGGACTAAAACTTTTTACAAATTGAGTTATCCT ATAGTGAGTCGTATTAATTTC | R | crRNA speM 3' |

| ***In vitro* tracrRNA and crRNAs of *Francisella novicida* U112 (*speM* spacer underlined)** | | | | |
|---|---|---|---|---|
| T7-tracrRNA | OLEC310 2 | GAAATTAATACGACTCACTATAGGGTACCAAATA ATTAATGCTCTG | F | T7-tracrRNA 5' |
| | OLEC310 | *GTTATTCAGACGTGTCAAACAG* | R | T7-tracrRNA 3' |
| | 3 | | | |
| T7-crR NA (template) | OLEC308 9 | GAAATTAATACGACTCACTATAGGATAACTCAAT TTGTAAAAAAGTTTCAGTTGCTGAATTATTTGGTA AAC | F | crRNA speM 5' |
| | OLEC309 0 | GTTTACCAAATAATTCAGCAACTGAAACTTTTTTA CAAATTGAGTTATCCTATAGTGAGTCGTATTAAT TTC | R | crRNA speM 3' |

| ***In vitro* tracrRNA and crRNAs of *Streptococcus thermophilus** LMD-9 (*speM* spacer underlined)** | | | | |
|---|---|---|---|---|
| T7-tracrRNA | OLEC310 4 | GAAATTAATACGACTCACTATAGGAACAACACA GCGAGTTAAAATAAGG | F | T7-tracrRNA 5' |
| | OLEC310 5 | *AAAAAAAACACCGAATCGGTG* | R | T7-tracrRNA 3' |
| T7-crR NA (template) | OLEC308 5 | GAAATTAATACGACTCACTATAGGATAACTCAAT TTGTAAAAAAGTTTTAGAGCTGTGTTGT | F | crRNA speM 5' |
| | OLEC308 6 | ACAACACAGCTCTAAAACTTTTTTACAAATTGAGT TATCCTATAGTGAGTCGTATTAATTTC | R | crRNA speM 3' |

| ***In vitro* tracrRNA and crRNAs of *Streptococcus thermophilus*** LMD-9 (*speM* spacer underlined)** | | | | |
|---|---|---|---|---|
| T7-tracrRNA | OLEC310 6 | GAAATTAATACGACTCACTATAGGCTTAAATCTT GCAGAAGCTACAAAG | F | T7-tracrRNA 5' |
| | OLEC310 7 | *AAATAACGAAAACACCCTGCC* | R | T7-tracrRNA 3' |
| T7-crRNA (template) | OLEC309 1 | GAAATTAATACGACTCACTATAGGATAACTCAAT TTGTAAAAAAGTTTTGTACTCTCAAGATTTA | F | crRNA speM 5' |
| | OLEC309 2 | TAAATCTTGAGAGTACAAAAACTTTTTACAAATT GAGTTATCCTATAGTGAGTCGTATTAATTTC | R | crRNA speM 3' |

| ***In vitro* tracrRNA and crRNAs of *Pasteurella multocida* Pm70 (*speM* spacer underlined)** | | | | |
|---|---|---|---|---|
| T7-tracrRNA | OLEC310 8 | GAAATTAATACGACTCACTATAGGCTGCGAAAT GAGAGACGTTGCTAC | F | T7-tracrRNA 5' |
| | OLEC310 9 | *AAAAACGATGCCCCTTGCAATTAAG* | R | T7-tracrRNA 3' |
| T7-crRNA (template) | OLEC309 3 | GAAATTAATACGACTCACTATAGGATAACTCAAT TTGTAAAAAAGTTGTAGTTCCCTCTCTCATTTCGC | F | crRNA speM 5' |
| | OLEC309 4 | GCGAAATGAGAGAGGGAACTACAACTTTTTTACA AATTGAGTTATCCTATAGTGAGTCGTATTAATTTC | R | crRNA speM 3' |

| **Primers for sequencing analysis** | | | | |
|---|---|---|---|---|
| ***cas9 Streptococcus mutans* UA159** | | | | |
| *cas9*(Smu) | OLEC279 2 | *ATGAAAAAACCTTACTCTATTGGA* | F | SEQ |
| | OLEC279 3 | *GATTTTAAAAAGCATTTTGAATTA* | F | SEQ |
| | OLEC279 4 | *TACTTGCCAAATCAAAAAGTTCTT* | F | SEQ |
| | OLEC279 5 | *ATTATGGGACATCAACCTGAAAAT* | F | SEQ |
| | OLEC279 6 | *TACCCACAATTGGAACCTGAATTT* | F | SEQ |

| ***cas9 Neisseria* *meningitidis* A Z2491** | | | | |
|---|---|---|---|---|
| *cas9*(Nme) | OLEC279 7 | *ATGGCTGCCTTCAAACCTAATCCA* | F | SEQ |
| | OLEC279 8 | *GTTCAAAAAATGTTGGGGCATTGC* | F | SEQ |
| | OLEC279 9 | *ATCCATATTGAAACTGCAAGGGAA* | F | SEQ |
| | OLEC280 0 | *AACGCGTTTGACGGTAAAACCATA* | F | SEQ |

| ***cas9 Streptococcus thermophilus** LMD-9** | | | | |
|---|---|---|---|---|
| *cas9*(Sth*) | OLEC280 7 | *ATGACTAAGCCATACTCAATTGGA* | F | SEQ |
| | OLEC280 8 | *GATTTTAGGAAATGTTTTAATTTA* | F | SEQ |
| | OLEC280 9 | *TATTTGCCAGAAGAGAAGGTACTT* | F | SEQ |
| | OLEC281 0 | *GTAATGGGAGGAAGAAAACCCGAG* | F | SEQ |
| | OLEC281 | *GCAAGTGCTTTACTTAAGAAATAC* | F | SEQ |
| | 1 | | | |
| | OLEC281 2 | *TTACTTTATCATGCTAAGAGAATA* | F | SEQ |

| ***cas9 Streptococcus thermophilus*** LMD-9** | | | | |
|---|---|---|---|---|
| *cas9*(Sth**) | OLEC281 7 | *ATGAGTGACTTAGTTTTAGGACTT* | F | SEQ |
| | OLEC281 8 | *ATTTTTGGAATTCTAATTGGGAAA* | F | SEQ |
| | OLEC281 9 | *GGAGACTTTGACAATATTGTCATC* | F | SEQ |
| | OLEC282 0 | *TTGAATTTGTGGAAAAAACAAAAG* | F | SEQ |
| | OLEC282 1 | *CAGGAAAAATACAATGACATTAAG* | F | SEQ |

| ***cas9 Pasteurella multocida* Pm70** | | | | |
|---|---|---|---|---|
| *cas9*(Pmu) | OLEC281 3 | *ATGCAAACAACAAATTTAAGTTAT* | F | SEQ |
| | OLEC281 4 | *ACGCATGAAAAAAATGAGTTTAAA* | F | SEQ |
| | OLEC281 5 | *CTTGGGAAATCTTTTAAAGAACGT* | F | SEQ |
| | OLEC281 6 | *TATGAAATGGTGGATCAAGAAAGC* | F | SEQ |

| **cas9 *Campylobacter* *jejuni* NCTC 11168** | | | | |
|---|---|---|---|---|
| *cas9*(Cje) | OLEC282 2 | *GTGGCAAGAATTTTGGCATTTGAT* | F | SEQ |
| | OLEC282 3 | *GATGAAAAAAGAGCGCCAAAAAAT* | F | SEQ |
| | OLEC282 4 | *AACTACAAGGCCAAAAAAGACGCC* | F | SEQ |
| | OLEC282 5 | *AACAAAAGGAAGTTTTTTGAGCCT* | F | SEQ |

| ***cas9 Francisella novicida* U112** | | | | |
|---|---|---|---|---|
| cas9(Fno) | OLEC286 9 | *ATGAATTTCAAAATATTGCCAATA* | F | SEQ |
| | OLEC287 0 | *TTAGATACTCTTTTAACTGATGAT* | F | SEQ |
| | OLEC287 1 | *TTAAAAGTCTTAAAGTCAAGTAAA* | F | SEQ |
| | OLEC287 2 | *GGTTCAGAAGATAAAAAAGGTAAT* | F | SEQ |
| | OLEC287 3 | *AGAATTTTCTGCCTACGTGATCTT* | F | SEQ |
| | OLEC287 4 | *CCAATACTAATCCATAAAGAACTA* | F | SEQ |
| | OLEC287 5 | *ACATCAAAAAATATTTTTTGGCTG* | F | SEQ |

| | | | | |
|---|---|---|---|---|
| ^{a} *italic,* sequence annealing to the template; underlined, restriction site; **bold,** T7 promoter. ^{b} F, forward primer; R, reverse primer. ^{c} NB, probe for Northern blot; SEQ, sequencing | | | | |

### Example 1

### Diversity of Cas9 orthologs.

To investigate the evolution and diversity of dual-RNA:Cas9 systems, publicly available genomes were subjected to multiple rounds of BLAST search using previously retrieved Cas9 sequences as queries (15). Cas9 orthologs were identified in 653 bacterial strains representing 347 species (Supplementary Table S2). After removing incomplete or highly similar sequences, we selected 83 diverse, representative Cas9 orthologs for multiple sequence alignment and phylogenetic tree reconstruction (Figure 1A, Supplementary Table S2, Supplementary Figures S2 and S4, see Materials and Methods). The Cas9 tree topology largely agrees with the phylogeny of the corresponding Cas1 proteins (Supplementary Table S2, Supplementary Figures S3 and S4) and fully supports the previously described classification of type II CRISPR-Cas into three subtypes, II-A (specified by *csn2*), II-B (characterized by long and most diverged cas9 variants (formerly *csx12*) and *cas4*), and II-C (three-*cas* gene operon) (15).

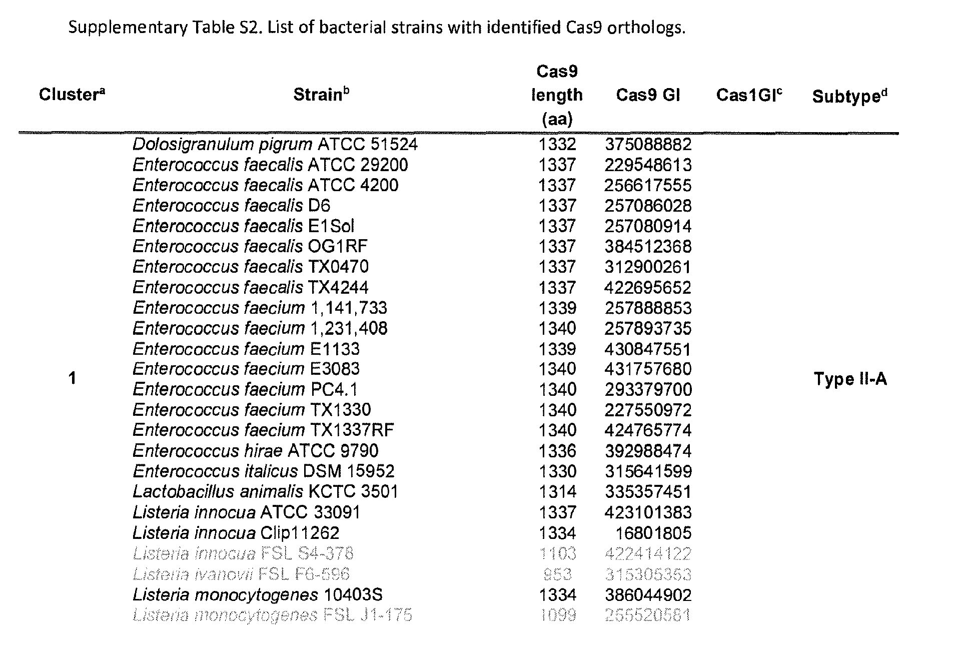

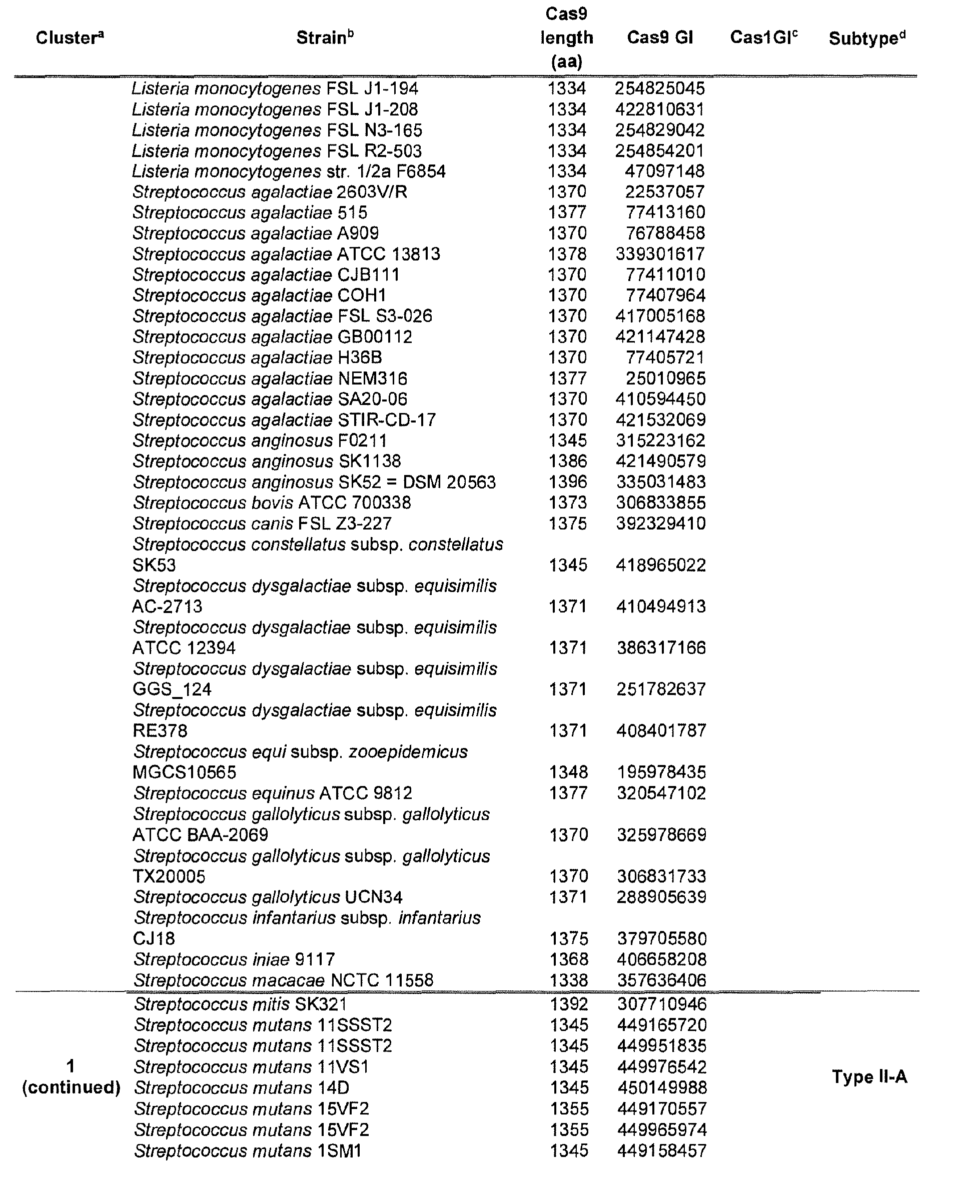

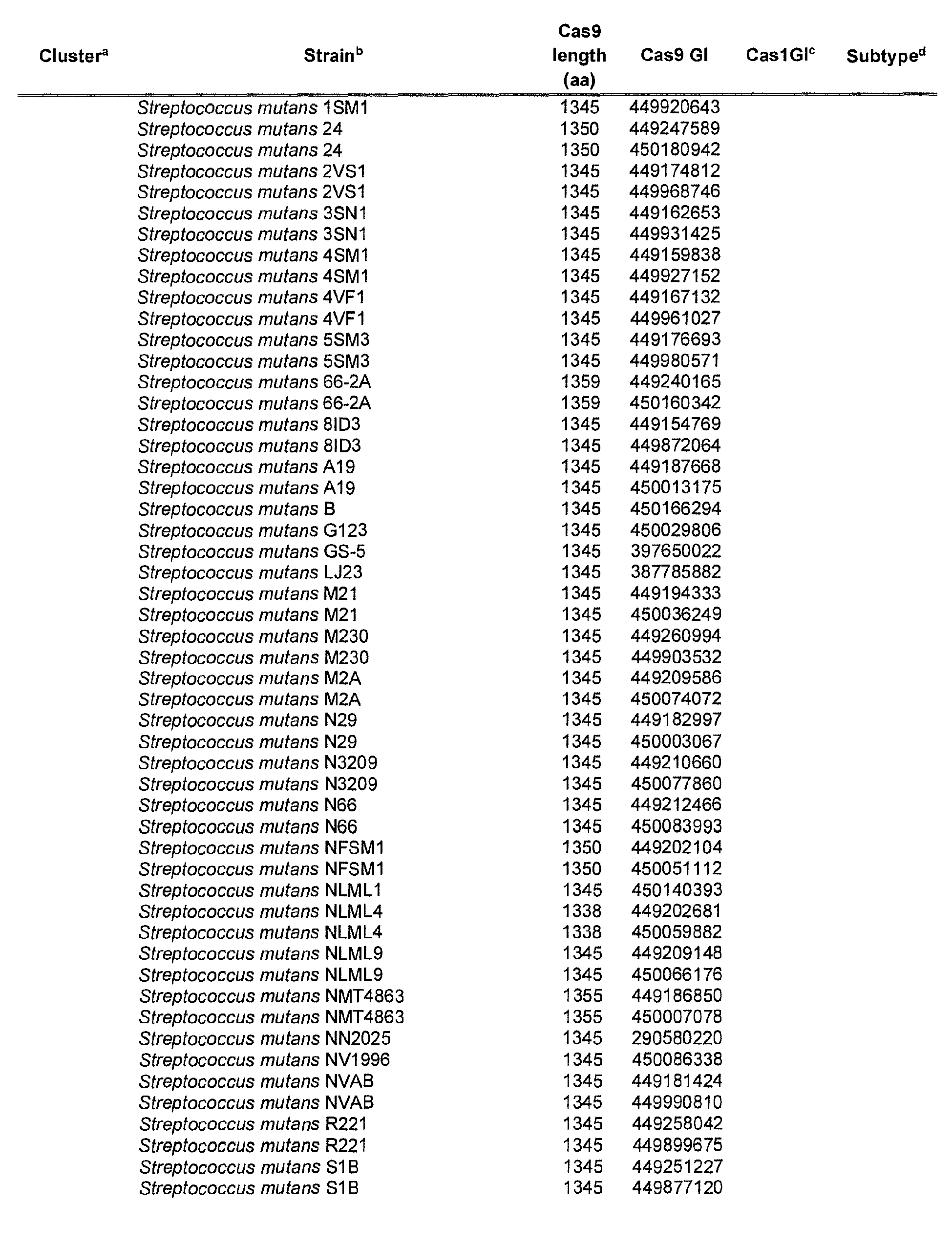

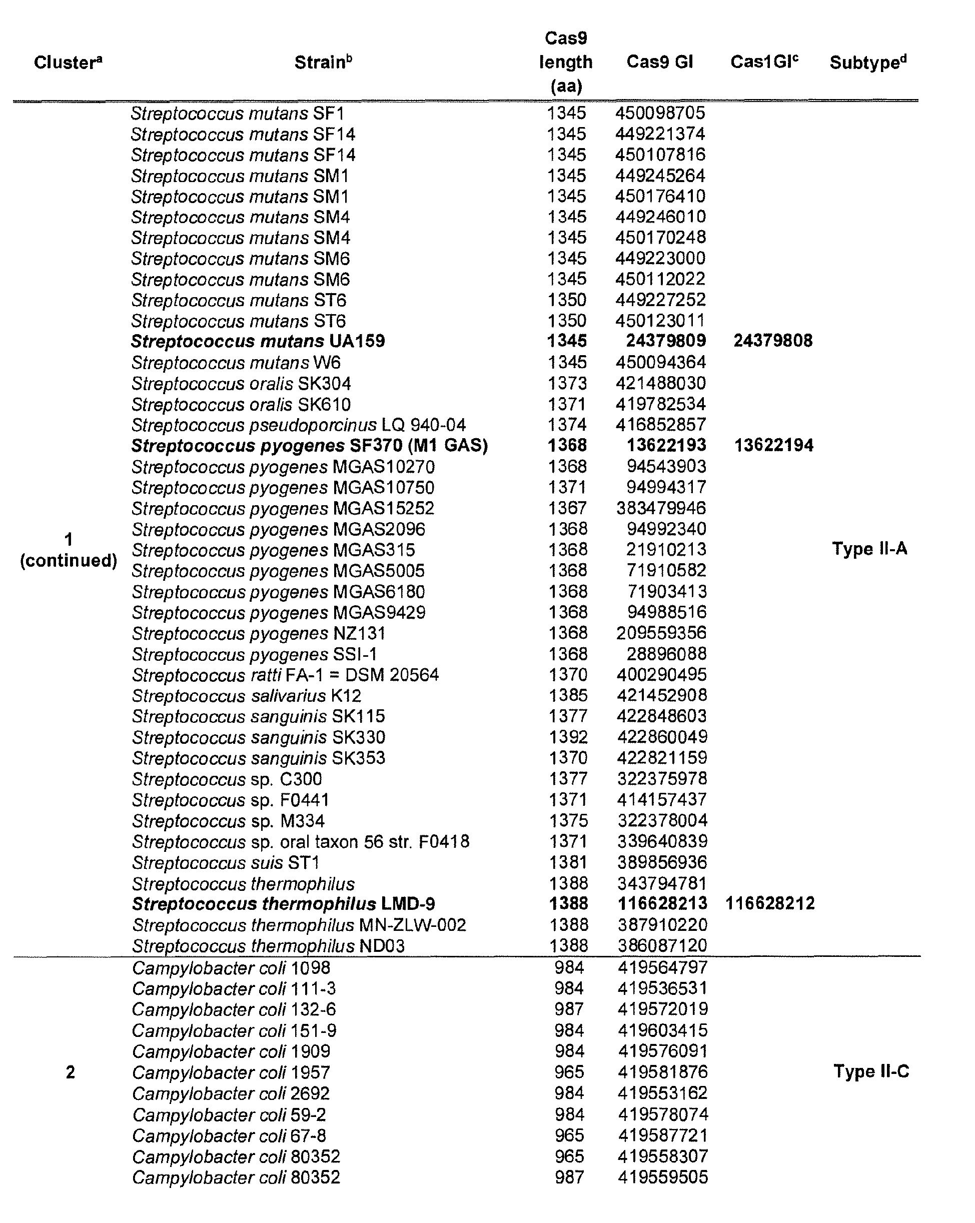

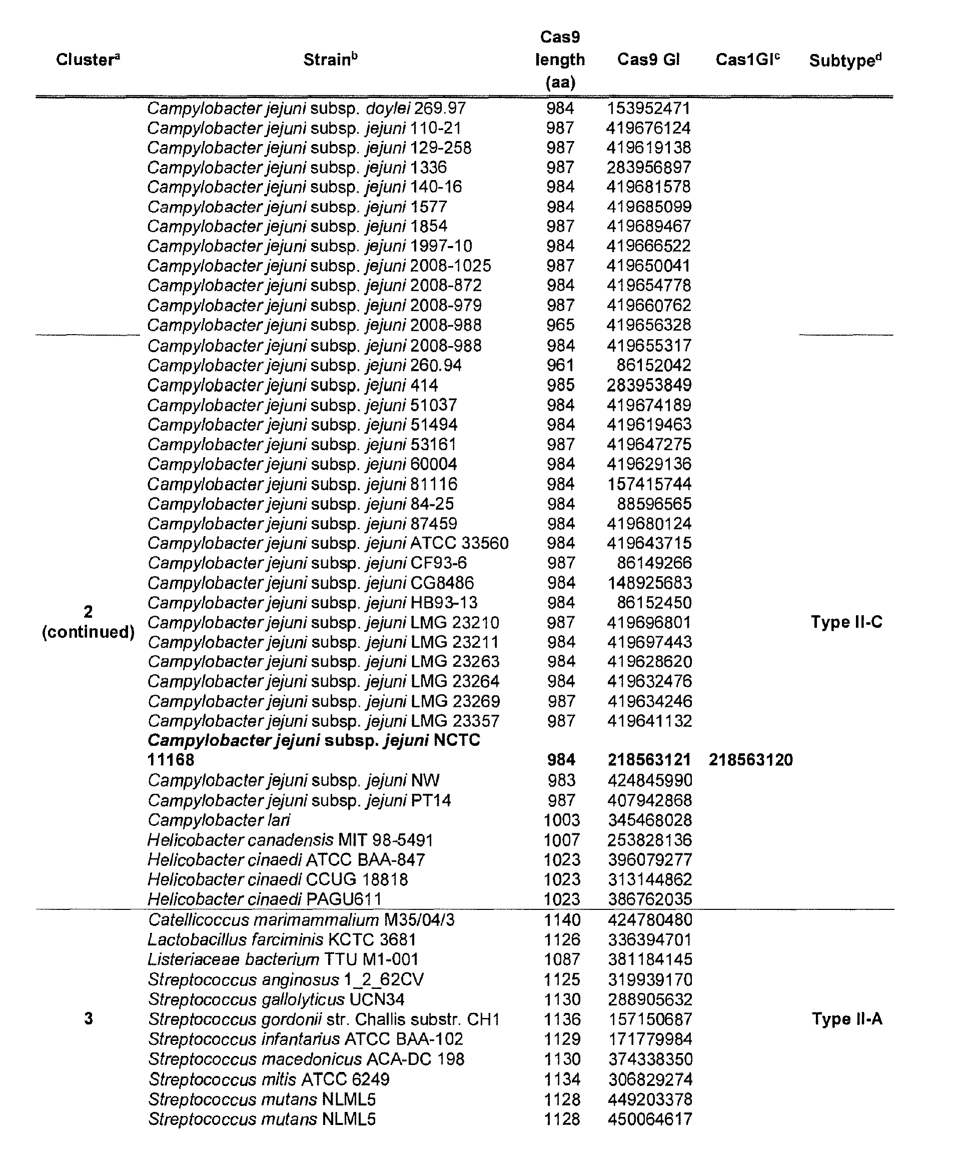

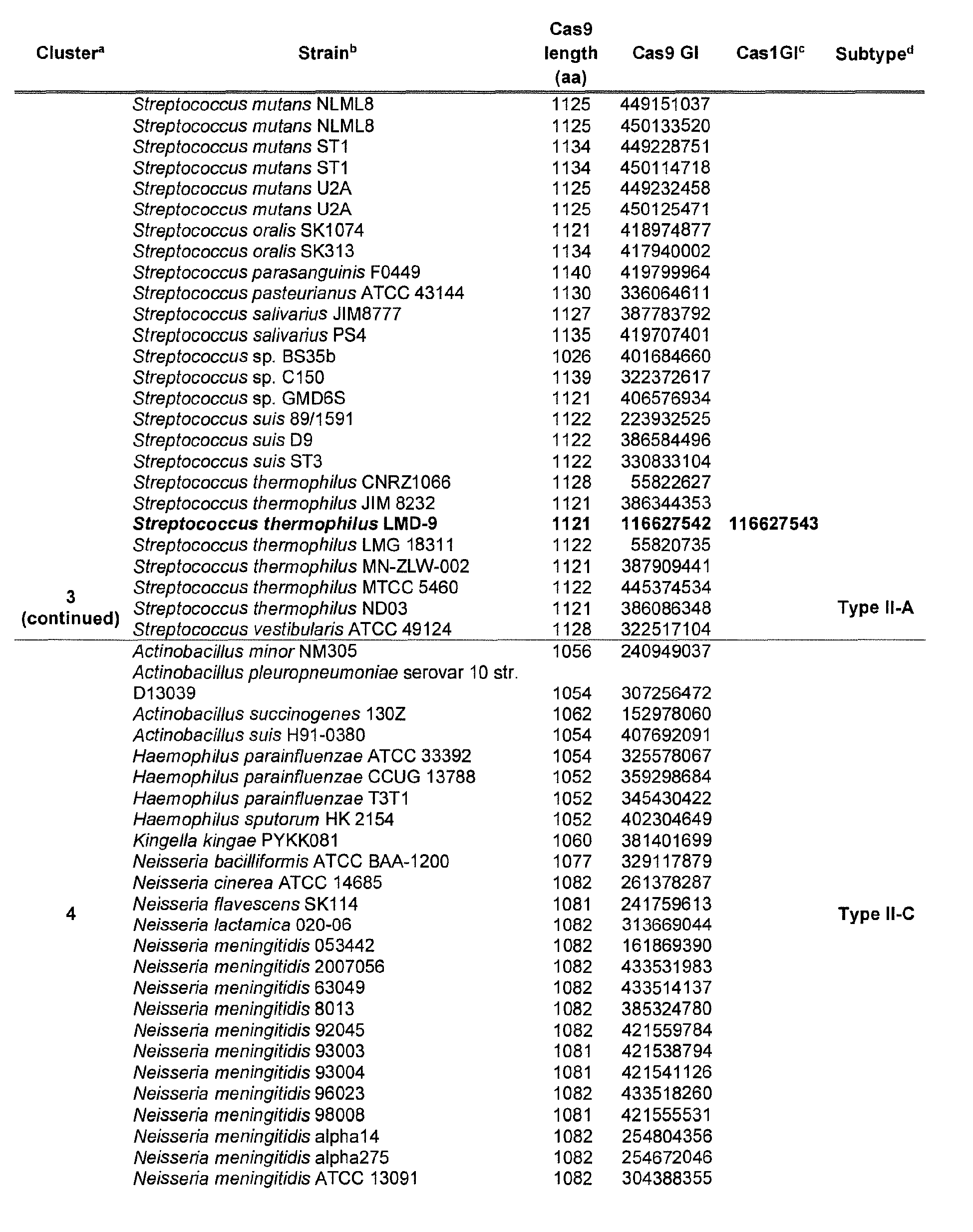

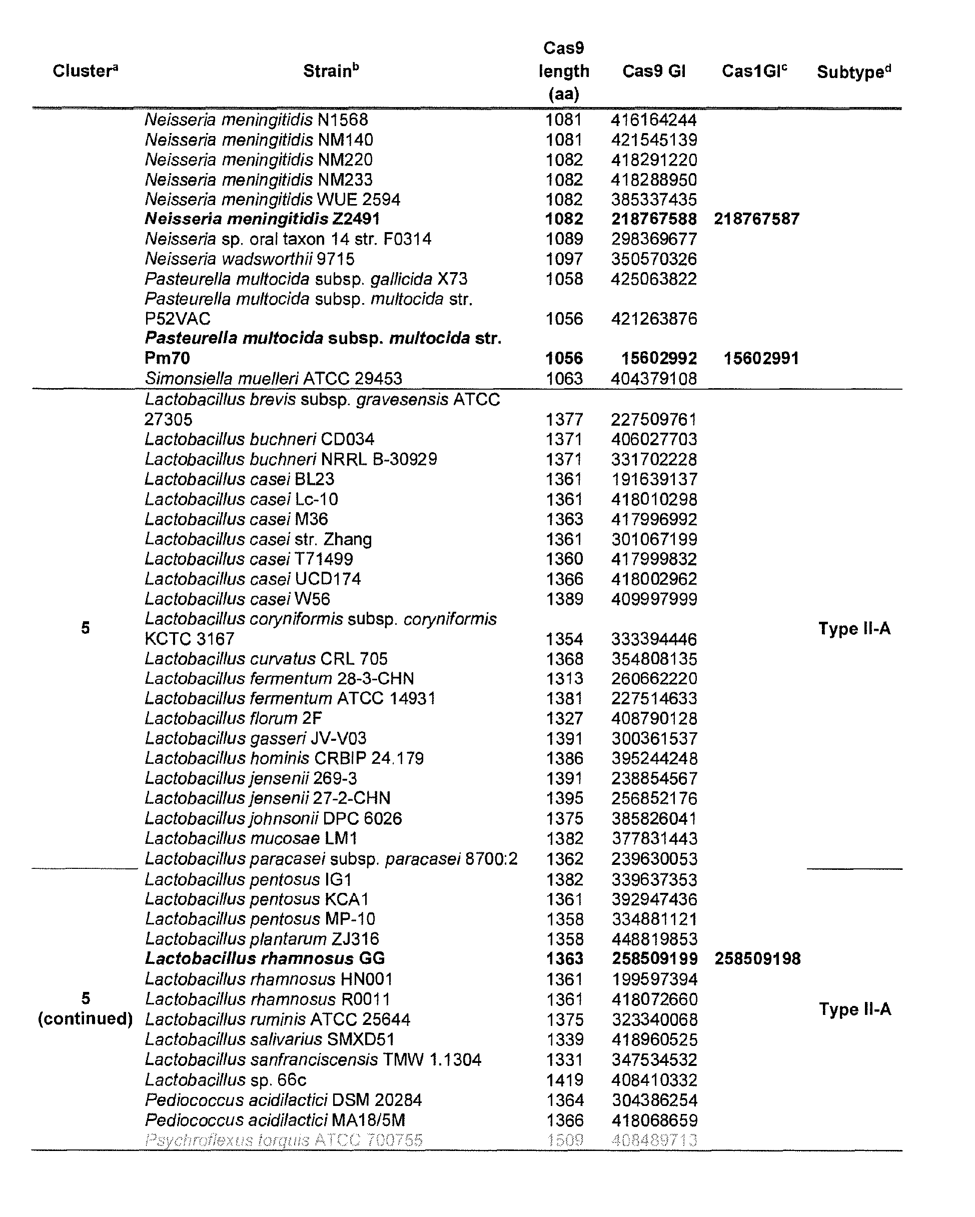

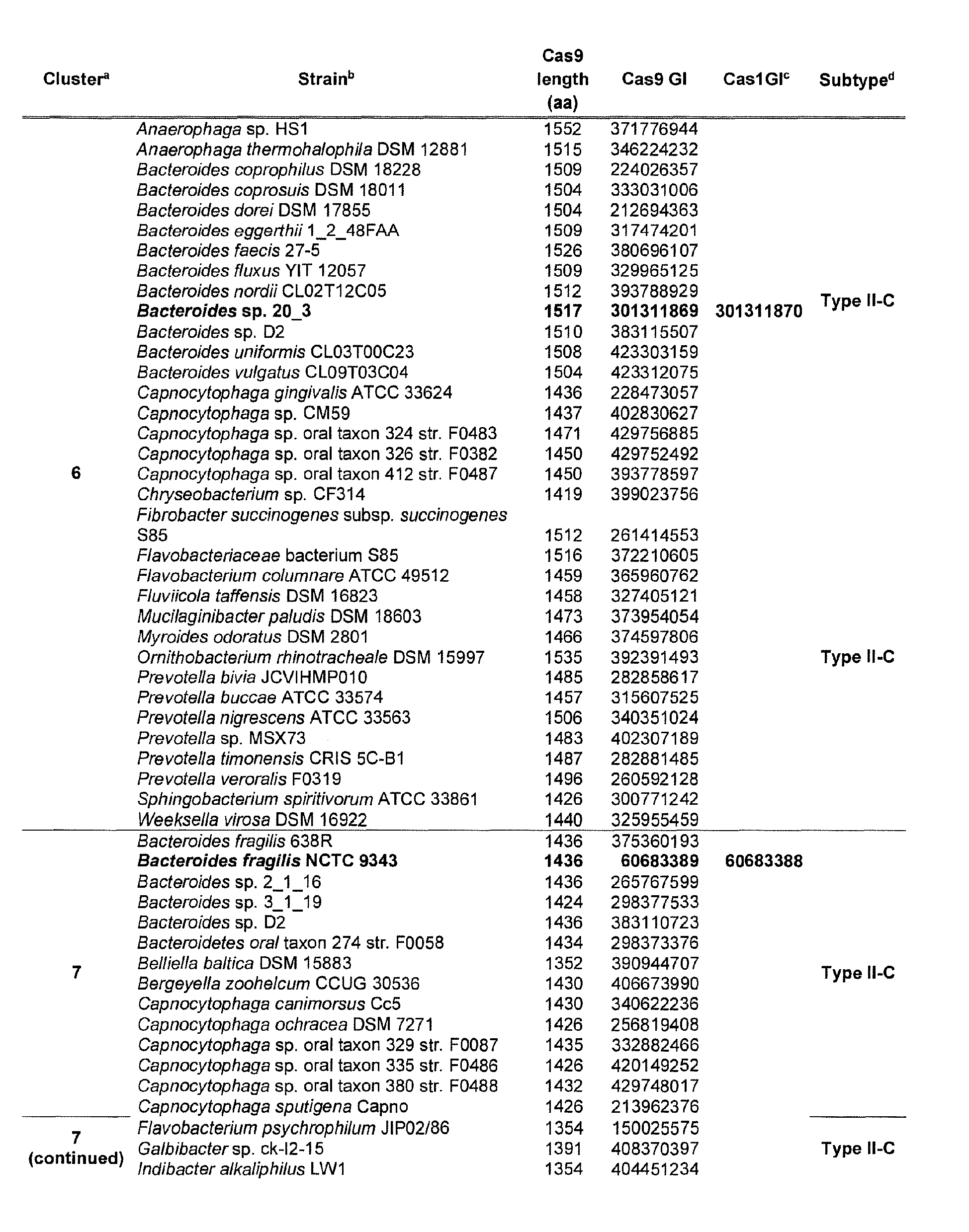

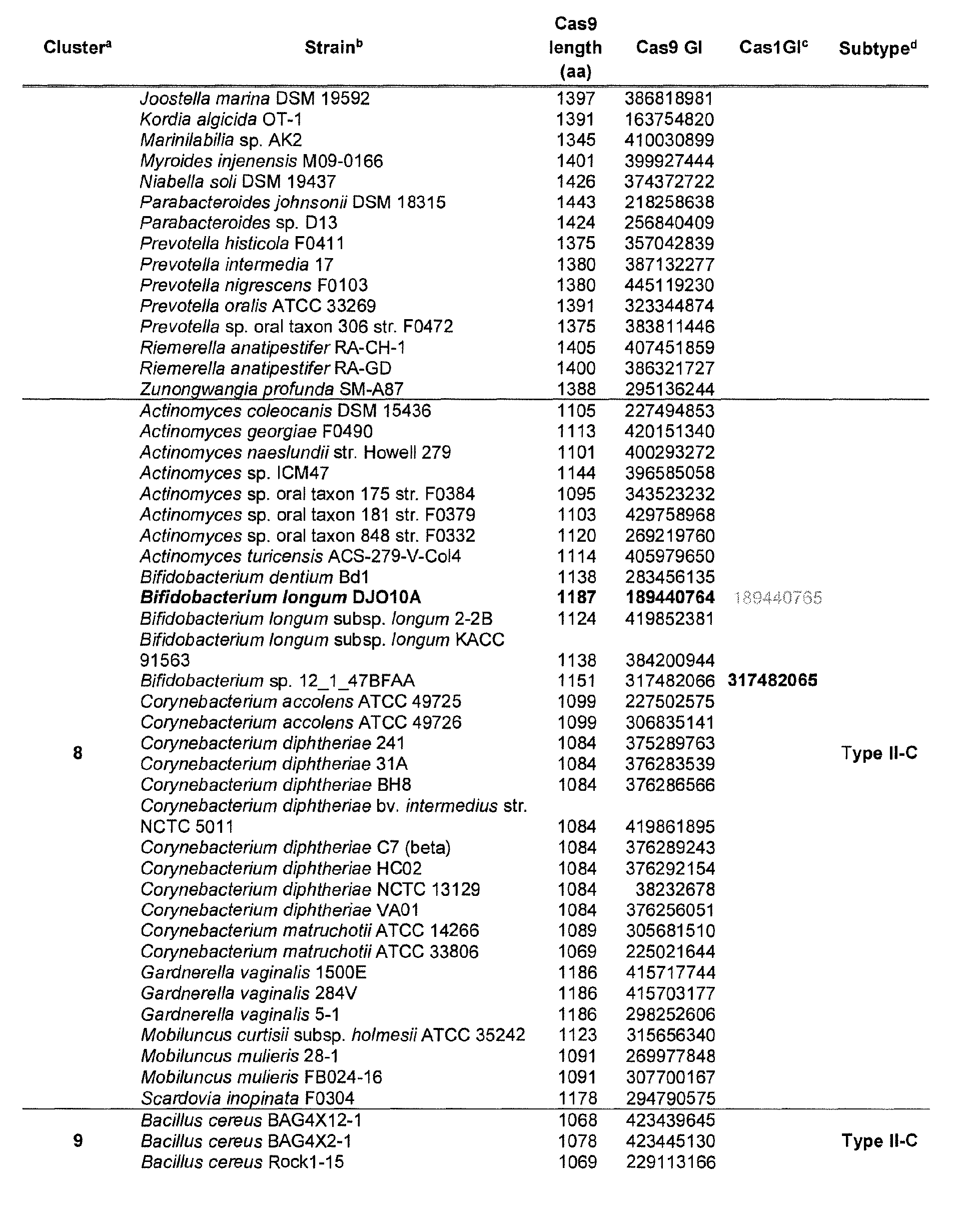

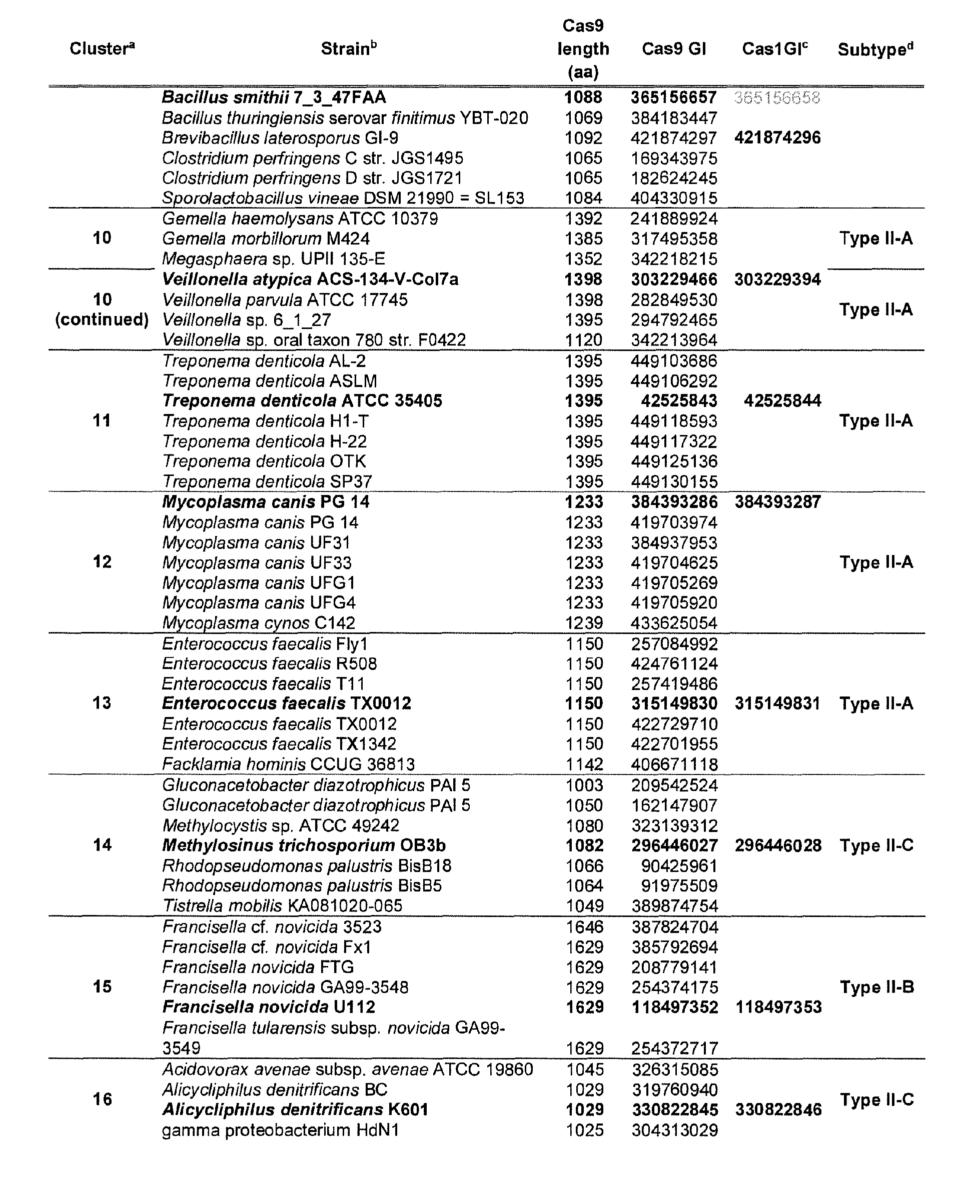

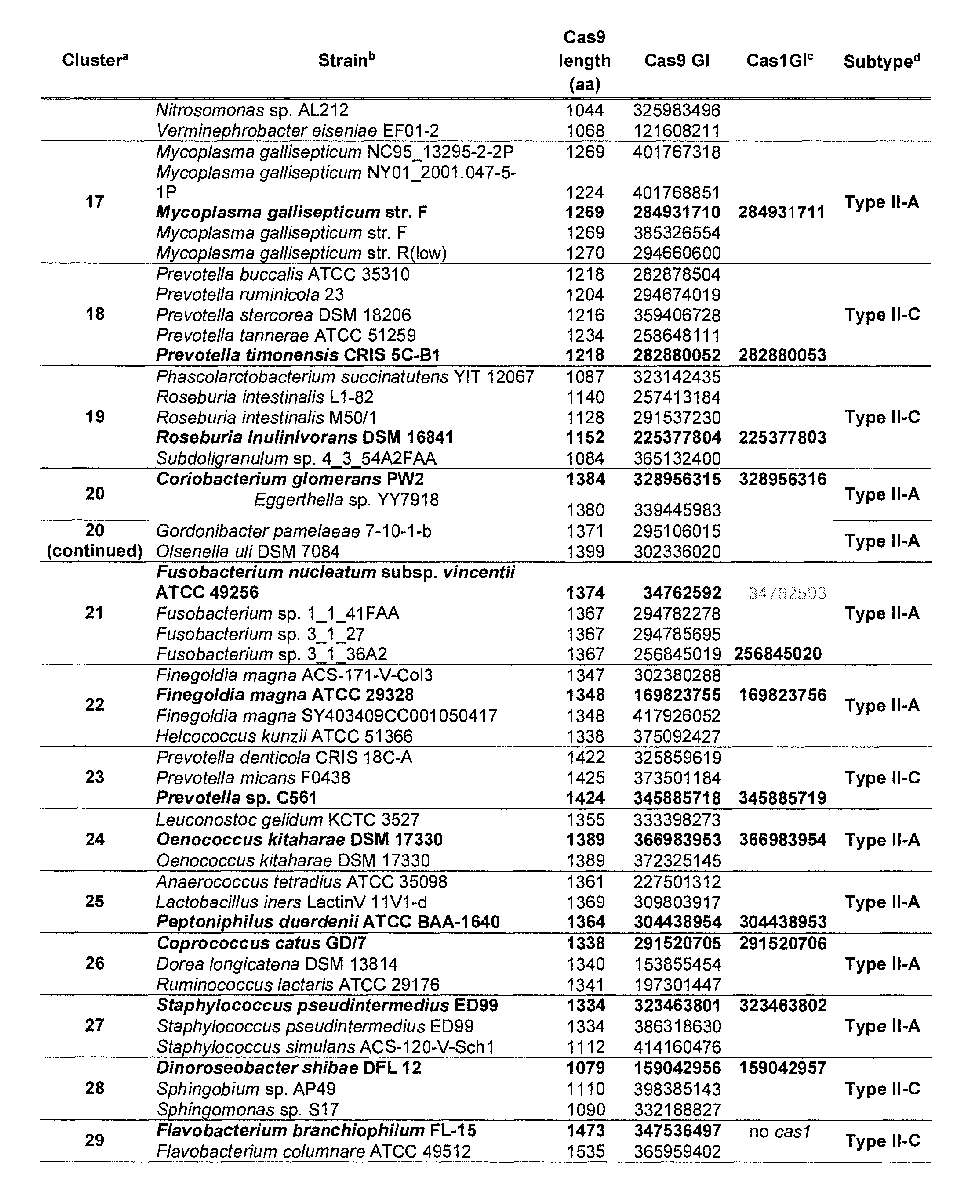

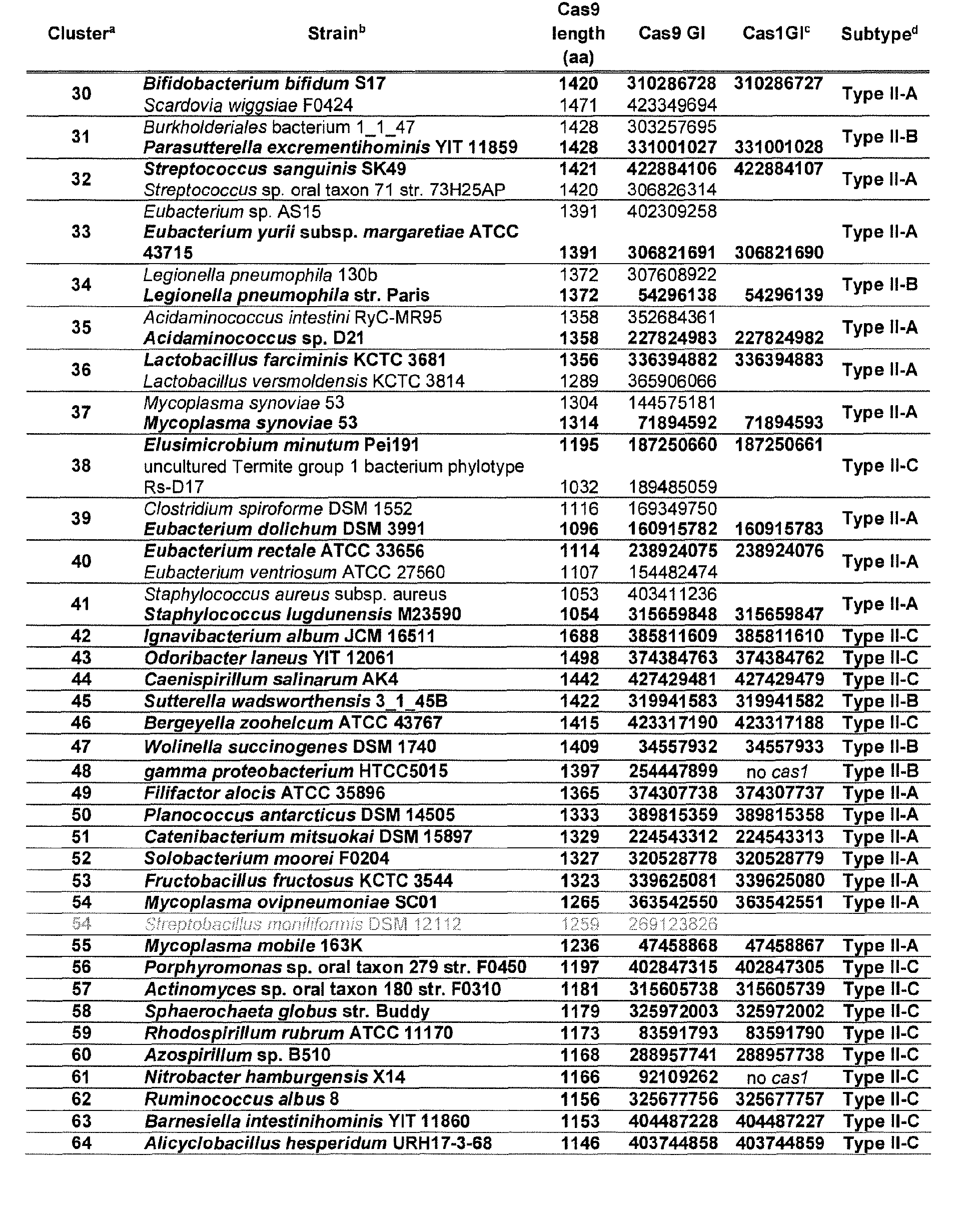

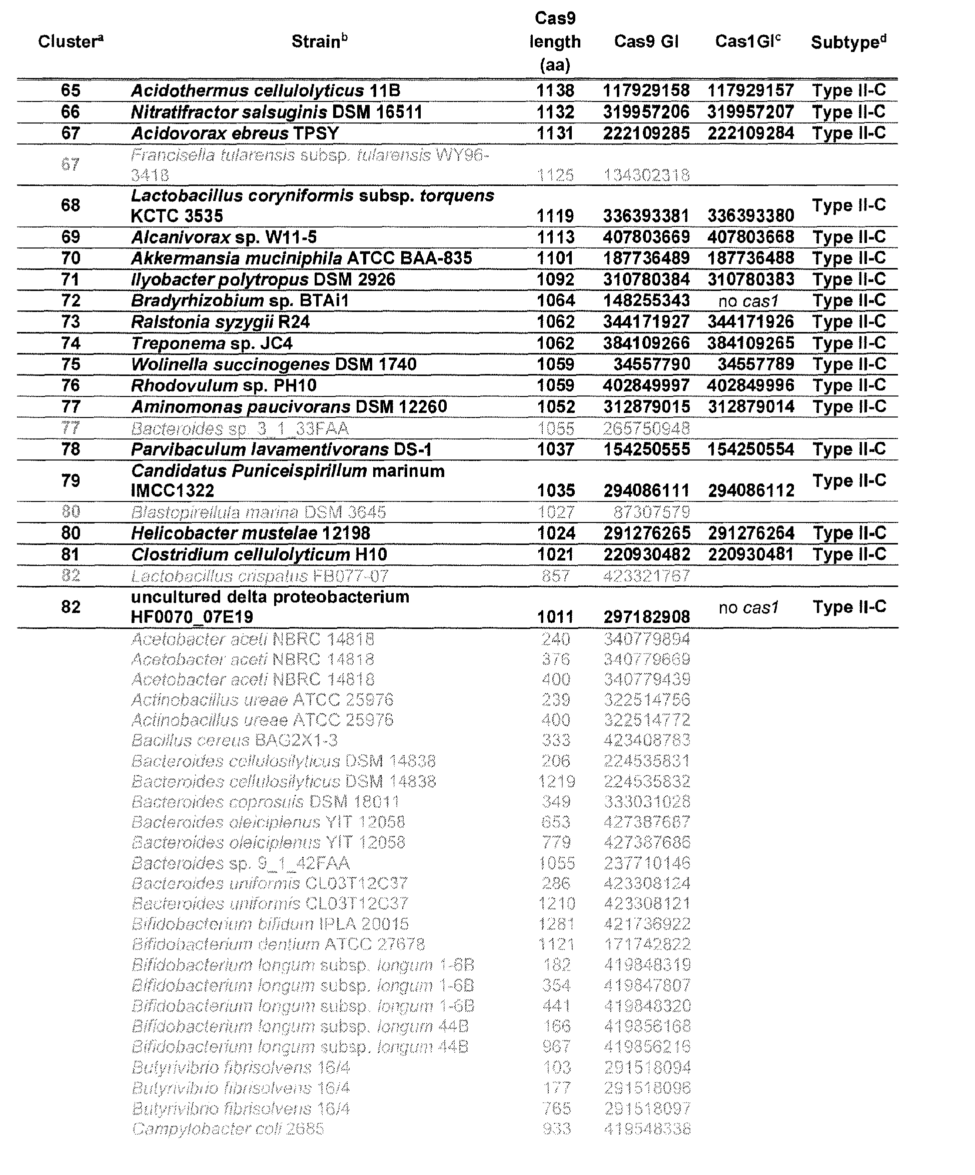

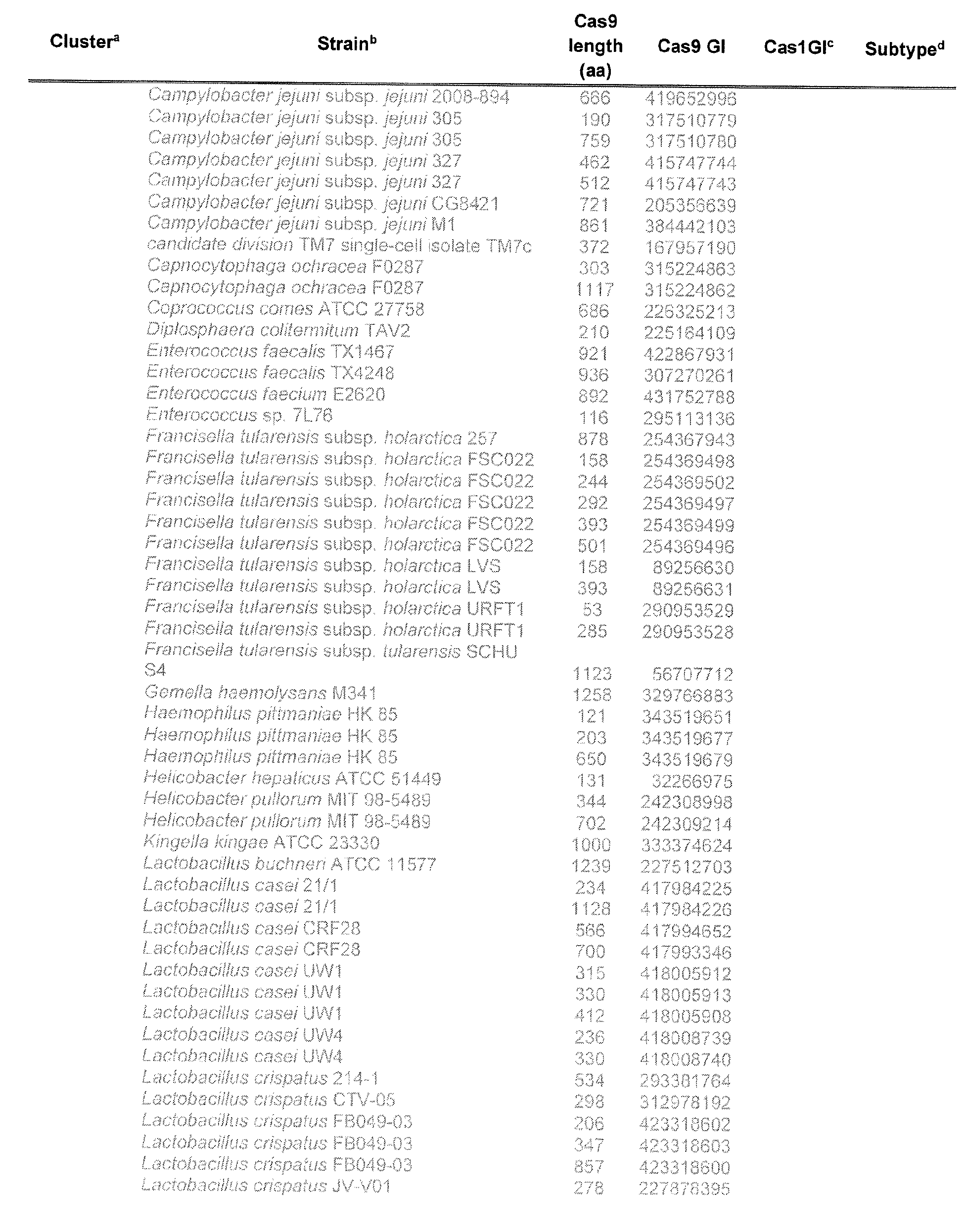

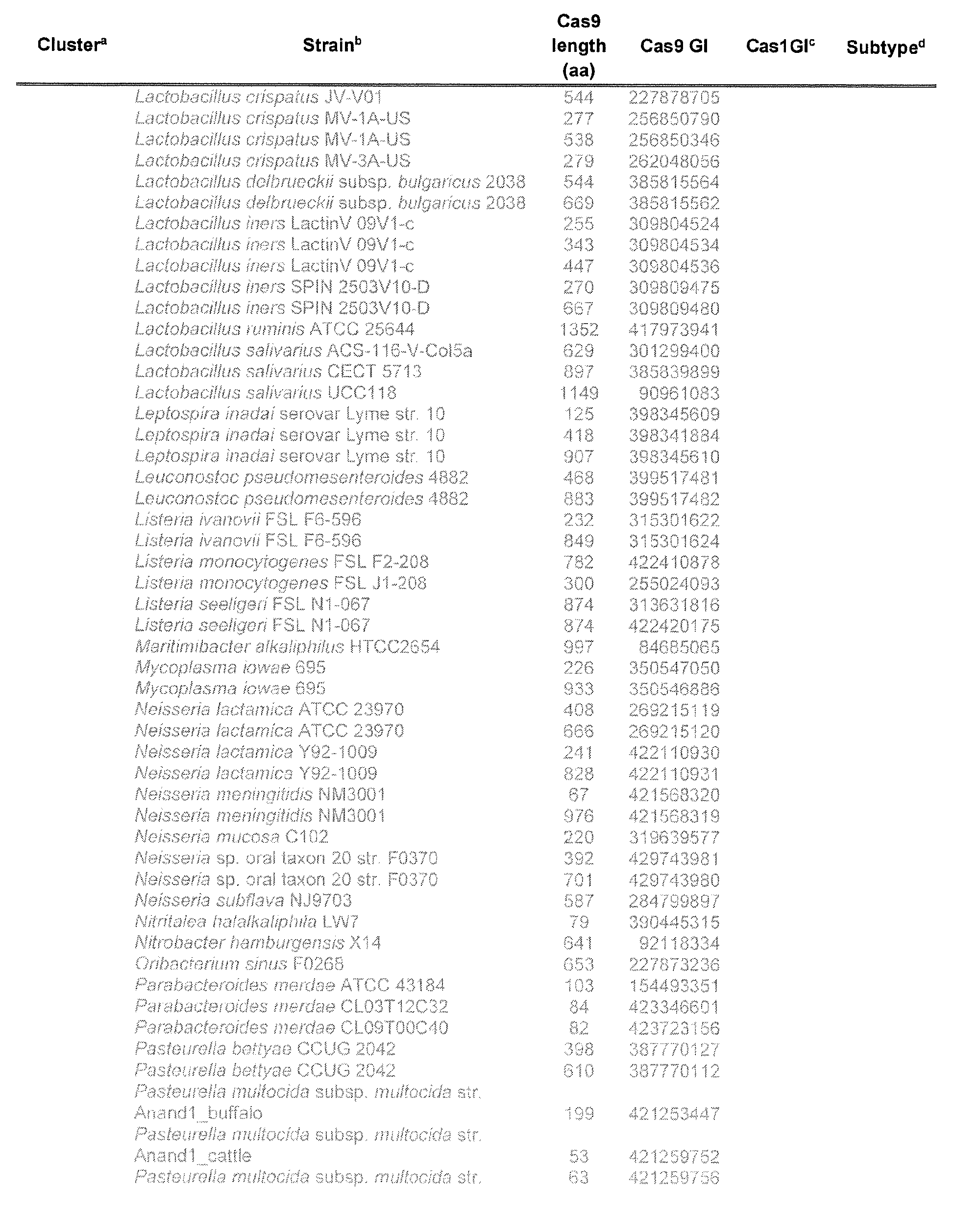

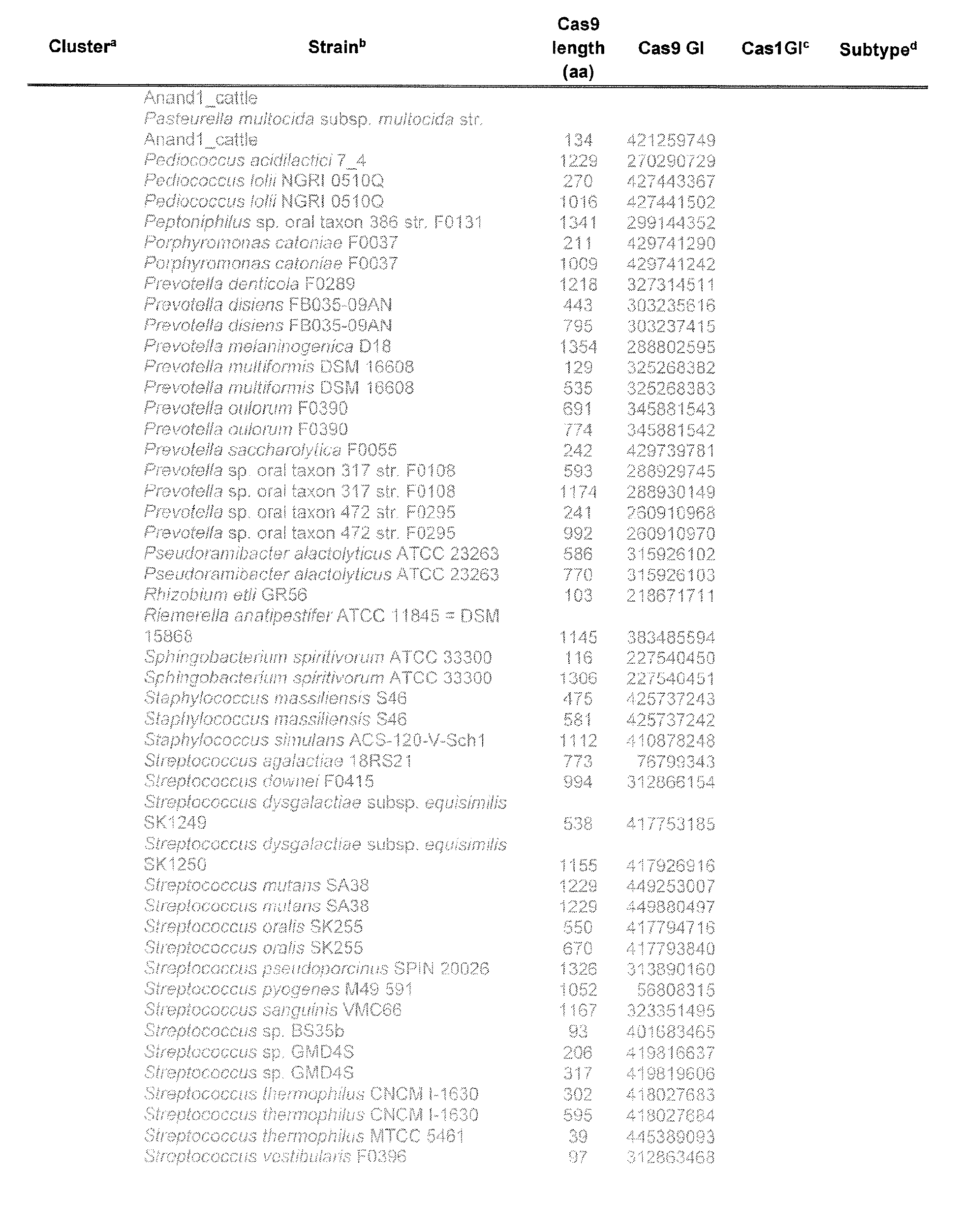

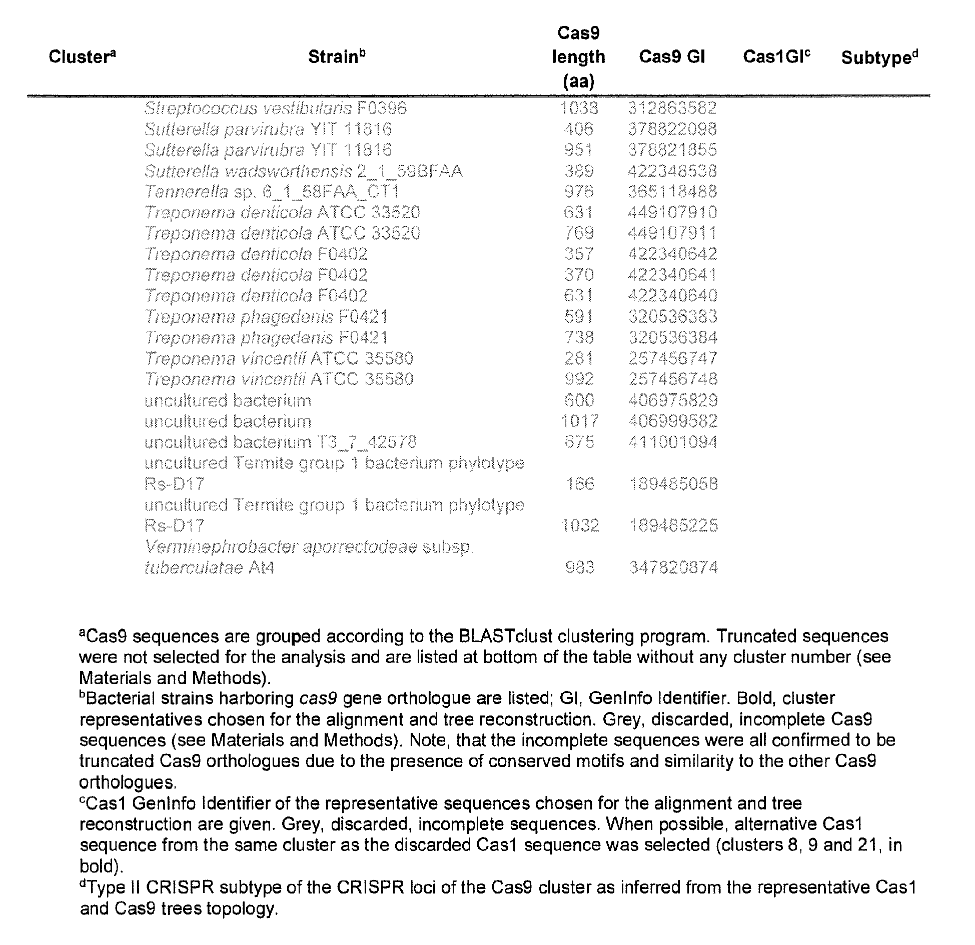

Analysis of the composition of cas genes, transcription direction of the CRISPR arrays with respect to that of the cas operon, and location and orientation of tracrRNAs resulted in the division of subtypes into groups with distinct locus characteristics, especially within the subtype II-A (Figure 1, clusters marked with different colors) (15). We selected Cas9 enzymes representative of the major type II groups. Cas9 orthologs of *S. pyogenes, S. thermophilus** (CRISPR3) and *S. mutans* were chosen for type II-A systems associated with shorter, ∼220 amino acid Csn2 variants (Csn2a). Cas9 of *S*. *thermophilus*** (CRISPR1) represents a distinct group of type II-A sequences associated with longer, ∼350 amino acid version of Csn2 orthologs (Csn2b). Cas9 of *F. novicida* was selected for type II-B. The closely related Cas9 orthologs of *P. multocida* and *N. meningitidis* and the distinct, short Cas9 of *C*. *jejuni* were chosen for type II-C (Figure 1B). Expression of associated tracrRNAs and crRNAs in *S*. *pyogenes, S. mutans, F. novicida, N. meningitidis* and *C. jejuni* was already validated by deep RNA sequencing (15,16). The RNAs in *S*. *thermophilus* and *P. multocida* were predicted bioinformatically based on the sequences from related species within the same type II group. Figure 1B shows the organization of the eight selected type II CRISPR-Cas loci and highlights our previous findings demonstrating that the type II loci architectures are highly variable among subtypes, yet conserved within each group (15). These variations are in good agreement with the clustering derived from the Cas9 and Cas1 phylogenetic trees (Figure 1A, Supplementary Figure S4).

Thus, to evaluate dual-RNA:Cas9 diversity, the bioinformatics analysis of type II CRISPR-Cas systems from available genomes identified Cas9 orthologs in a plethora of bacterial species that belong to 12 phyla and were isolated from diverse environments (Supplementary Tables S2 and S4). Most of the strains that harbor type II CRISPR-Cas systems (and accordingly Cas9) are pathogens and commensals of vertebrates. A majority of these strains were isolated from gastrointestinal tracts and feces of mammals, fish and birds, but also from wounds, abscesses and spinocereberal fluid of septicaemia patients. Strains were also isolated from invertebrates and environmental samples, including fresh and sea water, plant material, soil and food, the latter comprising species used in fermentation processes. Cas9 is also present in species from extreme environments such as deep sea sediments, hot springs and Antarctic ice, further demonstrating the wide spread of type II CRISPR-Cas systems in bacteria. A comparison of the taxonomy and habitats of representative strains with the phylogenetic clustering of Cas9 sequences shows little correlation (Supplementary Figure S11). In particular, clusters of Cas9 genes were identified from taxonomically distant bacteria that were isolated from similar habitats. Examples include diverse Firmicutes, Molicutes, Spirochaete and Fusobacteria, that were all isolated from gastrointestinal tracts of mammals, and members of different Proteobacteria, Firmicutes and Fusobacteria families mostly found in environmental samples (Supplementary Figure S11, clusters 1 and 3). A few exceptions involve grouping of Cas9 genes from closely related species isolated from diverse habitats such as Actinobacteria isolated from human and dog specimens but also from hot springs (Supplementary Figure S11, clusters 2, 4 and 5). This complex distribution of Cas9 across bacterial genomes indicates that evolution of dual-RNA:Cas9 systems in bacteria occurs both vertically and horizontally (55).

### Example 2

Bacterial RNases III are interchangeable in dual-RNA maturation.

As described in *S. pyogenes* and *S. thermophilus,* RNase III plays an essential role in the biogenesis of dual-RNA:Cas9 systems by co-processing tracrRNA and pre-crRNA at the level of antirepeat:repeat duplexes (16,17). The interchangeability of *S. pyogenes* RNase III with RNases III from selected bacterial species was analyzed in the co-processing of *S. pyogenes* tracrRNA:pre-crRNA, including strains that lack type II CRISPR-Cas (*S*. *aureus* COL, *E*. *coli* TOP10). Northern blot analysis showed that all RNases III studied can co-process the RNA duplex (Figure 2, Supplementary Figure S5), indicating that there is no species-specificity for tracrRNA:pre-crRNA cleavage by RNase III. Multiple sequence alignment of RNase III orthologs demonstrates conservation of the catalytic aspartate residue and the dsRNA binding domain (Figure 2, Supplementary Figure S6) that are both required for RNA co-processing (Figure 2, Supplementary Figure S5). These data imply that the conservation of tracrRNA:pre-crRNA co-processing by bacterial RNase III provides a degree of flexibility allowing the functionality of dual-RNA:Cas9 systems in multiple species upon horizontal transfer.

Thus, to investigate the basis for the horizontal dissemination of CRISPR-Cas modules among bacteria, the specificity of RNase III utilized by type II CRISPR-Cas for dual-RNA maturation was analyzed. Complementation analysis shows that RNase III from a variety of species, including bacteria that lack type II CRISPR-Cas, can process *S. pyogenes* tracrRNA:pre-crRNA, suggesting that type II CRISPR-Cas systems can exploit any double-stranded RNA cleavage activity. This finding is consistent with the observation of *S. pyogenes* dual-RNA maturation in human cells which is apparently mediated by host RNases (2).

### Example 3

Cas9 HNH and split RuvC domains are the catalytic moieties for DNA interference.

Comparison of Cas9 sequences revealed high diversity in amino acid composition and length (984 amino acid for *C*. *jejuni* to 1648 amino acids for *F. novicida*), especially in the linker sequence between the highly conserved N-terminal RuvC and central RuvC-HNH-RuvC regions and in the C-terminal extension (Supplementary Figure S2). Several studies demonstrated the importance of the nuclease motifs for dsDNA cleavage activity by mutating one aspartate in the N-terminal motif of the RuvC domain and one or several residues in the predicted catalytic motif of the HNH domain of the Cas9 enzyme (14,22,23). To investigate the relevance of all catalytic motifs for tracrRNA:pre-crRNA processing and/or DNA interference, alanine substitutions of selected residues were created (Figure 3A). In addition to the already published catalytic amino acids, we created Cas9 point mutants of conserved amino acid residues in the central RuvC motifs (14) (Figure 3A, Supplementary Figure S2). Northern blot analysis of *S*. *pyogenes cas9* deletion mutant complemented with each of the cas9 point mutants revealed the presence of mature tracrRNA and crRNA forms, demonstrating that none of the catalytic motifs is involved in dual-RNA maturation by RNase III. This is in agreement with previous data showing that RNase III is the enzyme that specifically cleaves tracrRNA:pre-crRNA duplex (16). Cas9 seems to have a stabilizing function on dual-RNA. We show that the catalytic motifs are not involved in RNA duplex stabilization (Figure 3B, Supplementary Figure S7).

To investigate the involvement of the conserved motifs of Cas9 in DNA interference *in vivo,* a previously described plasmid-based read-out system was used that mimics infection with invading protospacer-containing DNA elements (16). Transformation assays were done in *S. pyogenes* WT or a cas9 deletion mutant using plasmids containing the *speM* protospacer gene (complementary to the second spacer of *S. pyogenes* SF370 type II CRISPR array (16)) and WT or mutant cas9 (Figure 3C). In this assay, Cas9 expressed following plasmid delivery in bacterial cells catalyzes its own vector cleavage, when active. Control experiments showed that the *speM* protospacer-containing plasmid was not tolerated in WT *S. pyogenes,* demonstrating activity of WT CRISPR-Cas. Similarly, a plasmid containing the *speM* protospacer and encoding WT Cas9 could not be maintained in the cas9 deletion mutant, demonstrating that Cas9 is able to cleave the plasmid from which it is expressed. Except for Cas9 N854A, all plasmids encoding Cas9 mutants were tolerated in the cas9 deletion strain, indicating abrogation of Cas9 interference activity for these variants.

The *in vivo* DNA targeting data were confirmed with *in vitro* DNA cleavage assays. Purified WT and mutant Cas9 proteins were incubated with tracrRNA:crRNA targeting *speM* and subjected to cleavage of plasmid DNA containing the *speM* protospacer. WT and N854A Cas9 show dsDNA cleavage activity, whereas the other Cas9 mutants cleave only one strand of the dsDNA substrate, yielding nicked open circular plasmid DNA (Figure 3D). This corroborates the results obtained *in vivo* showing the importance of the conserved nuclease motifs for DNA interference by Cas9. In addition to the previously published data demonstrating the importance of the N-terminal RuvC motif and the catalytic motif of HNH, we thus defined new catalytic residues in the central RuvC motifs.

Dual-RNA and Cas9 sequences have widely evolved in bacteria (15). However, despite the high sequence variability among Cas9 sequences, certain motifs are conserved. In addition to the previously identified central HNH and N-terminal RuvC catalytic motifs (20,21,44,56), we show that the two middle RuvC motifs are required for interference activity *in vivo* and *in vitro.* In agreement with previous findings, deactivation of either one of the catalytic motifs (RuvC or HNH) results in nicking activity of Cas9 originating from the other motif (2,8,24,25). None of the mutations introduced in these conserved motifs affected the role of Cas9 in tracrRNA:pre-crRNA maturation by RNase III *in vivo.*

### Example 4

Only Cas9 from closely related CRISPR-Cas systems can substitute for *S. pyogenes* Cas9 in tracrRNA-directed pre-crRNA maturation by RNase III.

Beside the conservation of the HNH and split RuvC domains involved in DNA cleavage (14,15), the length of Cas9 orthologs and the amino acid sequences of Cas9 are highly variable among the different groups of type II CRISPR-Cas systems (Figure 4A, Supplementary Figure S2). Hence, whether this variability plays a role in the specificity of Cas9 with regard to tracrRNA:pre-crRNA duplex and mature crRNA stabilization was investigated. A *S*. *pyogenes cas9* deletion mutant was complemented with Cas9 from selected bacterial species representative of the various type II groups and analyzed tracrRNA:pre-crRNA processing by Northern blot. Cas9 proteins from *S. mutans* and *S. thermophilus** can substitute for the stabilizing role of *S. pyogenes* Cas9 in RNA processing by RNase III (Figure 4B, Supplementary Figure S8). By contrast, Cas9 from *S. thermophilus***, *C. jejuni, N. meningitidis, P. multocida* and *F. novicida* could not complement the lack of RNA processing in the *cas9* mutant of *S. pyogenes.* In these strains, the 75-nt processed form of tracrRNA is observed as a very weak signal of background level of dual-RNA processed by RNase III in the absence of Cas9. Overall, only Cas9 from closely related systems of *S. pyogenes* in the type II-A cluster can substitute endogenous Cas9 role in dual-RNA stabilization and subsequent maturation by RNase III.

Thus, substitution of orthologs from the selected species for the endogenous *S. pyogenes* Cas9 shows that only Cas9 proteins from the *S. pyogenes* subcluster are capable of assisting tracrRNA:pre-crRNA processing by RNase III. This result indicates that the less-conserved inter-motif regions, which are the basis for the Cas9 subgrouping, could be responsible for Cas9 specificity for certain dual-RNAs.

### Example 5

Cas9 orthologs require their specific PAM sequence for DNA cleavage activity.

In *S. pyogenes* and *S. thermophilus** types II-A, PAMs were identified as NGG and NGGNG, respectively. In these two species, mutating the PAM abrogates DNA interference by dual-RNA:Cas9 (14,22,23). To identify the functional PAMs for Cas9 from bacterial species other than *S. pyogenes* and S. *thermophilus,* potential protospacers matching spacer sequences in the selected CRISPR arrays were searched using BLAST. For *S. mutans* UA159, *C. jejuni* NCTC 11168, *P. multocida* Pm70 and *F. novicida* U112, potential protospacers were identified. Therefore, strains that harbor a closely related variant of Cas9 (Supplementary Table S2) were searched and their spacer sequences analyzed following the same approach (Supplementary Table S3). The identified 10 nt sequences located directly downstream of the protospacer sequence were aligned and the most common nucleotides that could represent PAM sequences were delineated. Based on the data visualized as a logo plot (Figure 5A), plasmid DNA substrates were designed containing the *speM* protospacer followed by different adjacent sequences either comprising the predicted PAM or not (Figure 5B). The Cas9 orthologous proteins were purified (Supplementary Figure S1) and dual-RNA orthologs were designed based on deep RNA sequencing data (15), with the spacer sequence of crRNA targeting *speM.* To determine the protospacer-adjacent sequences critical for efficient DNA targeting, the purified Cas9 orthologs and their cognate dual-RNAs were used in DNA cleavage assays with different plasmid substrates (Figure 5C, Supplementary Figure S9). The previously published PAMs for Cas9 from *S. pyogenes* (NGG), *S. mutans* (NGG), *S. thermophilus** (NGGNG) and *N. meningitidis* (NNNNGATT) (27,28,53,54) were confirmed by multiple sequence alignments and *in vitro* cleavage assay, validating our approach. However, dual-RNA guided Cas9 from *S. thermophilus** could efficiently cleave target DNA in the presence of only NGG instead of NGGNG (Supplementary Figure S9). This is in contrast to data obtained *in vivo,* where mutation of the third G abrogates interference by Cas9 of *S. thermophilus** (23). For *S*. *thermophilus***, the PAM was published as NNAGAAW (27), which differs by one base from the sequence that we derived (NNAAAAW). *In vitro* cleavage assays with these two sequences demonstrate that the DNA substrate with the "NNAAAAW" PAM is cleaved more efficiently by Cas9 of *S*. *thermophilus*** compared to the "NNAGAAW" PAM (Supplementary Figure S9).

Using the same approach, the PAM activity of the most common protospacer-downstream sequences for *C. jejuni, F. novicida* and *P*. *multocida* were validated by *in vitro* cleavage assays, resulting in the most probable PAM sequences being NNNNACA (*C*. *jejuni*), GNNNCNNA (*P*. *multocida*) and NG (*F. novicida*) (Figure 5C, Supplementary Figure S9). Analysis of the protospacer-adjacent sequence from *C. jejuni* shows the same frequency of C and A ("NNNNCCA" or "NNNNACA") at position 5 downstream of the protospacer (Supplementary Table S3). Hence, both substrates were tested for cleavage activity by *C. jejuni* dual-RNA:Cas9. Only the DNA target containing A at this position was cleaved efficiently (Supplementary Figure S9). This result could be explained by the origin of the protospacer, with the "NNNNCCA" PAM being mostly found in genomic DNA or prophages of *Campylobacter* strains. In this case, the mutated PAM sequence on the chromosomally located protospacer prevents self-targeting. The *P. multocida* PAM requires further verification given that the multiple sequence alignment was derived from only two protospacer sequences. Thus, a series of specific PAMs that enable dsDNA cleavage by dual-RNA:Cas9 complexes from different bacterial species *in vitro* were identified. For gene editing purposes, it is contemplated that a range of potential motifs be analyzed to select those PAMs that would allow efficient targeting with limited off-site effect.

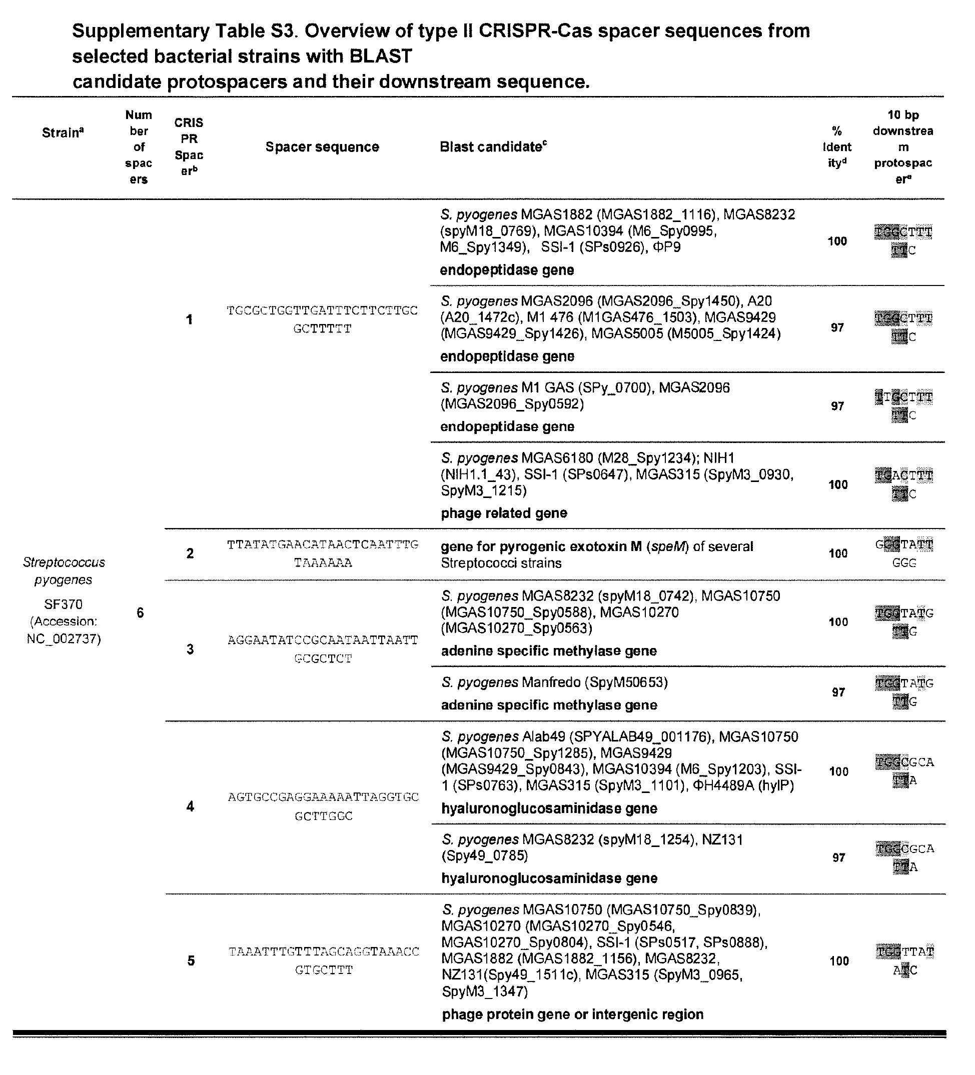

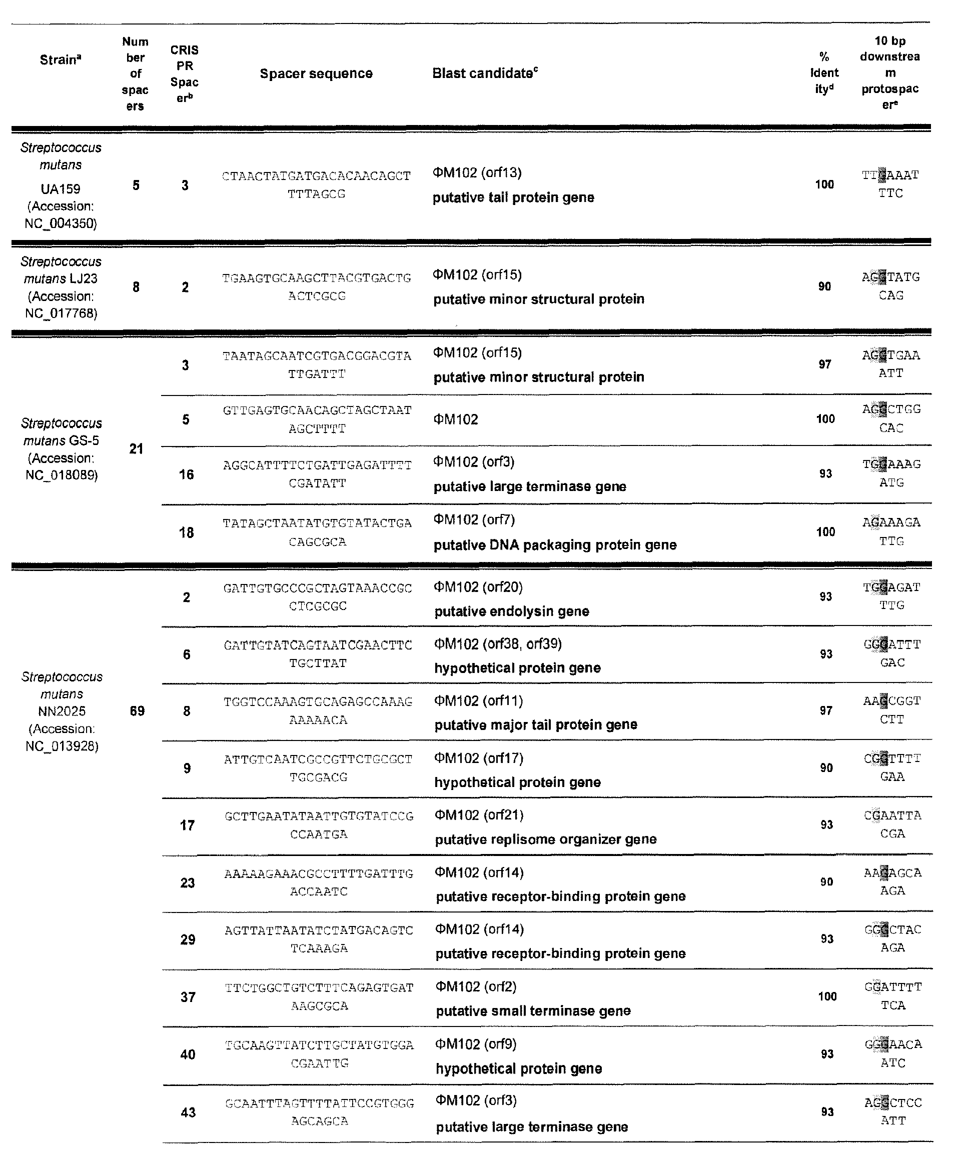

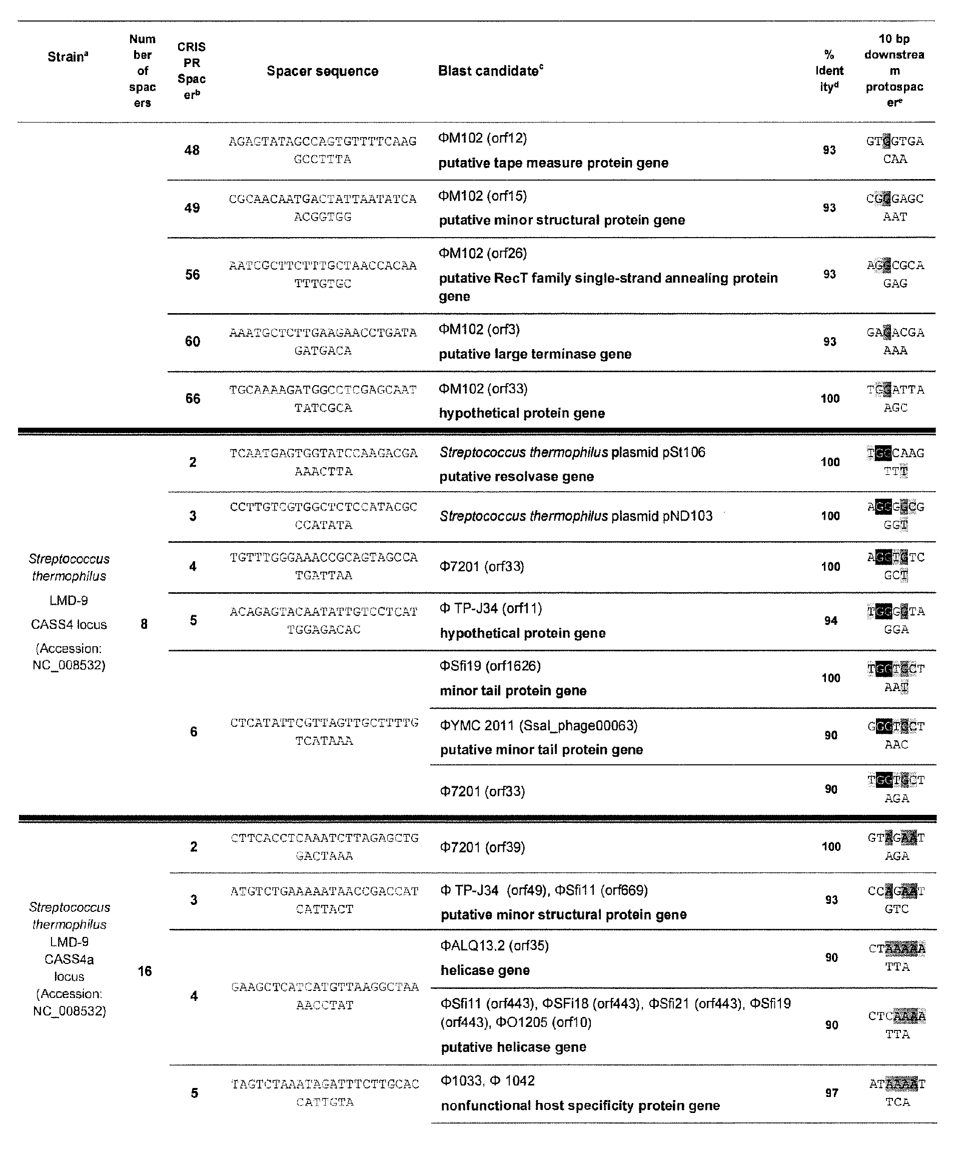

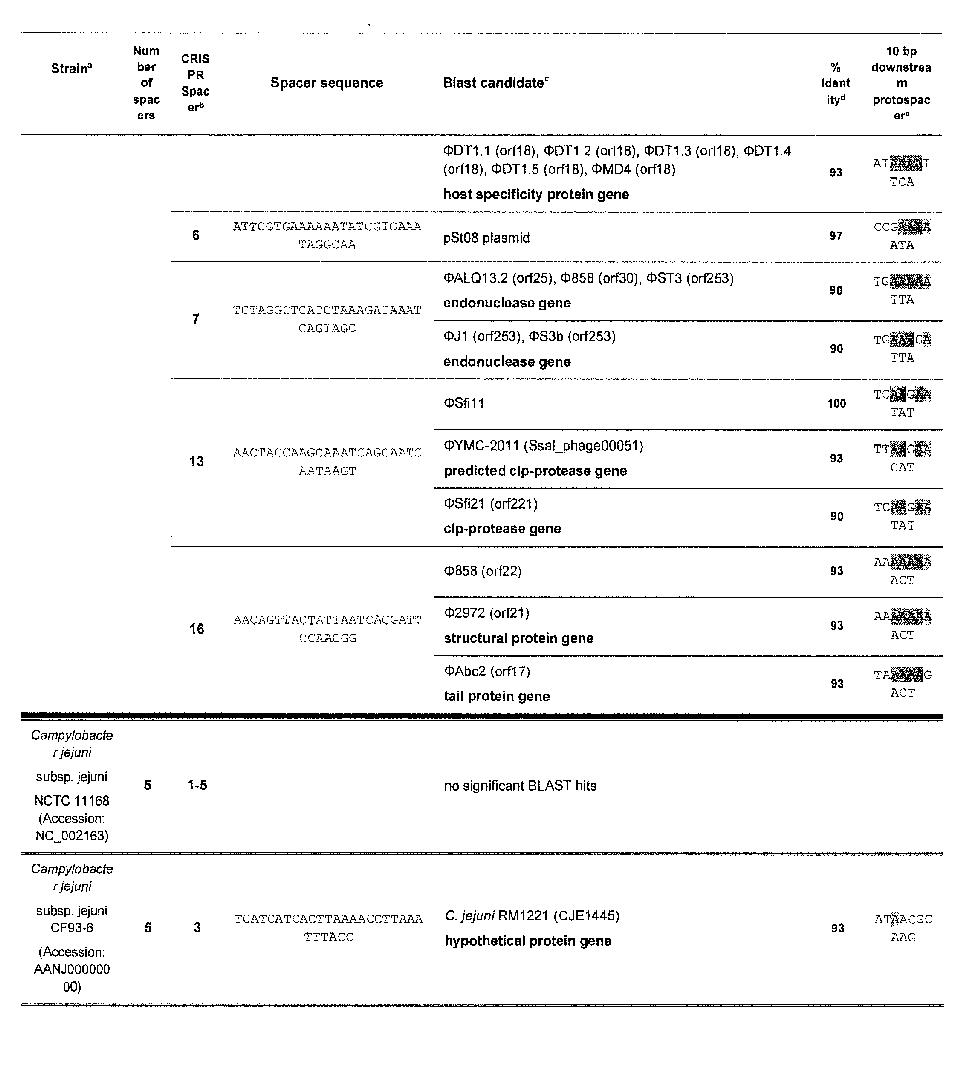

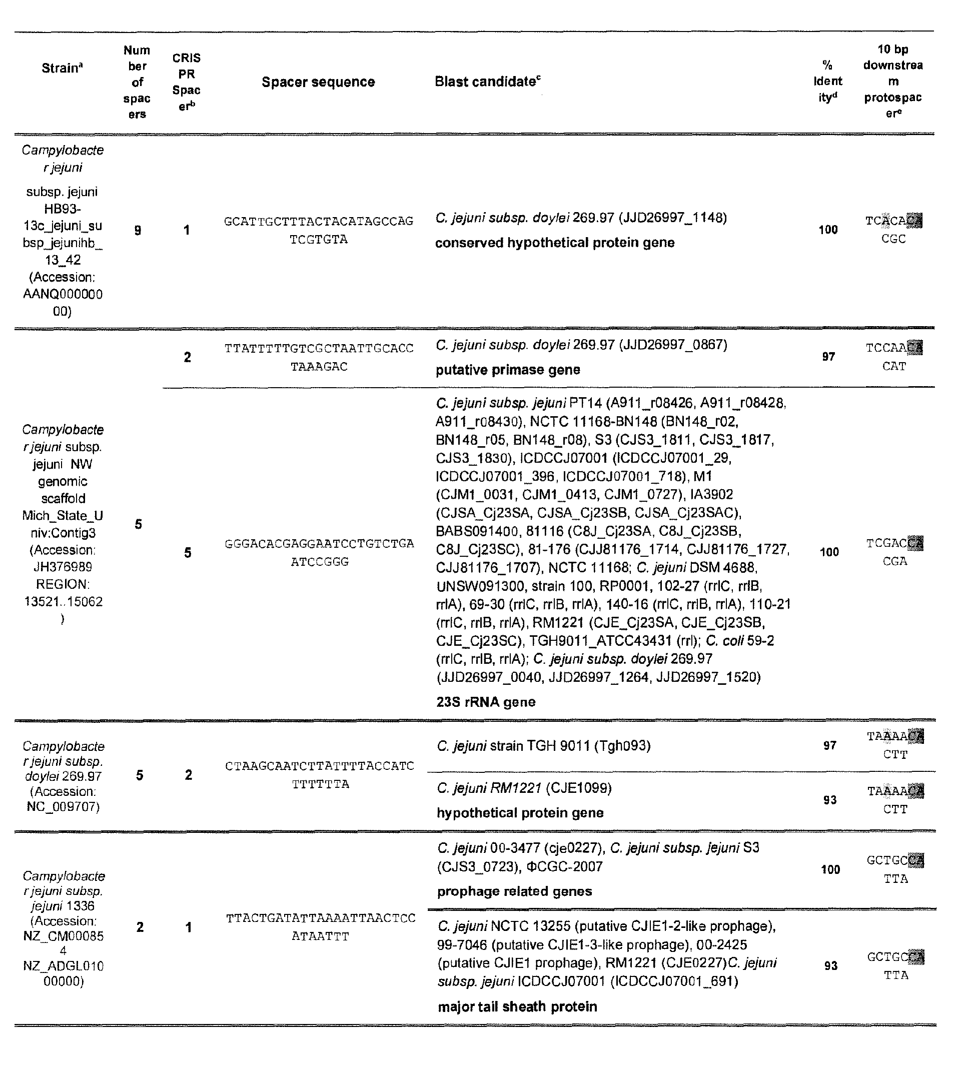

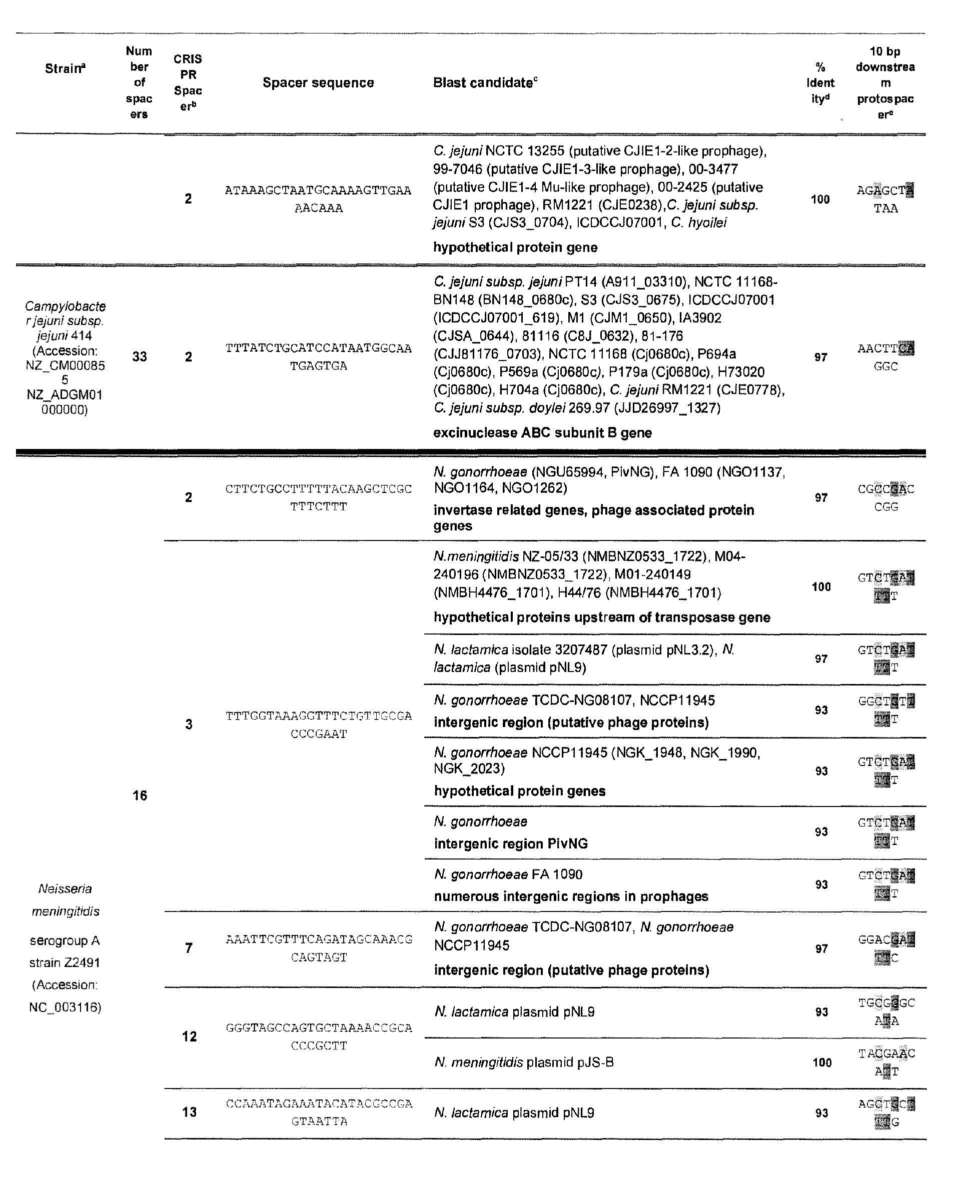

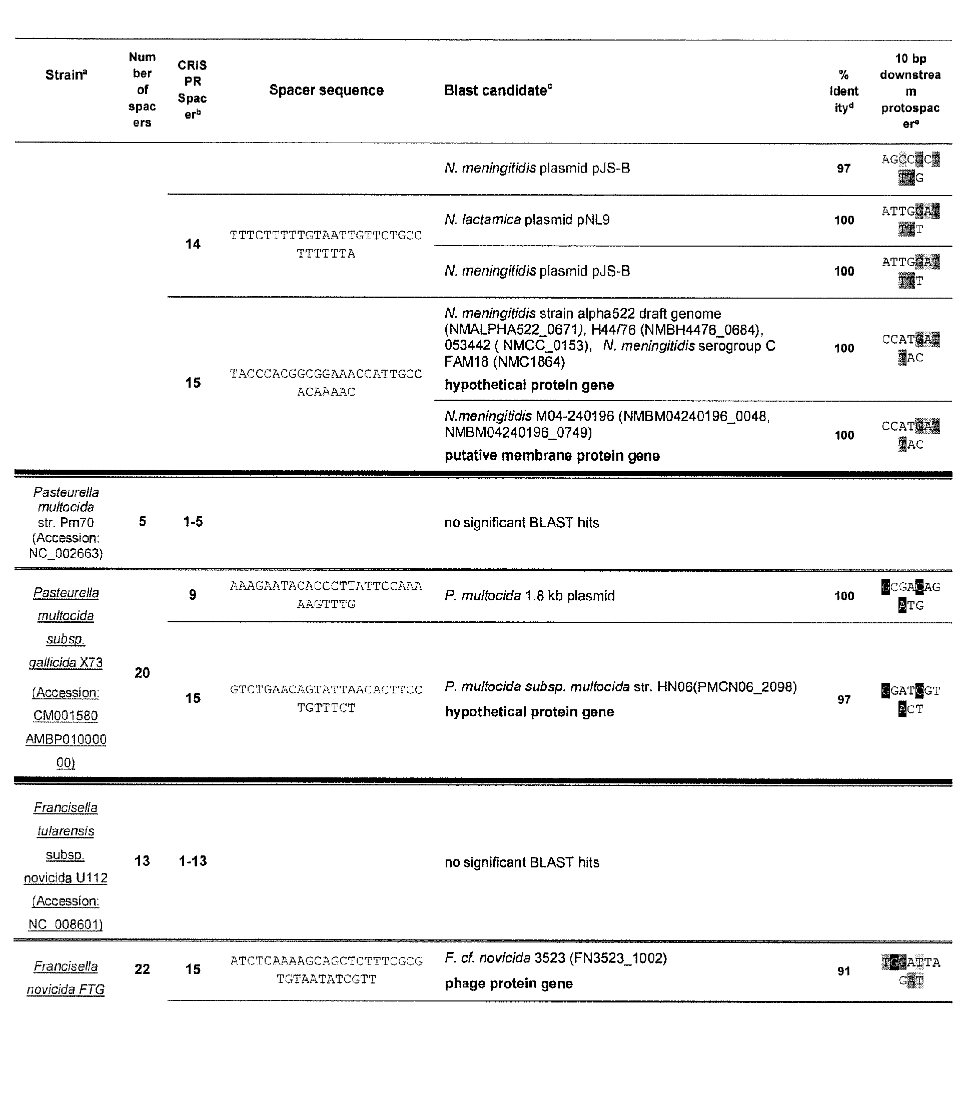

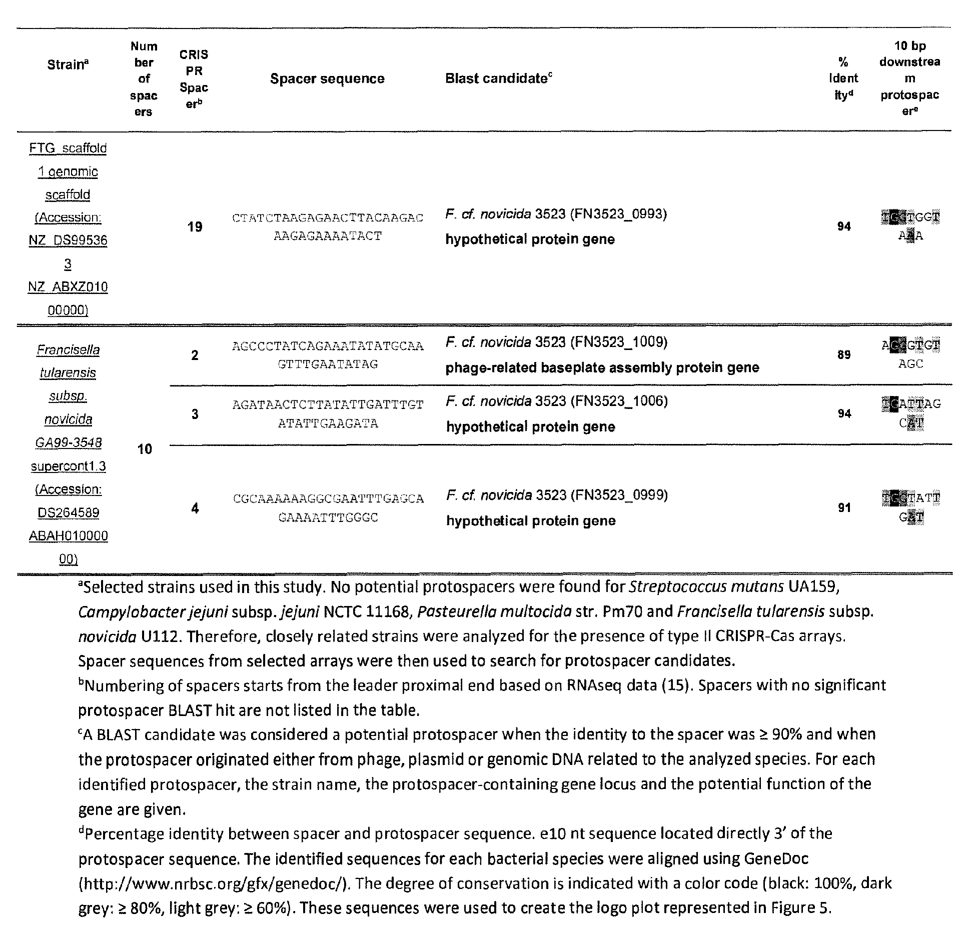

**Supplementary Table S4 - Cas9 is present in bacteria from 12 different phyla and diverse habitats**

| **Strain^{a}** | **Class** | **Isolation/habitat^{b}** |
|---|---|---|
| **Actinobacteria** | | |
| Actinobacteridae | | |
| *Acidothermus cellulolyticus* 11B | *Acidothermaceae* | extremophile (hot water spring) |
| *Actinomyces coleocanis* | *Actinomycetaceae* | dog genital tract |
| *Actinomyces georgiae* F0490 | *Actinomycetaceae* | oral cavity |
| *Actinomyces naeslundii* str. Howell 279 | *Actinomycetaceae* | oral cavity |
| *Actinomyces* sp. ICM47 | *Actinomycetaceae* | ND |
| *Actinomyces* sp. oral taxon 175 str. F0384 | *Actinomycetaceae* | oral cavity |
| *Actinomyces* sp. oral taxon 180 str. F0310 | *Actinomycetaceae* | oral cavity |
| *Actinomyces* sp. oral taxon 181 str. F0379 | *Actinomycetaceae* | oral cavity |
| *Actinomyces* sp. oral taxon 848 str. F0332 | *Actinomycetaceae* | oral cavity |
| *Actinomyces turicensis* ACS-279-V-Col4 | *Actinomycetaceae* | genital tract |
| *Bifidobacterium bifidum* S17 | *Bifidobacteriaceae* | gastrointestinal tract/feces |
| *Bifidobacterium dentium* Bd1 | *Bifidobacteriaceae* | oral cavity |
| *Bifidobacterium longum* DJO10A | *Bifidobacteriaceae* | gastrointestinal tract/feces |
| *Bifidobacterium* sp. 12_1_47BFAA | *Bifidobacteriaceae* | gastrointestinal tract/feces |
| *Corynebacterium accolens* ATCC 49726 | *Corynebacterineae* | wound |
| *Corynebacterium diphteriae* NCTC 13129 | *Corynebacterineae* | oral cavity |
| *Corynebacterium matruchotii* ATCC 14266 | *Corynebacterineae* | oral cavity |
| *Gardnerella vaginalis* 5-1 | *Bifidobacteriaceae* | genital tract |
| *Mobiluncus curtisii* ATCC 35242 | *Actinomycetaceae* | genital tract |
| *Mobiluncus mulieris* 28-1 | *Actinomycetaceae* | genital tract |
| *Scardovia inopinata* F0304 | *Bifidobacteriaceae* | oral cavity |
| *Scardovia wiggsiae* F0424 | *Bifidobacteriaceae* | oral cavity |

| Coriobacteridae | | |
|---|---|---|
| *Coriobacterium glomerans* PW2 | *Coriobacteriaceae* | invertebrate (red soldier bug) |
| *Eggerthella* sp. YY7918 | *Coriobacteriaceae* | gastrointestinal tract/feces |
| *Gordonibacter pamelaeae* 7-10-1-b | *Coriobacteriaceae* | gastrointestinal tract/feces |
| *Olsenella uli* DSM 7084 | *Coriobacteriaceae* | oral cavity |

| **Bacteroidetes** | | |
|---|---|---|
| Bacteroidia | | |
| *Anaerophaga* sp. HS1 | *Marinilabiliaceae* | extremophile (hot water spring) |
| *Anaerophaga thermohalophila* DSM 12881 | *Marinilabiliaceae* | environmental sample (oil residue) |
| *Bacteroides cellulosilyticus* DSM 14838 | *Bacteroidaceae* | gastrointestinal tract/feces |
| *Bacteroides coprophilus* DSM 18228 | *Bacteroidaceae* | gastrointestinal tract/feces |
| *Bacteroides coprosuis* DSM 18011 | *Bacteroidaceae* | pig feces |
| *Bacteroides dorei* DSM 17855 | *Bacteroidaceae* | gastrointestinal tract/feces |
| *Bacteroides eggerthii* 1_2_48FAA | *Bacteroidaceae* | gastrointestinal tract/feces |
| *Bacteroides faecis* MAJ27 | *Bacteroidaceae* | gastrointestinal tract/feces |
| *Bacteroides fluxus* YIT 12057 | *Bacteroidaceae* | gastrointestinal tract/feces |
| *Bacteroides fragilis* NCTC9343 | *Bacteroidaceae* | gastrointestinal tract/feces |
| *Bacteroides nordii* CL02T12C05 | *Bacteroidaceae* | gastrointestinal tract/feces |
| *Bacteroides oleiciplenus* YIT 12058 | *Bacteroidaceae* | gastrointestinal tract/feces |
| *Bacteroides* sp. 2_1_16 | *Bacteroidaceae* | gastrointestinal tract/feces |
| *Bacteroides* sp. 203 | *Bacteroidaceae* | gastrointestinal tract/feces |
| *Bacteroides* sp. 3_1_19 | *Bacteroidaceae* | gastrointestinal tract/feces |
| *Bacteroides* sp. 3_1_33FAA | *Bacteroidaceae* | gastrointestinal tract/feces |
| *Bacteroides* sp. 9_1_42FAA | *Bacteroidaceae* | gastrointestinal tract/feces |
| *Bacteroides* sp. D2 | *Bacteroidaceae* | gastrointestinal tract/feces |
| *Bacteroides uniformis* CL03T00C23 | *Bacteroidaceae* | gastrointestinal tract/feces |
| *Bacteroides vulgatus* CL09T03C04 | *Bacteroidaceae* | gastrointestinal tract/feces |
| *Bacteroidetes* oral taxon 274 str. F0058 | *Bacteroidaceae* | oral cavity |
| *Barnesiella intestinihominis* YIT 11860 | *Bacteroidaceae* | gastrointestinal tract/feces |
| *Marinilabilia* sp. AK2 | *Marinilabiliaceae* | extremophile (solar saltern) |
| *Odoribacter laneus* YIT 12061 | *Porphyromonadaceae* | gastrointestinal tract/feces |
| *Parabacteroides johnsonii* DSM 18315 | *Bacteroidaceae* | gastrointestinal tract/feces |
| *Parabacteroides* sp. D13 | *Bacteroidaceae* | gastrointestinal tract/feces |
| *Porphyromonas catoniae* F0037 | *Porphyromonadaceae* | oral cavity |
| *Porphyromonas* sp. oral taxon 279 str. F0450 | *Porphyromonadaceae* | oral cavity |
| *Prevotella bivia* JCVIHMP010 | *Prevotellaceae* | genital tract |
| *Prevotella buccae* ATCC 33574 | *Prevotellaceae* | oral cavity |
| *Prevotella buccalis* ATCC 35310 | *Prevotellaceae* | oral cavity |
| *Prevotella denticola* F0289 | *Prevotellaceae* | oral cavity |
| *Prevotella disiens* FB035-09AN | *Prevotellaceae* | oral cavity |
| *Prevotella histicola* F0411 | *Prevotellaceae* | oral cavity |
| *Prevotella intermedia 17* | *Prevotellaceae* | oral cavity |
| *Prevotella melaninogenica* D18 | *Prevotellaceae* | oral cavity/rumen |
| *Prevotella micans* F0438 | *Prevotellaceae* | oral cavity |
| *Prevotella multiformis* DSM 16608 | *Prevotellaceae* | oral cavity |
| *Prevotella nigrescens* ATCC 33563 | *Prevotellaceae* | oral cavity |
| *Prevotella oralis* ATCC 33269 | *Prevotellaceae* | oral cavity |
| *Prevotella oulorum* F0390 | *Prevotellaceae* | oral cavity |
| *Prevotella ruminicola* 23 | *Prevotellaceae* | rumen |
| *Prevotella saccharolytica* F0055 | *Prevotellaceae* | oral cavity |
| *Prevotella* sp. C561 | *Prevotellaceae* | oral cavity |
| *Prevotella* sp. MSX73 | *Prevotellaceae* | oral cavity |
| *Prevotella* sp. oral taxon 306 str. F0472 | *Prevotellaceae* | oral cavity |
| *Prevotella* sp. oral taxon 317 str. F0108 | *Prevotellaceae* | oral cavity |
| *Prevotella* sp. oral taxon 472 str. F0295 | *Prevotellaceae* | oral cavity |
| *Prevotella stercorea* DSM 18206 | *Prevotellaceae* | gastrointestinal tract/feces |
| *Prevotella tannerae* ATCC 51259 | *Prevotellaceae* | oral cavity |
| *Prevotella timonensis* CRIS 5C-B1 | *Prevotellaceae* | wound (breast abscess) |
| *Prevotella veroralis* F0319 | *Prevotellaceae* | oral cavity |
| *Tannerella* sp. 6_1_58FAA_CT1 | *Porphyromonadaceae* | gastrointestinal tract/feces |

| Cytophagia | | |
|---|---|---|
| *Belliella baltica* DSM 15883 | *Cyclobacteriaceae* | environmental sample (groundwater) |
| *Indibacter alkaliphilus* LW1 | *Cyclobacteriaceae* | extremophile (soda lake) |
| *Nitritalea halalkaliphila* LW7 | *Cyclobacteriaceae* | extremophile (saline soda lake) |

| Flavobacteria | | |
|---|---|---|
| *Bergeyella zoohelcum* ATCC 43767 | *Flavobacteriaceae* | oral cavity |
| | | dog and cat oral cavity/zoonotic |
| *Capnocytophaga canimorsus* Cc5 | *Flavobacteriaceae* | infections |
| *Capnocytophaga gingivalis* ATCC 33624 | *Flavobacteriaceae* | oral cavity |
| *Capnocytophaga ochracea* DSM 7271 | *Flavobacteriaceae* | oral cavity |
| *Capnocytophaga* sp. CM59 | *Flavobacteriaceae* | oral cavity |
| *Capnocytophaga* sp. oral taxon 324 str. F0483 | *Flavobacteriaceae* | oral cavity |
| *Capnocytophaga* sp. oral taxon 326 str. F0382 | *Fla vobacteriaceae* | oral cavity |
| *Capnocytophaga* sp. oral taxon 329 str. F0087 | *Fla vobacteriaceae* | oral cavity |
| *Capnocytophaga* sp. oral taxon 335 str. F0486 | *Flavobacteriaceae* | oral cavity |
| *Capnocytophaga* sp. oral taxon 380 str. F0488 | *Flavobacteriaceae* | oral cavity |
| *Capnocytophaga* sp. oral taxon 412 str. F0487 | *Flavobacteriaceae* | oral cavity |
| *Capnocytophaga sputigena* ATCC 33612 | *Flavobacteriaceae* | oral cavity |
| *Chryseobacterium* sp. CF314 | *Flavobacteriaceae* | vegetation |
| *Flavobacteriaceae* bacterium S85 | *Flavobacteriaceae* | environmental sample (seawater) |
| *Flavobacterium branchiophilum* FL-15 | *Flavobacteriaceae* | fish pathogen |
| *Flavobacterium columnare* ATCC 49512 | *Flavobacteriaceae* | fish pathogen |
| *Flavobacterium psychrophilum* JIP02/86 | *Flavobacteriaceae* | fish pathogen |
| *Fluviicola taffensis* DSM 16823 | *Cryomorphaceae* | environmental sample (fresh water) |
| *Galbibacter* sp. ck-12-15 | *Flavobacteriaceae* | extremophile (deep sea sediment) |
| *Joostella marina* DSM 19592 | *Flavobacteriaceae* | environmental sample (seawater) |
| *Kordia algicida* OT-1 | *Flavobacteriaceae* | environmental sample (seawater) |
| *Myroides injenensis* M09-0166 | *Flavobacteriaceae* | human clinical specimens |
| *Myroides odoratus* DSM 2801 | *Flavobacteriaceae* | fish |

| Flavobacteria (continued) | | |
|---|---|---|
| *Omithobacterium rhinotracheale* DSM 15997 | *Flavobacteriaceae* | bird respiratory tract |
| *Psychroflexus torquis* ATCC 700755 | *Flavobacteriaceae* | extremophile (antarctic ice) |
| *Riemerella anatipestifer* ATCC 11845 = DSM 15868 | *Flavobacteriaceae* | bird |
| *Weeksella virosa* DSM 16922 | *Flavobacteriaceae* | genital tract/urine |
| *Zunongwangia profunda* SM-A87 | *Flavobacteriaceae* | extremophile (deep sea sediment) |

| Sphingobacteria | | |
|---|---|---|
| *Mucilaginibacter paludis* DSM 18603 | *Sphingobacteriaceae* | food (fermented) |
| *Niabella soli* DSM 19437 | *Chitinophagaceae* | environmental sample (soil) |
| *Sphingobacterium spiritivorum* ATCC 33861 | *Sphingobacteriaceae* | human clinical specimens |

| **Firmicutes** | | |
|---|---|---|
| Bacilli | | |
| *Alicycliphilus denitrificans* | *Alicyclobacillaceae* | environmental sample (sewage) |
| *Alicyclobacillus hesperidum* URH17-3-68 | *Alicyclobacillaceae* | extremophile (hot water spring) |
| *Bacillus cereus* Rock1-15 | *Bacillaceae* | environmental sample (soil) |
| *Bacillus smithii* 7 3 47FAA | *Bacillaceae* | human clinical specimens |
| *Bacillus thuringiensis* serovar *finitimus* YBT-020 | *Bacillaceae* | environmental sample (soil) |
| *Brevibacillus laterosporus* GI-9 | *Paenibacillaceae* | environmental sample (soil) |
| *Catellicoccus marimammalium* M35/04/3 | *Enterococcaceae* | grey seal gastrointestinal tract |
| *Dolosigranulum pigrum* ATCC 51524 | *Carnobacteriaceae* | human clinical specimens |
| *Enterococcus faecalis* TX0012 | *Enterococcaceae* | gastrointestinal tract/feces |
| *Enterococcus faecium* 1231408 | *Enterococcaceae* | gastrointestinal tract/feces |
| *Enterococcus hirae* ATCC 9790 | *Enterococcaceae* | gastrointestinal tract/feces |
| *Enterococcus italicus* DSM 15952 | *Enterococcaceae* | food (fermented) |
| *Enterococcus* sp. 7L76 | *Enterococcaceae* | gastrointestinal tract/feces |
| *Facklamia hominis* CCUG 36813 | *Aerococcaceae* | burbuncle (human) |
| *Fructobacillus fructosus* KCTC 3544 | *Leuconostocaceae* | vegetation |
| *Gemella haemolysans* ATCC 10379 | *Streptococcaceae* | oral cavity |
| *Gemella moribillum* M424 | *Streptococcaceae* | gastrointestinal tract/feces |
| *Lactobacillus animalis* KCTC 3501 | *Lactobacillaceae* | food (fermented) |
| *Lactobacillus brevis* subsp. *gravesensis* ATCC 27305 | *Lactobacillaceae* | food (fermented) |
| *Lactobacillus buchneri* ATCC 11577 | *Lactobacillaceae* | food (fermented) |
| *Lactobacillus casei* str. Zhang | *Lactobacillaceae* | gastrointestinal tract/feces |
| *Lactobacillus coryniformis* subsp. *coryniformis* KCTC 3167 | *Lactobacillaceae* | food (fermented) |
| *Lactobacillus coryniformis* subsp. *torquens* KCTC 3535 | *Lactobacillaceae* | food (fermented) |
| *Lactobacillus crispatus* FB049-03 | *Lactobacillaceae* | genital tract |
| *Lactobacillus curvatus* CRL 705 | *Lactobacillaceae* | food (fermented) |
| *Lactobacillus delbrueckii* subsp. bulgaricus 2038 | *Lactobacillaceae* | food (fermented) |
| *Lactobacillus farciminis* KCTC 3681 | *Lactobacillaceae* | food (fermented) |
| *Lactobacillus fermentum* ATCC 14931 | *Lactobacillaceae* | food (fermented) |
| *Lactobacillus florum* 2F | *Lactobacillaceae* | vegetation |
| *Lactobacillus gasseri* JV-V03 | *Lactobacillaceae* | oral cavity |
| *Lactobacillus hominis* CRBIP 24.179 | *Lactobacillaceae* | gastrointestinal tract/feces |
| *Lactobacillus iners* LactinV 11V1-d | *Lactobacillaceae* | genital tract/urine |
| *Lactobacillus jensenii* 269-3 | *Lactobacillaceae* | genital tract/blood |
| *Lactobacillus johnsonii* DPC 6026 | *Lactobacillaceae* | pig gastrointestinal tract |
| *Lactobacillus mucosae* LM1 | *Lactobacillaceae* | wild pig gastrointestinal tract |
| *Lactobacillus paracasei* subsp. *paracasei* 8700:2 | *Lactobacillaceae* | food (fermented) |
| *Lactobacillus pentosus* IG1 | *Lactobacillaceae* | food (fermented) |
| *Lactobacillus plantarum* ZJ316 | *Lactobacillaceae* | gastrointestinal tract/feces |
| *Lactobacillus rhamnosus* GG | *Lactobacillaceae* | gastrointestinal tract/feces |
| *Lactobacillus ruminis* ATCC 25644 | *Lactobacillaceae* | rumen |
| *Lactobacillus salivarius* UCC118 | *Lactobacillaceae* | oral cavity |
| *Lactobacillus sanfranciscensis* TMW 1-1304 | *Lactobacillaceae* | food (fermented) |
| *Lactobacillus* sp. 66c | *Lactobacillaceae* | ND |
| *Lactobacillus versmoldensis* KCTC 3814 | *Lactobacillaceae* | food (fermented) |
| *Leuconostoc gelidum* KCTC 3527 | *Leuconostocaceae* | food (fermented) |
| *Leuconostoc pseudomesenteroides* 4882 | *Leuconostocaceae* | food (fermented) |

| Bacilli (continued) | | |
|---|---|---|
| *Listeria innocua* Clip11262 | *Listeriaceae* | environmental sample (soil) |
| *Listeria ivanovii* FSL F6-596 | *Listeriaceae* | animal and human/environmental samples |
| *Listeria monocytogenes* str. 1/2a F6854 | *Listeriaceae* | animal and human/environmental samples |
| *Listeria seeligeri* FSL N1-067 | *Listeriaceae* | animal and human/environmental samples |
| *Listeriaceae bacterium* TTU M1-001 | *Listeriaceae* | environmental sample (soil) |
| *Oenococcus kitaharae* DSM 17330 | *Leuconostocaceae* | food (fermented) |
| *Pediococcus acidilactici* DSM 20284 | *Lactobacillaceae* | vegetation |
| *Pediococcus lolii* NGRI 0510Q | *Lactobacillaceae* | vegetation (fermented) |
| *Planococcus antarcticus* DSM 14505 | *Planococcaceae* | extremophile (antarctic) |
| *Sporolactobacillus vineae* DSM 21990 = SL153 | *Sporolactobacillaceae* | environmental sample (soil) |
| *Staphylococcus aureus* subsp. *aureus* | *Staphylococcaceeae* | human clinical specimens |
| *Staphylococcus lugdunensis* M23590 | *Staphylococcaceeae* | human clinical specimens |
| *Staphylococcus massiliensis* S46 | *Staphylococcaceeae* | skin |
| *Staphylococcus pseudintermedius* ED99 | *Staphylococcaceeae* | dog skin |
| *Staphylococcus simulans* ACS-120-V-Sch1 | *Staphylococcaceeae* | genital tract |
| *Streptococcus agalactiae* 2603V/R | *Streptococcaceae* | gastrointestinal tract/feces |
| *Streptococcus anginosus* F0211 | *Streptococcaceae* | oral cavity |
| *Streptococcus bovis* ATCC 700338 | *Streptococcaceae* | rumen/zoonotic infections |
| *Streptococcus canis* FSL Z3-227 | *Streptococcaceae* | food (fermented) |
| *Streptococcus constellatus* subsp. *constellatus* SK53 | *Streptococcaceae* | human clinical specimens |
| *Streptococcus downei* F0415 | *Streptococcaceae* | monkey oral cavity |
| *Streptococcus dysgalactiae* DSM 12112 | *Streptococcaceae* | various animals/zoonotic infections |
| *Streptococcus equi* subsp. *zooepidemicus* MGCS10565 | *Streptococcaceae* | horse respiratory tract |
| *Streptococcus equinus* ATCC 9812 | *Streptococcaceae* | ruminants alimentary tract |
| *Streptococcus gallolyticus* UCN34 | *Streptococcaceae* | ruminants alimentary tract |
| *Streptococcus gordonii* str. Challis substr. CH1 | *Streptococcaceae* | oral cavity |
| *Streptococcus infantarius* ATCC BAA-102 | *Streptococcaceae* | gastrointestinal tract/feces |
| *Streptococcus iniae* 9117 | *Streptococcaceae* | fish/human pathogen |
| *Streptococcus macacae* NCTC 11558 | *Streptococcaceae* | monkey oral cavity |
| *Streptococcus macedonicus ACA-DC 198* | *Streptococcaceae* | food (fermented) |
| *Streptococcus mitis* ATCC 6249 | *Streptococcaceae* | oral cavity |
| *Streptococcus mutans* UA159 | *Streptococcaceae* | oral cavity |
| *Streptococcus oralis* SK1074 | *Streptococcaceae* | oral cavity |
| *Streptococcus parasanguinis* F0449 | *Streptococcaceae* | oral cavity |
| *Streptococcus pasteurianus* ATCC 43144 | *Streptococcaceae* | blood |
| *Streptococcus pseudoporcinus* SPIN 20026 | *Streptococcaceae* | genital tract |
| *Streptococcus pyogenes* SF370 | *Streptococcaceae* | oral cavity/wounds |
| *Streptococcus ratti* FA-1 = DSM 20564 | *Streptococcaceae* | rat oral cavity |
| *Streptococcus salivarius* JIM8777 | *Streptococcaceae* | oral cavity |
| *Streptococcus sanguinis* VMC66 | *Streptococcaceae* | oral cavity |
| *Streptococcus* sp. BS35b | *Streptococcaceae* | oral cavity |
| *Streptococcus* sp. C150 | *Streptococcaceae* | oral cavity (expectorated sputum) |
| *Streptococcus* sp. C300 | *Streptococcaceae* | oral cavity (expectorated sputum) |
| *Streptococcus* sp. F0441 | *Streptococcaceae* | oral cavity |
| *Streptococcus* sp. GMD4S | *Streptococcaceae* | oral cavity |
| *Streptococcus* sp. GMD6S | *Streptococcaceae* | oral cavity |
| *Streptococcus* sp. M334 | *Streptococcaceae* | oral cavity (expectorated sputum) |
| *Streptococcus* sp. oral taxon 056 str. F0418 | *Streptococcaceae* | oral cavity |
| *Streptococcus* sp. oral taxon 071 str. 73H25AP | *Streptococcaceae* | oral cavity |
| *Streptococcus suis* 89/1591 | *Streptococcaceae* | pig |
| *Streptococcus thermophilus* LMD-9 | *Streptococcaceae* | food (fermented) |
| *Streptococcus vestibularis* ATCC 49124 | *Streptococcaceae* | oral cavity |

| Clostridia | | |
|---|---|---|
| *Acidaminococcus intestini RyC-MR95* | *Acidaminococcaceae* | wound/abscess |
| *Acidaminococcus* sp. D21 | *Acidaminococcaceae* | gastrointestinal tract/feces |
| *Aminomonas paucivorans* DSM 12260 | *Syntrophoomonadaceae* | environmental sample (sewage) |
| *Anaerococcus tetradius* ATCC 35098 | *Peptostreptococcaceae* | human clinical specimens |
| *Butyrivibrio fibrisolvens* 16/4 | *Lachnospiraceae* | rumen |
| *Catenibacterium mitsuokai* DSM 15897 | *Lachnospiraceae* | gastrointestinal tract/feces |
| *Clostridium cellulolyticum* H10 | *Clostridiaceae* | vegetation (composted) |

| Clostridia (continued) | | |
|---|---|---|
| *Clostridium perfringens* D str. JGS1721 | *Clostridiaceae* | environmental sample (vegetation/marine sediment) |
| *Clostridium spiroforme* DSM 1552 | *Clostridiaceae* | gastrointestinal tract/feces |
| *Coprococcus catus* GD/7 | *Lachnospiraceae* | gastrointestinal tract/feces |
| *Coprococcus comes* ATCC 27758 | *Lachnospiraceae* | gastrointestinal tract/feces |
| *Dorea longicatena* DSM 13814 | *Clostridiaceae* | gastrointestinal tract/feces |
| *Eubacterium dolichum* DSM 3991 | *Eubacteriaceae* | gastrointestinal tract/feces |
| *Eubacterium rectale* ATCC 33656 | *Eubacteriaceae* | gastrointestinal tract/feces |
| *Eubacterium* sp. AS15 | *Eubacteriaceae* | oral cavity |
| *Eubacterium ventriosum* ATCC 27560 | *Eubacteriaceae* | gastrointestinal tract/feces |
| *Eubacterium yurii* subsp. margaretiae ATCC 43715 | *Peptostreptococcaceae* | oral cavity |
| *Filifactor alocis* ATCC 35896 | *Peptostreptococcaceae* | cat and human oral cavity |
| *Finegoldia magna* ATCC 29328 | *Peptostreptococcaceae* | oral cavity |
| *Helcococcus kunzii* ATCC 51366 | *Clostridiales Family XI* | wound |
| *Oribacterium sinus* F0268 | *Lachnospiraceae* | human clinical specimens |
| *Peptoniphilus duerdenii* ATCC BAA-1640 | *Peptostreptococcaceae* | wound |
| *Peptoniphilus* sp. oral taxon 386 str. F0131 | *Peptostreptococcaceae* | oral cavity |
| *Phascolarctobacterium* sp. YIT 12067 | *Acidaminococcaceae* | gastrointestinal tract/feces |
| *Phascolarctobacterium succinatutens* YIT 12067 | *Acidaminococcaceae* | gastrointestinal tract/feces |
| *Pseudoramibacter alactolyticus* ATCC 23263 | *Clostridiaceae* | oral cavity |
| *Roseburia intestinalis* L1-82 | *Lachnospiraceae* | gastrointestinal tract/feces |
| *Roseburia inulinivorans* DSM 16841 | *Lachnospiraceae* | gastrointestinal tract/feces |
| *Ruminococcus albus* 8 | *Ruminococcaceae* | gastrointestinal tract/feces |
| *Ruminococcus lactaris* ATCC 29176 | *Ruminococcaceae* | gastrointestinal tract/feces |
| *Subdoligranulum* sp. 4_3_54A2FAA | *Ruminococcaceae* | gastrointestinal tract/feces |

| Negativicutes | | |
|---|---|---|
| *Megasphaera* sp. UPII 135-E | *Veillonellaceae* | rumen |
| *Veillonella atypica* ACS-134-V-Col7a | *Veillonellaceae* | oral cavity |
| *Veillonella parvula* ATCC 17745 | *Veillonellaceae* | gastrointestinal/genital tract |
| *Veillonella* sp. 6_1_27 | *Veillonellaceae* | gastrointestinal tract/feces |
| *Veillonella* sp. oral taxon 780 str. F0422 | *Veillonellaceae* | oral cavity |

| **Proteobacteria** | | |
|---|---|---|
| Alphaproteobacteria | | |
| *Acetobacter aceti* NBRC 14818 | *Acetobacteraceae* | environmental sample |
| *Azospirillum* sp. B510 | *Rhodospirillaceae* | vegetation |
| *Bradyrhizobium* sp. BTAi1 | *Bradyrhizobiaceae* | vegetation |
| *Caenispirillum salinarum* AK4 | *Rhodospirillaceae* | extremophile (solar saltern) |
| *Dinoroseobacter shibae* DFL 12 | *Rhodobacteraceae* | environmental sample (seawater) |
| *Gluconacetobacter diazotrophicus* PAI5 | *Acetobacteriaceae* | vegetation |
| *Maritimibacter alkaliphilus* ATCC2654 | *Rhodobacteraceae* | environmental sample (seawater) |
| *Methylocystis* sp. ATCC 49242 | *Methylocystaceae* | environmental sample (sewage, fresh water) |
| *Methylosinus trichosporium* OB3b | *Methylocystaceae* | environmental sample (soil, fresh water) |
| *Nitrobacter hamburgensis* X14 | *Bradyrhizobiaceae* | environmental sample (soil) |
| *Parvibaculum lavamentivorans* DS-1 | *Phyllobacteriaceae* | environmental sample (sewage) |
| *Puniceispirillum marinum* IMCC1322 | *SAR16 clade* | environmental sample (seawater) |
| *Rhodopseudomonas palustris* BisB18 | *Bradyrhizobiaceae* | environmental sample (soil) |
| *Rhodospirillum rubrum* ATCC 11170 | *Rhodospirillaceae* | environmental sample (sea mud) |
| *Rhodovulum* sp. PH10 | *Rhodobacteraceae* | environmental sample (soil) |
| *Sphingobium* sp. AP49 | *Sphingomonadaceae* | vegetation |
| *Sphingomonas* sp. S17 | *Sphingomonadaceae* | environmental sample (stromatolite) |
| *Tistrella mobilis* KA081020-065 | *Rhodospirillaceae* | environmental sample (seawater) |

| Betaproteobacteria | | |
|---|---|---|
| *Acidovorax avenae* subsp. *avenae* ATCC 19860 | *Comamonadaceae* | environmental sample (soil) |
| *Acidovorax ebreus* TPSY | *Comamonadaceae* | environmental sample (water) |
| *Burkholderiales* bacterium 1 -1 47 | *Burkholderiales* | gastrointestinal tract/feces |
| *Kingella kingae* ATCC 23330 | *Neisseriaceae* | oral cavity |
| *Neisseria bacilliformis* ATCC BAA-1200 | *Neisseriaceae* | oral cavity |

| Betaproteobacteria (continued) | | |
|---|---|---|
| *Neisseria cinerea* ATCC 14685 | *Neisseriaceae* | oral cavity |
| *Neisseria flavescens* SK114 | *Neisseriaceae* | human clinical specimens |
| *Neisseria lactamica* 020-06 | *Neisseriaceae* | oral cavity |
| *Neisseria meningitidis A Z2491* | *Neisseriaceae* | oral cavity |
| *Neisseria mucosa* C102 | *Neisseriaceae* | oral cavity (expectorated sputum) |
| *Neisseria* sp. oral taxon 014 str. F0314 | *Neisseriaceae* | oral cavity |
| *Neisseria subflava* NJ9703 | *Neisseriaceae* | oral cavity |
| *Neisseria wadsworthii* 9715 | *Neisseriaceae* | skin |
| *Nitrosomonas* sp. AL212 | *Nitrosomonadaceae* | environmental sample (fresh water) |
| *Parasutterella excrementihominis* YIT 11859 | *Alcaligenaceae* | gastrointestinal tract/feces |
| *Ralstonia syzygii* R24 | *Burkholderiaceae* | environmental sample (soil) |
| *Simonsiella muelleri* ATCC 29453 | *Neisseriaceae* | oral cavity |
| *Sutterella parvirubra* YIT 11816 | *Alcaligenaceae* | gastrointestinal tract/feces |
| *Sutterella wadsworthensis* 3 1 45B | *Alcaligenaceae* | gastrointestinal tract/feces |
| *Verminephrobacter aporrectodeae* subsp. *tuberculatae* At4 | *Comamonadaceae* | invertebrate (earthworm) |
| *Verminephrobacter eiseniae* EF01-2 | *Comamonadaceae* | invertebrate (earthworm) |

| Gammaproteobacteria | | |
|---|---|---|
| *Actinobacillus minor* NM305 | *Pasteurellaceae* | pig respiratory tract |
| *Actinobacillus pleuropneumoniae* serovar 10 D13039 | *Pasteurellaceae* | pig respiratory tract |
| *Actinobacillus succinogenes* 130Z | *Pasteurellaceae* | rumen |
| *Actinobacillus suis* H91-0380 | *Pasteurellaceae* | pig pathogen |
| *Actinobacillus ureae* ATCC 25976 | *Pasteurellaceae* | respiratory tract |
| *Alcanivorax* sp. W11-5 | *Alcanivoracaceae* | extremophile (deep sea sediment) |
| *Francisella tularensis* subsp. *holarctica* LVS | *Francisellaceae* | engineered live vaccine strain human/environmental sample |
| *Francisella tularensis* subsp. *novicida* U112 | *Francisellaceae* | (water) |
| *Francisella tularensis* subsp. *tularensis* WY96-3418 | *Francisellaceae* | wound |
| gamma proteobacterium HTCC5015 | *Unclassified* | environmental sample (seawater) |
| gammaproteobacterium HdN1 | *Unclassified* | environmental sample (sewage) |
| *Haemophilus parainfluenzae* T3T1 | *Pasteurellaceae* | oral cavity/genital tract |
| *Haemophilus pittmaniae* HK 85 | *Pasteurellaceae* | oral cavity |
| *Haemophilus sputorum* HK 2154 | *Pasteurellaceae* | oral cavity |
| *Legionella pneumophila* str. Paris | *Legionellaceae* | human clinical specimens |
| *Pasteurella bettyae* CCUG 2042 | *Pasteurellaceae* | genital tract |
| *Pasteurella multocida* subsp. *gallicida* X73 | *Pasteurellaceae* | bird pathogen |
| *Pasturella multocida* Pm70 | *Pasteurellaceae* | bird respiratory tract/zoonotic infections |

| Deltaproteobacteria | | |
|---|---|---|
| uncultured delta proteobacterium HF0070_ 07E19 | *Unclassified* | environmental sample (seawater) |

| Eps Epsilonproteobacteria | | |
|---|---|---|
| *Campylobacter coli* 2962 | *Campylobacteraceae* | animals/human pathogen |
| *Campylobacter jejuni* NCTC11168 | *Campylobacteraceae* | bird |
| *Campylobacter jejuni* subsp. *doylei* 269-97 | *Campylobacteraceae* | blood |
| *Campylobacter lari* | *Campylobacteraceae* | gastrointestinal tract/feces |
| *Helicobacter canadensis* MIT 98-5491 | *Helicobacteriaceae* | gastrointestinal tract/feces |
| *Helicobacter cinaedi* CCUG 18818 | *Helicobacteriaceae* | gastrointestinal tract/feces |
| *Helicobacter hepaticus* ATCC 51449 | *Helicobacteriaceae* | mouse liver |
| *Helicobacter mustelae* 12198 | *Helicobacteriaceae* | ferret |
| *Helicobacter pullorum* MIT 98-5489 | *Helicobacteriaceae* | bird/zoonotic infections |
| *Nitratifractor salsuginis* DSM 16511 | *Unclassified* | extremophile (deep sea sediment) |
| *Wolinella succinogenes* DSM 1740 | *Helicobacteraceae* | rumen |

| **Fusobacteria** | | |
|---|---|---|
| *Fusobacterium nucleatum* subsp. *vincentii* ATCC | *Fusobacteriaceae* | oral cavity |
| 49256 | | |
| *Fusobacterium* sp. 1_1_41FAA | *Fusobacteriaceae* | gastrointestinal tract/feces |
| *Fusobacterium* sp. 3_1_27 | *Fusobacteriaceae* | gastrointestinal tract/feces |
| *Fusobacterium* sp. 3_1_36A2 | *Fusobacteriaceae* | gastrointestinal tract/feces |
| *Ilyobacter polytropus* DSM 2926 | *Fusobacteriaceae* | environmental sample (sea mud) |
| *Streptobacillus moniliformis* DSM 12112 | *Leptotrichiaceae* | rodent/human pathogen |

| **Spirochaetes** | | |
|---|---|---|
| *Leptospira inadai* serovar Lyme str. 10 | *Leptospiraceae* | human clinical specimens |
| *Sphaerochaeta globus* str. Buddy | *Spirochaetaceae* | extremophile (marine hot spring) |
| *Treponema denticola* ATCC 35405 | *Spirochaetaceae* | oral cavity |
| *Treponema phagedenis* F0421 | *Spirochaetaceae* | monkey genital tracts |
| *Treponema* sp. JC4 | *Spirochaetaceae* | rumen |
| *Treponema vincentii* ATCC 35580 | *Spirochaetaceae* | oral cavity |

| **Tenericutes** | | |
|---|---|---|
| Mollicutes | | |
| *Mycoplasma canis* PG 14 | *Mycoplasmataceae* | dog oral cavity |
| *Mycoplasma cynos* C142 | *Mycoplasmataceae* | dog respiratory tract |
| *Mycoplasma gallisepticum* str. F | *Mycoplasmataceae* | bord pathogen |
| *Mycoplasma iowae* 695 | *Mycoplasmataceae* | bird |
| *Mycoplasma mobile* 163K | *Mycoplasmataceae* | fish pathogen |
| *Mycoplasma ovipneumoniae* SC01 | *Mycoplasmataceae* | goat respiratory tract |
| *Mycoplasma synoviae* 53 | *Mycoplasmataceae* | bird pathogen |
| *Solobacterium moorei* F0204 | *Erysipelotrichaceae* | gastrointestinal tract/feces |

| **Elusimicrobia** | | |
|---|---|---|
| *Elusimicrobium minutum* Pei191 | *Elusimicrobiaceae* | invertebrate (scarab beetle) |
| Uncultured Termite group 1 bacterium phylotype Rs-D17 | *Elusimicrobiaceae* | invertebrate |

| **Fibrobacteres** | | |
|---|---|---|
| *Fibrobacter succinogenes* S85 | *Fibrobacteraceae* | rumen |

| **Ignavibacteria** | | |
|---|---|---|
| *Ignavibacterium album* JCM 16511 | *Ignavibacteriaceae* | extremophile (hot water spring) |

| **Planktomycetes** | | |
|---|---|---|
| *Blastopirellula marina* DSM 3645 | *Planctomycetaceae* | environmental sample (seawater) |

| **Verrucomicrobia** | | |
|---|---|---|
| *Diplosphaera colitermitum* TAV2 | *Opitutaceae* | invertebrate (termite) |
| *Akkermansia muciniphila* ATCC BAA-835 | *Verrucomicrobiaceae* | gastrointestinal tract/feces |

| **Unclassified** | | |
|---|---|---|
| candidate division TM7 single-cell isolate TM7c | *Unclassified* | oral cavity |
| uncultured bacterium | *Unclassified* | environmental sample (groundwater) |
| uncultured bacterium | *Unclassified* | environmental sample (groundwater) |
| uncultured bacterium T3_7_42578 | *Unclassified* | invertebrate (honeybee) |

| | | |
|---|---|---|
| ^{a}Single strains representing every species found to harbor the cas9 gene are listed. ^{b}The origin of the specific strain and/or typical habitat of the species are given for every strain. ND, no data available. Note that if not specified otherwise, isolates from body sites and feces are human commensals and pathogens. | | |

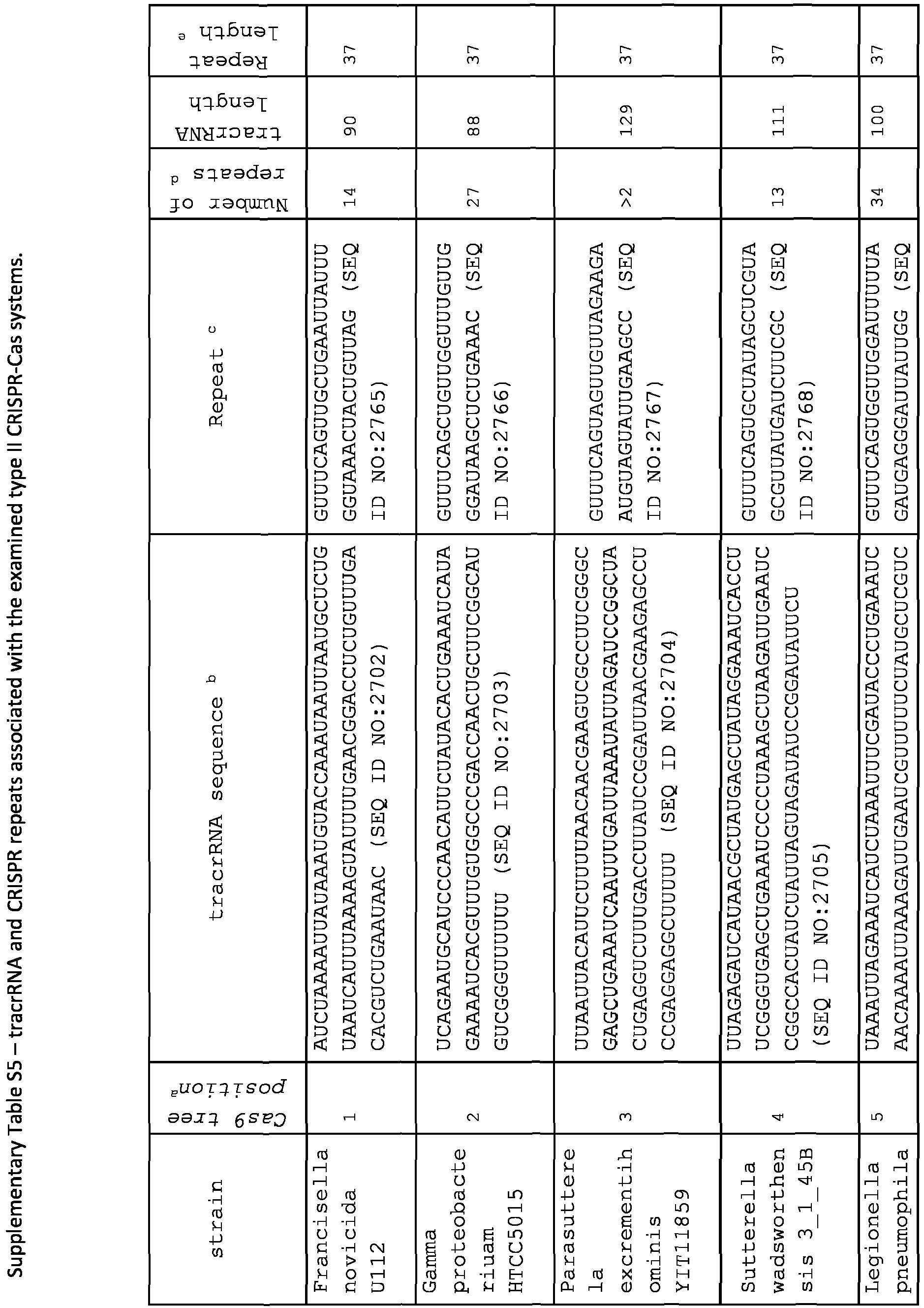

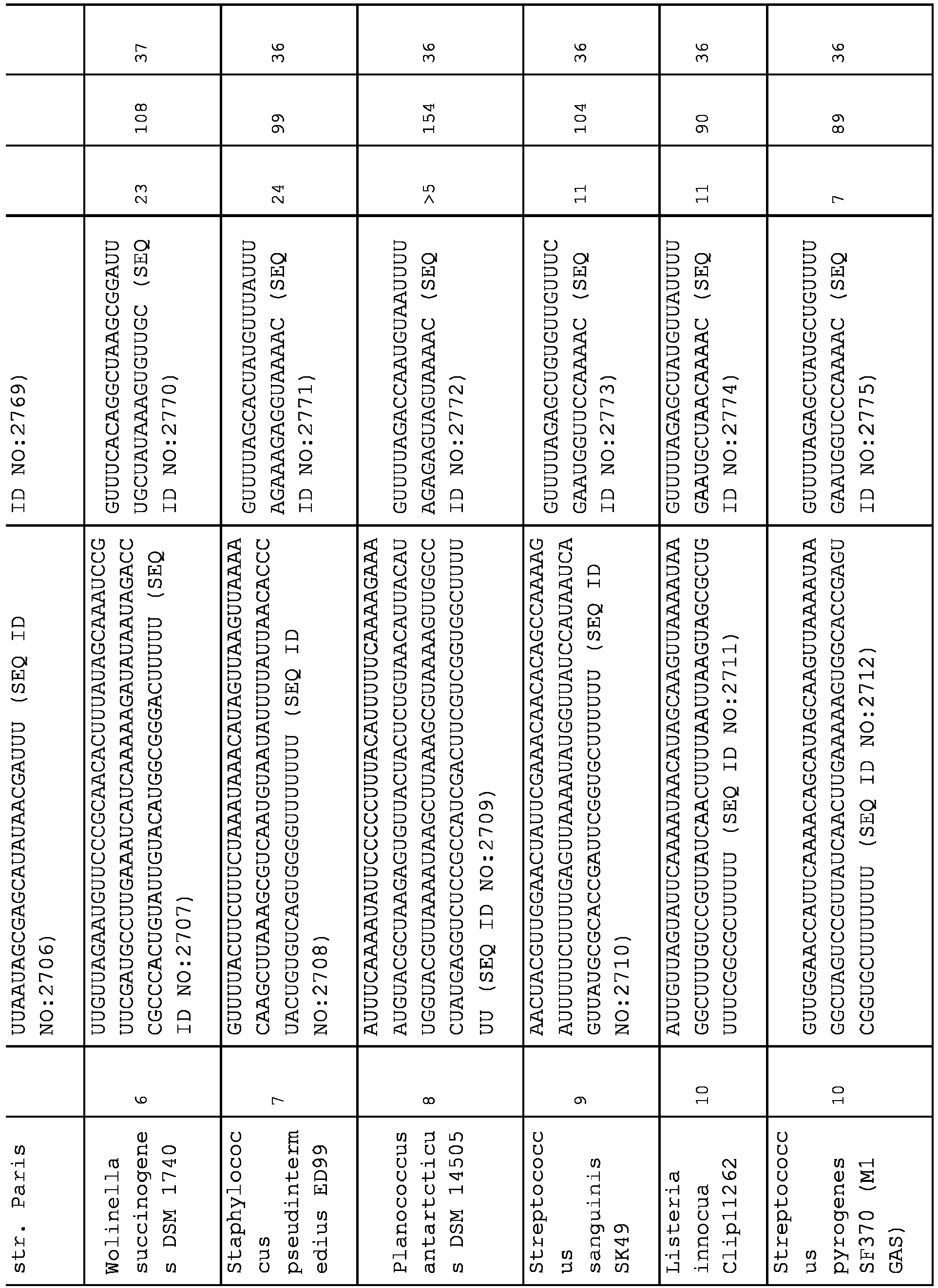

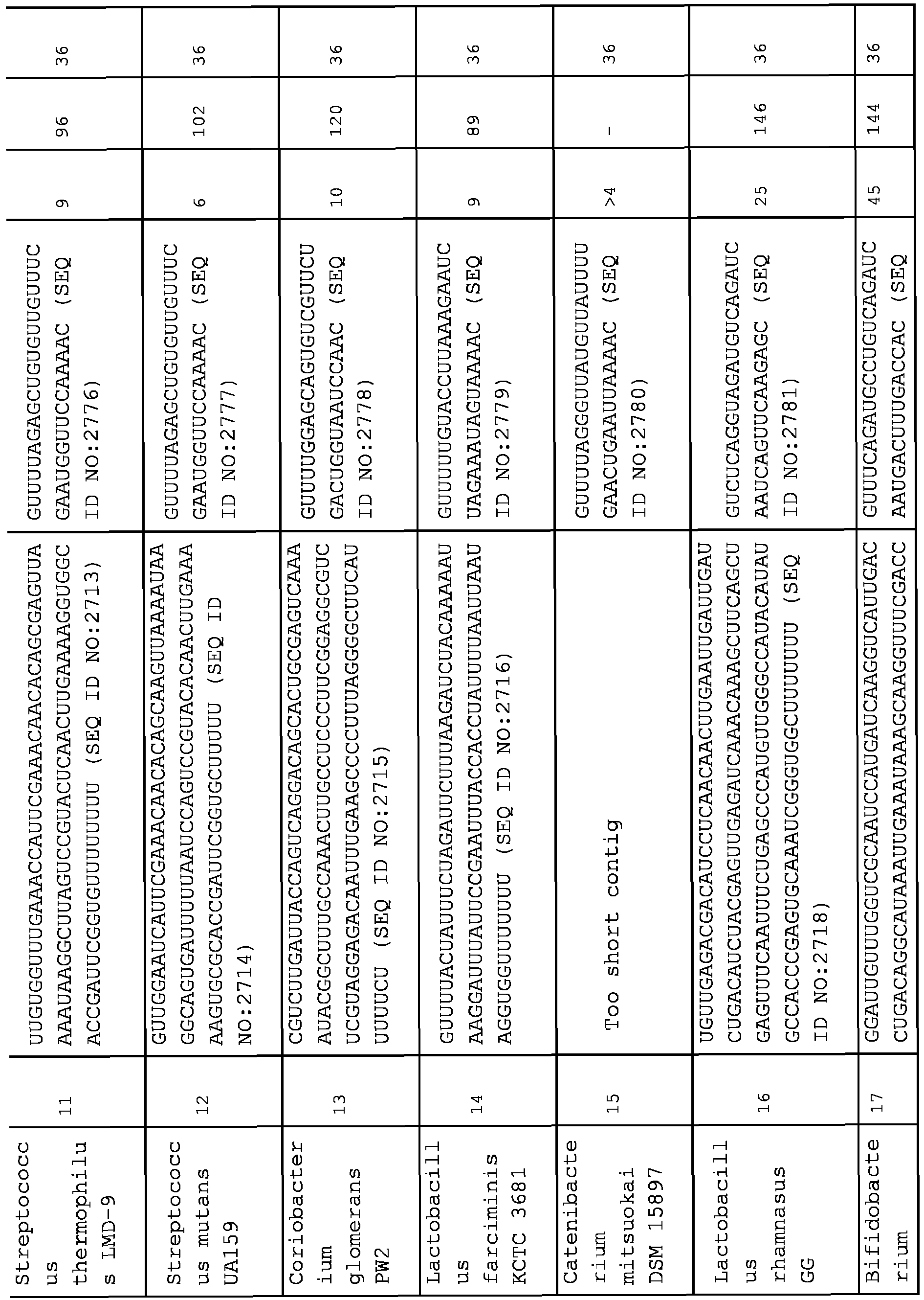

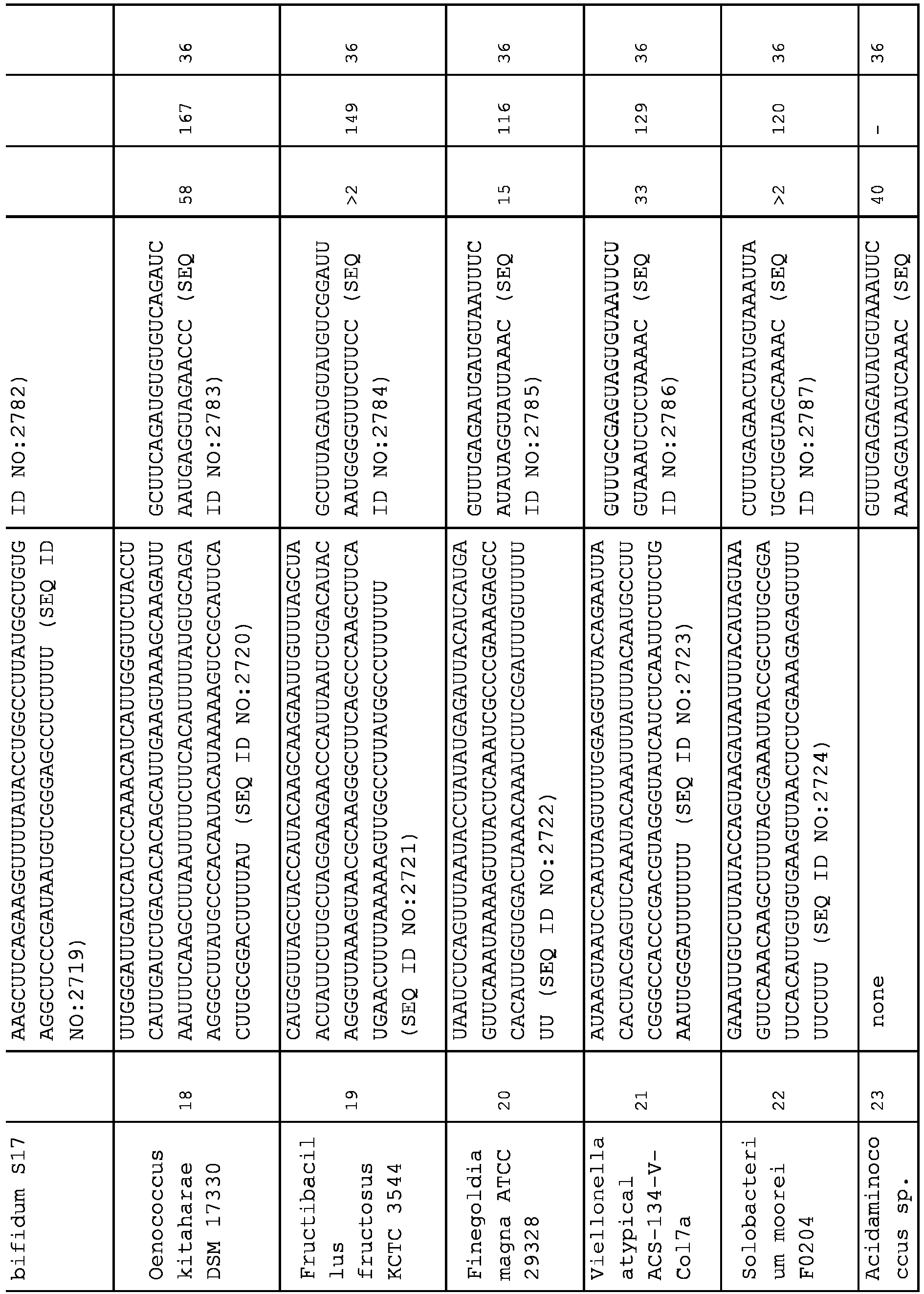

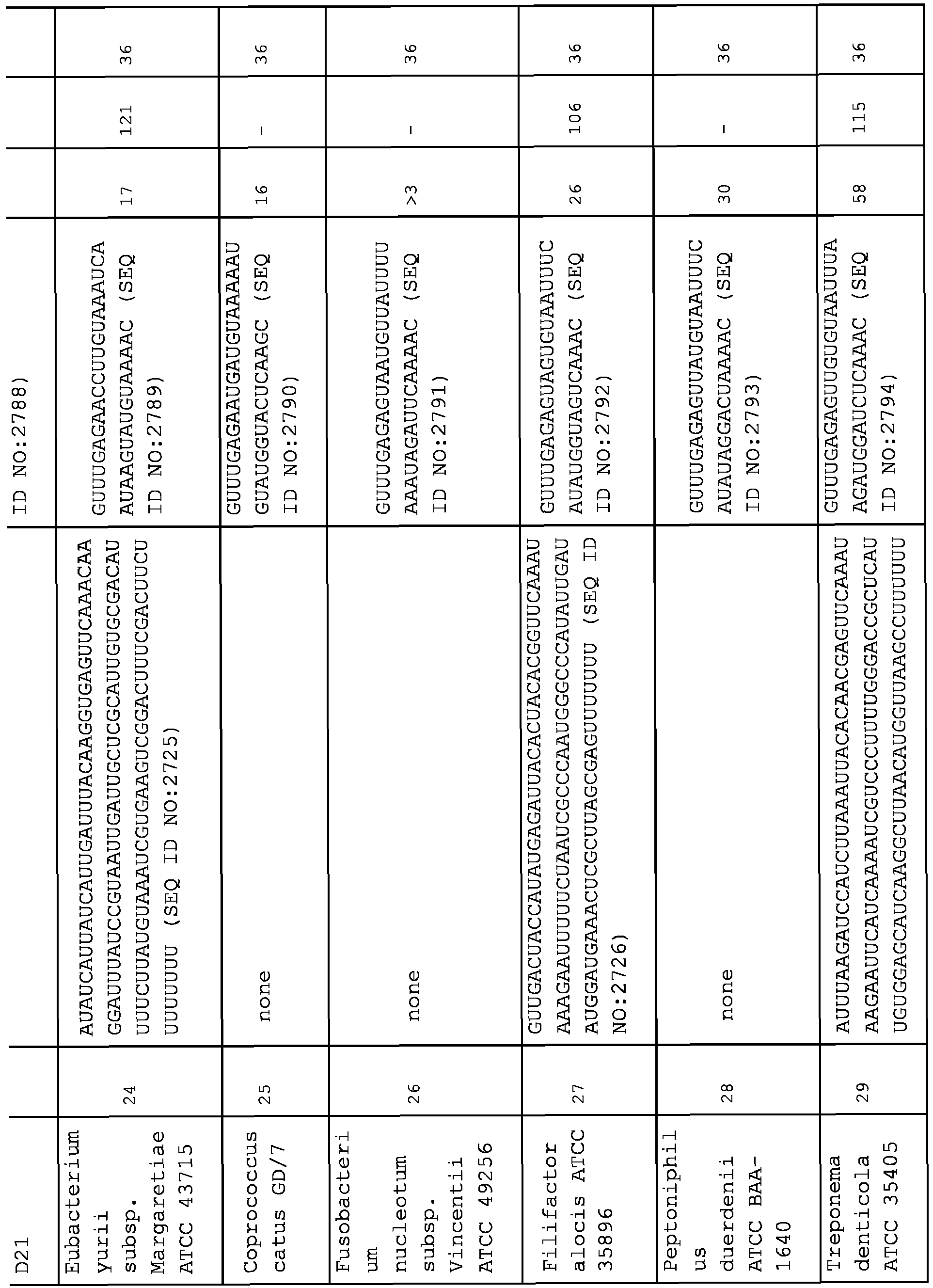

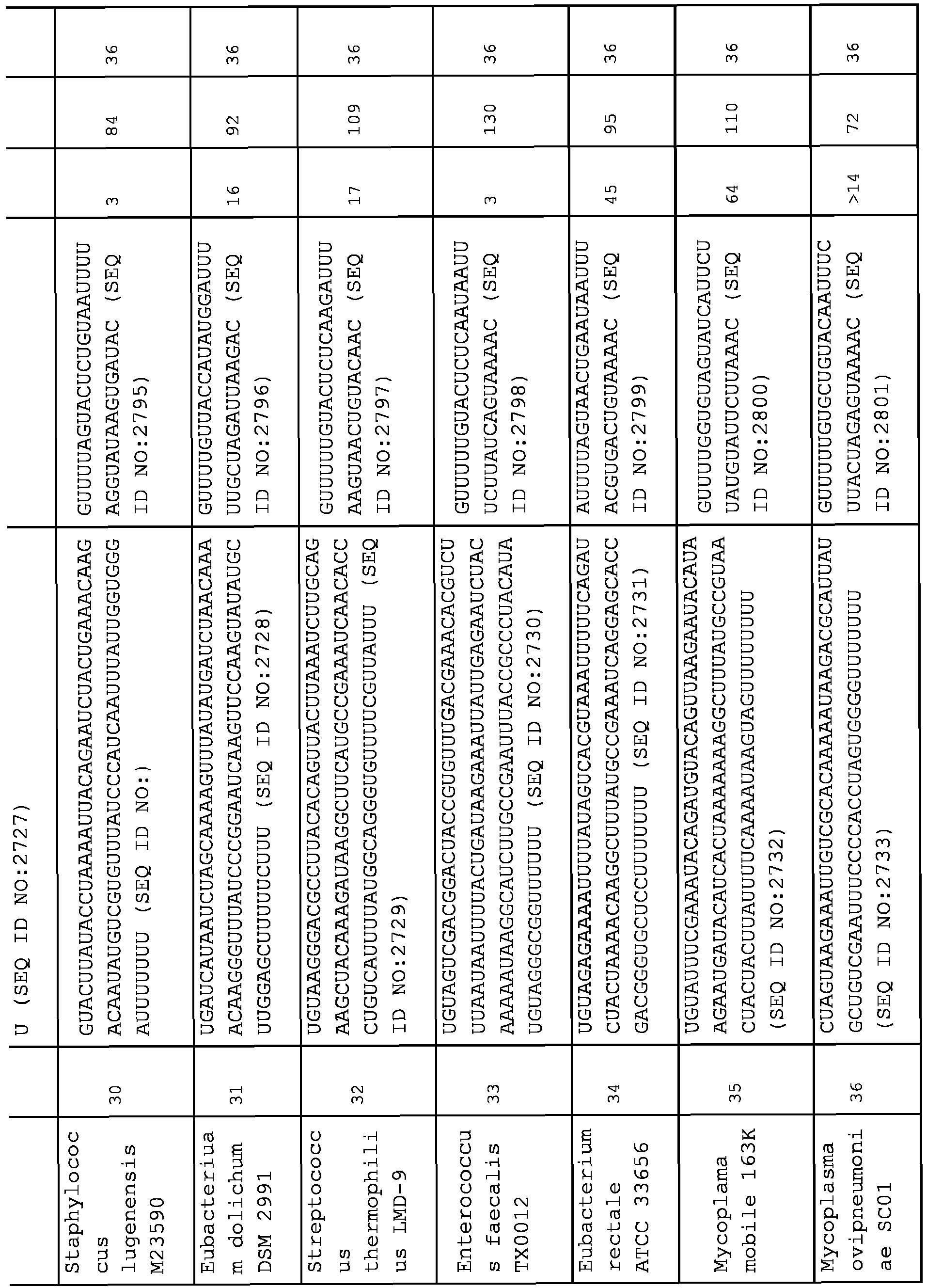

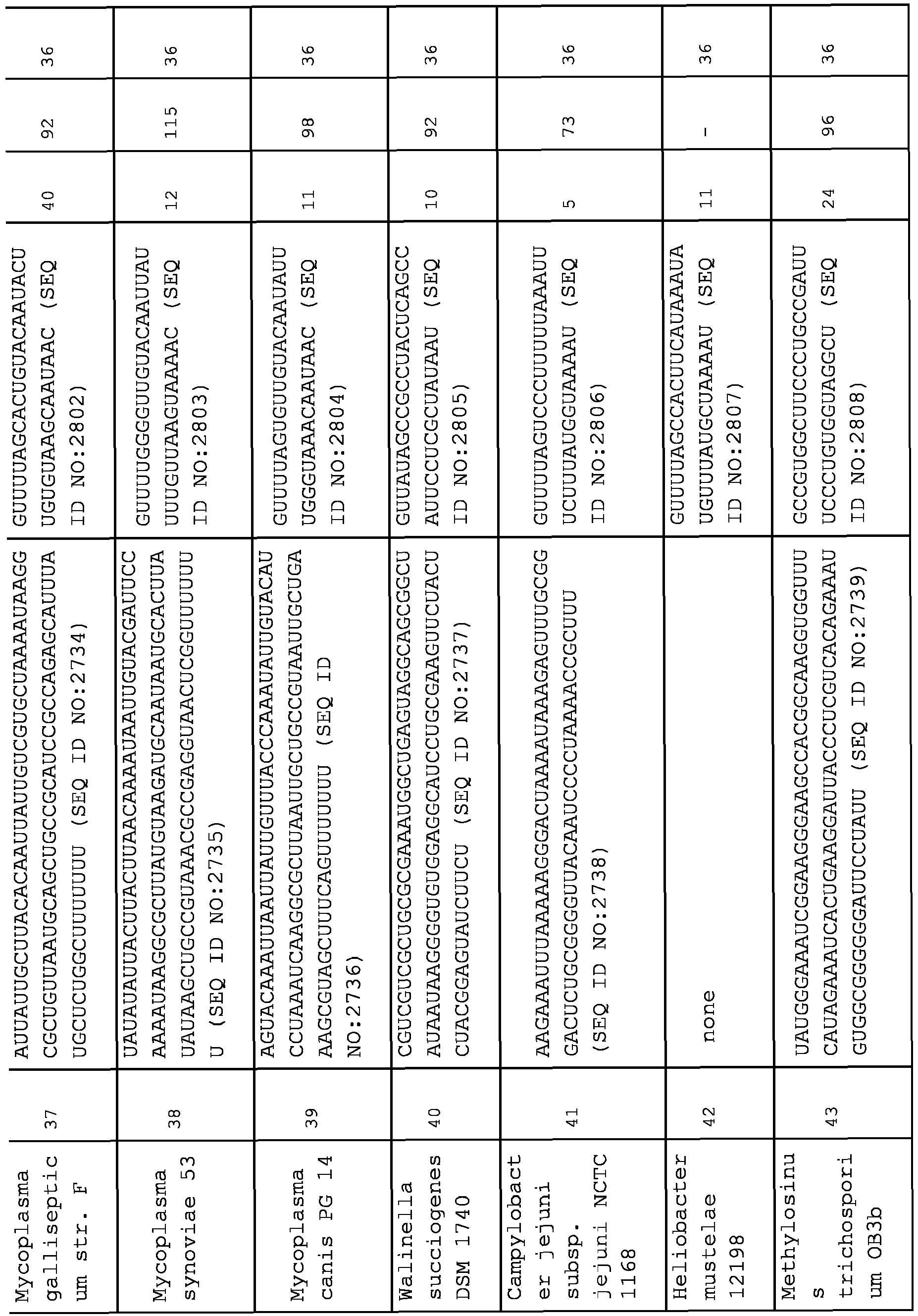

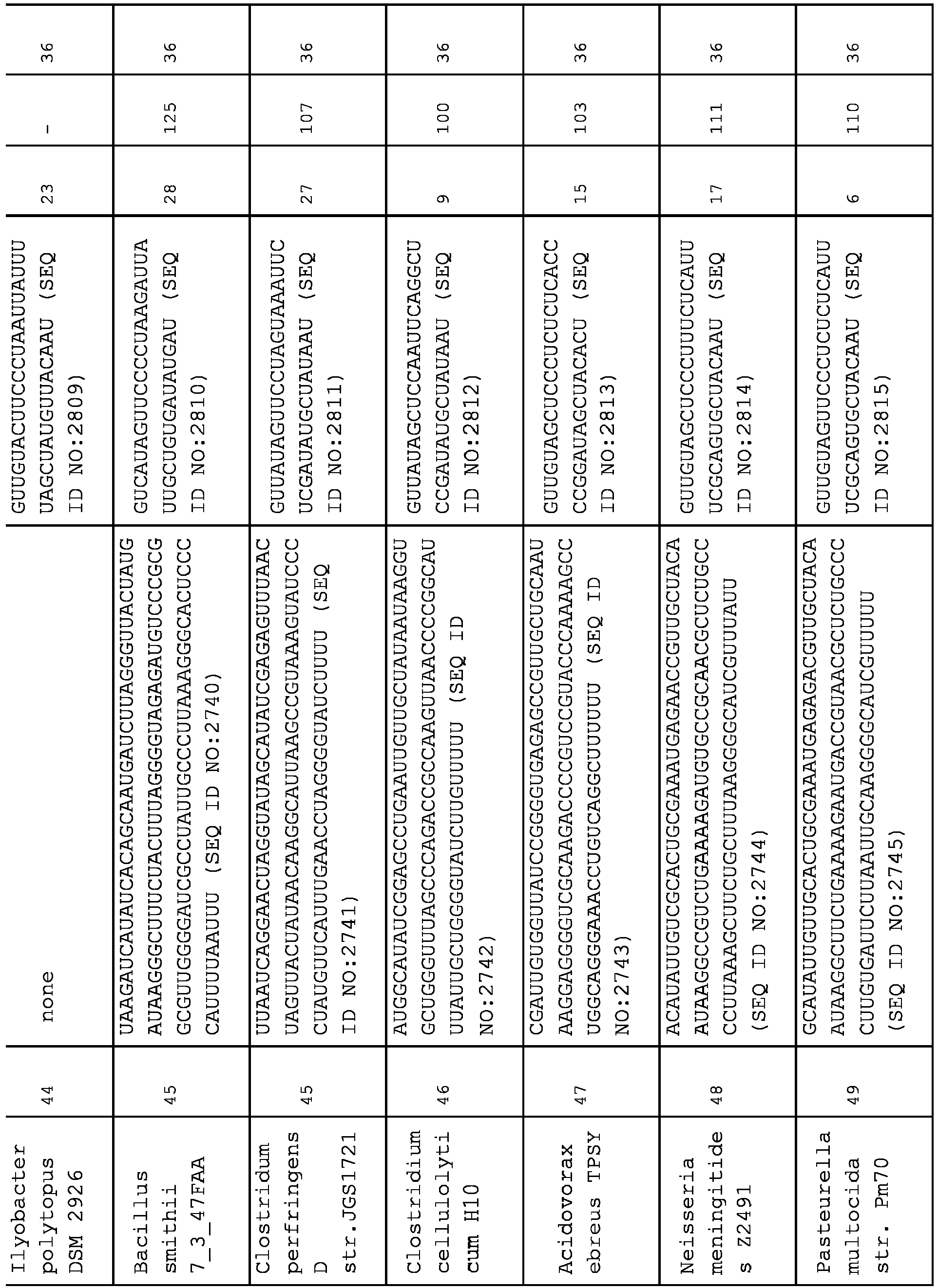

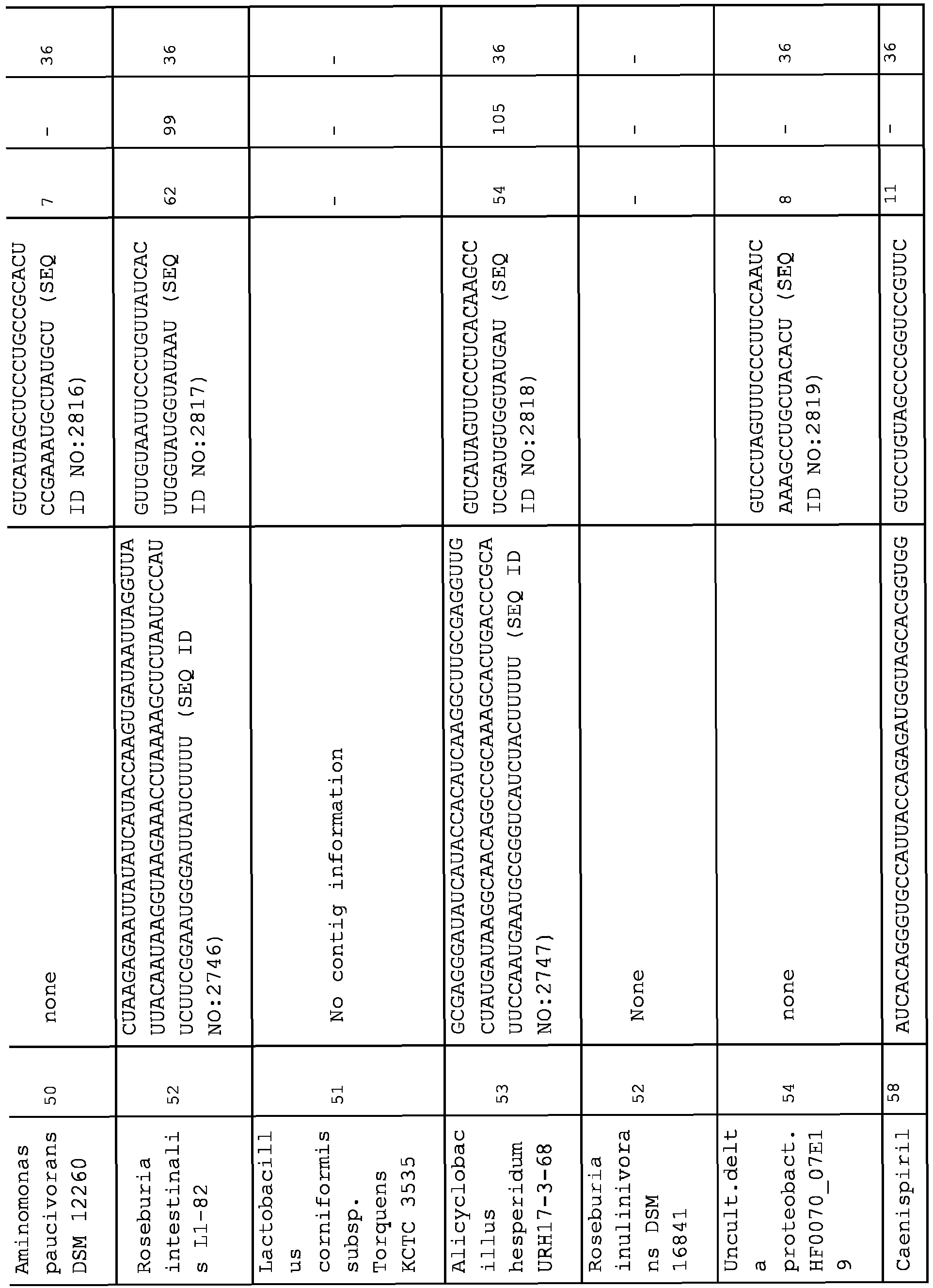

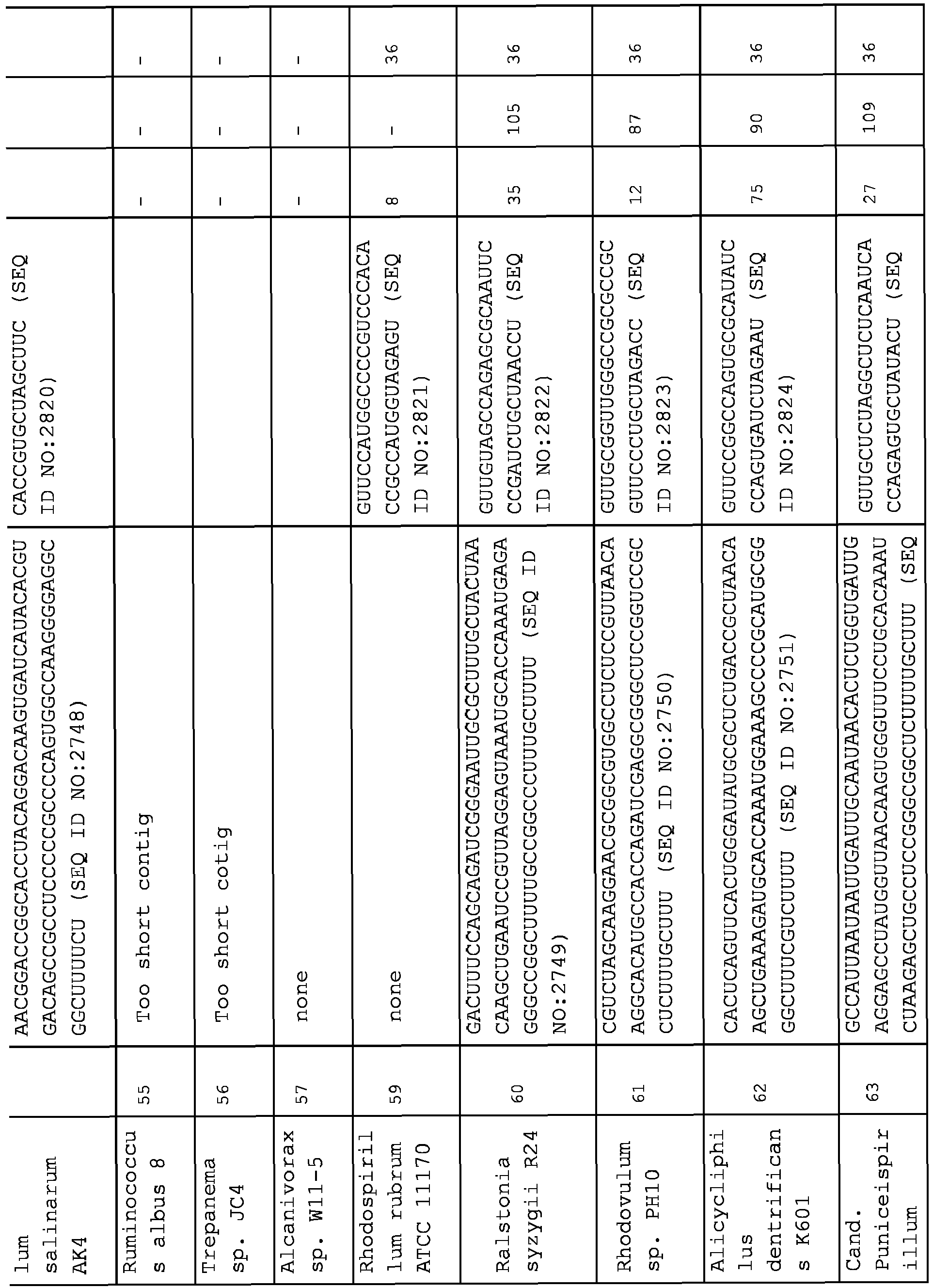

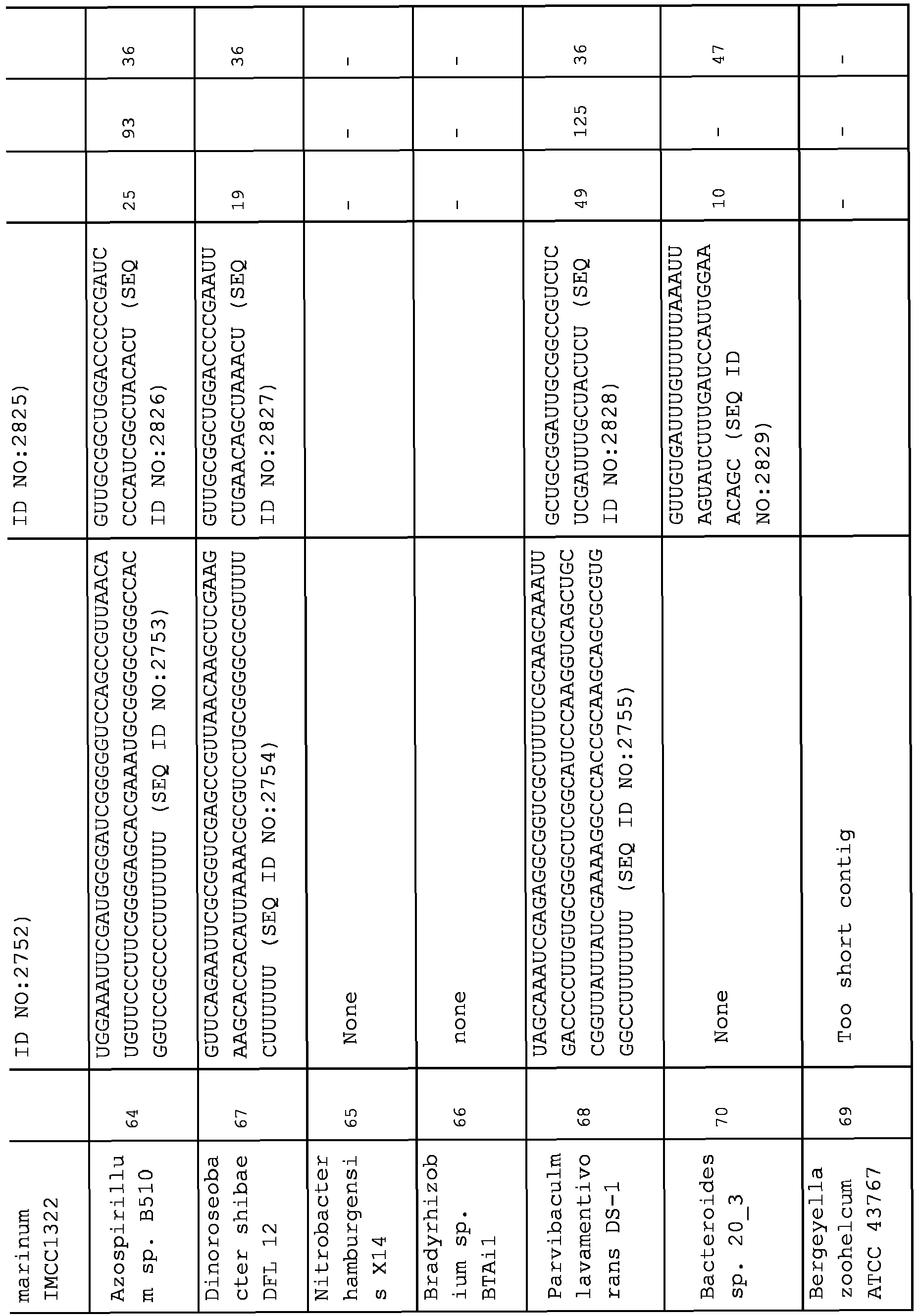

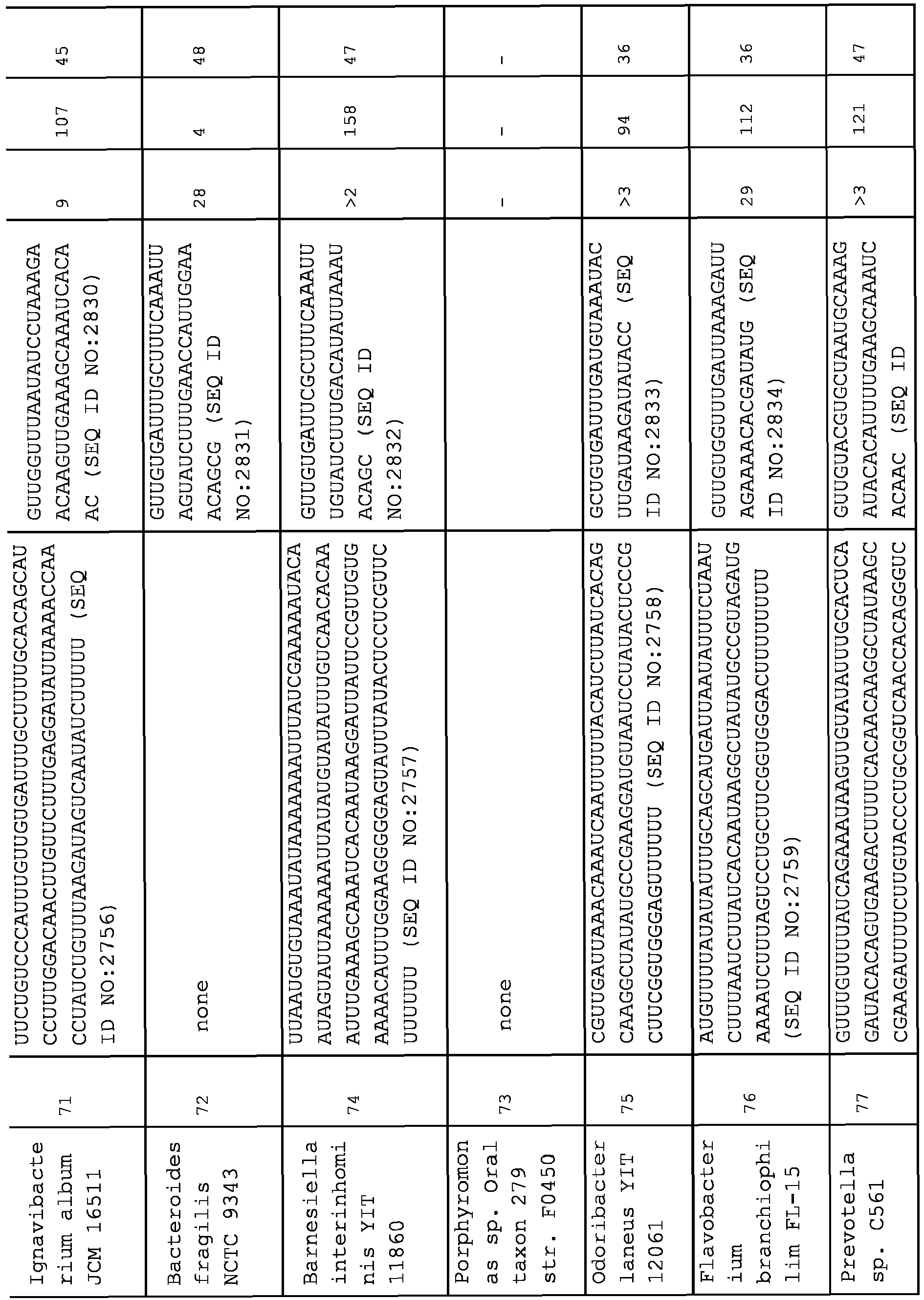

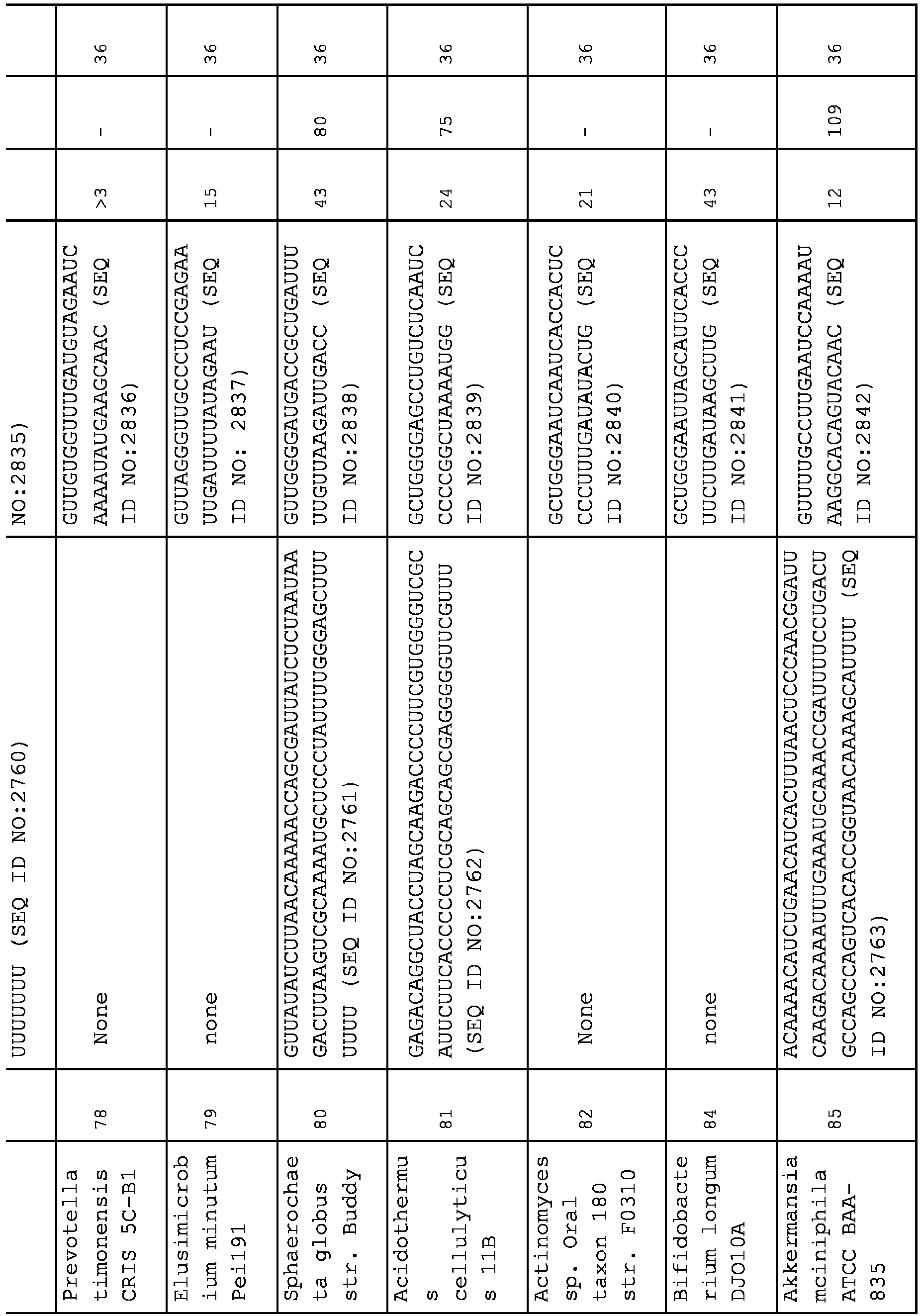

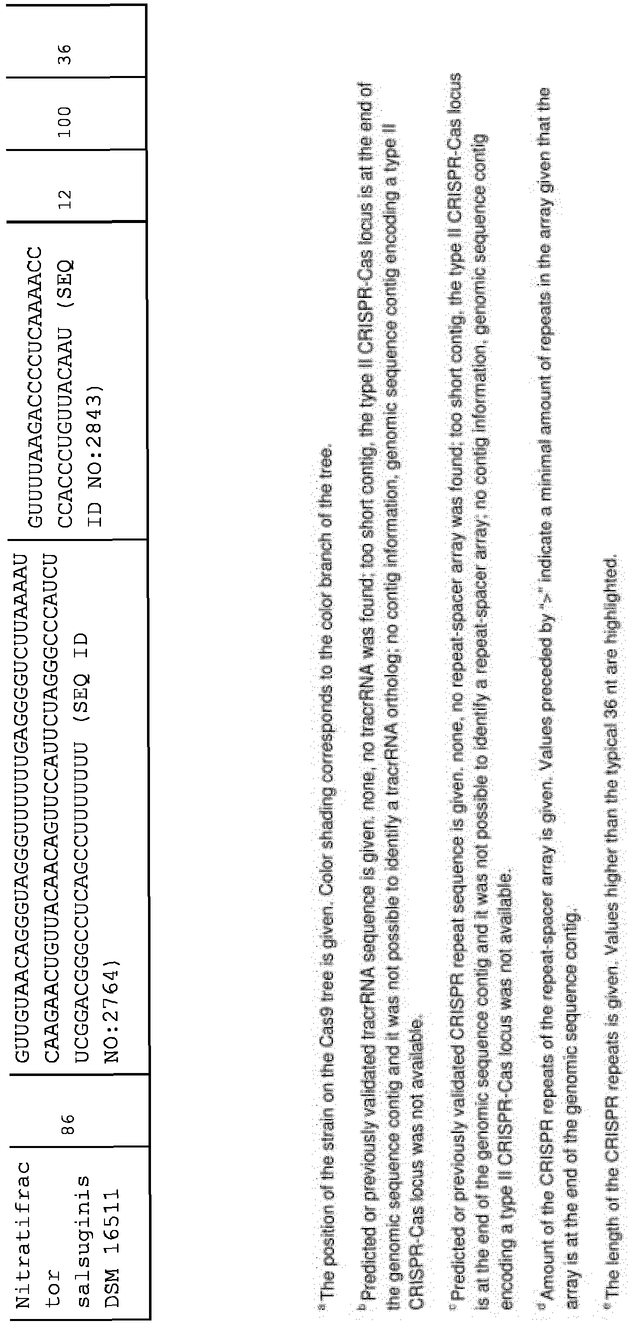

**Supplemental Table S6**

| **Strain** | **Cas9 GI** | **Cluster** |
|---|---|---|
| *Acidaminococcus intestini RyC-MR95* | 352684361 | 1 |
| *Acidaminococcus sp. D21* | 227824983 | 1 |
| *Anaerococcus tetradius ATCC 35098* | 227501312 | 1 |
| *Bifidobacterium bifidum S17* | 310286728 | 1 |
| *Catenibacterium mitsuokai DSM 15897* | 224543312 | 1 |
| *Coprococcus catus GD*/*7* | 291520705 | 1 |
| *Coriobacterium glomerans PW2* | 328956315 | 1 |
| *Dolosigranulum pigrum ATCC51524* | 375088882 | 1 |
| *Dorea longicatena DSM 13814* | 153855454 | 1 |
| *Eggerthella sp. YY7918* | 339445983 | 1 |
| *Enterococcus faecalis ATCC 29200* | 229548613 | 1 |
| *Enterococcus faecalis ATCC 4200* | 256617555 | 1 |
| *Enterococcus faecalis D6* | 257086028 | 1 |
| *Enterococcus faecalis E1Sol* | 257080914 | 1 |
| *Enterococcus faecalis OG1RF* | 384512368 | 1 |
| *Enterococcus faecalis TX0470* | 312900261 | 1 |
| *Enterococcus faecalis TX4244* | 422695652 | 1 |
| *Enterococcus faecium 1,141,733* | 257888853 | 1 |
| *Enterococcus faecium 1,231,408* | 257893735 | 1 |
| *Enterococcus faecium E1133* | 430847551 | 1 |
| *Enterococcus faecium E3083* | 431757680 | 1 |
| *Enterococcus faecium PC4.1* | 293379700 | 1 |
| *Enterococcus faecium TX1330* | 227550972 | 1 |
| *Enterococcus faecium TX1337RF* | 424765774 | 1 |
| *Enterococcus hirae ATCC 9790* | 392988474 | 1 |
| *Enterococcus italicus DSM 15952* | 315641599 | 1 |
| *Eubacterium sp. AS15* | 402309258 | 1 |
| *Eubacterium yurii subsp. margaretiae ATCC 43715* | 306821691 | 1 |
| *Filifactor alocis ATCC 35896* | 374307738 | 1 |
| *Finegoldia magna ACS-171-V-Col3* | 302380288 | 1 |
| *Finegoldia magna ATCC 29328* | 169823755 | 1 |
| *Finegoldia magna SY403409CC001050417* | 417926052 | 1 |
| *Fructobacillus fructosus KCTC 3544* | 339625081 | 1 |
| *Fusobacterium nucleatum subsp. vincentii ATCC 49256* | 34762592 | 1 |
| *Fusobacterium sp. 1_1_41FAA* | 294782278 | 1 |
| *Fusobacterium sp. 3_1_27* | 294785695 | 1 |
| *Fusobacterium sp. 3_1_36A2* | 256845019 | 1 |
| *Gemella haemolysans ATCC 10379* | 241889924 | 1 |
| *Gemella morbillorum M424* | 317495358 | 1 |
| *Gordonibacter pamelaeoe 7-10-1-b* | 295106015 | 1 |
| *Helcococcus kunzii ATCC 51366* | 375092427 | 1 |
| *Lactobacillus animalis KCTC 3501* | 335357451 | 1 |
| *Lactobacillus brevis subsp. gravesensis ATCC 27305* | 227509761 | 1 |
| *Lactobacillus buchneri CD034* | 406027703 | 1 |
| *Lactobacillus buchneri NRRL B-30929* | 331702228 | 1 |
| *Lactobacillus casei BL23* | 191639137 | 1 |
| *Lactobacillus casei Lc-10* | 418010298 | 1 |
| *Lactobacillus casei M36* | 417996992 | 1 |
| *Lactobacillus casei str. Zhang* | 301067199 | 1 |
| *Lactobacillus casei T71499* | 417999832 | 1 |
| *Lactobacillus casei UCD174* | 418002962 | 1 |
| *Lactobacillus casei W56* | 409997999 | 1 |
| *Lactobacillus coryniformis subsp. coryniformis KCTC 3167* | 333394446 | 1 |
| *Lactobacillus curvatus CRL 705* | 354808135 | 1 |
| *Lactobacillus farciminis KCTC 3681* | 336394882 | 1 |
| *Lactobacillus fermentum 28-3-CHN* | 260662220 | 1 |
| *Lactobacillus fermentum ATCC 14931* | 227514633 | 1 |
| *Lactobacillus florum 2F* | 408790128 | 1 |
| *Lactobacillus gasseri JV-V03* | 300361537 | 1 |
| *Lactobacillus hominis CRBIP 24.179* | 395244248 | 1 |
| *Lactobacillus iners LactinV 11V1-d* | 309803917 | 1 |
| *Lactobacillus jensenii 269-3* | 238854567 | 1 |
| *Lactobacillus jensenii 27-2-CHN* | 256852176 | 1 |
| *Lactobacillus johnsonii DPC 6026* | 385826041 | 1 |
| *Lactobacillus mucosae LM1* | 377831443 | 1 |
| *Lactobacillus paracasei subsp. paracasei 8700:2* | 239630053 | 1 |
| *Lactobacillus pentosus IG1* | 339637353 | 1 |
| *Lactobacillus pentosus KCA1* | 392947436 | 1 |
| *Lactobacillus pentosus MP-10* | 334881121 | 1 |
| *Lactobacillus plantarum ZI316* | 448819853 | 1 |
| *Lactobacillus rhamnosus GG* | 258509199 | 1 |
| *Lactobacillus rhamnosus HN001* | 199597394 | 1 |
| *Lactobacillus rhamnosus R0011* | 418072660 | 1 |
| *Lactobacillus ruminis ATCC 25644* | 323340068 | 1 |
| *Lactobacillus salivarius SMXD51* | 418960525 | 1 |
| *Lactobacillus sanfranciscensis TMW 1.1304* | 347534532 | 1 |
| *Lactobacillus sp. 66c* | 408410332 | 1 |
| *Lactobacillus versmoldensis KCTC3814* | 365906066 | 1 |
| *Leuconostoc gelidum KCTC 3527* | 333398273 | 1 |
| *Listeria innocua ATCC 33091* | 423101383 | 1 |
| *Listeria innocua Clip11262* | 16801805 | 1 |
| *Listeria innocua FSL S4-378* | 422414122 | 1 |
| *Listeria ivanovii FSL F6-596* | 315305353 | 1 |
| *Listeria monocytogenes 10403S* | 386044902 | 1 |
| *Listeria monocytogenes FSL J1-175* | 255520581 | 1 |
| *Listeria monocytogenes FSL J1-194* | 254825045 | 1 |
| *Listeria monocytogenes FSL J1-208* | 422810631 | 1 |
| *Listeria monocytogenes FSL N3-165* | 254829042 | 1 |
| *Listeria monocytogenes FSL R2-503* | 254854201 | 1 |
| *Listeria monocytogenes str. 1*/*2a F6854* | 47097148 | 1 |
| *Megasphaera sp. UPII 135-E* | 342218215 | 1 |
| *Oenococcus kitaharae DSM 17330* | 366983953 | 1 |
| *Oenococcus kitaharae DSM 17330* | 372325145 | 1 |
| *Olsenella uli DSM 7084* | 302336020 | 1 |
| *Pediococcus acidilactici DSM 20284* | 304386254 | 1 |
| *Pediococcus acidilactici MA18*/*5M* | 418068659 | 1 |
| *Peptoniphilus duerdenii ATCC BAA-1640* | 304438954 | 1 |
| *Planococcus antarcticus DSM 14505* | 389815359 | 1 |
| *Psychroflexus torquis ATCC 700755* | 408489713 | 1 |
| *Ruminococcus lactaris ATCC29176* | 197301447 | 1 |
| *Scardovia wiggsiae F0424* | 423349694 | 1 |
| *Solobacterium moorei F0204* | 320528778 | 1 |
| *Staphylococcus pseudintermedius ED99* | 323463801 | 1 |
| *Staphylococcus pseudintermedius ED99* | 386318630 | 1 |
| *Staphylococcus simulans ACS-120-V-Sch1* | 414160476 | 1 |
| *Streptococcus agalactiae 2603V*/*R* | 22537057 | 1 |
| *Streptococcus agalactiae 515* | 77413160 | 1 |
| *Streptococcus agalactiae A909* | 76788458 | 1 |
| *Streptococcus agalactiae ATCC 13813* | 339301617 | 1 |
| *Streptococcus agalactiae CJB111* | 77411010 | 1 |
| *Streptococcus agalactiae COH1* | 77407964 | 1 |
| *Streptococcus agalactiae FSL S3-026* | 417005168 | 1 |
| *Streptococcus agalactiae GB00112* | 421147428 | 1 |
| *Streptococcus agalactiae H36B* | 77405721 | 1 |
| *Streptococcus agalactiae NEM316* | 25010965 | 1 |
| *Streptococcus agalactiae SA20-06* | 410594450 | 1 |
| *Streptococcus agalactiae STIR-CD-17* | 421532069 | 1 |
| *Streptococcus anginosus F0211* | 315223162 | 1 |
| *Streptococcus anginosus SK1138* | 421490579 | 1 |
| *Streptococcus anginosus SK52* = *DSM 20563* | 335031483 | 1 |
| *Streptococcus bovis ATCC 700338* | 306833855 | 1 |
| *Streptococcus canis FSL Z3-227* | 392329410 | 1 |
| *Streptococcus constellatus subsp. constellatus SK53* | 418965022 | 1 |
| *Streptococcus dysgalactiae subsp. equisimilis AC-2713* | 410494913 | 1 |
| *Streptococcus dysgalactiae subsp. equisimilis ATCC 12394* | 386317166 | 1 |
| *Streptococcus dysgalactiae subsp. equisimilis GGS_124* | 251782637 | 1 |
| *Streptococcus dysgalactiae subsp. equisimilis RE378* | 408401787 | 1 |
| *Streptococcus equi subsp. zooepidemicus MGCS10565* | 195978435 | 1 |
| *Streptococcus equinus ATCC 9812* | 320547102 | 1 |
| *Streptococcus gallolyticus subsp. gallolyticus ATCC BAA-2069* | 325978669 | 1 |
| *Streptococcus gallolyticus subsp. gallolyticus TX20005* | 306831733 | 1 |
| *Streptococcus gallolyticus UCN34* | 288905639 | 1 |
| *Streptococcus infantarius subsp. infantarius G18* | 379705580 | 1 |
| *Streptococcus iniae 9117* | 406658208 | 1 |
| *Streptococcus macacae NCTC 11558* | 357636406 | 1 |
| *Streptococcus mitis SK321* | 307710946 | 1 |
| *Streptococcus mutans 11SSST2* | 449165720 | 1 |
| *Streptococcus mutans 11SSST2* | 449951835 | 1 |
| *Streptococcus mutans 11VS1* | 449976542 | 1 |
| *Streptococcus mutans 14D* | 450149988 | 1 |
| *Streptococcus mutans 15VF2* | 449170557 | 1 |
| *Streptococcus mutans 15VF2* | 449965974 | 1 |
| *Streptococcus mutans 1SM1* | 449158457 | 1 |
| *Streptococcus mutans 1SM1* | 449920643 | 1 |
| *Streptococcus mutans 24* | 449247589 | 1 |
| *Streptococcus mutans 24* | 450180942 | 1 |
| *Streptococcus mutans 2VS1* | 449174812 | 1 |
| *Streptococcus mutans 2VS1* | 449968746 | 1 |
| *Streptococcus mutans 3SN1* | 449162653 | 1 |
| *Streptococcus mutans 3SN1* | 449931425 | 1 |
| *Streptococcus mutans 4SM1* | 449159838 | 1 |
| *Streptococcus mutans 4SM1* | 449927152 | 1 |
| *Streptococcus mutans 4VF1* | 449167132 | 1 |
| *Streptococcus mutans 4VF1* | 449961027 | 1 |
| *Streptococcus mutans 5SM3* | 449176693 | 1 |
| *Streptococcus mutans 5SM3* | 449980571 | 1 |
| *Streptococcus mutans 66-2A* | 449240165 | 1 |
| *Streptococcus mutans 66-2A* | 450160342 | 1 |
| *Streptococcus mutans 8ID3* | 449154769 | 1 |
| *Streptococcus mutans 8ID3* | 449872064 | 1 |
| *Streptococcus mutans A19* | 449187668 | 1 |
| *Streptococcus mutans A19* | 450013175 | 1 |
| *Streptococcus mutans B* | 450166294 | 1 |
| *Streptococcus mutans G123* | 450029806 | 1 |
| *Streptococcus mutans GS-5* | 397650022 | 1 |
| *Streptococcus mutans LI23* | 387785882 | 1 |
| *Streptococcus mutans M21* | 449194333 | 1 |
| *Streptococcus mutans M21* | 450036249 | 1 |
| *Streptococcus mutans M230* | 449260994 | 1 |
| *Streptococcus mutans M230* | 449903532 | 1 |
| *Streptococcus mutans M2A* | 449209586 | 1 |
| *Streptococcus mutans M2A* | 450074072 | 1 |
| *Streptococcus mutans N29* | 449182997 | 1 |
| *Streptococcus mutans N29* | 450003067 | 1 |
| *Streptococcus mutans N3209* | 449210660 | 1 |
| *Streptococcus mutans N3209* | 450077860 | 1 |
| *Streptococcus mutans N66* | 449212466 | 1 |
| *Streptococcus mutans N66* | 450083993 | 1 |
| *Streptococcus mutans NFSM1* | 449202104 | 1 |
| *Streptococcus mutans NFSM1* | 450051112 | 1 |
| *Streptococcus mutans NLML1* | 450140393 | 1 |
| *Streptococcus mutans NLML4* | 449202681 | 1 |
| *Streptococcus mutans NLML4* | 450059882 | 1 |
| *Streptococcus mutans NLML9* | 449209148 | 1 |
| *Streptococcus mutans NLML9* | 450066176 | 1 |
| *Streptococcus mutans NMT4863* | 449186850 | 1 |
| *Streptococcus mutans NMT4863* | 450007078 | 1 |
| *Streptococcus mutans NN2025* | 290580220 | 1 |
| *Streptococcus mutans NV1996* | 450086338 | 1 |
| *Streptococcus mutans NVAB* | 449181424 | 1 |
| *Streptococcus mutans NVAB* | 449990810 | 1 |
| *Streptococcus mutans R221* | 449258042 | 1 |
| *Streptococcus mutans R221* | 449899675 | 1 |
| *Streptococcus mutans S1B* | 449251227 | 1 |
| *Streptococcus mutans S1B* | 449877120 | 1 |
| *Streptococcus mutans SF1* | 450098705 | 1 |
| *Streptococcus mutans SF14* | 449221374 | 1 |
| *Streptococcus mutans SF14* | 450107816 | 1 |
| *Streptococcus mutans SM1* | 449245264 | 1 |
| *Streptococcus mutans SM1* | 450176410 | 1 |
| *Streptococcus mutans SM4* | 449246010 | 1 |
| *Streptococcus mutans SM4* | 450170248 | 1 |
| *Streptococcus mutans SM6* | 449223000 | 1 |
| *Streptococcus mutans SM6* | 450112022 | 1 |
| *Streptococcus mutans ST6* | 449227252 | 1 |
| *Streptococcus mutans ST6* | 450123011 | 1 |
| *Streptococcus mutans UA159* | 24379809 | 1 |
| *Streptococcus mutans W6* | 450094364 | 1 |
| *Streptococcus oralis SK304* | 421488030 | 1 |
| *Streptococcus aralis SK610* | 419782534 | 1 |
| *Streptococcus pseudoporcinus LQ 940-04* | 416852857 | 1 |
| *Streptococcus pyogenes M1* | 13622193 | 1 |
| *Streptococcus pyogenes MGAS10750* | 94543903 | 1 |
| *Streptococcus pyogenes MGA515252* | 94994317 | 1 |
| *Streptococcus pyogenes MGAS2096* | 383479946 | 1 |
| *Streptococcus pyogenes MGAS315* | 94992340 | 1 |
| *Streptococcus pyogenes MGAS5005* | 21910213 | 1 |
| *Streptococcus pyogenes MGAS6180* | 71910582 | 1 |
| *Streptococcus pyogenes MGAS9429* | 71903413 | 1 |
| *Streptococcus pyogenes NZ131* | 94988516 | 1 |
| *Streptococcus pyogenes SSI-1* | 28896088 | 1 |
| *Streptococcus ratti FA-1 = DSM 20564* | 400290495 | 1 |
| *Streptococcus salivarius K12* | 421452908 | 1 |
| *Streptococcus sanguinis SK115* | 422848603 | 1 |
| *Streptococcus sanguinis SK330* | 422860049 | 1 |
| *Streptococcus sanguinis SK353* | 422821159 | 1 |
| *Streptococcus sanguinis SK49* | 422884106 | 1 |
| *Streptococcus sp. C300* | 322375978 | 1 |
| *Streptococcus sp. F0441* | 414157437 | 1 |
| *Streptococcus sp. M334* | 322378004 | 1 |
| *Streptococcus sp. oral taxon* 56 *str. F0418* | 339640839 | 1 |
| *Streptococcus sp. oral taxon 71 str. 73H25AP* | 306826314 | 1 |
| *Streptococcus suis ST1* | 389856936 | 1 |
| *Streptococcus thermophilus* | 343794781 | 1 |
| *Streptococcus thermophilus LMD-9* | 116628213 | 1 |
| *Streptococcus thermophilus MN-ZLW-002* | 387910220 | 1 |
| *Streptococcus thermophilus ND03* | 386087120 | 1 |
| *Treponema denticola AL-2* | 449103686 | 1 |
| *Treponema denticola ASLM* | 449106292 | 1 |
| *Treponema denticola ATCC 35405* | 42525843 | 1 |
| *Treponema denticola H1-T* | 449118593 | 1 |
| *Treponema denticola H-22* | 449117322 | 1 |
| *Treponema denticola OTK* | 449125136 | 1 |
| *Treponema denticola SP37* | 449130155 | 1 |
| *Veillonella atypica ACS-134-V-Col7a* | 303229466 | 1 |
| *Veillonella parvula ATCC 17745* | 282849530 | 1 |
| *Veillonella sp. 6_1_27* | 294792465 | 1 |
| *Veillonella sp. oral taxon 780 str. F0422* | 342213964 | 1 |
| *Streptococcus pyogenes SF370 (M1 GAS)* | 209559356 | 1 |
| *Streptococcus pyogenes MGAS10270* | 56808315 | 1 |
| *Acidovorax ebreus TPSY* | 222109285 | 2 |
| *Actinobacillus minor NM305* | 240949037 | 2 |
| *Actinobacillus pleuropneumoniae serovar 10 str. D13039* | 307256472 | 2 |
| *Actinobacillus succinogenes 130Z* | 152978060 | 2 |
| *Actinobacillus suis H91-0380* | 407692091 | 2 |
| *Alicyclobacillus hesperidum URH173-68* | 403744858 | 2 |
| *Aminomonas paucivorans DSM 12260* | 312879015 | 2 |
| *Bacillus cereus BAG4X12-1* | 423439645 | 2 |
| *Bacillus cereus BAG4X2-1* | 423445130 | 2 |
| *Bacillus cereus Rockl-15* | 229113166 | 2 |
| *Bacillus smithii 7_3_47FAA* | 365156657 | 2 |
| *Bacillus thuringiensis serovar finitimus YBT-020* | 384183447 | 2 |
| *Bacteroides sp. 3_1_33FAA* | 265750948 | 2 |
| *Brevibacillus laterosporus GI-9* | 421874297 | 2 |
| *Campylobacter coli 1098* | 419564797 | 2 |
| *Campylobacter coli 111-3* | 419536531 | 2 |
| *Campylobacter coli 132-6* | 419572019 | 2 |
| *Campylobacter coli 151-9* | 419603415 | 2 |
| *Campylobacter coli 1909* | 419576091 | 2 |
| *Campylobacter coli 1957* | 419581876 | 2 |
| *Campylobacter coli 2692* | 419553162 | 2 |
| *Campylobacter coli 59-2* | 419578074 | 2 |
| *Campylobacter coli 67-8* | 419587721 | 2 |
| *Campylobacter coli 80352* | 419559505 | 2 |
| *Campylobacter coli 80352* | 419558307 | 2 |
| *Campylobacter jejuni subsp. doylei 269.97* | 153952471 | 2 |
| *Campylobacter jejuni subsp. jejuni 110-21* | 419676124 | 2 |
| *Campylobacter jejuni subsp. jejuni 129-258* | 419619138 | 2 |
| *Campylobacter jejuni subsp. jejuni 1336* | 283956897 | 2 |
| *Campylobacter jejuni subsp. jejuni 140-16* | 419681578 | 2 |
| *Campylobacter jejuni subsp. jejuni 1577* | 419685099 | 2 |
| *Campylobacter jejuni subsp. jejuni 1854* | 419689467 | 2 |
| *Campylobacter jejuni subsp. jejuni 1997-10* | 419666522 | 2 |
| *Campylobacter jejuni subsp. jejuni 2008-1025* | 419650041 | 2 |
| *Campylobacter jejuni subsp. jejuni 2008-872* | 419654778 | 2 |
| *Campylobacter jejuni subsp. jejuni 2008-979* | 419660762 | 2 |
| *Campylobacter jejuni subsp. jejuni 2008-988* | 419655317 | 2 |
| *Campylobacter jejuni subsp. jejuni 2008-988* | 419656328 | 2 |
| *Campylobacter jejuni subsp. jejuni 260.94* | 86152042 | 2 |
| *Campylobacter jejuni subsp. jejuni 414* | 283953849 | 2 |
| *Campylobacter jejuni subsp. jejuni 51037* | 419674189 | 2 |
| *Campylobacter jejuni subsp. jejuni 51494* | 419619463 | 2 |
| *Campylobacter jejuni subsp. jejuni 53161* | 419647275 | 2 |
| *Campylobacter jejuni subsp. jejuni 60004* | 419629136 | 2 |
| *Campylobacter jejuni subsp. jejuni 81116* | 157415744 | 2 |
| *Campylobacter jejuni subsp. jejuni 84-25* | 88596565 | 2 |
| *Campylobacter jejuni subsp. jejuni 87459* | 419680124 | 2 |
| *Campylobacter jejuni subsp. jejuni ATCC 33560* | 419643715 | 2 |
| *Campylobacter jejuni subsp. jejuni CF93-6* | 86149266 | 2 |
| *Campylobacter jejuni subsp. jejuni CG8486* | 148925683 | 2 |
| *Campylobacter jejuni subsp. jejuni HB93-13* | 86152450 | 2 |
| *Campylobacter jejuni subsp. jejuni LMG 23210* | 419696801 | 2 |
| *Campylobacter jejuni subsp. jejuni LMG 23211* | 419697443 | 2 |
| *Campylobacter jejuni subsp. jejuni LMG 23263* | 419628620 | 2 |
| *Campylobacter jejuni subsp. jejuni LMG* 23264 | 419632476 | 2 |
| *Campylobacter jejuni subsp. jejuni LMG 23269* | 419634246 | 2 |
| *Campylobacter jejuni subsp. jejuni LMG 23357* | 419641132 | 2 |
| *Campylobacter jejuni subsp. jejuni NCTC 11168* | 218563121 | 2 |
| *Campylobacter jejuni subsp. jejuni NW* | 424845990 | 2 |
| *Campylobacter jejuni subsp. jejuni PT14* | 407942868 | 2 |
| *Campylobacter lari* | 345468028 | 2 |
| *Clostridium cellulolyticum H10* | 220930482 | 2 |
| *Clostridium perfringens Cstr. JGS1495* | 169343975 | 2 |
| *Clostridium perfringens D str. JGS1721* | 182624245 | 2 |
| *Haemophilus parainfluenzae ATCC 33392* | 325578067 | 2 |
| *Haemophilus parainfluenzae CCUG* 13788 | 359298684 | 2 |
| *Haemophilus parainfluenzae T3T1* | 345430422 | 2 |
| *Haemophilus sputorum HK 2154* | 402304649 | 2 |
| *Helicobacter canadensis MIT 98-5491* | 253828136 | 2 |
| *Helicobacter cinaedi ATCC BAA-847* | 396079277 | 2 |
| *Helicobacter cinaedi CCUG* 18818 | 313144862 | 2 |
| *Helicobacter cinaedi PAGU611* | 386762035 | 2 |
| *Helicobacter mustelae 12198* | 291276265 | 2 |
| *Ilyobacter polytropus DSM 2926* | 310780384 | 2 |
| *Kingella kingae PYKK081* | 381401699 | 2 |
| *Lactobacillus coryniformis subsp. torquens KCTC 3535* | 336393381 | 2 |
| *Neisseria bacilliformis ATCC BAA-1200* | 329117879 | 2 |
| *Neisseria cinerea ATCC 14685* | 261378287 | 2 |
| *Neisseria flavescens SK114* | 241759613 | 2 |
| *Neisseria lactamica 020-06* | 313669044 | 2 |
| *Neisseria meningitidis 053442* | 161869390 | 2 |
| *Neisseria meningitidis 2007056* | 433531983 | 2 |
| *Neisseria meningitidis 63049* | 433514137 | 2 |
| *Neisseria meningitidis 8013* | 385324780 | 2 |
| *Neisseria meningitidis 92045* | 421559784 | 2 |
| *Neisseria meningitidis 93003* | 421538794 | 2 |
| *Neisseria meningitidis 93004* | 421541126 | 2 |
| *Neisseria meningitidis 96023* | 433518260 | 2 |
| *Neisseria meningitidis 98008* | 421555531 | 2 |
| *Neisseria meningitidis alpha14* | 254804356 | 2 |
| *Neisseria meningitidis alpha275* | 254672046 | 2 |
| *Neisseria meningitidis ATCC 13091* | 304388355 | 2 |
| *Neisseria meningitidis N1568* | 416164244 | 2 |
| *Neisseria meningitidis NM140* | 421545139 | 2 |
| *Neisseria meningitidis NM220* | 418291220 | 2 |
| *Neisseria meningitidis NM233* | 418288950 | 2 |
| *Neisseria meningitidis WUE 2594* | 385337435 | 2 |
| *Neisseria meningitidis Z2491* | 218767588 | 2 |
| *Neisseria sp. oral taxon 14 str. F0314* | 298369677 | 2 |
| *Neisseria wadsworthii 9715* | 350570326 | 2 |
| *Pasteurella multocida subsp. gallicida X73* | 425063822 | 2 |
| *Pasteurella multocida subsp. multocida str. P52VAC* | 421263876 | 2 |
| *Pasteurella multocida subsp. multocida str. Pm70* | 15602992 | 2 |
| *Phascolarctobacterium succinatutens YIT 12067* | 323142435 | 2 |
| *Roseburia intestinalis L1-82* | 257413184 | 2 |
| *Roseburia intestinalis M50*/*1* | 291537230 | 2 |
| *Roseburia inulinivorans DSM 16841* | 225377804 | 2 |
| *Simonsiella muelleri ATCC 29453* | 404379108 | 2 |
| *Sporalactobacillus vineae DSM 21990 = SL153* | 404330915 | 2 |
| *Subdoligranulum sp. 4_3_54A2FAA* | 365132400 | 2 |
| *Wolinella succinogenes DSM 1740* | 34557790 | 2 |
| *Catellicaccus marimammalium M35*/*04*/*3* | 424780480 | 3 |
| *Clostridium spiroforme DSM 1552* | 169349750 | 3 |
| *Enterococcus faecalis Flyl* | 257084992 | 3 |
| *Enterococcus faecalis R508* | 424761124 | 3 |
| *Enterococcus faecalis T11* | 257419486 | 3 |
| *Enterococcus faecalis TX0012* | 315149830 | 3 |
| *Enterococcus faecalis TX0012* | 422729710 | 3 |
| *Enterococcus faecalis TX1342* | 422701955 | 3 |
| *Eubacterium dolichum DSM 3991* | 160915782 | 3 |
| *Eubacterium rectale ATCC* 33656 | 238924075 | 3 |
| *Eubacterium ventriosum ATCC 27560* | 154482474 | 3 |
| *Facklamia hominis CCUG 36813* | 406671118 | 3 |
| *Lactobacillus farciminis KCTC 3681* | 336394701 | 3 |
| *Listeriaceae bacterium TTU M1-001* | 381184145 | 3 |
| *Staphylococcus aureus subsp. aureus* | 403411236 | 3 |
| *Staphylococcus lugdunensis M23590* | 315659848 | 3 |
| *Streptococcus anginosus 1_2_62CV* | 319939170 | 3 |
| *Streptococcus gallolyticus UCN34* | 288905632 | 3 |
| *Streptococcus gordonii str. Challis substr. CH1* | 157150687 | 3 |
| *Streptococcus infantarius ATCC BAA-102* | 171779984 | 3 |
| *Streptococcus macedonicus ACA-DC 198* | 374338350 | 3 |
| *Streptococcus mitis ATCC 6249* | 306829274 | 3 |
| *Streptococcus mutans NLML5* | 449203378 | 3 |
| *Streptococcus mutans NLML5* | 450064617 | 3 |
| *Streptococcus mutans NLML8* | 449151037 | 3 |
| *Streptococcus mutans NLML8* | 450133520 | 3 |
| *Streptococcus mutans ST1* | 449228751 | 3 |
| *Streptococcus mutans ST1* | 450114718 | 3 |
| *Streptococcus mutans U2A* | 449232458 | 3 |
| *Streptococcus mutans U2A* | 450125471 | 3 |
| *Streptococcus oralis SK1074* | 418974877 | 3 |
| *Streptococcus oralis SK313* | 417940002 | 3 |
| *Streptococcus parasanguinis F0449* | 419799964 | 3 |
| *Streptococcus pasteurianus ATCC 43144* | 336064611 | 3 |
| *Streptococcus salivarius JIM8777* | 387783792 | 3 |
| *Streptococcus salivarius PS4* | 419707401 | 3 |
| *Streptococcus sp. BS35b* | 401684660 | 3 |
| *Streptococcus sp. C150* | 322372617 | 3 |
| *Streptococcus sp. GMD6S* | 406576934 | 3 |
| *Streptococcus suis 89*/*1591* | 223932525 | 3 |
| *Streptococcus suis D9* | 386584496 | 3 |
| *Streptococcus suis ST3* | 330833104 | 3 |
| *Streptococcus thermophilus CNRZ1066* | 55822627 | 3 |
| *Streptococcus thermophilus JIM 8232* | 386344353 | 3 |
| *Streptacoccus thermophilus LMD-9* | 116627542 | 3 |
| *Streptococcus thermophilus LMG 18311* | 55820735 | 3 |
| *Streptococcus thermophilus MN-ZLW-002* | 387909441 | 3 |
| *Streptococcus thermophilus MTCC 5460* | 445374534 | 3 |
| *Streptococcus thermophilus ND03* | 386086348 | 3 |
| *Streptococcus vestibularis ATCC 49124* | 322517104 | 3 |
| *Anaerophaga sp. HS1* | 371776944 | 4 |
| *Anaerophaga thermohalophila DSM 12881* | 346224232 | 4 |
| *Bacteroides coprophilus DSM 18228* | 224026357 | 4 |
| *Bacteroides coprosuis DSM 18011* | 333031006 | 4 |
| *Bacteroides dorei DSM 17855* | 212694363 | 4 |
| *Bacteroides eggerthii 1_2_48FAA* | 317474201 | 4 |
| *Bacteroides faecis 27-5* | 380696107 | 4 |
| *Bacteroides fluxus YIT 12057* | 329965125 | 4 |
| *Bacteroides nordii CL02T12C05* | 393788929 | 4 |
| *Bacteroides sp. 20_3* | 301311869 | 4 |
| *Bacteroides sp. D2* | 383115507 | 4 |
| *Bacteroides uniformis CL03T00C23* | 423303159 | 4 |
| *Bacteroides vulgatus CL09T03C04* | 423312075 | 4 |
| *Bergeyella zoohelcum ATCC 43767* | 423317190 | 4 |
| *Capnocytophaga gingivalis ATCC 33624* | 228473057 | 4 |
| *Capnocytophaga sp. CM59* | 402830627 | 4 |
| *Capnocytophaga sp. oral taxon 324 str. F0483* | 429756885 | 4 |
| *Capnocytophaga sp. oral taxon 326 str. F0382* | 429752492 | 4 |
| *Capnocytophaga sp. oral taxon 412 str. F0487* | 393778597 | 4 |
| *Chryseobacterium sp. CF314* | 399023756 | 4 |
| *Fibrobacter succinogenes subsp. succinogenes S85* | 261414553 | 4 |
| *Flavobacteriaceae bacterium S85* | 372210605 | 4 |
| *Flavobacterium columnare ATCC 49512* | 365960762 | 4 |
| *Fluviicola taffensis DSM 16823* | 327405121 | 4 |
| *Ignavibacterium album JCM 16511* | 385811609 | 4 |
| *Mucilaginibacter paludis DSM 18603* | 373954054 | 4 |
| *Myroides odoratus DSM 2801* | 374597806 | 4 |
| *Ornithobacterium rhinotracheale DSM 15997* | 392391493 | 4 |
| *Prevotella bivia JCVIHMP010* | 282858617 | 4 |
| *Prevotella buccae ATCC 33574* | 315607525 | 4 |
| *Prevotella nigrescens ATCC 33563* | 340351024 | 4 |
| *Prevotella sp. MSX73* | 402307189 | 4 |
| *Prevotella timonensis CRIS 5C-B1* | 282881485 | 4 |
| *Prevotella veroralis F0319* | 260592128 | 4 |
| *Sphingobacterium spiritivorum ATCC 33861* | 300771242 | 4 |
| *Weeksella virosa DSM 16922* | 325955459 | 4 |
| *Acidovorax avenae subsp. avenae ATCC 19860* | 326315085 | 5 |
| *Alicycliphilus denitrificans BC* | 319760940 | 5 |
| *Alicycliphilus denitrificans K601* | 330822845 | 5 |
| *Azospirillum sp. B510* | 288957741 | 5 |
| *Bradyrhizabium sp. BTAi1* | 148255343 | 5 |
| *Candidatus Puniceispirillum marinum IMCC1322* | 294086111 | 5 |
| *Dinoroseabacter shibae DFL 12* | 159042956 | 5 |
| gamma proteobacterium HdN1 | 304313029 | 5 |
| *Nitrobacter hamburgensis X14* | 92109262 | 5 |
| *Nitrosomonas sp. AL212* | 325983496 | 5 |
| *Ralstonia syzygii R24* | 344171927 | 5 |
| *Rhodovulum sp. PH10* | 402849997 | 5 |
| *Sphingobium sp. AP49* | 398385143 | 5 |
| *Sphingomonas sp. S17* | 332188827 | 5 |
| *Verminephrobacter eiseniae EF01-2* | 121608211 | 5 |
| *Bacteroides fragilis 638R* | 375360193 | 6 |
| *Bacteroides fragilis NCTC 9343* | 60683389 | 6 |
| *Bacteroides sp. 2_1_16* | 265767599 | 6 |
| *Bacteroides sp. 3_1_19* | 298377533 | 6 |
| *Bacteroides sp. D2* | 383110723 | 6 |
| *Bacteroidetes oral taxon 274 str. F0058* | 298373376 | 6 |
| *Barnesiella intestinihominis YIT 11860* | 404487228 | 6 |
| *Belliella baltica DSM 15883* | 390944707 | 6 |
| *Bergeyella zoohelcum CCUG 30536* | 406673990 | 6 |
| *Capnocytophaga canimorsus Cc5* | 340622236 | 6 |
| *Capnocytophaga ochracea DSM 7271* | 256819408 | 6 |
| *Capnocytophaga sp. oral taxon 329 str. F0087* | 332882466 | 6 |
| *Capnocytophaga sp. oral taxon 335 str. F0486* | 420149252 | 6 |
| *Capnocytophaga sp. oral taxon 380 str. F0488* | 429748017 | 6 |
| *Capnocytophaga sputigena Capno* | 213962376 | 6 |
| *Flavobacterium psychrophilum JIP02*/*86* | 150025575 | 6 |
| *Galbibacter sp. ck-l2-15* | 408370397 | 6 |
| *Indibacter alkaliphilus LW1* | 404451234 | 6 |
| *Joostella marina DSM 19592* | 386818981 | 6 |
| *Kordia algicida OT-1* | 163754820 | 6 |
| *Marinilabilia sp. AK2* | 410030899 | 6 |
| *Myroides injenensis M09-0166* | 399927444 | 6 |
| *Niabella soli DSM 19437* | 374372722 | 6 |
| *Parabacteroides johnsonii DSM 18315* | 218258638 | 6 |
| *Parabacteroides sp. D13* | 256840409 | 6 |
| *Porphyromonas sp. oral taxon 279 str. F0450* | 402847315 | 6 |
| *Prevotella histicola F0411* | 357042839 | 6 |
| *Prevotella intermedia 17* | 387132277 | 6 |
| *Prevotella nigrescens F0103* | 445119230 | 6 |
| *Prevotella oralis ATCC 33269* | 323344874 | 6 |
| *Prevotella sp. oral taxon 306 str. F0472* | 383811446 | 6 |
| *Riemerella anatipestifer RA-CH-1* | 407451859 | 6 |
| *Riemerella anatipestifer RA-GD* | 386321727 | 6 |
| *Zunongwangia profunda SM-A87* | 295136244 | 6 |
| *Actinomyces coleocanis DSM 15436* | 227494853 | 7 |
| *Actinomyces georgiae F0490* | 420151340 | 7 |
| *Actinomyces naeslundii str. Howell 279* | 400293272 | 7 |
| *Actinomyces sp. ICM47* | 396585058 | 7 |
| *Actinomyces sp. oral taxon 175 str. F0384* | 343523232 | 7 |
| *Actinomyces sp. oral taxon 181 str. F0379* | 429758968 | 7 |
| *Actinomyces sp. oral taxon 848 str. F0332* | 269219760 | 7 |
| *Actinomyces turicensis ACS-279-V-Col4* | 405979650 | 7 |
| *Bifidobacterium dentium Bd1* | 283456135 | 7 |
| *Bifidobacterium longum DJO10A* | 189440764 | 7 |
| *Bifidobacterium longum subsp. longum 2-2B* | 419852381 | 7 |
| *Bifidobacterium longum subsp. longum KACC 91563* | 384200944 | 7 |
| *Bifidobacterium sp. 12_1_47BFAA* | 317482066 | 7 |
| *Corynebacterium accolens ATCC 49725* | 227502575 | 7 |
| *Corynebacterium accolens ATCC 49726* | 306835141 | 7 |
| *Corynebacterium diphtheriae 241* | 375289763 | 7 |
| *Corynebacterium diphtheriae 31A* | 376283539 | 7 |
| *Corynebacterium diphtheriae BH8* | 376286566 | 7 |
| *Corynebacterium diphtheriae bv. intermedius str. NCTC 5011* | 419861895 | 7 |
| *Corynebacterium diphtheriae C7 (beta)* | 376289243 | 7 |
| *Corynebacterium diphtheriae HC02* | 376292154 | 7 |
| *Corynebacterium diphtheriae NCTC 13129* | 38232678 | 7 |
| *Corynebacterium diphtheriae VA01* | 376256051 | 7 |
| *Corynebacterium matruchotii ATCC 14266* | 305681510 | 7 |
| *Corynebacterium matruchotii ATCC 33806* | 225021644 | 7 |
| *Gardnerella vaginalis 1500E* | 415717744 | 7 |
| *Gardnerella vaginalis 284V* | 415703177 | 7 |
| *Gardnerella vaginalis 5-1* | 298252606 | 7 |
| *Mobiluncus curtisii subsp. holmesii ATCC 35242* | 315656340 | 7 |
| *Mobiluncus mulieris 28-1* | 269977848 | 7 |
| *Mobiluncus mulieris FB024-16* | 307700167 | 7 |
| *Scardovia inopinata F0304* | 294790575 | 7 |
| *Actinomyces sp. oral taxon 180 str. F0310* | 315605738 | 8 |
| *Gluconacetobacter diazotrophicus PAl 5* | 209542524 | 8 |
| *Gluconacetobacter diazotrophicus PAl 5* | 162147907 | 8 |
| *Methylocystis sp. ATCC 49242* | 323139312 | 8 |
| *Methylosinus trichosporium OB3b* | 296446027 | 8 |
| *Rhodopseudomonas palustris BisB18* | 90425961 | 8 |
| *Rhodopseudomonas palustris BisB5* | 91975509 | 8 |
| *Tistrella mobilis KA081020-065* | 389874754 | 8 |
| *Mycoplasma canis PG 14* | 384393286 | 9 |
| *Mycoplasma canis PG 14* | 419703974 | 9 |
| *Mycoplasma canis UF31* | 384937953 | 9 |
| *Mycoplasma canis UF33* | 419704625 | 9 |
| *Mycoplasma canis UFG1* | 419705269 | 9 |
| *Mycoplasma canis UFG4* | 419705920 | 9 |
| *Mycoplasma cynos C142* | 433625054 | 9 |
| *Mycoplasma gallisepticum NC95_13295-2-2P* | 401767318 | 9 |
| *Mycoplasma gallisepticum NY01_2001.047-5-1P* | 401768851 | 9 |
| *Mycoplasma gallisepticum str. F* | 284931710 | 9 |
| *Mycoplasma gallisepticum str. F* | 385326554 | 9 |
| *Mycoplasma gallisepticum str. R(low)* | 294660600 | 9 |
| *Mycoplasma synoviae 53* | 71894592 | 9 |
| *Mycoplasma synoviae 53* | 144575181 | 9 |
| *Prevotella buccalis ATCC 35310* | 282878504 | 10 |
| *Prevotella ruminicola 23* | 294674019 | 10 |
| *Prevotella stercorea DSM 18206* | 359406728 | 10 |
| *Prevotella tannerae ATCC 51259* | 258648111 | 10 |
| *Prevotella timonensis CRIS 5C-B1* | 282880052 | 10 |
| *Burkholderiales bacterium 1_1_47* | 303257695 | 11 |
| *Parasutterella excrementihominis YIT 11859* | 331001027 | 11 |
| *Sutterella wadsworthensis 3_1_45B* | 319941583 | 11 |
| *Elusimicrobium minutum Pei191* | 187250660 | 12 |
| *Sphaerochaeta globus str. Buddy* | 325972003 | 12 |
| uncultured Termite group 1 bacterium phylotype Rs-D17 | 189485059 | 12 |
| *Flavobacterium branchiophilum FL-15* | 347536497 | 13 |
| *Flavobacterium columnare ATCC 49512* | 365959402 | 13 |
| *Odoribacter laneus YIT 12061* | 374384763 | 13 |
| *Prevotella denticola CRIS 18C-A* | 325859619 | 14 |
| *Prevotella micans F0438* | 373501184 | 14 |
| *Prevotella sp. C561* | 345885718 | 14 |
| *Francisella tularensis subsp. tularensis WY96-3418* | 134302318 | 15 |
| *Francisella cf. novicida 3523* | 387824704 | 16 |
| *Francisella cf. novicida Fx1* | 385792694 | 16 |
| *Francisella novicida FTG* | 208779141 | 16 |
| *Francisella novicida GA99-3548* | 254374175 | 16 |
| *Francisella novicida U112* | 118497352 | 16 |
| *Francisella tularensis subsp. novicida GA99-3549* | 254372717 | 16 |
| *Wolinella succinogenes DSM 1740* | 34557932 | 17 |
| *gamma proteobacterium HTCC5015* | 254447899 | 18 |
| *Legionella pneumophila 130b* | 307608922 | 19 |
| *Legionella pneumophila str. Paris* | 54296138 | 19 |
| *Mycoplasma ovipneumoniae SC01* | 363542550 | 20 |
| *Streptobacillus moniliformis DSM 12112* | 269123826 | 21 |
| *Mycoplasma mobile 163K* | 47458868 | 22 |
| *Alcanivorax sp. W11-5* | 407803669 | 23 |
| *Caenispirillum salinarum AK4* | 427429481 | 23 |
| *Rhodospirillum rubrum ATCC 11170* | 83591793 | 23 |
| *Treponema sp. JC4* | 384109266 | 23 |
| *Ruminococcus albus 8* | 325677756 | 24 |
| uncultured delta proteobacterium HF0070_07E19 | 297182908 | 24 |
| *Acidothermus cellulolyticus 11B* | 117929158 | 25 |
| *Nitratifractor salsuginis DSM 16511* | 319957206 | 26 |
| *Akkermansia muciniphila ATCC BAA-835* | 187736489 | 27 |
| *Parvibaculum lavamentivorans DS-1* | 154250555 | 28 |

### Example 6

Phylogenetic clustering of Cas9 defines dual-RNA:Cas9 exchangeability.

As described above, clustering of Cas9 orthologs correlates with the ability to substitute for the RNA-stabilizing role of *S. pyogenes* Cas9 in tracrRNA:pre-crRNA processing by RNase III *in vivo* (Figure 4B). The exchangeability between Cas9 and dual-RNA in closely related CRISPR-Cas systems was investigated at the level of DNA interference.

Plasmid cleavage assays were performed using *S. pyogenes* Cas9 complexed with dual-RNAs from selected CRISPR-Cas systems representative of the clustering of the type II CRISPR-Cas systems. As shown in Figure 6A (upper panel), *S. pyogenes* Cas9 can cleave target DNA in the presence of dual-RNAs from *S. mutans* and *S. thermophilus** (type II-A, yellow subcluster), but not from any other tested species. The same result was observed when the dual-RNA from *S. pyogenes* was incubated with Cas9 orthologs from different bacteria (Figure 6A, lower panel). Cleavage assays were also performed with all Cas9 orthologs incubated with cognate and non-cognate dual-RNAs on their PAM-specific plasmid DNA. Only the combinations of Cas9 and dual-RNA within the same type II subcluster conferred dsDNA cleavage activity (Figure 6B, Supplementary Figure S10). More striking was the gradient of activity dependent on how closely related the species are in the corresponding type II group. This effect can be observed for *C. jejuni* Cas9 that is able to cleave DNA in the presence of dual-RNA from *P. multocida* and *N. meningitidis,* but not as efficient as with its own RNA (type II-C, blue subcluster). This finding is in good agreement with the phylogenetic tree of Cas9 (Figure 1A) showing that all three Cas9 orthologs belong to type II-C but C. *jejuni* Cas9 clusters more distantly from *P. multocida* and *N. meningitidis* Cas9. This effect was even greater for *S. thermophilus*** Cas9 which belongs to type II-A together with *S. pyogenes, S. mutans* and *S. thermophilus**. However, none of the dual-RNAs from the three latter loci could direct DNA cleavage by *S. thermophilus*** Cas9. This result supports the recent findings demonstrating the lack of exchangeability between Cas9 from CRISPR1 and CRISPR3 of *S. thermophilus* DGCC7710 with regard to dual-RNA binding (17). Cas9 and tracrRNA:crRNA interchangeabilty is contemplated to directly result from Cas9 co-evolution with dual-RNA and follows the Cas9 phylogeny that may differ from the phylogeny of the respective bacterial species due to horizontal transfer.

Thus, to investigate the interchangeability between type II subgroups at the level of DNA interference, the PAMs specific for each of the 8 selected Cas9 orthologs (28) were determined. By aligning potential crRNA-targeted sequences, conserved motifs adjacent to the protospacers in all selected species were identified. These motifs were then shown to be essential for DNA interference activity of the cognate dual-RNA:Cas9 complex *in vitro.* The interchangeability between dual-RNA and Cas9 from different subclusters was tested using plasmid cleavage assays. Only closely related Cas9 proteins can exchange their cognate dual-RNAs and still exert cleavage activity when using the Cas9 specific PAM. The specificity of Cas9 towards dual-RNAs is highly sensitive to the Cas9 sequence relatedness. This sensitivity is observed with Cas9 from *C. jejuni* that displays full cleavage activity with its cognate dual-RNA but reduced activity with dual-RNAs from *N. meningitidis* or *P. multocida* which belong to different subclusters of type II-C. It is contemplated that Cas9 possesses specificity for the secondary structure of dual-RNAs, given that bioinformatics predictions suggest similar structures of repeat:antirepeat duplexes in closely related CRISPR-Cas systems (Supplementary Figure S12).

While the present invention has been described in terms of specific embodiments, it is understood that variations and modifications will occur to those skilled in the art. Accordingly, only such limitations as appear in the claims should be placed on the invention.

### Documents Cited

All publications and patents cited in this specification are herein incorporated by reference as if each individual publication or patent were specifically and individually indicated to be incorporated by reference and are incorporated herein by reference to disclose and describe the methods and/or materials in connection with which the publications are cited. The citation of any publication is for its disclosure prior to the filing date and should not be construed as an admission that the present invention is not entitled to antedate such publication by virtue of prior invention. Further, the dates of publication provided may be different from the actual publication dates which may need to be independently confirmed.
1. Cho, S.W., Kim, S., Kim, J.M. and Kim, J.S. (2013) Targeted genome engineering in human cells with the Cas9 RNA-guided endonuclease. Nat. Biotechnol., 31, 230-232.
2. Cong, L., Ran, F.A., Cox, D., Lin, S., Barretto, R., Habib, N., Hsu, P.D., Wu, X., Jiang, W., Marraffini, L.A. et al. (2013) Multiplex genome engineering using CRISPR/Cas systems. Science, 339, 819-823.
3. DiCarlo, J.E., Norville, J.E., Mali, P., Rios, X., Aach, J. and Church, G.M. (2013) Genome engineering in Saccharomyces cerevisiae using CRISPR-Cas systems. Nucleic Acids Res., 41, 4336-4343.
4. Friedland, A.E., Tzur, Y.B., Esvelt, K.M., Colaiacovo, M.P.,Church, G.M. and Calarco, J.A. (2013) Heritable genome editing in C. elegans via a CRISPR-Cas9 system. Nat. Methods, 10, 741-743.
5. Gratz, S.J., Cummings, A.M., Nguyen, J.N., Hamm, D.C., Donohue, L.K., Harrison, M.M., Wildonger, J. and O'Connor-Giles, K.M. (2013) Genome engineering of Drosophila with the CRISPR RNA-guided Cas9 nuclease. Genetics, 194, 1029-1035.
6. Hwang, W.Y., Fu, Y., Reyon, D., Maeder, M.L., Tsai, S.Q., Sander, J.D., Peterson, R.T., Yeh, J.R. and Joung, J.K. (2013) Efficient genome editing in zebrafish using a CRISPR-Cas system. Nat. Biotechnol., 31, 227-229.
7. Jiang, W., Bikard, D., Cox, D., Zhang, F. and Marraffini, L.A. (2013) RNA-guided editing of bacterial genomes using CRISPR-Cas systems. Nat. Biotechnol., 31, 233-239.
8. Mali, P., Yang, L., Esvelt, K.M., Aach, J., Guell, M., Dicarlo, J.E., Norville, J.E. and Church, G.M. (2013) RNA-guided human genome engineering via Cas9. Science, 339, 823-826.
9. Shen, B., Zhang, J., Wu, H., Wang, J., Ma, K., Li, Z., Zhang, X., Zhang, P. and Huang, X. (2013) Generation of gene-modified mice via Cas9/RNA-mediated gene targeting. Cell Res., 23, 720-723.
10. Wang, H., Yang, H., Shivalila, C.S., Dawlaty, M.M., Cheng, A.W., Zhang, F. and Jaenisch, R. (2013) One-step generation of mice carrying mutations in multiple genes by CRISPR/Cas-mediated genome engineering. Cell, 153, 910-918.
11. Jinek, M., East, A., Cheng, A., Lin, S., Ma, E. and Doudna, J. (2013) RNA-programmed genome editing in human cells. eLIFE, 2, e00471.
12. Li, J.F., Norville, J.E., Aach, J., McCormack, M., Zhang, D., Bush, J., Church, G.M. and Sheen, J. (2013) Multiplex and homologous recombination-mediated genome editing in Arabidopsis and Nicotiana benthamiana using guide RNA and Cas9. Nat. Biotechnol., 31, 688-691.
13. Nekrasov, V., Staskawicz, B., Weigel, D., Jones, J.D. and Kamoun, S. (2013) Targeted mutagenesis in the model plant Nicotiana benthamiana using Cas9 RNA-guided endonuclease. Nat. Biotechnol., 31, 691-693.
14. Jinek, M., Chylinski, K., Fonfara, I., Hauer, M., Doudna, J.A. and Charpentier, E. (2012) A programmable dual-RNA-guided DNA endonuclease in adaptive bacterial immunity. Science, 337, 816-821.
15. Chylinski, K., Le Rhun, A. and Charpentier, E. (2013) The tracrRNA and Cas9 families of type II CRISPR-Cas immunity systems. RNA Biol., 10, 726-737.
16. Deltcheva, E., Chylinski, K., Sharma, C.M., Gonzales, K., Chao, Y., Pirzada, Z.A., Eckert, M.R., Vogel, J. and Charpentier, E. (2011) CRISPR RNA maturation by trans-encoded small RNA and host factor RNase III. Nature, 471, 602-607.
17. Karvelis, T., Gasiunas, G., Miksys, A., Barrangou, R., Horvath, P. and Siksnys, V. (2013) crRNA and tracrRNA guide Cas9-mediated DNA interference in Streptococcus thermophilus. RNA Biol., 10, 841-851.
18. Garneau, J.E., Dupuis, M.E., Villion, M., Romero, D.A., Barrangou, R., Boyaval, P., Fremaux, C., Horvath, P., Magadan, A.H. and Moineau, S. (2010) The CRISPR/Cas bacterial immune system cleaves bacteriophage and plasmid DNA. Nature, 468, 67-71.
19. Magadan, A.H., Dupuis, M.E., Villion, M. and Moineau, S. (2012) Cleavage of phage DNA by the Streptococcus thermophilus CRISPR3-Cas system. PLoS One, 7, e40913.
20. Haft, D.H., Selengut, J., Mongodin, E.F. and Nelson, K.E. (2005) A guild of 45 CRISPR-associated (Cas) protein families and multiple CRISPR/Cas subtypes exist in prokaryotic genomes. PLoS Comput. Biol., 1, e60.
21. Makarova, K.S., Grishin, N.V., Shabalina, S.A., Wolf, Y.I. and Koonin, E.V. (2006) A putative RNA-interference-based immune system in prokaryotes: computational analysis of the predicted enzymatic machinery, functional analogies with eukaryotic RNAi, and hypothetical mechanisms of action. Biol. Direct, 1, 7.
22. Gasiunas, G., Barrangou, R., Horvath, P. and Siksnys, V. (2012) Cas9-crRNA ribonucleoprotein complex mediates specific DNA cleavage for adaptive immunity in bacteria. Proc. Natl. Acad. Sci. U. S. A., 109, E2579-2586.
23. Sapranauskas, R., Gasiunas, G., Fremaux, C., Barrangou, R., Horvath, P. and Siksnys, V. (2011) The Streptococcus thermophilus CRISPR/Cas system provides immunity in Escherichia coli. Nucleic Acids Res., 39, 9275-9282.
24. Mali, P., Aach, J., Stranges, P.B., Esvelt, K.M., Moosburner, M., Kosuri, S., Yang, L. and Church, G.M. (2013) Cas9 transcriptional activators for target specificity screening and paired nickases for cooperative genome engineering. Nat Biotechnol., 31, 833-838.
25. Ran, F.A., Hsu, P.D., Lin, C.Y., Gootenberg, J.S., Konermann, S., Trevino, A.E., Scott, D.A., Inoue, A., Matoba, S., Zhang, Y. et al. (2013) Double nicking by RNA-guided CRISPR Cas9 for enhanced genome editing specificity. Cell, 154, 1380-1389.
26. Deveau, H., Barrangou, R., Garneau, J.E., Labonte, J., Fremaux, C., Boyaval, P., Romero, D.A., Horvath, P. and Moineau, S. (2008) Phage response to CRISPR-encoded resistance in Streptococcus thermophilus. J. Bacteriol., 190, 1390-1400.
27. Horvath, P., Romero, D.A., Coute-Monvoisin, A.C., Richards, M., Deveau, H., Moineau, S., Boyaval, P., Fremaux, C. and Barrangou, R. (2008) Diversity, activity, and evolution of CRISPR loci in Streptococcus thermophilus. J. Bacteriol., 190, 1401-1412.
28. Mojica, F.J., Diez-Villasenor, C., Garcia-Martinez, J. and Almendros, C. (2009) Short motif sequences determine the targets of the prokaryotic CRISPR defence system. Microbiology, 155, 733-740.
29. Bikard, D., Jiang, W., Samai, P., Hochschild, A., Zhang, F. and Marraffini, L.A. (2013) Programmable repression and activation of bacterial gene expression using an engineered CRISPR-Cas system. Nucleic Acids Res., 41, 7429-7437.
30. Qi, L.S., Larson, M.H., Gilbert, L.A., Doudna, J.A., Weissman, J.S., Arkin, A.P. and Lim, W.A. (2013) Repurposing CRISPR as an RNA-guided platform for sequence-specific control of gene expression. Cell, 152, 1173-1183.
31. Charpentier, E. and Doudna, J.A. (2013) Biotechnology: Rewriting a genome. Nature, 495, 50-51.
32. Horvath, P. and Barrangou, R. (2013) RNA-guided genome editing a la carte. Cell Res., 23, 733-734.
33. van der Oost, J. (2013) Molecular biology. New tool for genome surgery. Science, 339, 768-770.
34. Hou, Z., Zhang, Y., Propson, N.E., Howden, S.E., Chu, L.F., Sontheimer, E.J. and Thomson, J.A. (2013) Efficient genome engineering in human pluripotent stem cells using Cas9 from Neisseria meningitidis. Proc. Natl. Acad. Sci. U. S. A., 110, 15644-15649.
35. Sambrook, J., Fritsch, E.F. and Maniatis, T. (1989) Molecular Cloning: a Laboratory Manual. 2nd edn. Cold Spring Harbor, N. Y. ed. Cold Spring Harbor Laboratory Press.
36. Caparon, M.G. and Scott, J.R. (1991) Genetic manipulation of pathogenic streptococci. Methods Enzymol., 204, 556-586.
37. Kirsch, R.D. and Joly, E. (1998) An improved PCR-mutagenesis strategy for two-site mutagenesis or sequence swapping between related genes. Nucleic Acids Res., 26, 1848-1850.
38. Siller, M., Janapatla, R.P., Pirzada, Z.A., Hassler, C., Zinkl, D. and Charpentier, E. (2008) Functional analysis of the group A streptococcal luxS/Al-2 system in metabolism, adaptation to stress and interaction with host cells. BMC Microbiol., 8, 188.
39. Mangold, M., Siller, M., Roppenser, B., Vlaminckx, B.J., Penfound, T.A., Klein, R., Novak, R., Novick, R.P. and Charpentier, E. (2004) Synthesis of group A streptococcal virulence factors is controlled by a regulatory RNA molecule. Mol. Microbiol., 53, 1515-1527.
40. Herbert, S., Barry, P. and Novick, R.P. (2001) Subinhibitory clindamycin differentially inhibits transcription of exoprotein genes in Staphylococcus aureus. Infect. Immun., 69, 2996-3003.
41. Pall, G.S. and Hamilton, A.J. (2008) Improved northern blot method for enhanced detection of small RNA. Nat. Protoc., 3, 1077-1084.
42. Urban, J.H. and Vogel, J. (2007) Translational control and target recognition by Escherichia coli small RNAs in vivo. Nucleic Acids Res., 35, 1018-1037.
43. McClelland, M., Hanish, J., Nelson, M. and Patel, Y. (1988) KGB: a single buffer for all restriction endonucleases. Nucleic Acids Res., 16, 364.
44. Makarova, K.S., Haft, D.H., Barrangou, R., Brouns, S.J., Charpentier, E., Horvath, P., Moineau, S., Mojica, F.J., Wolf, Y.I., Yakunin, A.F. et al. (2011) Evolution and classification of the CRISPR-Cas systems. Nat. Rev. Microbiol., 9, 467-477.
45. Altschul, S.F., Madden, T.L., Schaffer, A.A., Zhang, J., Zhang, Z., Miller, W. and Lipman, D.J. (1997) Gapped BLAST and PSI-BLAST: a new generation of protein database search programs. Nucleic Acids Res., 25, 3389-3402.
46. Wheeler, D. and Bhagwat, M. (2007) BLAST QuickStart: example-driven web-based BLAST tutorial. Methods Mol. Biol., 395, 149-176.
47. Edgar, R.C. (2004) MUSCLE: multiple sequence alignment with high accuracy and high throughput. Nucleic Acids Res., 32, 1792-1797.
48. Soding, J., Biegert, A. and Lupas, A.N. (2005) The HHpred interactive server for protein homology detection and structure prediction. Nucleic Acids Res., 33, W244-248.
49. Price, M.N., Dehal, P.S. and Arkin, A.P. (2010) FastTree 2--approximately maximum-likelihood trees for large alignments. PLoS One, 5, e9490.
50. Bernhart, S.H., Tafer, H., Muckstein, U., Flamm, C., Stadler, P.F. and Hofacker, I.L. (2006) Partition function and base pairing probabilities of RNA heterodimers. Algorithms Mol. Biol., 1, 3.
51. Hofacker, I.L., Fekete, M. and Stadler, P.F. (2002) Secondary structure prediction for aligned RNA sequences. Journal of molecular biology, 319, 1059-1066.
52. Darty, K., Denise, A. and Ponty, Y. (2009) VARNA: Interactive drawing and editing of the RNA secondary structure. Bioinformatics, 25, 1974-1975.
53. Bhaya, D., Davison, M. and Barrangou, R. (2011) CRISPR-Cas systems in bacteria and archaea: versatile small RNAs for adaptive defense and regulation. Annu. Rev. Genet., 45, 273-297.
54. Zhang, Y., Heidrich, N., Ampattu, B.J., Gunderson, C.W., Seifert, H.S., Schoen, C., Vogel, J. and Sontheimer, E.J. (2013) Processing-independent CRISPR RNAs limit natural transformation in Neisseria meningitidis. Mol. Cell, 50, 488-503.
55. Takeuchi, N., Wolf, Y.I., Makarova, K.S. and Koonin, E.V. (2012) Nature and intensity of selection pressure on CRISPR-associated genes. J. Bacteriol., 194, 1216-1225.
56. Makarova, K.S., Aravind, L., Wolf, Y.I. and Koonin, E.V. (2011) Unification of Cas protein families and a simple scenario for the origin and evolution of CRISPR-Cas systems. Biol. Direct., 6, 38.
57. Barrangou, R., Fremaux, C., Deveau, H., Richards, M., Boyaval, P., Moineau, S., Romero, D.A. and Horvath, P. (2007) CRISPR provides acquired resistance against viruses in prokaryotes. Science, 315, 1709-1712.
58. Sun, W., Li, G. and Nicholson, A.W. (2004) Mutational analysis of the nuclease domain of Escherichia coli ribonuclease III. Identification of conserved acidic residues that are important for catalytic function in vitro. Biochemistry, 43, 13054-13062.
59. Sun, W., Jun, E. and Nicholson, A.W. (2001) Intrinsic double-stranded-RNA processing activity of Escherichia coli ribonuclease III lacking the dsRNA-binding domain. Biochemistry, 40, 14976-14984.

### Aspects of the invention

1. A single-molecule guide RNA comprising:
   a DNA-targeting segment and a protein-binding segment wherein the protein-binding segment comprises a tracrRNA set out in Supplementary Table S5.
2. The single-molecule guide RNA of aspect 1 wherein the protein-binding segment comprises a CRISPR repeat set out in Supplementary Table S5 that is the cognate CRISPR repeat of the tracrRNA of the protein-binding segment.
3. The single-molecule guide RNA of aspect 1 or 2 wherein the DNA-targeting segment further comprises RNA complementary to a protospacer-like sequence in a target DNA 5' to a PAM sequence.
4. The single-molecule guide RNA of aspect 3 wherein the tracrRNA and CRISPR repeat are respectively at least 80% identical to the C. jejuni tracrRNA and CRISPR repeat set out in Supplementary Table S5 and wherein the PAM sequence is NNNNACA.
5. The single-molecule guide RNA of aspect 4 or 8 wherein the RNA complementary to a protospacer-like sequence is RNA complementary to the target sequences set out in one of SEQ ID NOs: 801-973, 1079-1222, 1313-1348, 1372-1415, 1444-1900, 2163-2482 or 2667-2686.
6. A single-molecule guide RNA comprising:
   a DNA-targeting segment and a protein-binding segment, wherein the protein-binding segment comprises a tracrRNA at least 80% identical over at least 20 nucleotides to a tracrRNA set out in Supplementary Table S5.
7. A single-molecule guide RNA comprising:
   a DNA-targeting segment and a protein-binding segment, wherein the protein-binding segment comprises a tracrRNA at least 80% identical over at least 20 nucleotides to a tracrRNA set out in Supplementary Table S5, a CRISPR repeat at least 80% identical to a CRISPR repeat set out in Supplementary Table S5, or both.
8. The single-molecule guide RNA of aspect 7 wherein the tracrRNA and CRISPR repeat are respectively at least 80% identical to the C. jejuni tracrRNA and CRISPR repeat set out in Supplementary Table S5 and wherein the PAM sequence is NNNNACA.
9. The single-molecule guide RNA of aspect 1 or 6 comprising a linker between the DNA-targeting segment and the protein-binding segment.
10. A DNA encoding a single-molecule guide RNA comprising:
   a DNA-targeting segment and a protein-binding segment, wherein the protein-binding segment comprises a tracrRNA set out in Supplementary Table S5.
11. A DNA encoding a single-molecule guide RNA comprising:
   a DNA-targeting segment and a protein-binding segment, wherein the protein-binding segment comprises a tracrRNA at least 80% identical over at least 20 nucleotides to a tracrRNA set out in Supplementary Table S5, a CRISPR repeat at least 80% identical to a CRISPR repeat set out in Supplementary Table S5, or both.
12. A vector comprising a DNA encoding a single-molecule guide RNA comprising:
   a DNA-targeting segment and a protein-binding segment wherein the protein-binding segment comprises a tracrRNA set out in Supplementary Table S5.
13. A vector comprising a DNA encoding a single-molecule guide RNA comprising:
   a DNA-targeting segment and a protein-binding segment, wherein the protein-binding segment comprises a tracrRNA at least 80% identical over at least 20 nucleotides to a tracrRNA set out in Supplementary Table S5, a CRISPR repeat at least 80% identical to a CRISPR repeat set out in Supplementary Table S5, or both.
14. A cell comprising a DNA encoding a single-molecule guide RNA comprising:
   a DNA-targeting segment and a protein-binding segment, wherein the protein-binding segment comprises a tracrRNA set out in Supplementary Table S5.
15. A cell comprising a DNA encoding a single-molecule guide RNA comprising:
   a DNA-targeting segment and a protein-binding segment, wherein the protein-binding segment comprises a tracrRNA at least 80% identical over at least 20 nucleotides to a tracrRNA set out in Supplementary Table S5, a CRISPR repeat at least 80% identical to a CRISPR repeat set out in Supplementary Table S5, or both.
16. A double-molecule guide RNA comprising:
   a targeter-RNA and an activator-RNA complementary thereto, wherein the activator-RNA comprises a tracrRNA set out in Supplementary Table S5, and
   wherein the guide RNA comprises a modified backbone, a non-natural internucleoside linkage, a nucleic acid mimetic, a modified sugar moiety, a base modification, a modification or sequence that provides for modified or regulated stability, a modification or sequence that provides for subcellular tracking, a modification or sequence that provides for tracking, or a modification or sequence that provides for a binding site for a protein or protein complex.
17. The double-molecule guide RNA of aspect 16, wherein the targeter-RNA comprises a CRISPR repeat set out in Supplementary Table S5 that is the cognate CRISPR repeat of the tracrRNA of the protein-binding segment.
18. The double-molecule guide RNA of aspect 16 or 17 wherein the targeter-RNA further comprises RNA complementary to a protospacer-like sequence in a target DNA 5' to a PAM sequence.
19. The double-molecule guide RNA of aspect 18 wherein the tracrRNA and CRISPR repeat are respectively at least 80% identical to the C. jejuni tracrRNA and CRISPR repeat set out in Supplementary Table S5 and wherein the PAM sequence is NNNNACA.
20. The double-molecule guide RNA of aspect 19 or aspect 23 wherein the RNA complementary to a protospacer-like sequence is RNA complementary to the target sequences set out in one of SEQ ID NOs: 801-973, 1079-1222, 1313-1348, 1372-1415, 1444-1900, 2163-2482 or 2667-2686.
21. A double-molecule guide RNA comprising:
   a targeter-RNA and a activator-RNA, wherein the activator-RNA comprises a tracrRNA at least 80% identical over at least 20 nucleotides to a tracrRNA set out in Supplementary Table S5.
22. The double-molecule guide RNA of aspect 21, wherein the targeter-RNA comprises a CRISPR repeat set out in Supplementary Table S5, the cognate CRISPR repeat of the tracrRNA of the activator-RNA set out in Supplementary Table S5, or a CRISPR repeat at least 80% identical to a CRISPR repeat set out in Supplementary Table S5.
23. The double-molecule guide RNA of aspect 21 wherein the tracrRNA and CRISPR repeat are respectively at least 80% identical to the C. jejuni tracrRNA and CRISPR repeat set out in Supplementary Table S5 and wherein the PAM sequence is NNNNACA.
24. The double-molecule guide RNA of aspect 16 or 21 comprising a linker between the targeter-RNA and the activator-RNA.
25. A DNA encoding a double-molecule guide RNA comprising:
   a targeter-RNA and a activator-RNA complementary thereto, wherein the activator-RNA comprises a tracrRNA set out in Supplementary Table S5.
26. A DNA encoding a double-molecule guide RNA comprising:
   a targeter-RNA and a activator-RNA complementary thereto, wherein the activator-RNA comprises a tracrRNA at least 80% identical over at least 20 nucleotides to a tracrRNA set out in Supplementary Table S5, a CRISPR repeat at least 80% identical to a CRISPR repeat set out in Supplementary Table S5, or both.
27. A vector comprising a DNA encoding a double-molecule guide RNA comprising:
   a targeter-RNA and a activator-RNA complementary thereto, wherein the activator-RNA comprises a tracrRNA set out in Supplementary Table S5.
28. A vector comprising a DNA encoding a double-molecule guide RNA comprising:
   a targeter-RNA and a activator-RNA complementary thereto, wherein the activator-RNA comprises a tracrRNA at least 80% identical over at least 20 nucleotides to a tracrRNA set out in Supplementary Table S5, a CRISPR repeat at least 80% identical to a CRISPR repeat set out in Supplementary Table S5, or both.
29. A cell comprising a DNA encoding a double-molecule guide RNA comprising:
   a targeter-RNA and a activator-RNA complementary thereto, wherein the activator-RNA comprises a tracrRNA set out in Supplementary Table S5.
30. A cell comprising a DNA encoding a double-molecule guide RNA comprising:
   a targeter-RNA and a activator-RNA complementary thereto, wherein the activator-RNA comprises a tracrRNA at least 80% identical over at least 20 nucleotides to a tracrRNA set out in Supplementary Table S5, a CRISPR repeat at least 80% identical to a CRISPR repeat set out in Supplementary Table S5, or both.
31. A method for manipulating DNA in a cell, comprising contacting the DNA with a Cas9 ortholog-guideRNA complex, wherein the complex comprises:
   (a) a C. jejuni Cas9 endonuclease heterologous to the cell or an endonuclease with an activity portion at least 90% identical to the activity portion of the C. jejuni Cas9 endonuclease, and a guide RNA targeting the complex to a protospacer-like sequence in the DNA 5' to the PAM sequence NNNNACA;
   (b) a P. multocida Cas9 endonuclease heterologous to the cell or an endonuclease with an activity portion at least 90% identical to the activity portion of the P. multocida Cas9 endonuclease, and a guide RNA targeting the complex to a protospacer-like sequence in the DNA 5' to the PAM sequence GNNNCNNA or NNNNC;
   (c) an F. novicida Cas9 endonuclease heterologous to the cell or an endonuclease with an activity portion at least 90% identical to the activity portion of the F. novicida Cas9 endonuclease, and a guide RNA targeting the complex to a protospacer-like sequence in the DNA 5' to the PAM sequence NG;
   (d) an S. thermophilus** Cas9 endonuclease heterologous to the cell or an endonuclease with an activity portion at least 90% identical to the activity portion of the S. thermophilus** Cas9 endonuclease, and a guide RNA targeting the complex to a protospacer-like sequence in the DNA 5' to the PAM sequence NNAAAAW;
   (e) an L. innocua Cas9 endonuclease heterologous to the cell or an endonuclease with an activity portion at least 90% identical to the activity portion of the L. innocua Cas9 endonuclease, and a guide RNA targeting the complex to a protospacer-like sequence in the DNA 5' to the PAM sequence NGG; or
   (f) an S. dysgalactiae Cas9 endonuclease heterologous to the cell or an endonuclease with an activity portion at least 90% identical to the activity portion of the S. dysgalactiae Cas9 endonuclease, and a guide RNA targeting the complex to a protospacer-like sequence in the DNA 5' to the PAM sequence NGG.
32. The method of aspect 31 wherein the cell is a bacterial cell, a fungal cell, an archaea cell, a plant cell or an animal cell.
33. The method of aspect 31 wherein the guide RNA is a single-molecule guide RNA.
34. The method of aspect 31 wherein the guide RNA is a double-molecule guide RNA.
35. The method of aspect 31 wherein the endonuclease is a nickase.
36. The method of aspect 31 wherein the endonuclease comprises a mutation corresponding to S pyogenes E762A, HH983AA or D986A.
37. The method of aspect 31 wherein the endonuclease is a dead mutant/DNA binding protein.
38. The method of aspect 31 wherein the protospacer-like sequence targeted is in a CCR5, CXCR4, KRT5, KRT14, PLEC or COL7A1 gene.
39. The method of aspect 31 wherein the protospacer-like sequence is in a chronic granulomatous disease (CGD)-related gene CYBA, CYBB, NCF1, NCF2 or NCF4.
40. The method of aspect 31 wherein the protospacer-like sequence targeted is in, or is up to 1000 nucleotides upstream of, a gene encoding B-cell lymphoma/leukemia IIA (BCL11A) protein, an erythroid enhancer of BCL11A or a BCL11A binding site.
41. The method of aspect 31 wherein the endonuclease and the guide RNA are introduced to the cell by the same or different recombinant vectors encoding the endonuclease and the guide RNA.
42. The method of aspect 31 wherein at least one recombinant vector is a recombinant viral vector.
43. A recombinant vector encoding:
   (a) a guide RNA, wherein the guide RNA comprises a DNA-targeting segment complementary to a protospacer-like sequence in the DNA 5' to the PAM sequence NNNNACA; and
   (b) s C. jejuni Cas9 endonuclease or an endonuclease with an activity portion at least 90% identical to the activity portion of the C. jejuni Cas9 endonuclease.
44. A recombinant vector encoding:
   (a) a guide RNA, wherein the guide RNA comprises a DNA-targeting segment complementary to a protospacer-like sequence in the DNA 5' to the PAM sequence GNNNCNNA or NNNNC; and
   (b) a P. multocida Cas9 endonuclease or an endonuclease with an activity portion at least 90% identical to the activity portion the P. multocida Cas9 endonuclease.
45. A recombinant vector encoding:
   (a) a guide RNA, wherein the guide RNA comprises a DNA-targeting segment complementary to a protospacer-like sequence in the DNA 5' to the PAM sequence NG; and
   (b) a F. novicida Cas9 endonuclease or an endonuclease with an activity portion at least 90% identical to the activity portion of the F. novicida Cas9 endonuclease.
46. A recombinant vector encoding:
   (a) a guide RNA, wherein the guide RNA comprises a DNA-targeting segment complementary to a protospacer-like sequence in the DNA 5' to the PAM sequence NNAAAAW; and
   (b) a S. thermophilus** Cas9 endonuclease or an endonuclease with an activity portion at least 90% identical to the activity portion of the S. thermophilus** Cas9 endonuclease.
47. A recombinant vector encoding:
   (a) a guide RNA, wherein the guide RNA comprises a DNA-targeting segment complementary to a protospacer-like sequence in the DNA 5' to the PAM sequence NGG; and
   (b) a L. innocua Cas9 endonuclease or an endonuclease with an activity portion at least 90% identical to the activity portion of the L. innocua Cas9 endonuclease.
48. A recombinant vector encoding:
   (a) a guide RNA, wherein the guide RNA comprises a DNA-targeting segment complementary to a protospacer-like sequence in the DNA 5' to the PAM sequence NGG; and
   (b) a S. dysgalactiae Cas9 endonuclease or an endonuclease with an activity portion at least 90% identical to the activity portion of the S. dysgalactiae Cas9 endonuclease.
49. The recombinant vector of aspect 43, 44, 45, 46, 47 or 48 wherein the recombinant vector is a recombinant viral vector.
50. A modified Cas9 endonuclease comprising one or more mutations corresponding to S. pyogenes mutation E762A, HH983AA or D986A.
51. The modified Cas 9 endonuclease of aspect 50 further comprising one or more mutations corresponding to S. pyogenes mutation D10A, H840A, G12A, G17A, N854A, N863A, N982A or A984A.
52. A method for manipulating DNA in a cell, comprising contacting the DNA with a Cas9 ortholog-guide RNA complex, wherein the complex comprises:
   (a) a Cas9 endonuclease heterologous to the cell and
   (b) a cognate guide RNA of the Cas9 endonuclease comprising a tracrRNA set out in Supplementary Table S5 or a guide RNA comprising a tracrRNA at least 80% identical to a cognate tracrRNA set out in Supplementary Table S5 over at least 20 nucleotides.
53. The method of aspect 52 wherein the cell is a bacterial cell, a fungal cell, an archaea cell, a plant cell or an animal cell.
54. The method of aspect 52 wherein the guide RNA is a single-molecule guide RNA.
55. The method of aspect 52 wherein the guide RNA is a double-molecule guide RNA.
56. The method of aspect 52 wherein the endonuclease is a nickase.
57. The method of aspect 52 wherein the endonuclease comprises a mutation corresponding to S pyogenes mutations E762, HH983AA or D986A.
58. The method of aspect 52 wherein the endonuclease is a dead mutant/DNA binding protein.
59. The method of aspect 52 wherein the protospacer-like sequence targeted is in a CCR5, CXCR4, KRT5, KRT14, PLEC or COL7A1 gene or a sequence up to 1000 nucleotides upstream of the gene.
60. The method of aspect 52 wherein the protospacer-like sequence is in a chronic granulomatous disease (CGD)-related gene CYBA, CYBB, NCF1, NCF2 or NCF4 or a sequence up to 1000 nucleotides upstream of the gene.
61. The method of aspect 52 wherein the protospacer-like sequence targeted is in, or is up to 1000 nucleotides upstream of, a gene encoding B-cell lymphoma/leukemia IIA (BCL11A) protein, an erythroid enhancer of BCL11A or a BCL11A binding site.
62. The method of aspect 52 wherein the enodnuclease and the guide RNA are introduced to the cell by the same or different recombinant vectors encoding the endonuclease and the guide RNA.
63. The method of aspect 52 wherein at least one recombinant vector is a recombinant viral vector.

## Claims

1. A single-molecule guide RNA comprising:
i) a DNA-targeting segment comprising RNA complementary to a protospacer-like sequence in a target DNA 5' to a PAM sequence of NNNNACA, and
ii) a protein-binding segment.

2. The single-molecule guide RNA of claim 1 wherein the protospacer-like sequence in a target DNA 5' to a PAM sequence has a nucleic acid sequence selected from the group consisting of SEQ ID NOs: 801-973, 1313-1348 and 1372-1415.

3. The single-molecule guide RNA of claim 1,
wherein the protein-binding segment comprises a tracrRNA at least 80% identical over at least 20 nucleotides to a tracrRNA set out in Supplementary Table S5.

4. The single-molecule guide RNA of claim 1, wherein the protein-binding segment comprises a tracrRNA as set out in Supplementary Table S5.

5. The single-molecule guide RNA of claim 1 further comprising a linker between the DNA-targeting segment and the protein-binding segment.

6. A DNA encoding the single-molecule guide RNA of any of the preceding claims.

7. A vector comprising the DNA molecule of claim 6.

8. A cell comprising the vector of claim 7.

9. An *in vitro* method for manipulating DNA in a cell comprising_contacting the DNA with a *C. jejuni* Cas9 endonuclease heterologous to the cell or an endonuclease with an activity portion at least 90% identical to the activity portion of the *C. jejuni* Cas9 endonuclease, and
the single-molecule guide RNA of claim 1.

10. The method of claim 9, wherein the protospacer-like sequence in a target DNA 5' to the PAM sequence has a nucleic acid sequence selected from the group consisting of SEQ ID NOs: 801-973, 1313-1348 and 1372-1415.

11. The method of claim 9 or 10, wherein the protein-binding segment comprises a tracrRNA in Supplementary Table S5.

12. The method of claim 9, wherein the endonuclease is a polypeptide.

13. The method of claim 9, wherein the endonuclease is encoded by a nucleic acid.

14. The method of claim 13, wherein the nucleic acid is a recombinant expression vector.

15. A recombinant vector encoding:
(a) the single-molecule guide RNA of claim 1; and
(b) a *C. jejuni* Cas9 endonuclease or an endonuclease with an activity portion at least 90% identical to the activity portion of the *C. jejuni* Cas9 endonuclease.

16. The method of claim 9, wherein the endonuclease and the guide RNA are introduced to the cell by the same or different recombinant vectors encoding the endonuclease and the guide RNA, optionally wherein at least one recombinant vector is a recombinant viral vector.

17. A *C. jejuni* Cas9 endonuclease heterologous to the cell or an endonuclease with an activity portion at least 90% identical to the activity portion of the *C. jejuni* Cas9 endonunclease, and the single-molecule guide RNA of Claim 1 for use in a therapeutic method for manipulating DNA in a cell.
